# EUROPEAN PATENT SPECIFICATION

(11) **EP 3 433 239 B1**
(45) Date of publication and mention of the grant of the patent: **27.05.2026**
(21) Application number: 17771272.6
(22) Date of filing: 24.03.2017
(51) Int. Cl.: A61K 31/407, C07D 295/185, A61P 35/00, A61K 31/12, A61K 31/498, A61K 31/46, A61K 31/445, A61K 31/42, A61K 31/496, A61K 31/495, C07C 49/563, C07D 217/16, C07D 487/04, C07D 405/06, C07D 401/08, C07D 403/06, A61K 31/4166, A61K 31/454, A61K 31/4709, A61K 31/58, A61P 13/08

(54) **SMALL MOLECULE INHIBITORS OF THE NUCLEAR TRANSLOCATION OF ANDROGEN RECEPTOR FOR THE TREATMENT OF CASTRATION-RESISTANT PROSTATE CANCER**
KLEINMOLEKÜLIGE INHIBITOREN DER NUKLEAREN TRANSLOKATION DES ANDROGENREZEPTORS ZUR BEHANDLUNG VON KASTRATIONSRESISTENTEM PROSTATAKREBS
INHIBITEURS DE TYPE PETITE MOLÉCULE DE LA TRANSLOCATION NUCLÉAIRE DU RÉCEPTEUR AUX ANDROGÈNES POUR LE TRAITEMENT DU CANCER DE LA PROSTATE RÉSISTANT À LA CASTRATION

(30) Priority: 24.03.2016 US 201615080237
(43) Date of publication of application: 30.01.2019
(73) Proprietor: University of Pittsburgh- Of the Commonwealth System of Higher Education, Pittsburgh, PA 15260 (US)
(72) Inventor: WIPF, Peter, Pittsburgh, PA 15260 (US); JOHNSON, James, K., Pittsburgh, PA 15260 (US); SKODA, Erin, M., Columbia, MD 21046 (US); NELSON, Joel, B., Pittsburgh, PA 15213-2582 (US); WANG, Zhou, Pittsburgh, PA 15232 (US); TAI, Serene, Pittsburgh, PA 15260 (US); TAKUBO, Keita, Pittsburgh, PA 15260 (US); MILLIGAN, John, Philadelphia, PA 19104 (US)
(74) Representative: HGF
(86) International application number: PCT/US2017/024105
(87) International publication number: WO 2017/165822

(56) References cited:
- WO-A1-2013/055793
- WO-A1-2015/042297
- WO-A1-2015/042297
- US-A1- 2014 371 235
- US-A1- 2016 257 657
- US-A1- 2016 257 657
- JAMES K. JOHNSON ET AL: "Small Molecule Antagonists of the Nuclear Androgen Receptor for the Treatment of Castration-Resistant Prostate Cancer", ACS MEDICINAL CHEMISTRY LETTERS, vol. 7, no. 8, 27 May 2016 (2016-05-27), US, pages 785 - 790, XP055425250, ISSN: 1948-5875, DOI: 10.1021/acsmedchemlett.6b00186
- DATABASE Pubmed Compound [online] 29 May 2009 (2009-05-29), "Compound Summary for CID 36925632", XP055425240, retrieved from NCBI Database accession no. ZINC303692363
- DATABASE PUBMED [online] 11 March 2016 (2016-03-11), "ZINC303692363", retrieved from NCBI Database accession no. ZINC303692363
- DATABASE PUBMED [online] 11 March 2016 (2016-03-11), "ZINC303410047", retrieved from Ncbi Database accession no. ZINC303410047
- DATABASE REGISTRY [online] CHEMICAL ABSTRACTS SERVICE, COLUMBUS, OHIO, US; 5 June 2015 (2015-06-05), CHEMICAL LIBRARY SUPPLIER: AURORA FINE CHEMICALS: "1793934-53-8", retrieved from REGISTRY Database accession no. 1793934-53-8
- DATABASE REGISTRY [online] CHEMICAL ABSTRACTS SERVICE, COLUMBUS, OHIO, US; 18 September 2014 (2014-09-18), CHEMICAL LIBRARY SUPPLIER: UKRORGSYNTEZ LTD.: "1623461-69-7", retrieved from REGISTRY Database accession no. 1623461-69-7
- DATABASE PubMed [online] 29 May 2009 (2009-05-29), "CID 36925618", retrieved from NCBI Database accession no. CID 36925618
- DATABASE REGISTRY 15 February 2015 (2015-02-15), ANONYMOUS, XP055581363, retrieved from STN Database accession no. 1647452-68-3
- DATABASE REGISTRY 13 February 2015 (2015-02-13), ANONYMOUS, XP055581366, retrieved from STN Database accession no. 1646734-73-7
- DATABASE REGISTRY 24 September 2014 (2014-09-24), ANONYMOUS, XP055581370, retrieved from STN Database accession no. 1624387-89-8
- DATABASE REGISTRY 23 September 2014 (2014-09-23), ANONYMOUS, XP055581373, retrieved from STN Database accession no. 1624152-83-5
- DATABASE PubChem [O] National Center for Biotechnology Information; 29 May 2009 (2009-05-29), XP055425240, retrieved from NCBI Database accession no. 18061517
- DATABASE PubChem [O] National Center for Biotechnology Information; 29 May 2009 (2009-05-29), "SCHEMBL18061526", XP055425236, Database accession no. 18061526
- JOHNSON, J.K. ET AL.: "Small Molecule Antagonists of the Nuclear Androgen Receptor for the Treatment of Castration-Resistant Prostate Cancer", ACS MEDICINAL CHEMISTRY LETTERS, vol. 7, no. 8, 2 June 2016 (2016-06-02), pages 785 - 790, XP055425250, Retrieved from the Internet <URL:http:// pubs.acs.org/doi/pdf/10.1021/acsmedchemlett.6b00186> [retrieved on 20160602]
- VACHAL, P .: "l-Sulfonyl-4-acylpiperazines as selective cannabinoid-1 receptor (CB1R) inverse agonists for the treatment of obesity", JOURNAL OF MEDICINAL CHEMISTRY, vol. 52, no. 8, 25 March 2009 (2009-03-25), pages 2550 - 2558, XP055425255, Retrieved from the Internet <URL:http://dlx.booksc.org/21100000/libgen. scimag21132000-21132999.zip/browse/10.1021/jm900063x.pdf>

## Description

### BACKGROUND

Castration-resistant prostate cancer (CRPC) is currently incurable and makes prostate cancer the second most common cause of cancer death among men in the United States. The androgen receptor (AR) is activated via multiple mechanisms including AR overexpression, mutation, hypersensitization, and/or intratumoral androgen synthesis in patients relapsed after androgen deprivation therapy (ADT). The steroidal hormones testosterone and dihydrotestosterone are the major endogenous androgens that cause nuclear translocation and subsequent activation of androgen receptor (AR). Overexpression and knockdown studies have demonstrated that AR is a key molecular determinant and an excellent therapeutic target for CRPC. Clinical use of abiraterone, a potent inhibitor of testosterone synthesis, and MDV3 100 (enzalutamide) and bicalutamide, AR antagonists, indicates that AR remains a viable target in a significant number of CRPC patients.

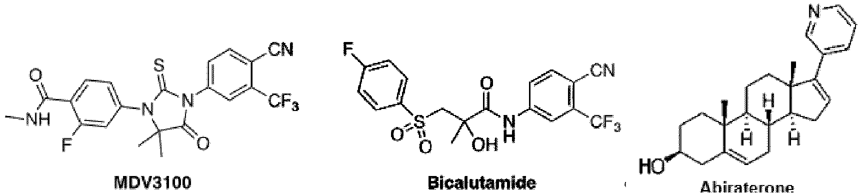

Androgen receptor (AR), a member of the steroid receptor superfamily, is a ligand-dependent transcription factor that controls the expression of androgen-responsive genes. Intracellular trafficking is an important mechanism in the regulation of many transcription factors, including AR. In order to access its target genes, a transcription factor requires localization to the nucleus. Retention of a transcription factor in the cytoplasm prevents its activity. Thus, a key regulatory step in the action of AR is its nuclear translocation. In androgen-sensitive cells, AR is localized to the cytoplasm in the absence of ligand. Upon addition of androgens, AR translocates to the nucleus and transactivates target genes. However, in CRPC cells, AR remains in the nucleus even in the absence of androgen and transactivates androgen-responsive genes, leading to uncontrolled growth of prostate tumors. Therefore, novel approaches that can block the nuclear localization of AR, degrade nuclear AR, and/or suppress nuclear AR activity may provide an effective therapy against CRPC.

US 2014/371235 A1 and WO 2015/042297 A1 disclose certain phenyl-substituted imidazoles and amide derivatives for use in the treatment of prostate cancer. The compounds are stated to target androgen nuclear localization and/or level.

US 2016/257657 A1, and Johnson, J.K. et al., "Small Molecule Antagonists of the Nuclear Androgen Receptor for the Treatment of Castration-Resistant Prostate Cancer", ACS medicinal chemistry letters, vol. 7, no. 8, (20160602), pages 785 - 790, both published after the priority date of this patent application, disclose certain compounds, including some cyclopropane amide derivatives which are described as androgen nuclear localization inhibitors useful in the treatment of prostate cancer.

### SUMMARY

The invention is defined by the claims. Any subject matter falling outside the scope of the claims is provided for information purposes only. Any references in the description to methods for treatment of the human or animal body by therapy refer to the compounds and pharmaceutical compositions of the present invention for use in the methods for treatment of the human or animal body by therapy.

According to one aspect of the invention, there is provided a compound selected from: 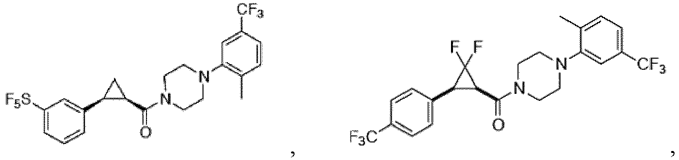 and 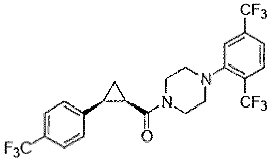 or a pharmaceutically acceptable salt thereof.

According to another aspect of the invention there is provided a compound selected from: 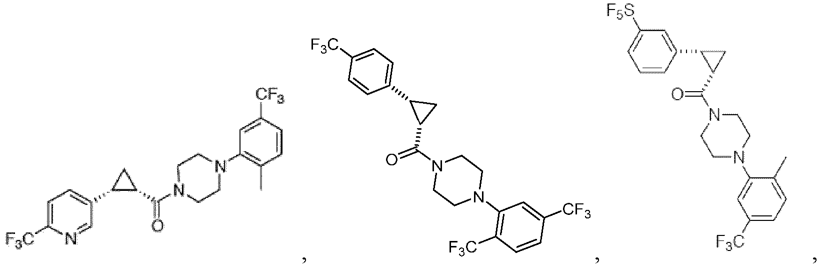 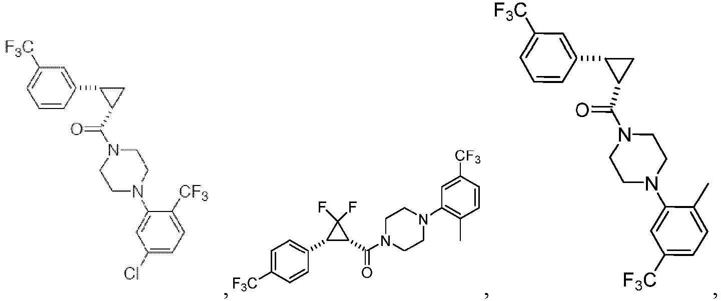 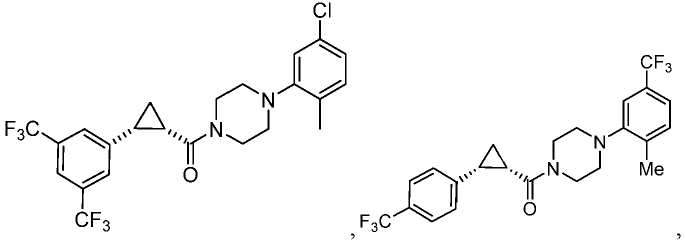 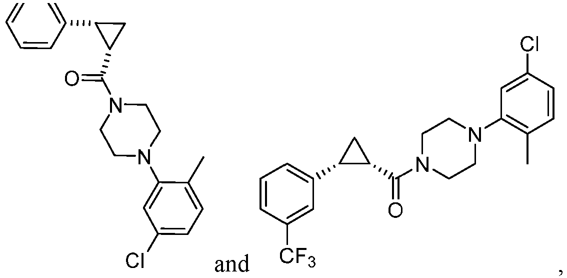 or a pharmaceutically acceptable salt thereof.

According to another aspect of the invention there is provided a compound selected from: 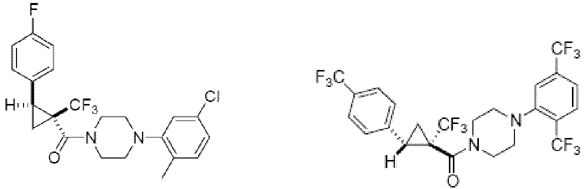 and 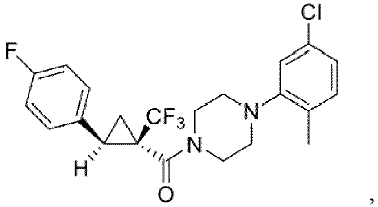 or a pharmaceutically acceptable salt thereof.

According to a further aspect of the invention there is provided a pharmaceutical composition comprising at least one pharmaceutically acceptable additive, and 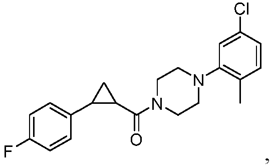 or a pharmaceutically acceptable salt thereof.

According to a further aspect of the invention there is compound for use in a method for treating prostate cancer in a subject, wherein the subject is administered a therapeutically effective amount of the compound, wherein the compound is 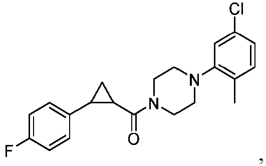 or a pharmaceutically acceptable salt thereof, optionally wherein the prostate cancer is castration-resistant cancer. It may be that the compound for use is: 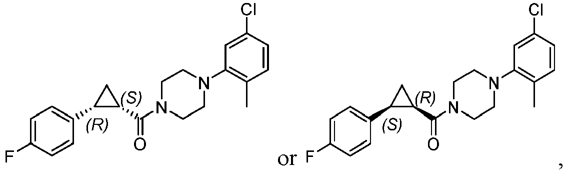 or a pharmaceutically acceptable salt thereof.

The foregoing will become more apparent from the following detailed description, which proceeds with reference to the accompanying figures.

### BRIEF DESCRIPTION OF THE DRAWINGS

Figs. 1A through 1D are a table showing compound structures.
Figs. 2A through 2I show additional compound structures.

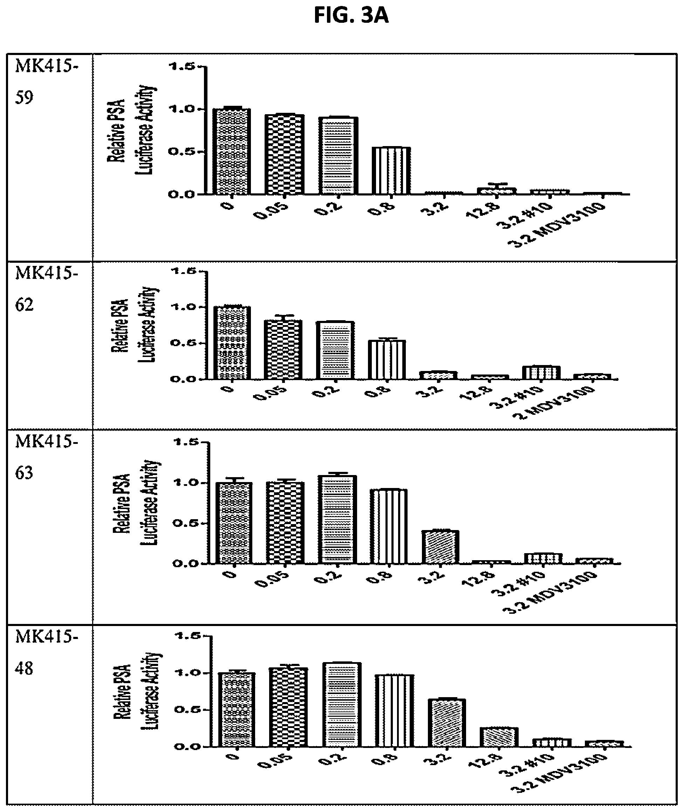

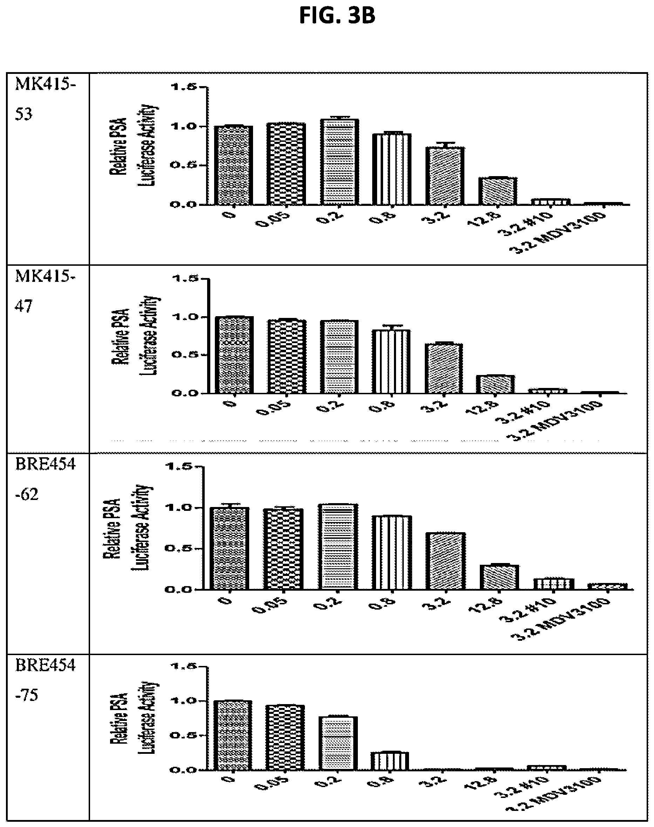

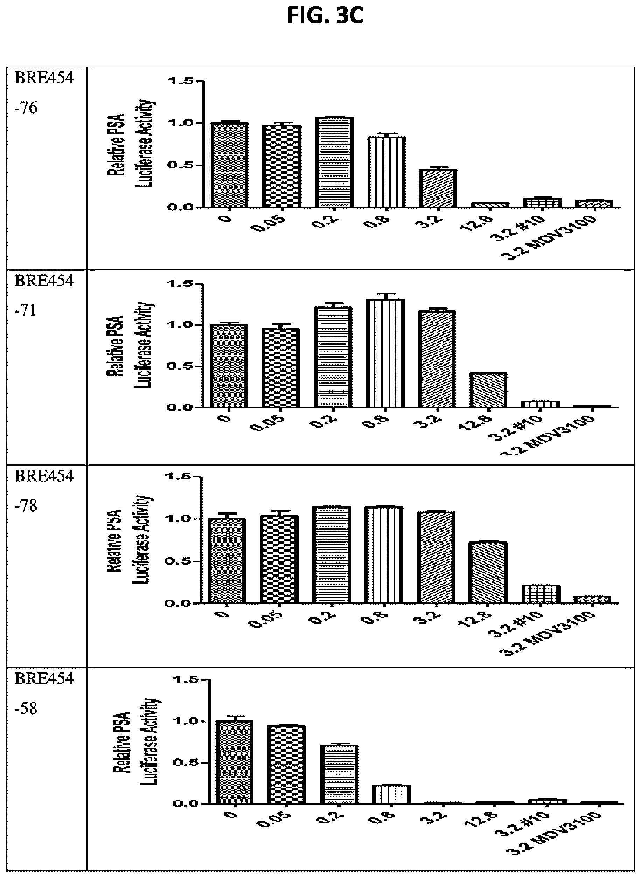

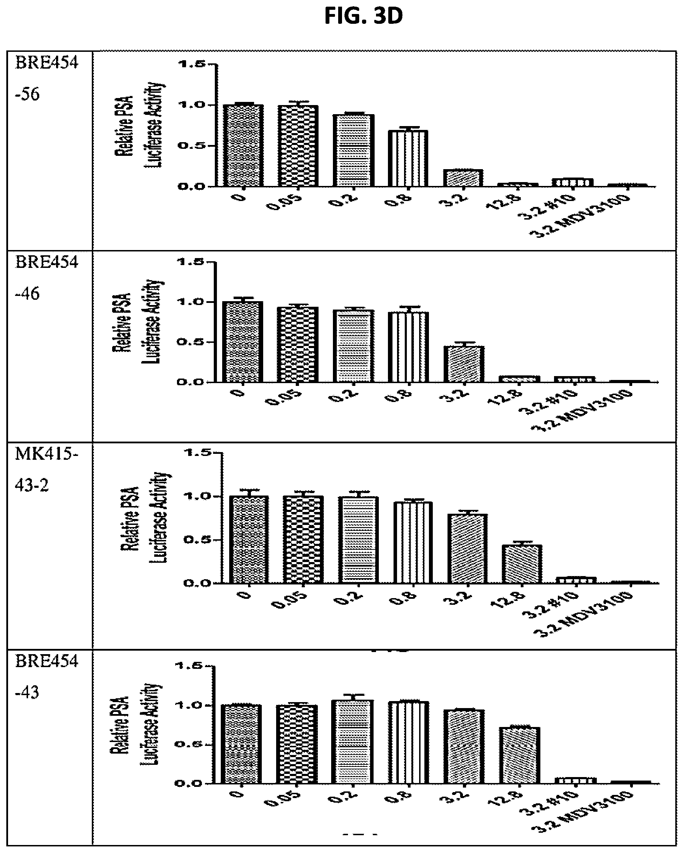

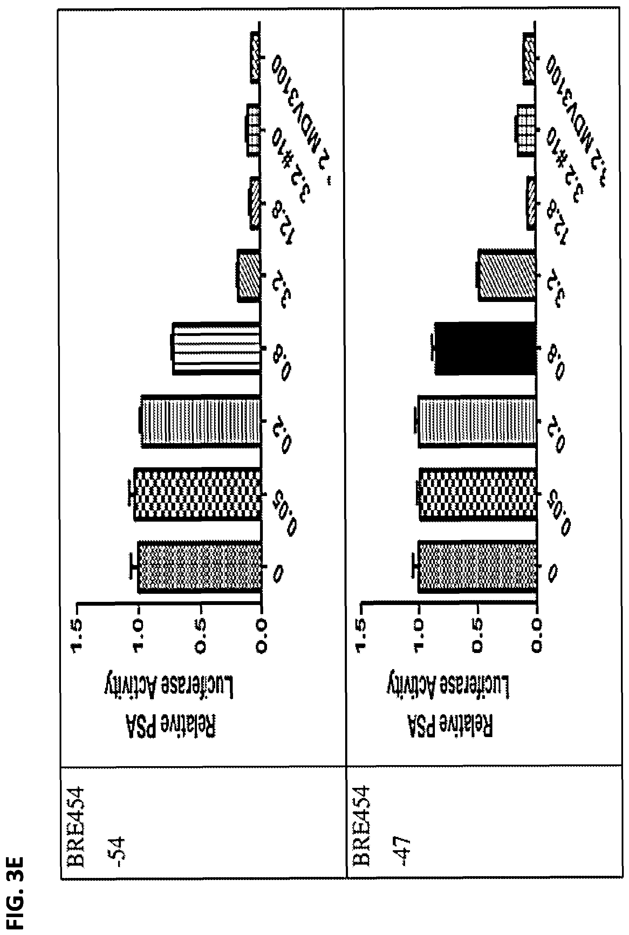

Figs. 3A through 3E show assay results for several of the compounds. C4-2 cells were transfected with PSA6.1-Luc, GFP-AR, and pRL-CMV and then treated with indicated doses for 24 hours. For luciferase assays, cells were lysed with passive lysis buffer (Promega) and both Firefly and Renilla luciferase activities were read using a Dual-Luciferase Reporter Assay kit (Promega) on a LmaxII384 luminometer (Molecular Devices). Firefly luciferase values were normalized to Renilla (pRL-CMV). Plotted values represent averaged normalized Firefly luciferase activities, each performed in triplicate, relative to DMSO control. This assay is described in more detail in PCT Patent Application Publication WO 2013055793.
Fig. 4 is a reaction scheme showing the synthesis of 2-((isoxazol-4-ylmethyl)thio)-1-(4-phenylpiperazin-1-yl)ethanone **1.**
Fig. 5 is a chemical structure of 2-((isoxazol-4-ylmethyl)thio)-1-(4-phenylpiperazin-1-yl)ethanone showing zones of modification.
Fig. 6 is a reaction scheme showing synthesis of certain disclosed compounds. Reagents and conditions: (a) T3P (propylphosphonic anhydride), Et₃N (triethylamine), CH₂Cl₂, rt (room temperature), overnight, 52-98%; (b) LiAlH₄, dry THF (tetrahydrofuran), 0 °C, 1 h, 42%; (c) NaIO₄, MeOH (methanol), H₂O, rt, 15 h, 68%; (d) *m*-CPBA (*meta*-chloroperoxybenzoic acid), CH₂Cl₂, rt, 15 h, 44%.
Fig. 7 is a reaction scheme showing synthesis of certain disclosed compounds. Reagents and conditions: (a) T3P, Et₃N, CH₂Cl₂, rt, overnight, 62-96%; (b) Lindlar's catalyst, quinoline, H₂, EtOAc (ethyl acetate), quant.; (c) CrCl₂, CH₂ICl, THF, reflux, overnight, 57%.
Fig. 8 is a reaction scheme showing synthesis of certain disclosed compounds. Reagents and conditions: (a) 2-chloroacetyl chloride, Et₃N, CH₂Cl₂, rt, overnight, 99%; (b) chloromethanesulfonyl chloride, Et₃N, CH₂Cl₂, rt, overnight, 85%; (c) NaH, THF, rt, 1-2 d, 4-99%; (d) DPPA (diphenyl phosphoryl azide), Et₃N, toluene, reflux, overnight, 17-65%.
Fig. 9 is a reaction scheme showing synthesis of certain disclosed compounds. Reagents and conditions: (a) Boc₂O, DMAP, CH₂Cl₂, rt, overnight, 78%; (b) NaHMDS (sodium bis(trimethylsilyl)amide), PhNTf₂ (N-phenyl-bis(tifluoromethanesulfonamide), THF, -78 °C to rt, 4 h, 78%; (c) Pd(PPh₃)₄, LiCl, Na₂CO₃, (2-Me)PhB(OH)₂, DME (dimethoxyethane), H₂O, 60 °C, 3 h, 78%; (d) H₂, Pd/C, EtOH (ethanol), rt, 14 h, 90%; (e) TFA (trifluoroacetic acid), CH₂Cl₂, rt, 16 h, quant.; (f) 2-chloroacetyl chloride, Et₃N, THF, rt, 22 h, 79%; (g) **25,** NaH, THF, rt, 1 d, 30%.
Fig. 10A is a graph showing the effect of compound #583 at indicated concentrations on PSA-driven luciferase activity in C4-2 cells.
Fig. 10B shows the effect of compound #583 at indicated concentrations on C4-2 cell proliferation in BrdU assay.
Fig. 10C shows the effect of analog #583 at indicated concentrations on PC3 cell proliferation in BrdU assay.
Fig. 11 is a graph showing the effect of compound #571 at indicated concentrations on PSA-driven luciferase activity in C4-2 cells.
Fig. 12 is a graph showing the effect of compound JJ-450 at indicated concentrations on 22Rv1 xenograft tumor volume. JJ-450 was injected i.p. daily.
Fig. 13 is a graph showing the effect of compound JJ-450 at indicated concentrations and administration route on LNCaP xenograft tumor volume. JJ-450 was administered 6 times, from Monday to Saturday, every week
Figs. 14-35 are reaction schemes showing synthesis of certain embodiments of the disclosed compounds.

### DETAILED DESCRIPTION

The following explanations of terms and methods are provided to better describe the present compounds, compositions and methods, and to guide those of ordinary skill in the art in the practice of the present disclosure. It is also to be understood that the terminology used in the disclosure is for the purpose of describing particular embodiments and examples only and is not intended to be limiting.

"Administration of" and "administering a" compound should be understood to mean providing a compound, a prodrug of a compound, or a pharmaceutical composition as described herein. The compound or composition can be administered by another person to the subject (e.g., intravenously) or it can be self-administered by the subject (e.g., tablets).

"Alkanediyl" or "cycloalkanediyl" refers to a divalent radical of the general formula -CₙH₂ₙ- or -CₙH₂ₙ₋₂-, respectively, derived from aliphatic or cycloaliphatic hydrocarbons. "Cycloalkenediyl" refers to a divalent radical of the general formula -CₙH₂ₙ₋₄- derived from a cycloalkene.

The term "aliphatic" is defined as including alkyl, alkenyl, alkynyl, halogenated alkyl and cycloalkyl groups as described above. A "lower aliphatic" group is a branched or unbranched aliphatic group having from 1 to 10 carbon atoms.

The term "alkyl" refers to a branched or unbranched saturated hydrocarbon group of 1 to 24 carbon atoms, such as methyl, ethyl, n-propyl, isopropyl, n-butyl, isobutyl, t-butyl, pentyl, hexyl, heptyl, octyl, decyl, tetradecyl, hexadecyl, eicosyl, tetracosyl and the like. A "lower alkyl" group is a saturated branched or unbranched hydrocarbon having from 1 to 6 carbon atoms. Preferred alkyl groups have 1 to 4 carbon atoms. Alkyl groups may be "substituted alkyls" wherein one or more hydrogen atoms are substituted with a substituent such as halogen, cycloalkyl, alkoxy, amino, hydroxyl, aryl, alkenyl, or carboxyl. For example, a lower alkyl or (C₁-C₆)alkyl can be methyl, ethyl, propyl, isopropyl, butyl, iso-butyl, sec-butyl, pentyl, 3-pentyl, or hexyl; (C₃-C₆)cycloalkyl can be cyclopropyl, cyclobutyl, cyclopentyl, or cyclohexyl; (C₃-C₆)cycloalkyl(C₁-C₆)alkyl can be cyclopropylmethyl, cyclobutylmethyl, cyclopentylmethyl, cyclohexylmethyl, 2-cyclopropylethyl, 2-cyclobutylethyl, 2-cyclopentylethyl, or 2-cyclohexylethyl; (C₁-C₆)alkoxy can be methoxy, ethoxy, propoxy, isopropoxy, butoxy, iso-butoxy, sec-butoxy, pentoxy, 3-pentoxy, or hexyloxy; (C₂-C₆)alkenyl can be vinyl, allyl, 1-propenyl, 2-propenyl, 1-butenyl, 2-butenyl, 3-butenyl, 1,-pentenyl, 2-pentenyl, 3-pentenyl, 4-pentenyl, 1- hexenyl, 2-hexenyl, 3-hexenyl, 4-hexenyl, or 5-hexenyl; (C₂-C₆)alkynyl can be ethynyl, 1-propynyl, 2-propynyl, 1-butynyl, 2-butynyl, 3-butynyl, 1-pentynyl, 2-pentynyl, 3-pentynyl, 4-pentynyl, 1- hexynyl, 2-hexynyl, 3-hexynyl, 4-hexynyl, or 5-hexynyl; (C₁-C₆)alkanoyl can be acetyl, propanoyl or butanoyl; halo(C₁-C₆)alkyl can be iodomethyl, bromomethyl, chloromethyl, fluoromethyl, trifluoromethyl, 2-chloroethyl, 2-fluoroethyl, 2,2,2-trifluoroethyl, or pentafluoroethyl; hydroxy(C₁-C₆)alkyl can be hydroxymethyl, 1-hydroxyethyl, 2-hydroxyethyl, 1-hydroxypropyl, 2-hydroxypropyl, 3-hydroxypropyl, 1-hydroxybutyl, 4-hydroxybutyl, 1-hydroxypentyl, 5-hydroxypentyl, 1-hydroxyhexyl, or 6-hydroxyhexyl; (C₁-C₆)alkoxycarbonyl can be methoxycarbonyl, ethoxycarbonyl, propoxycarbonyl, isopropoxycarbonyl, butoxycarbonyl, pentoxycarbonyl, or hexyloxycarbonyl; (C₁-C₆)alkylthio can be methylthio, ethylthio, propylthio, isopropylthio, butylthio, isobutylthio, pentylthio, or hexylthio; (C₂-C₆)alkanoyloxy can be acetoxy, propanoyloxy, butanoyloxy, isobutanoyloxy, pentanoyloxy, or hexanoyloxy.

The term "alkylaryl" refers to a group in which an alkyl group is substituted for a hydrogen atom of an aryl group. An example is -Ar-R, wherein Ar is an arylene group and R is an alkyl group.

The term "alkoxy" refers to a straight, branched or cyclic hydrocarbon configuration and combinations thereof, including from 1 to 20 carbon atoms, preferably from 1 to 8 carbon atoms (referred to as a "lower alkoxy"), more preferably from 1 to 4 carbon atoms, that include an oxygen atom at the point of attachment. An example of an "alkoxy group" is represented by the formula -OR, where R can be an alkyl group, optionally substituted with an alkenyl, alkynyl, aryl, aralkyl, cycloalkyl, halogenated alkyl, alkoxy or heterocycloalkyl group. Suitable alkoxy groups include methoxy, ethoxy, n-propoxy, i-propoxy, n-butoxy, i-butoxy, sec-butoxy, tert-butoxy cyclopropoxy, cyclohexyloxy, and the like.

"Alkoxycarbonyl" refers to an alkoxy substituted carbonyl radical, -C(O)OR, wherein R represents an optionally substituted alkyl, aryl, aralkyl, cycloalkyl, cycloalkylalkyl or similar moiety.

"Alkynyl" refers to a cyclic, branched or straight chain group containing only carbon and hydrogen, and unless otherwise mentioned typically contains one to twelve carbon atoms, and contains one or more triple bonds. Alkynyl groups may be unsubstituted or substituted. "Lower alkynyl" groups are those that contain one to six carbon atoms.

The term "amide" or "amido" is represented by the formula -C(O)NRR', where R and R' independently can be a hydrogen, alkyl, alkenyl, alkynyl, aryl, arylalkyl, cycloalkyl, halogenated alkyl, or heterocycloalkyl group described above. A suitable amido group is acetamido.

The term "amine" or "amino" refers to a group of the formula -NRR', where R and R' can be, independently, hydrogen or an alkyl, alkenyl, alkynyl, aryl, arylalkyl, carbonyl (e.g, -C(O)R", where R" can be hydrogen, an alkyl, alkenyl, alkynyl, aryl, or an arylalkyl), cycloalkyl, halogenated alkyl, or heterocycloalkyl group. For example, an "alkylamino" or "alkylated amino" refers to -NRR', wherein at least one of R or R' is an alkyl.

"Aminocarbonyl" alone or in combination, means an amino substituted carbonyl (carbamoyl) radical, wherein the amino radical may optionally be mono- or di-substituted, such as with alkyl, aryl, arylalkyl, cycloalkyl, cycloalkylalkyl, alkanoyl, alkoxycarbonyl, aralkoxycarbonyl and the like. An aminocarbonyl group may be -C(O)-N(R) (wherein R is a substituted group or H). An "aminocarbonyl" is inclusive of an amido group. A suitable aminocarbonyl group is acetamido.

An "analog" is a molecule that differs in chemical structure from a parent compound, for example a homolog (differing by an increment in the chemical structure or mass, such as a difference in the length of an alkyl chain or the inclusion of one of more isotopes), a molecular fragment, a structure that differs by one or more functional groups, or a change in ionization. An analog is not necessarily synthesized from the parent compound. Structural analogs are often found using quantitative structure activity relationships (QSAR), with techniques such as those disclosed in Remington (The Science and Practice of Pharmacology, 19th Edition (1995), chapter 28). A derivative is a molecule derived from the base structure.

An "animal" refers to living multi-cellular vertebrate organisms, a category that includes, for example, mammals and birds. The term mammal includes both human and non-human mammals. Similarly, the term "subject" includes both human and non-human subjects, including birds and non-human mammals, such as non-human primates, companion animals (such as dogs and cats), livestock (such as pigs, sheep, cows), as well as non-domesticated animals, such as the big cats. The term subject applies regardless of the stage in the organism's life-cycle. Thus, the term subject applies to an organism *in utero* or *in ovo,* depending on the organism (that is, whether the organism is a mammal or a bird, such as a domesticated or wild fowl).

The term "aryl" refers to any carbon-based aromatic group including, but not limited to, phenyl, naphthyl, etc. The term "aryl" also includes "heteroaryl group," which is defined as an aromatic group that has at least one heteroatom incorporated within the ring of the aromatic group. Examples of heteroatoms include, but are not limited to, nitrogen, oxygen, sulfur, and phosphorous. The aryl group can be substituted with one or more groups including, but not limited to, alkyl, alkynyl, alkenyl, aryl, halide, nitro, amino, ester, ketone, aldehyde, hydroxy, carboxylic acid, or alkoxy, or the aryl group can be unsubstituted.

The term "arylalkyl" refers to an alkyl group where at least one hydrogen atom is substituted by an aryl group. An example of an arylalkyl group is a benzyl group.

"Carbonyl" refers to a group of the formula -C(O)-. Carbonyl-containing groups include any substituent containing a carbon-oxygen double bond (C=O), including acyl groups, amides, carboxy groups, esters, ureas, carbamates, carbonates and ketones and aldehydes, such as substituents based on -COR or - RCHO where R is an aliphatic, heteroaliphatic, alkyl, heteroalkyl, hydroxyl, or a secondary, tertiary, or quaternary amine.

"Carboxyl" refers to a -COO group. Substituted carboxyl refers to -COOR where R is aliphatic, heteroaliphatic, alkyl, heteroalkyl, or a carboxylic acid or ester.

The term "cycloalkyl" refers to a non-aromatic carbon-based ring composed of at least three carbon atoms. Examples of cycloalkyl groups include, but are not limited to, cyclopropyl, cyclobutyl, cyclopentyl, cyclohexyl, and the like. The term "heterocycloalkyl group" is a cycloalkyl group as defined above where at least one of the carbon atoms of the ring is substituted with a heteroatom such as, but not limited to, nitrogen, oxygen, sulfur, or phosphorous.

The term "co-administration" or "co-administering" refers to administration of a first agent with a second agent within the same general time period, and does not require administration at the same exact moment in time (although co-administration is inclusive of administering at the same exact moment in time). Thus, co-administration may be on the same day or on different days, or in the same week or in different weeks. The first agent and the second agent may be included in the same composition or they may each individually be included in separate compositions. In certain embodiments, the two agents may be administered during a time frame wherein their respective periods of biological activity overlap. Thus, the term includes sequential as well as coextensive administration of two or more agents.

"Derivative" refers to a compound or portion of a compound that is derived from or is theoretically derivable from a parent compound.

The terms "halogenated alkyl" or "haloalkyl group" refer to an alkyl group as defined above with one or more hydrogen atoms present on these groups substituted with a halogen (F, Cl, Br, I).

The term "hydroxyl" is represented by the formula -OH.

The term "hydroxyalkyl" refers to an alkyl group that has at least one hydrogen atom substituted with a hydroxyl group. The term "alkoxyalkyl group" is defined as an alkyl group that has at least one hydrogen atom substituted with an alkoxy group described above.

"Inhibiting" refers to inhibiting the full development of a disease or condition. "Inhibiting" also refers to any quantitative or qualitative reduction in biological activity or binding, relative to a control.

"N-heterocyclic" refers to mono or bicyclic rings or ring systems that include at least one nitrogen heteroatom. The rings or ring systems generally include 1 to 9 carbon atoms in addition to the heteroatom(s) and may be saturated, unsaturated or aromatic (including pseudoaromatic). The term "pseudoaromatic" refers to a ring system which is not strictly aromatic, but which is stabilized by means of delocalization of electrons and behaves in a similar manner to aromatic rings. Aromatic includes pseudoaromatic ring systems, such as pyrrolyl rings.

Examples of 5-membered monocyclic N-heterocycles include pyrrolyl, H-pyrrolyl, pyrrolinyl, pyrrolidinyl, oxazolyl, oxadiazolyl, (including 1,2,3 and 1,2,4 oxadiazolyls) isoxazolyl, furazanyl, thiazolyl, isothiazolyl, pyrazolyl, pyrazolinyl, pyrazolidinyl, imidazolyl, imidazolinyl, triazolyl (including 1,2,3 and 1,3,4 triazolyls), tetrazolyl, thiadiazolyl (including 1,2,3 and 1,3,4 thiadiazolyls), and dithiazolyl. Examples of 6-membered monocyclic N-heterocycles include pyridyl, pyrimidinyl, pyridazinyl, pyrazinyl, piperidinyl, morpholinyl, thiomorpholinyl, piperazinyl, and triazinyl. The heterocycles may be optionally substituted with a broad range of substituents, and preferably with C₁₋₆ alkyl, C₁₋₆ alkoxy, C₂₋₆ alkenyl, C₂₋₆ alkynyl, halo, hydroxy, mercapto, trifluoromethyl, amino, cyano or mono or di(C₁₋₆alkyl)amino. The N-heterocyclic group may be fused to a carbocyclic ring such as phenyl, naphthyl, indenyl, azulenyl, fluorenyl, and anthracenyl.

Examples of 8, 9 and 10-membered bicyclic heterocycles include 1H thieno[2,3-c]pyrazolyl, indolyl, isoindolyl, benzoxazolyl, benzothiazolyl, benzisoxazolyl, benzisothiazolyl, benzimidazolyl, indazolyl, isoquinolinyl, quinolinyl, quinoxalinyl, purinyl, cinnolinyl, phthalazinyl, quinazolinyl, quinoxalinyl, benzotriazinyl, and the like. These heterocycles may be optionally substituted, for example with C₁₋₆ alkyl, C₁₋₆ alkoxy, C₂₋₆ alkenyl, C₂₋₆ alkynyl, halo, hydroxy, mercapto, trifluoromethyl, amino, cyano or mono or di(C₁₋₆alkyl)amino. Unless otherwise defined optionally substituted N-heterocyclics includes pyridinium salts and the N-oxide form of suitable ring nitrogens.

Examples of N-heterocycles also include bridged groups such as, for example, azabicyclo (for example, azabicyclooctane).

"Stereoisomers" are isomers that have the same molecular formula and sequence of bonded atoms, but which differ only in the three-dimensional orientation of the atoms in space. By convention, bold wedge bonds are used to indicate bonds coming out of the page toward the reader, and hashed wedge bonds are used to indicate bonds going behind the page away from the reader. Pairs of bold and hashed bonds that are not wedged are used to indicate bonds of the same orientation, i.e., a pair of bonds that are both coming out of the page or going behind the page.

"Pharmaceutical compositions" are compositions that include an amount (for example, a unit dosage) of one or more of the disclosed compounds together with one or more non-toxic pharmaceutically acceptable additives, including carriers, diluents, and/or adjuvants, and optionally other biologically active ingredients. Such pharmaceutical compositions can be prepared by standard pharmaceutical formulation techniques such as those disclosed in Remington's Pharmaceutical Sciences, Mack Publishing Co., Easton, PA (19th Edition).

The terms "pharmaceutically acceptable salt or ester" refers to salts or esters prepared by conventional means that include salts, e.g., of inorganic and organic acids, including but not limited to hydrochloric acid, hydrobromic acid, sulfuric acid, phosphoric acid, methanesulfonic acid, ethanesulfonic acid, malic acid, acetic acid, oxalic acid, tartaric acid, citric acid, lactic acid, fumaric acid, succinic acid, maleic acid, salicylic acid, benzoic acid, phenylacetic acid, mandelic acid and the like. "Pharmaceutically acceptable salts" of the presently disclosed compounds also include those formed from cations such as sodium, potassium, aluminum, calcium, lithium, magnesium, zinc, and from bases such as ammonia, ethylenediamine, N-methyl-glutamine, lysine, arginine, ornithine, choline, N,N'-dibenzylethylenediamine, chloroprocaine, diethanolamine, procaine, N-benzylphenethylamine, diethylamine, piperazine, tris(hydroxymethyl)aminomethane, and tetramethylammonium hydroxide. These salts may be prepared by standard procedures, for example by reacting the free acid with a suitable organic or inorganic base. Any chemical compound recited in this specification may alternatively be administered as a pharmaceutically acceptable salt thereof. "Pharmaceutically acceptable salts" are also inclusive of the free acid, base, and zwitterionic forms. Descriptions of suitable pharmaceutically acceptable salts can be found in Handbook of Pharmaceutical Salts, Properties, Selection and Use, Wiley VCH (2002). When compounds disclosed herein include an acidic function such as a carboxy group, then suitable pharmaceutically acceptable cation pairs for the carboxy group are well known to those skilled in the art and include alkaline, alkaline earth, ammonium, quaternary ammonium cations and the like. Such salts are known to those of skill in the art. For additional examples of "pharmacologically acceptable salts," see Berge et al., J. Pharm. Sci. 66:1 (1977).

"Pharmaceutically acceptable esters" includes those derived from compounds described herein that are modified to include a carboxyl group. An *in vivo* hydrolysable ester is an ester which is hydrolysed in the human or animal body to produce the parent acid or alcohol. Representative esters thus include carboxylic acid esters in which the non-carbonyl moiety of the carboxylic acid portion of the ester grouping is selected from straight or branched chain alkyl (for example, methyl, n-propyl, t-butyl, or n-butyl), cycloalkyl, alkoxyalkyl (for example, methoxymethyl), arylalkyl (for example benzyl), aryloxyalkyl (for example, phenoxymethyl), aryl (for example, phenyl, optionally substituted by, for example, halogen, C.sub.1-4 alkyl, or C.sub.1-4 alkoxy) or amino); sulphonate esters, such as alkyl- or arylalkylsulphonyl (for example, methanesulphonyl); or amino acid esters (for example, L-valyl or L-isoleucyl). A "pharmaceutically acceptable ester" also includes inorganic esters such as mono-, di-, or tri-phosphate esters. In such esters, unless otherwise specified, any alkyl moiety present advantageously contains from 1 to 18 carbon atoms, particularly from 1 to 6 carbon atoms, more particularly from 1 to 4 carbon atoms. Any cycloalkyl moiety present in such esters advantageously contains from 3 to 6 carbon atoms. Any aryl moiety present in such esters advantageously comprises a phenyl group, optionally substituted as shown in the definition of carbocycylyl above. Pharmaceutically acceptable esters thus include C₁-C₂₂ fatty acid esters, such as acetyl, t-butyl or long chain straight or branched unsaturated or omega-6 monounsaturated fatty acids such as palmoyl, stearoyl and the like. Alternative aryl or heteroaryl esters include benzoyl, pyridylmethyloyl and the like any of which may be substituted, as defined in carbocyclyl above. Additional pharmaceutically acceptable esters include aliphatic L-amino acid esters such as leucyl, isoleucyl and especially valyl.

For therapeutic use, salts of the compounds are those wherein the counter-ion is pharmaceutically acceptable. However, salts of acids and bases which are non-pharmaceutically acceptable may also find use, for example, in the preparation or purification of a pharmaceutically acceptable compound.

The pharmaceutically acceptable acid and base addition salts as mentioned hereinabove are meant to comprise the therapeutically active non-toxic acid and base addition salt forms which the compounds are able to form. The pharmaceutically acceptable acid addition salts can conveniently be obtained by treating the base form with such appropriate acid. Appropriate acids comprise, for example, inorganic acids such as hydrohalic acids, e.g. hydrochloric or hydrobromic acid, sulfuric, nitric, phosphoric and the like acids; or organic acids such as, for example, acetic, propanoic, hydroxyacetic, lactic, pyruvic, oxalic (i.e. ethanedioic), malonic, succinic (i.e. butanedioic acid), maleic, fumaric, malic (i.e. hydroxybutanedioic acid), tartaric, citric, methanesulfonic, ethanesulfonic, benzenesulfonic, p-toluenesulfonic, cyclamic, salicylic, p-aminosalicylic, pamoic and the like acids. Conversely said salt forms can be converted by treatment with an appropriate base into the free base form.

The compounds containing an acidic proton may also be converted into their non-toxic metal or amine addition salt forms by treatment with appropriate organic and inorganic bases. Appropriate base salt forms comprise, for example, the ammonium salts, the alkali and earth alkaline metal salts, e.g. the lithium, sodium, potassium, magnesium, calcium salts and the like, salts with organic bases, e.g. the benzathine, N-methyl-D-glucamine, hydrabamine salts, and salts with amino acids such as, for example, arginine, lysine and the like.

The term "addition salt" as used hereinabove also comprises the solvates which the compounds described herein are able to form. Such solvates are for example hydrates, alcoholates and the like.

The term "quaternary amine" as used hereinbefore defines the quaternary ammonium salts which the compounds are able to form by reaction between a basic nitrogen of a compound and an appropriate quaternizing agent, such as, for example, an optionally substituted alkylhalide, arylhalide or arylalkylhalide, e.g. methyliodide or benzyliodide. Other reactants with good leaving groups may also be used, such as alkyl trifluoromethanesulfonates, alkyl methanesulfonates, and alkyl p-toluenesulfonates. A quaternary amine has a positively charged nitrogen. Pharmaceutically acceptable counterions include chloro, bromo, iodo, trifluoroacetate and acetate. The counterion of choice can be introduced using ion exchange resins.

It will be appreciated that the compounds described herein may have metal binding, chelating, complex forming properties and therefore may exist as metal complexes or metal chelates.

Some of the compounds described herein may also exist in their tautomeric form.

The term "subject" includes both human and veterinary subjects.

A "therapeutically effective amount" or "diagnostically effective amount" refers to a quantity of a specified agent sufficient to achieve a desired effect in a subject being treated with that agent. Ideally, a therapeutically effective amount or diagnostically effective amount of an agent is an amount sufficient to inhibit or treat the disease without causing a substantial cytotoxic effect in the subject. The therapeutically effective amount or diagnostically effective amount of an agent will be dependent on the subject being treated, the severity of the affliction, and the manner of administration of the therapeutic composition.

"Treatment" refers to a therapeutic intervention that ameliorates a sign or symptom of a disease or pathological condition after it has begun to develop. As used herein, the term "ameliorating," with reference to a disease or pathological condition, refers to any observable beneficial effect of the treatment. The beneficial effect can be evidenced, for example, by a delayed onset of clinical symptoms of the disease in a susceptible subject, a reduction in severity of some or all clinical symptoms of the disease, a slower progression of the disease, an improvement in the overall health or well-being of the subject, or by other parameters well known in the art that are specific to the particular disease. The phrase "treating a disease" is inclusive of inhibiting the full development of a disease or condition, for example, in a subject who is at risk for a disease, or who has a disease, such as cancer or a disease associated with a compromised immune system. "Preventing" a disease or condition refers to prophylactic administering a composition to a subject who does not exhibit signs of a disease or exhibits only early signs for the purpose of decreasing the risk of developing a pathology or condition, or diminishing the severity of a pathology or condition.

Prodrugs of the disclosed compounds also are contemplated herein. A prodrug is an active or inactive compound that is modified chemically through *in vivo* physiological action, such as hydrolysis, metabolism and the like, into an active compound following administration of the prodrug to a subject. The term "prodrug" as used throughout this text means the pharmacologically acceptable derivatives such as esters, amides and phosphates, such that the resulting *in vivo* biotransformation product of the derivative is the active drug as defined in the compounds described herein. Prodrugs preferably have excellent aqueous solubility, increased bioavailability and are readily metabolized into the active inhibitors *in vivo.* Prodrugs of a compounds described herein may be prepared by modifying functional groups present in the compound in such a way that the modifications are cleaved, either by routine manipulation or *in vivo,* to the parent compound. The suitability and techniques involved in making and using prodrugs are well known by those skilled in the art. For a general discussion of prodrugs involving esters see Svensson and Tunek, Drug Metabolism Reviews 165 (1988) and Bundgaard, Design of Prodrugs, Elsevier (1985).

The term "prodrug" also is intended to include any covalently bonded carriers that release an active parent drug of the present invention *in vivo* when the prodrug is administered to a subject. Since prodrugs often have enhanced properties relative to the active agent pharmaceutical, such as, solubility and bioavailability, the compounds disclosed herein can be delivered in prodrug form. Thus, also contemplated are prodrugs of the presently disclosed compounds, methods of delivering prodrugs and compositions containing such prodrugs. Prodrugs of the disclosed compounds typically are prepared by modifying one or more functional groups present in the compound in such a way that the modifications are cleaved, either in routine manipulation or *in vivo,* to yield the parent compound. Prodrugs include compounds having a phosphonate and/or amino group functionalized with any group that is cleaved *in vivo* to yield the corresponding amino and/or phosphonate group, respectively. Examples of prodrugs include, without limitation, compounds having an acylated amino group and/or a phosphonate ester or phosphonate amide group. In particular examples, a prodrug is a lower alkyl phosphonate ester, such as an isopropyl phosphonate ester.

Protected derivatives of the disclosed compounds also are contemplated. A variety of suitable protecting groups for use with the disclosed compounds are disclosed in Greene and Wuts, Protective Groups in Organic Synthesis; 3rd Ed.; John Wiley & Sons, New York, 1999.

In general, protecting groups are removed under conditions which will not affect the remaining portion of the molecule. These methods are well known in the art and include acid hydrolysis, hydrogenolysis and the like. One preferred method involves the removal of an ester, such as cleavage of a phosphonate ester using Lewis acidic conditions, such as in TMS-Br mediated ester cleavage to yield the free phosphonate. A second preferred method involves removal of a protecting group, such as removal of a benzyl group by hydrogenolysis utilizing palladium on carbon in a suitable solvent system such as an alcohol, acetic acid, and the like or mixtures thereof. A t-butoxy-based group, including t-butoxy carbonyl protecting groups can be removed utilizing an inorganic or organic acid, such as HCl or trifluoroacetic acid, in a suitable solvent system, such as water, dioxane and/or methylene chloride. Another exemplary protecting group, suitable for protecting amino and hydroxy functions amino is trityl. Other conventional protecting groups are known and suitable protecting groups can be selected by those of skill in the art in consultation with Greene and Wuts, Protective Groups in Organic Synthesis; 3rd Ed.; John Wiley & Sons, New York, 1999. When an amine is deprotected, the resulting salt can readily be neutralized to yield the free amine. Similarly, when an acid moiety, such as a phosphonic acid moiety is unveiled, the compound may be isolated as the acid compound or as a salt thereof.

Particular examples of the presently disclosed compounds include one or more asymmetric centers; thus these compounds can exist in different stereoisomeric forms. Accordingly, compounds and compositions may be provided as individual pure enantiomers or as stereoisomeric mixtures, including racemic mixtures. In certain embodiments the compounds disclosed herein are synthesized in or are purified to be in substantially enantiopure form, such as in a 90% enantiomeric excess, a 95% enantiomeric excess, a 97% enantiomeric excess or even in greater than a 99% enantiomeric excess, such as in enantiopure form.

Groups which are substituted (e.g. substituted alkyl), may in some embodiments be substituted with a group which is substituted (e.g. substituted aryl). In some embodiments, the number of substituted groups linked together is limited to two (e.g. substituted alkyl is substituted with substituted aryl, wherein the substituent present on the aryl is not further substituted). In some embodiments, a substituted group is not substituted with another substituted group (e.g. substituted alkyl is substituted with unsubstituted aryl).

### Overview

CRPC is responsible for all prostate cancer deaths, and eventually all prostate cancer will develop into CRPC. The current best treatment for CRPC is MDV3100 (enzalutamide), which binds to androgen receptor. It is effective against a number of androgen-dependent prostate cancer cell lines. However, it is ineffective against the androgen-dependent prostate cancer cell line 22Rv1. Compounds disclosed herein are effective against all androgen-dependent cell lines tested including 22Rv1, a promising and unique property.

Several of the compounds show sub-micromolar inhibition of PSA-luciferase expression in C4-2 cells. Further, cell proliferation in androgen-dependent cell lines is significantly decreased while proliferation in androgen-independent cell lines is unaffected.

### Agents

Disclosed herein are agents that can be used for treating prostate cancer, particularly castration-resistant prostate cancer. The agents may inhibit AR nuclear localization and/or reduce AR levels in castration-resistant prostate cancer.

Disclosed herein, but not claimed, the agent is a compound, or a pharmaceutically acceptable salt or ester thereof, having a formula I of:

R²⁰ - (Z)_{b} - (Y)_{c} - (R²¹)ₐ - (X)_{d} - R²² - R²³

wherein R²⁰ is an aryl, substituted aryl, heteroaryl, substituted heteroaryl, cycloalkyl, substituted cycloalkyl, heterocycloalkyl, substituted heterocycloalkyl, alkoxy, aryloxy, a thio-containing group, a seleno-containing group, halide, or a nitro-containing group;
Z is alkanediyl, substituted alkanediyl, cycloalkanediyl, or substituted cycloalkanediyl;
Y is S, **O,** S(=O), -S(=O)(=O)-, or NR¹⁰, wherein R¹⁰ is H or alkyl (preferably methyl);
R²¹ is alkanediyl, substituted alkanediyl, cycloalkanediyl, substituted cycloalkanediyl, alkadienyl, substituted alkadienyl, cycloalkenediyl, substituted cycloalkenediyl, alkatrienyl, or substituted alkatrienyl;
X is -C(=O)-, -S(=O)(=O)-, or -N(H)C(=O)-:
R²² is a moiety that includes at least one divalent amino radical;
R²³ is an aryl, substituted aryl, heteroaryl, substituted heteroaryl, cycloalkyl, substituted cycloalkyl, heterocycloalkyl, substituted heterocycloalkyl, alkoxy, aryloxy, amino, a thio-containing group, or a seleno-containing group;
a is 0 or 1;
b is 0 or 1;
c is 0 or 1; and
d is 0 or 1.

Also disclosed but not claimed, if X is -C(=O)- then Y is not S. In certain embodiments, R²¹ is cycloalkanediyl, such as cyclopropanediyl. When R²¹ is cycloalkanediyl, R²⁰ may be a phenyl optionally substituted with at least one halogen and/or R²³ may be a phenyl substituted with at least one halogen and or at least one alkyl.

In certain embodiments, R²⁰ is selected from isoxazolyl, substituted isoxazolyl (e.g, dialkyl-substituted such as dimethyl, hydroxy-substituted, hydroxyalkyl-substituted, or a combination thereof), oxazolyl, substituted oxazolyl (e.g, dialkyl-substituted such as dimethyl, hydroxy-substituted, hydroxyalkyl-substituted, or a combination thereof) cyclohexyl, substituted cyclohexyl (e.g., hydroxy-substituted cyclohexyl), piperidinyl, substituted piperidinyl (e.g., hydroxy-substituted piperidinyl), oxacyclopentyl, substituted oxacyclopentyl (e.g., hydroxyalkyl-substituted), oxacyclohexanyl, substituted oxacyclopentyl (e.g., hydroxyalkyl-substituted), thiophenyl, substituted thiophenyl (e.g., hydroxyalkyl-substituted), phenyl, substituted phenyl (e.g., hydroxyalkyl-substituted or halogen-substituted), pyridinyl, substituted pyridinyl (e.g., hydroxyalkyl-substituted), indolyl, substituted indolyl (e.g., hydroxyalkyl-substituted), furanyl, substituted furanyl (e.g., hydroxyalkyl-substituted), imidazolyl, substituted imidazolyl (e.g., hydroxyalkyl-substituted). In preferred embodiments, R²⁰ is substituted isoxazolyl, particularly dialkyl (e.g., dimethyl)-substituted isooxazolyl, phenyl, or substituted phenyl, particularly halogen-substituted phenyl (e.g., fluorophenyl).

In certain embodiments, R²¹ is selected from C₁-C₃ alkanediyl or substituted C₁-C₃ alkanediyl (e.g., alkyl-substituted such as methyl or dimethyl), preferably C₁ alkanediyl (-CH₂-), C₃ alkanediyl (-(CH₂)₃-), or cycloalkanediyl, preferably cyclopropanediyl. In certain embodiments, R²¹ is:

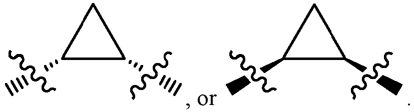

In certain embodiments, R²² is selected from: 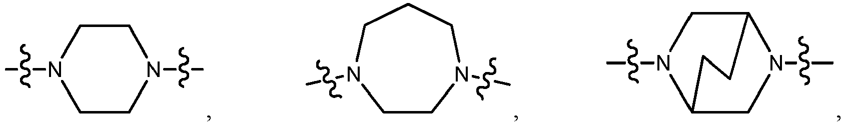 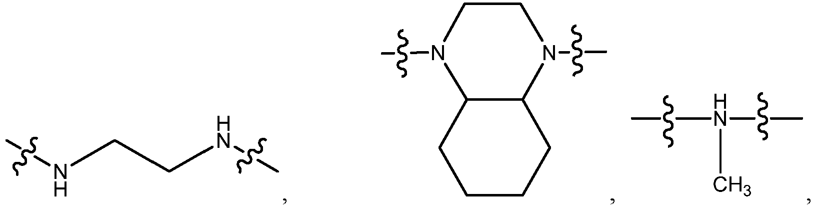 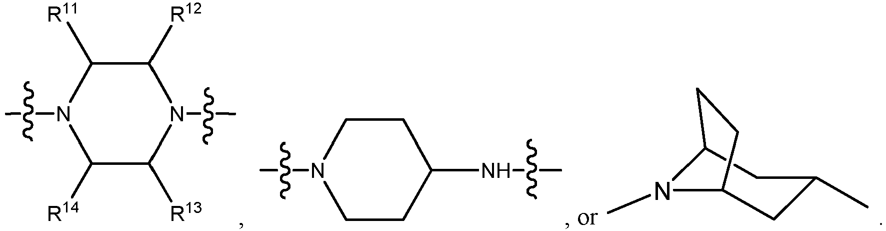 wherein R¹¹ to R¹⁴ are each individually H or alkyl, provided that at least one of R¹¹ to R¹⁴ is alkyl. In certain embodiments, R¹² and R¹³ are each alkyl (e.g., methyl) and R¹¹ and R¹⁴ are each H. In certain embodiments, R¹¹ and R¹⁴ are each alkyl (e.g., methyl) and R¹² and R¹³ are each H.

In certain embodiments, R²² is a divalent radical of a N-heterocyclic group. Illustrative N-heterocylic groups include piperazinyl, substituted piperazinyl, azabicyclo (for example, azabicyclooctane), and substituted azabicyclo.

In certain embodiments, R²³ is selected from phenyl, substituted phenyl (e.g., alkyl-substituted phenyl such as dimethyl-substituted, or halogen substituted, such as chloro- or fluoro-substituted, or amino-substituted, or aminoalkyl-substituted; alkynyl-substituted phenyl), piperidinyl, substituted piperidinyl (e.g., amino-substituted), furanyl, substituted furanyl (e.g., aminoalkyl-substituted or amino-substituted), pyridinyl, substituted pyridinyl (e.g., aminoalkyl-substituted or amino-substituted), pyrimidinyl, substituted pyrimidinyl (e.g., aminoalkyl-substituted or amino-substituted), naphthenyl, substituted naphthenyl, (e.g., aminoalkyl-substituted or amino-substituted), thiazole, substituted thiazole (e.g., aminoalkyl-substituted or amino-substituted); isoindazolyl, substituted isoindazolyl (e.g., aminoalkyl-substituted or amino-substituted); triazolyl, or substituted triazolyl (e.g., aminoalkyl-substituted or amino-substituted). R²³ may have two or more substituents, such as an alkyl substituent and a halogen substituent. Preferably, R²³ is a substituted phenyl having a structure of: 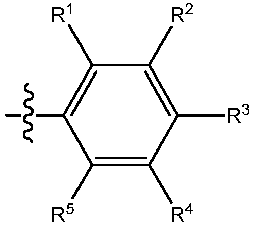 wherein each of R¹-R⁵ is individually H, alkyl, substituted alkyl, alkynyl, substituted alkynyl, halogen, or cyano, provided that at least one of R¹-R⁵ is not H. In certain embodiments, at least one of R¹-R⁵ is alkyl (such as methyl), halogen or cyano. In certain embodiments, R¹ is alkyl, halogen or cyano. In certain embodiments, R¹ is alkyl and R⁴ is halogen. In certain embodiments, at least one of R¹-R⁵ is hydroxy-substituted alkynyl.

In certain embodiments, Z is selected from C₁-C₃ alkanediyl, preferably -CH₂-.

In certain embodiments, R²⁰ is phenyl or substituted isoxazolyl, b is 0; c is 1; a is 1; R²¹ is -CH₂-, Y is S; X is -S(=O)(=O)-, R²² is: 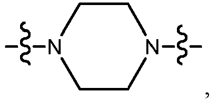 and R²³ is substituted phenyl.

In certain embodiments, R²⁰ is substituted phenyl, b is 0, c is 0, R²¹ is cyclopropanediyl, a is 1, X is -C(=O)-, d is 1, R²² is 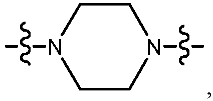 and R²³ is substituted phenyl. In one such embodiment, R²⁰ is halophenyl and R²³ is halo- and alkyl-substituted phenyl.

In certain embodiments, R²¹ is -CH₂-, Y is S; and X is -S(=O)(=O)-.

In certain embodiments, R²² is:

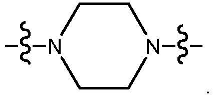

In certain embodiments, Y is S, O, S(=O), -S(=O)(=O)-; and X is -C(=O)-.

In certain embodiments, b is 0; c is 0; a is 1; and X is -C(=O)-.

In certain embodiments, b is 0; c is 0; a is 1; X is -C(=O)-; and R²¹ is alkanediyl (particularly - CH₂CH₂-),

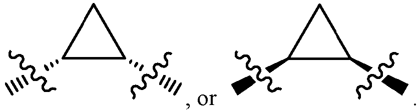

In certain embodiments, b is 0; c is 0; a is 1; X is -C(=O)-; R²¹ is alkanediyl (particularly -CH₂CH₂-), 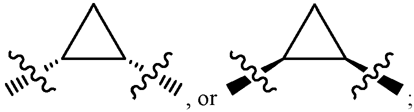 and R²² is

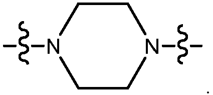

In certain embodiments, b is 0; c is 0; a is 1; X is -C(=O)-; R²¹ is alkanediyl (particularly -CH₂CH₂-), 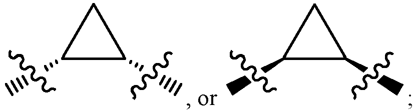 R²² is 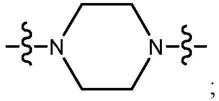 R²⁰ is phenyl, substituted phenyl, or substituted isoxazolyl; and R²³ is substituted phenyl.

In a further embodiment, the agent is a compound, or a pharmaceutically acceptable salt or ester thereof, having a formula II of:

R³⁰ - (Z')_{b} - (Y') - (R³¹)ₐ - X' - R³² - R³³

wherein R³⁰ is an aryl, substituted aryl, heteroaryl, substituted heteroaryl, heterocycloalkyl, substituted heterocycloalkyl, alkoxy, aryloxy, amino, a thio-containing group, or a seleno-containing group;
Z' is alkanediyl, or substituted alkanediyl;
Y' is S;
R³¹ is alkanediyl or substituted alkanediyl;
X is -C(=O)-;
R³² is a moiety that includes at least one divalent amino radical;
R³³ is a phenyl substituted with at least one halogen or cyano;
a is 0 or 1; and
b is 0 or 1.

In certain embodiments, R³⁰ is selected from isoxazolyl, substituted isoxazolyl (e.g, dialkyl-substituted such as dimethyl, hydroxy-substituted, hydroxyalkyl-substituted, or a combination thereof), oxazolyl, substituted oxazolyl (e.g, dialkyl-substituted such as dimethyl, hydroxy-substituted, hydroxyalkyl-substituted, or a combination thereof) cyclohexyl, substituted cyclohexyl (e.g., hydroxy-substituted cyclohexyl), piperidinyl, substituted piperidinyl (e.g., hydroxy-substituted piperidinyl), oxacyclopentyl, substituted oxacyclopentyl (e.g., hydroxyalkyl-substituted), oxacyclohexanyl, substituted oxacyclopentyl (e.g., hydroxyalkyl-substituted), thiophenyl, substituted thiophenyl (e.g., hydroxyalkyl-substituted), phenyl, substituted phenyl (e.g., hydroxyalkyl-substituted), pyridinyl, substituted pyridinyl (e.g., hydroxyalkyl-substituted), indolyl, substituted indolyl (e.g., hydroxyalkyl-substituted), furanyl, substituted furanyl (e.g., hydroxyalkyl-substituted), imidazolyl, substituted imidazolyl (e.g., hydroxyalkyl-substituted). In preferred embodiments, R³⁰ is substituted isoxazolyl, particularly dialkyl (e.g., dimethyl)-substituted isooxazolyl, or phenyl.

In certain embodiments, Z' is selected from C₁-C₃ alkanediyl, preferably -CH₂-.

In certain embodiments, R³¹ is selected from C₁-C₃ alkanediyl or substituted C₁-C₃ alkanediyl (e.g., alkyl-substituted such as methyl or dimethyl), preferably C₁ alkanediyl.

In certain embodiments, R³² is selected from:

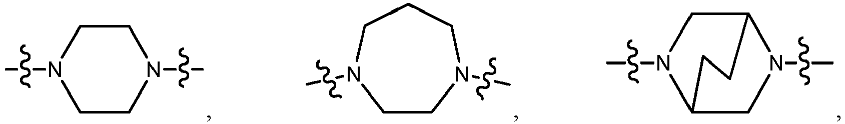

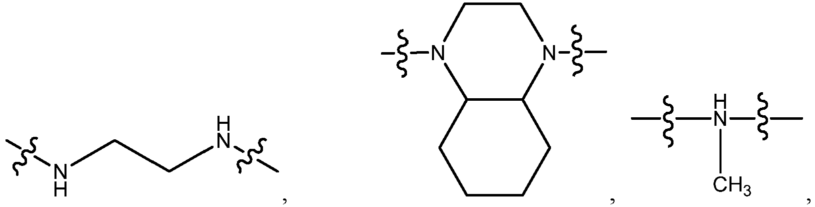

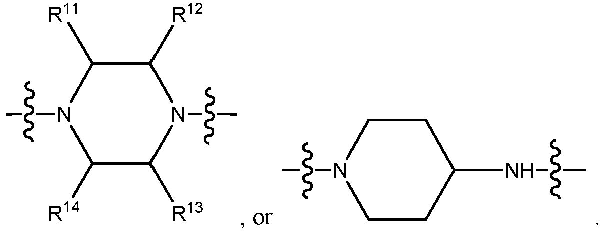

Preferably, R³³ is a substituted phenyl having a structure of: 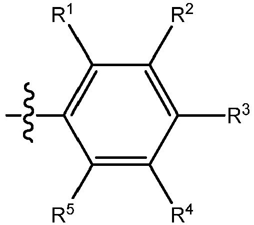 wherein each of R¹-R⁵ is individually H, alkyl, halogen, or cyano, provided that at least one of R¹-R⁵ is halogen or cyano. In certain embodiments, R¹ is alkyl, halogen or cyano.

In certain embodiments, R³⁰ is substituted isoxazolyl, b is 1; a is 1; R²¹ is -CH₂-; and R³² is:

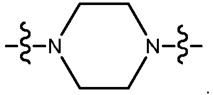

In a further embodiment, the agent is a compound, or stereoisomer, pharmaceutically acceptable salt, or an ester thereof according to formula III: 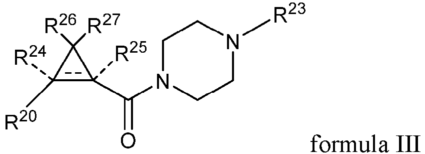 where the bond represented by "- - - - -" is a single or double bond. R²⁰ is (a) phenyl substituted with C₁-C₃ perfluoroalkyl, halo, or pentafluorosulfanyl, (b) thiophenyl substituted with C₁-C₃ alkyl, or (c) cycloalkyl substituted with C₁-C₃ perfluoroalkyl. R²³ is (a) phenyl mono- or di-substituted with substituents selected from halo, C₁-C₃ alkyl, C₁-C₃ perfluoroalkyl, pentafluorosulfanyl (-SF₅), cyano, -C(O)Oalkyl, or -C(O)N(H)alkyl (b) pyrimidinyl, (c) cycloalkyl, or (d) heterocycloalkyl. R²⁴ and R²⁵ are absent if the bond represented by "-----" is a double bond, or R²⁴ and R²⁵ independently are hydrogen, deuterium, C₁-C₃ perhaloalkyl, halo, or cyano, or R²⁴ and R²⁵ together form -CH₂-. R²⁶ and R²⁷ independently are hydrogen, deuterium, or halo. In some embodiments, when R²⁴-R²⁷ are hydrogen, then R²⁰ is not halo-substituted phenyl.

In some examples, R²⁰ is phenyl substituted with -CF₃, -SF₅, or -F; thiophenyl substituted with -CH₃; or cyclohexyl substituted with -CF₃. In certain examples, R²⁰ is phenyl or cyclohexyl and is substituted at the C4 position. In other examples, R²⁰ is phenyl substituted at the C3 position.

In any of the foregoing examples, R²³ may be phenyl substituted with -CF₃; phenyl disubstituted with two halo substituents, halo and -CF₃, halo and -CH₃, or halo and cyano; pyrimidinyl; cyclohexyl; or heterocyclohexyl (e.g., containing a heteroatom or group selected from O, N(H), or N(CH₃)). In some exmaples, R²³ is a disubstituted phenyl and the two substituents are para to one another.

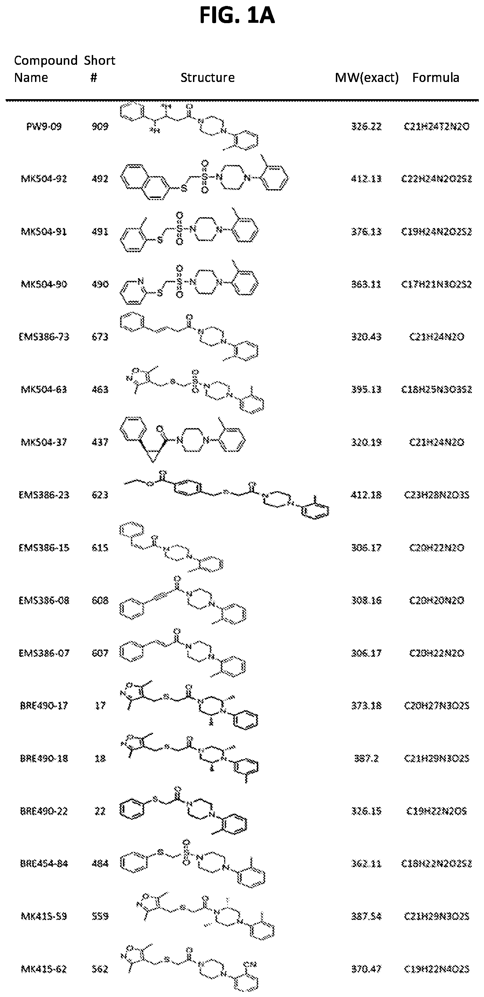

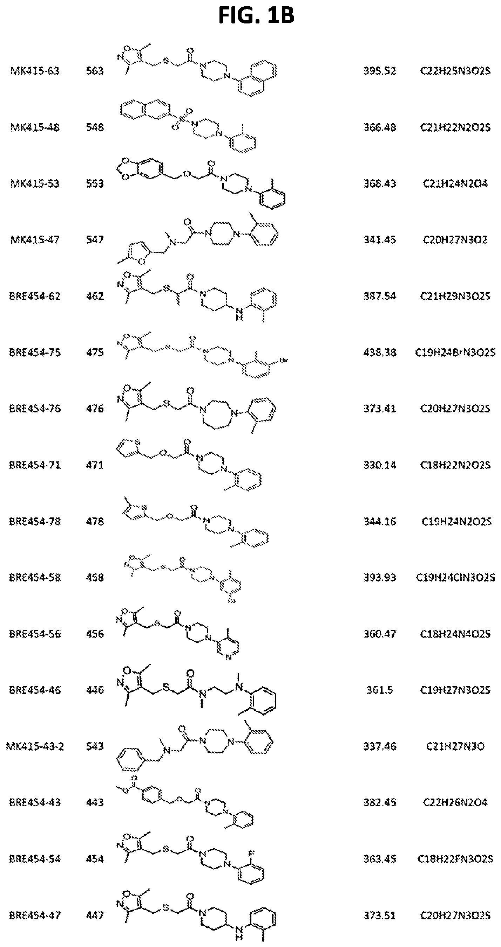

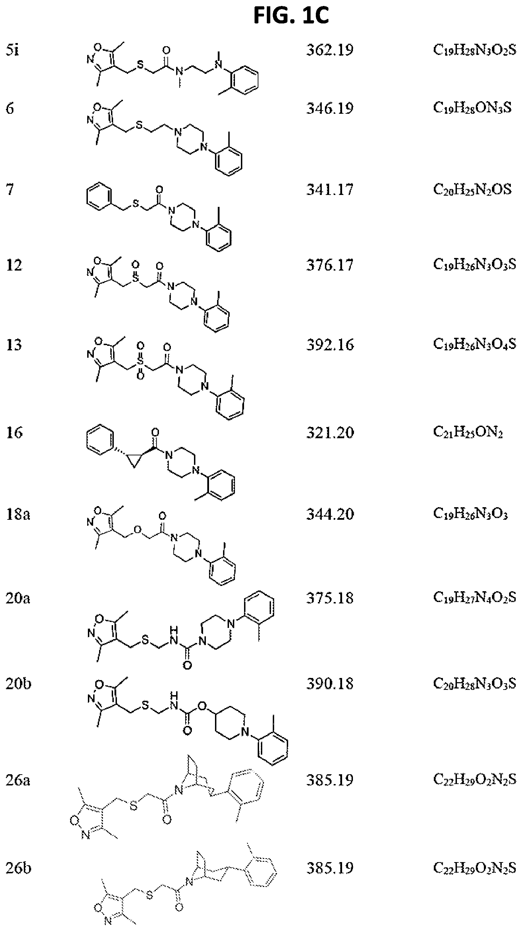

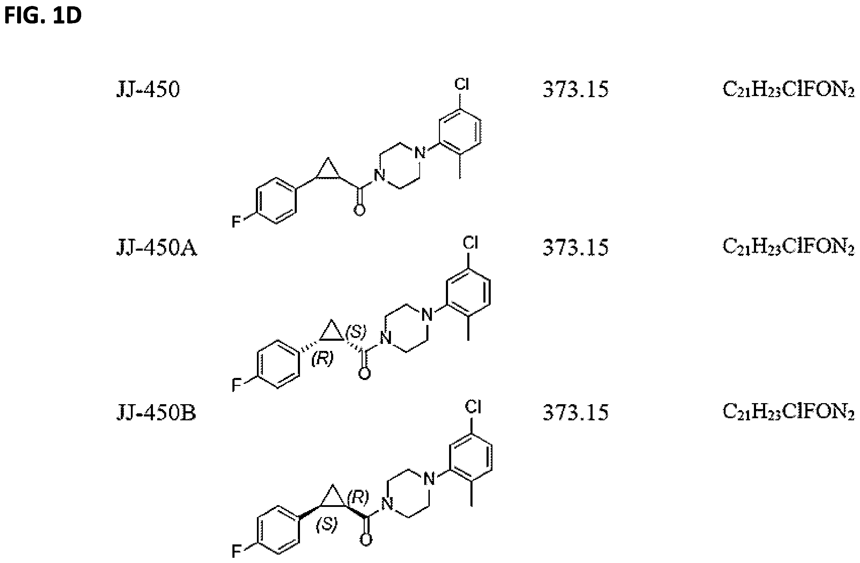

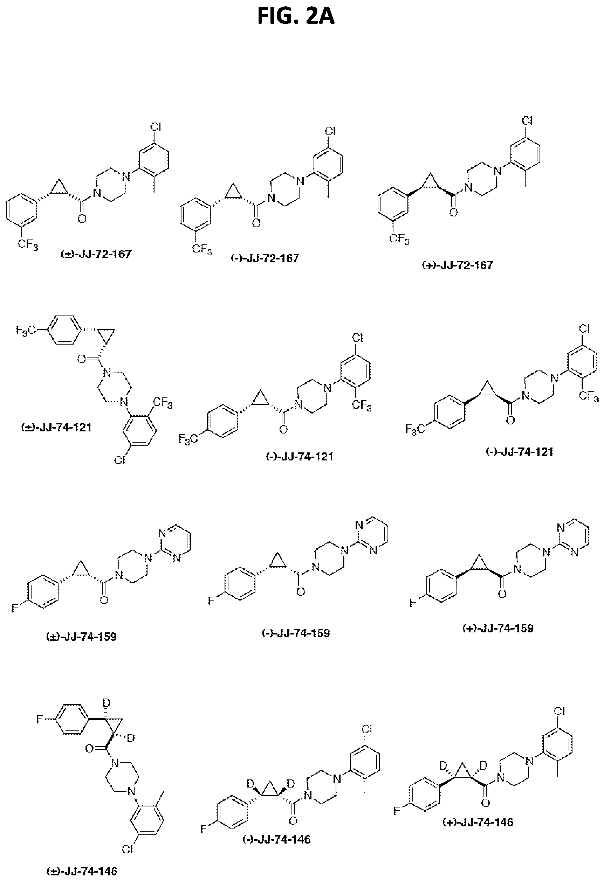

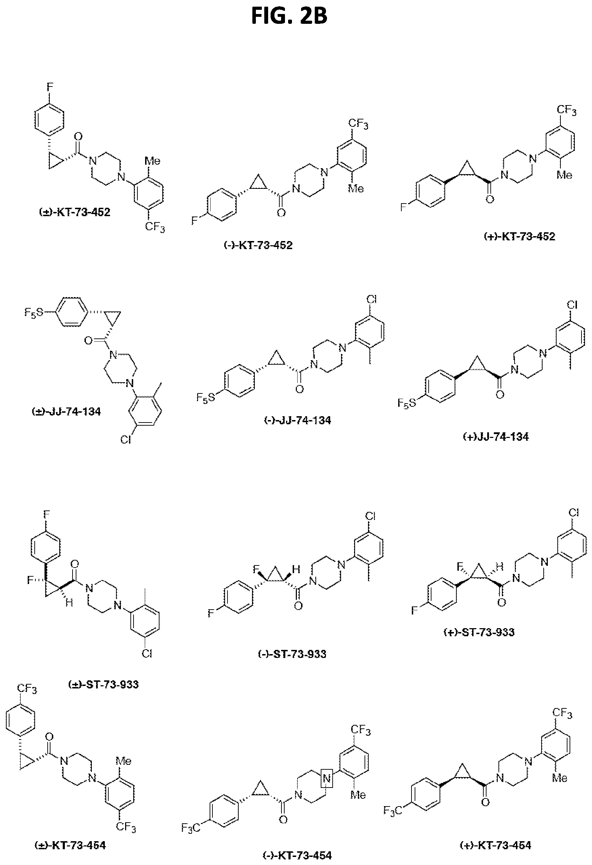

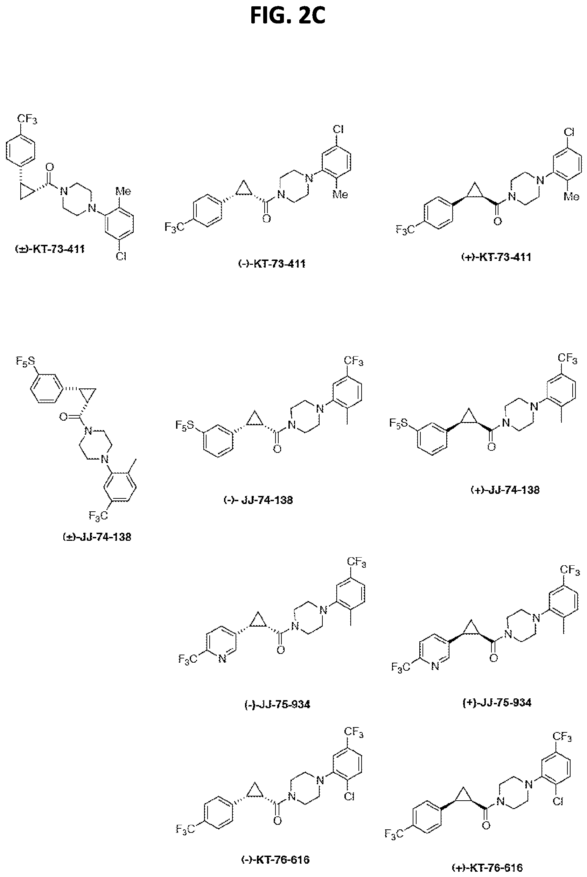

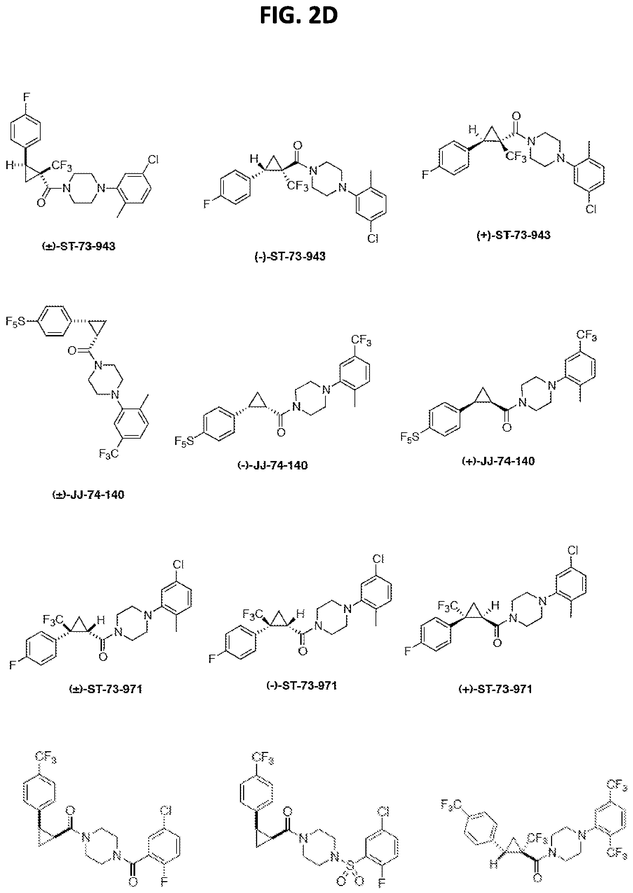

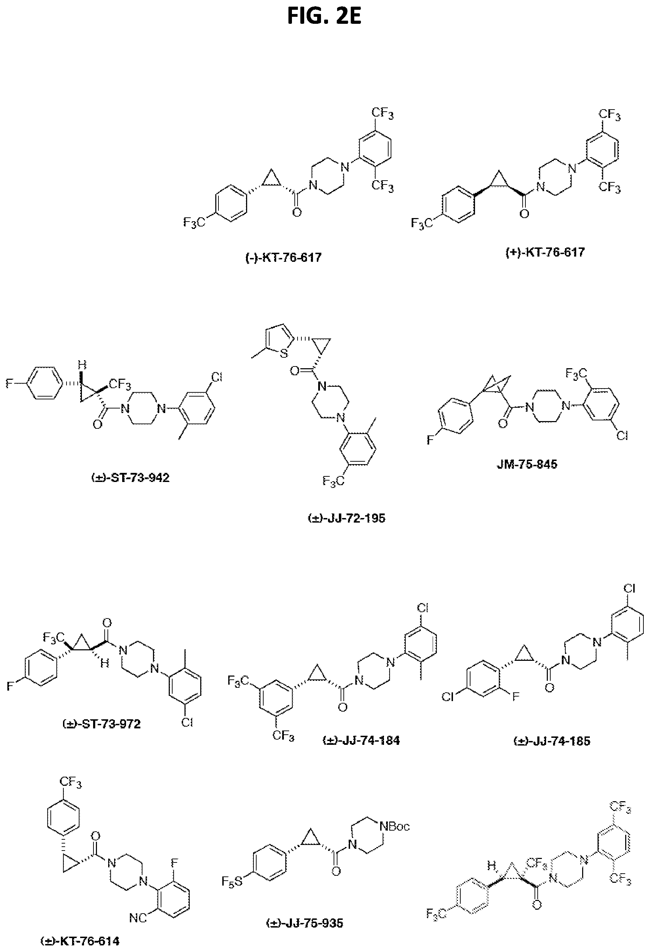

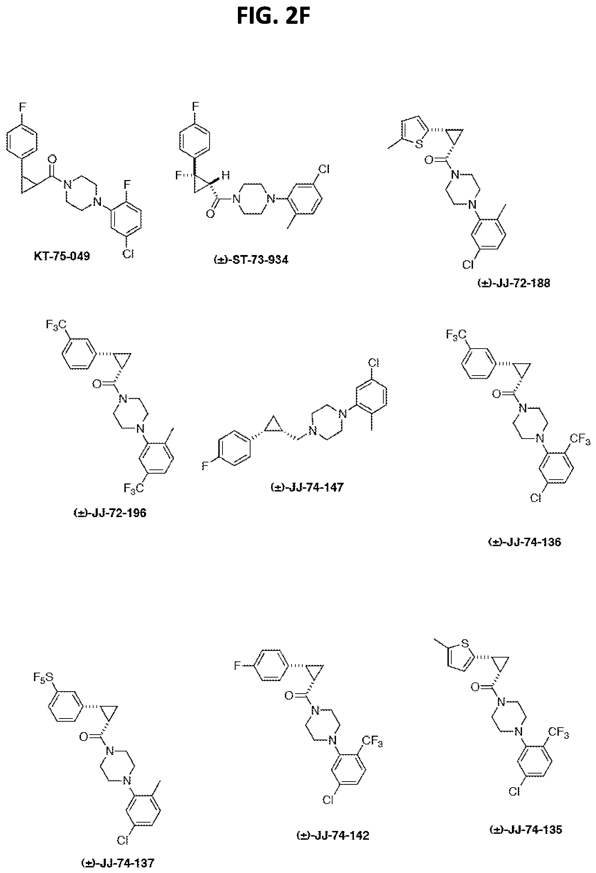

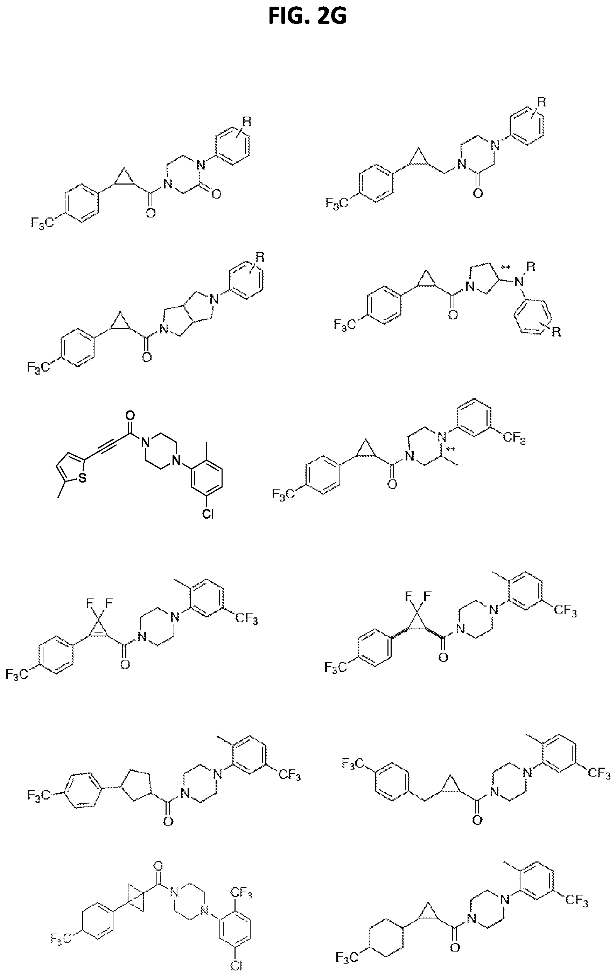

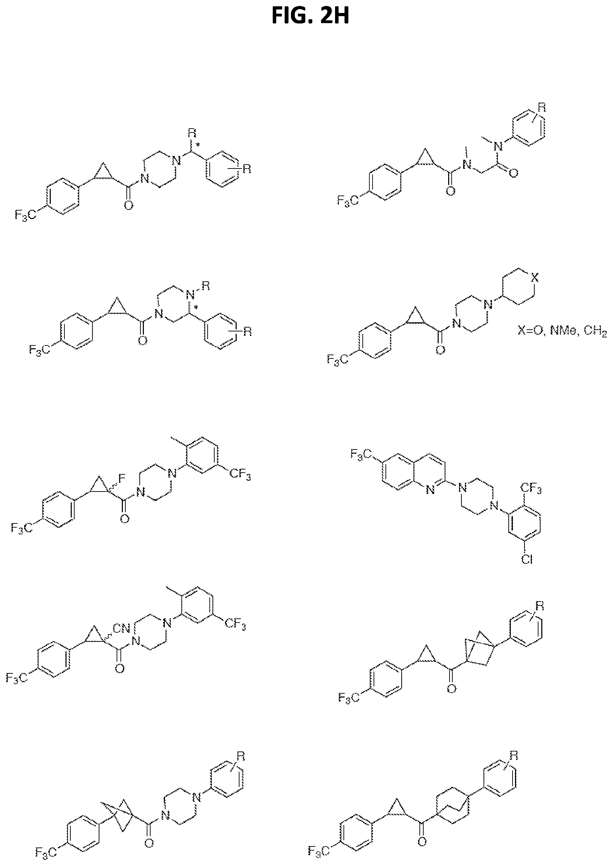

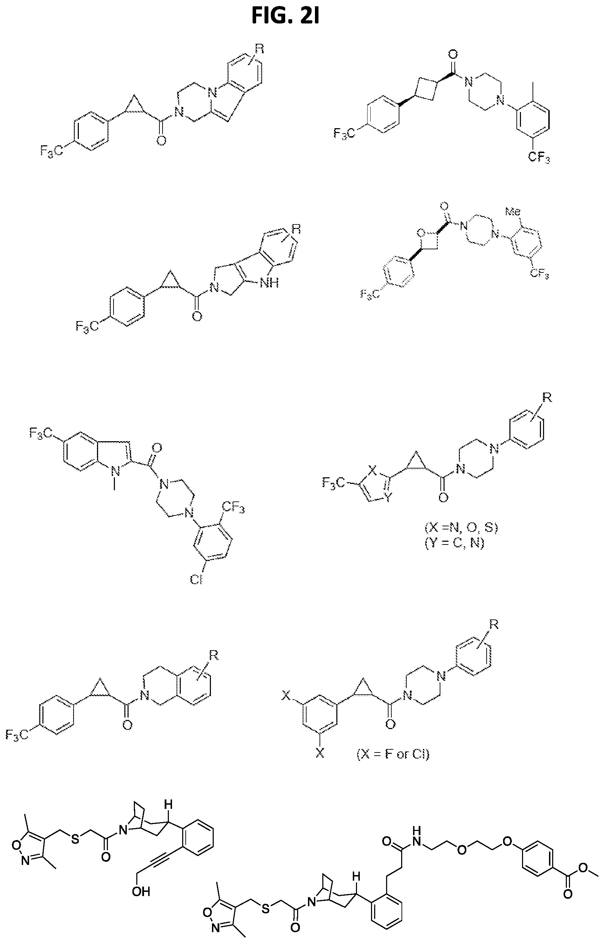

Illustrative compounds are shown in Figs. 1A-1D and 2A-2I. With respect to Fig. 2A-2I, each R independently is C₁-C₃ perfluoroalkyl, halo, pentafluorosulfanyl, -C(O)Oalkyl, or C(O)N(H)alkyl.

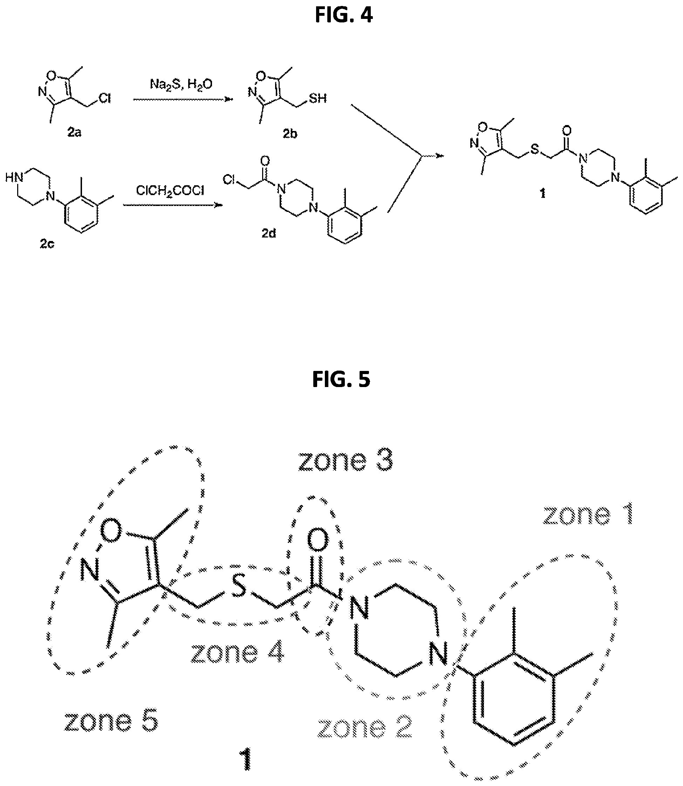

Fig. 4 shows a synthesis of a parent structure that is amenable to the modifications lined out in a zone model. Isoxazole **2a** can be obtained from the chloromethylation of 3,5-dimethylisoxazole, or via the corresponding alcohol, and can be converted to thiol **2b.** In situ alkylation of **2b** with chloride **2d** under the basic conditions of thiolate formation leads to **1.** There are many methods known for pyridazine synthesis, and the preparation of **2c** can follow one of these methods, for example starting with the aniline. Acylation of **2c** with chloroacetyl chloride provides **2d.** FIG. 5 shows zones of modification for compound **1.** The building blocks for zones 1 and 4 have been selected to cover a large range of chemical diversity; in addition, they are commercially available and are therefore readily funneled into the segment-based synthesis plan. Zone 2 contains a few diamines that preserve the distance between zone 1 and zone 3, i.e. where the nitrogens are appropriately spaced, but this zone can also be contracted to a simple nitrogen linker in order to probe the need to maintain the overall distance and orientation between zone 1 and zone 4. Zone 3 contains another spacer functionality, but the amide carbonyl group might also be involved in specific interactions with the binding site on the protein. Therefore, the distance between the carboxyl function and the halide electrophile can be varied, and the carbonyl group can also be replaced by a sulfonyl function.

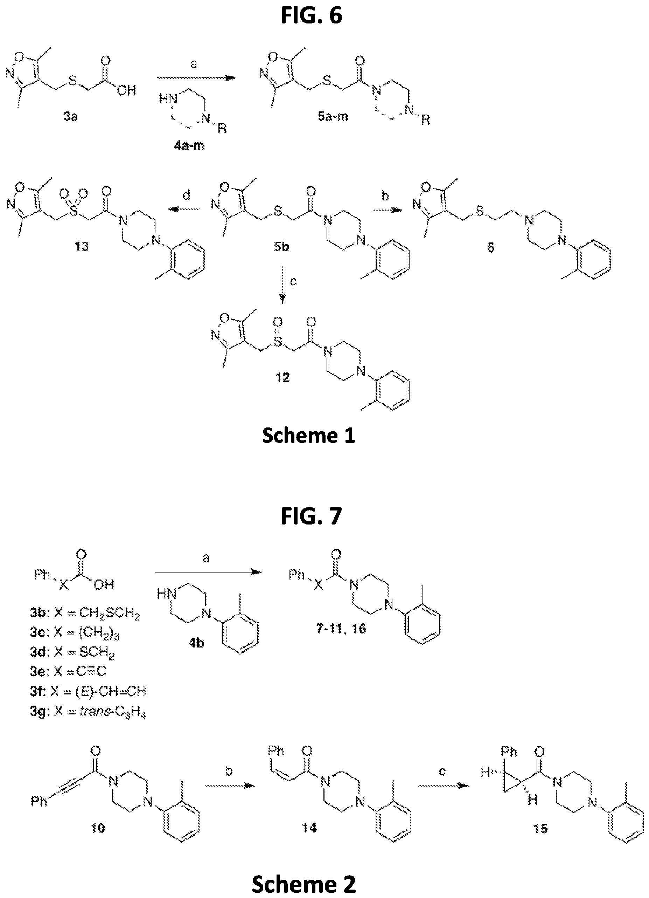

As described below, compounds **5a-h** were synthesized directly from commercially available carboxylic acids **3a** and *N*-arylated piperazines **4a-h** under amide coupling conditions with T3P (Scheme 1 (Fig. 6) and Table 1) (Basavaprabhhu et al., Synthesis 2013, 45, 1569-1601). The diamine linker in zone 2 was examined in more detail through the synthesis of analogs **5i-5m.** For these target molecules, the requisite diamines **4i-m** were prepared by a Buchwald-Hartwig cross-coupling of mono Boc-protected diamines with bromoarenes (Cabello-Sanchez et al., J. Org. Chem. 2007, 72, 2030-2039; Larsen et al., Tetrahedron 2008, 64, 2938-2950). Reduction of amide **5b** with lithium aluminum hydride led to diamine **6.** For an initial set of zone 4 analogs, thioether **5b** was also oxidized to sulfoxide **12** and sulfone **13** in good yields with sodium periodate and *m*-chloroperbenzoate, respectively (Scheme 1, Fig. 6).

Additional zone 4 and zone 5 analogs with a phenyl group in place of the isoxazole ring were obtained from carboxylic acids **3b-3g** (Scheme 2 (Fig. 7) and Table 1). Coupling to piperazine **4b** provided amides **7-11** and **16** in high yields. Alkynyl amide **10** was further hydrogenated to *cis*-alkene **14** using a Lindlar catalyst. The cis-cyclopropane **15** was prepared by a Simmons-Smith cyclopropanation of *cis*-alkene **14** (Concellón et al., Org. Lett. 2007, 9, 2981-2984), whereas the *trans-*cyclopropane **16** was obtained by coupling of commercially available *trans*-2-phenylcyclopropanecarboxylic acid **3g** with piperazine **4b.**

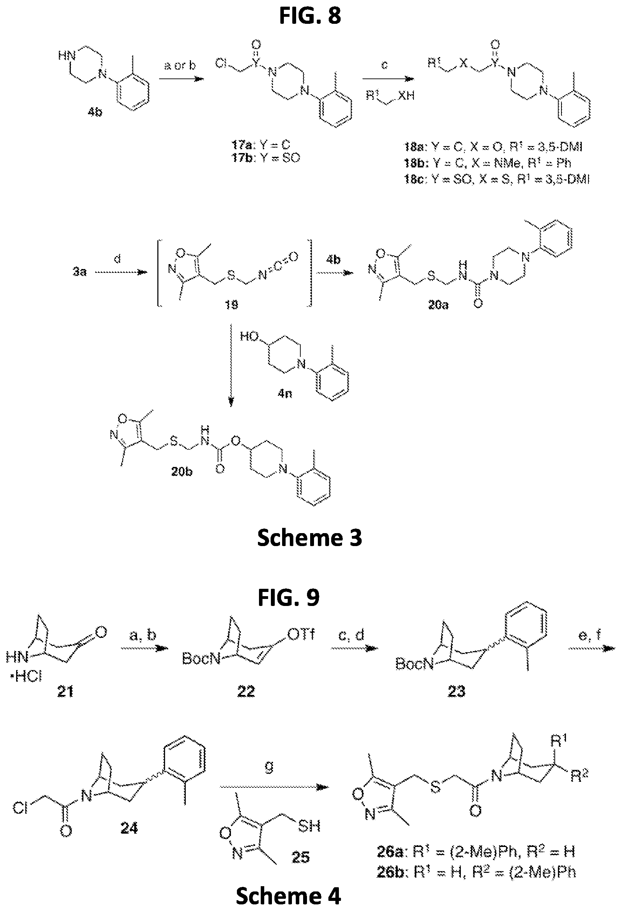

Further modifications in zones 3-4 were accomplished by acylation of piperazine **4b** with either 2-chloroacetyl chloride or chloromethanesulfonyl chloride to form the corresponding amide **17a** or sulfonamide **17b** in good yields (Scheme 3 (FIG. 8) and Table 1). S_{N}2-reaction of **17a** and **17b** led to ether **18a,** amine **18b,** and thioether **18c.** Starting with carboxylic acid **3a,** urea **20a** and carbamate **20b** were obtained in moderate yields via a Curtius rearrangement and addition of the intermediate isocyanate **19** to amine **4b** and alcohol **4n,** respectively (Scheme 3, Fig. 8) (WO 2005/085275).

A bridged bicyclic ring was introduced to add a strong conformational constraint in zone 2 (Scheme 4 (Fig. 9) and Table 1). Boc-protection of nortropinone hydrochloride **21** followed by enolization with NaHMDS and trapping of the enolate with N-phenyltriflimide provided vinyl triflate **22** in good yield. A Suzuki coupling was used to install the o-tolyl group, and the styrene double bond was reduced with Pd/C to afford **23** as a mixture of diastereomers. Without separation, this mixture was deprotected and acylated with α-chloroacetyl chloride. Finally, the chloride was displaced using thiol **25** and sodium hydride to afford the thioether. Diastereomers **26a** and **26b** were separated by chromatography on SiO₂ to afford both analogs in modest yields.

**Table 1. Structures of amine building blocks 4 and analogs 5, 7-11, and 16.**

| **Analog** | **Amine 4** | **R** | **X** |
|---|---|---|---|
| **5a** | 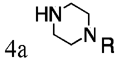 | Ph | - |
| **5b** | 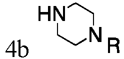 | (2-Me)Ph | - |
| **5c** | 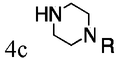 | (3-Me)Ph | - |
| **5d** | 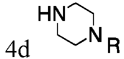 | (4-Me)Ph | - |
| **5e** | 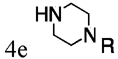 | (2-NC)Ph | - |
| **5f** | 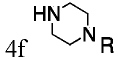 | (2-F)Ph | - |
| **5g** | 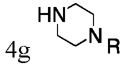 | 1-Naphthyl | - |
| **5h** | 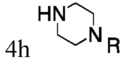 | (2-MeO)Ph | - |
| **5i** | 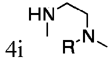 | (2-Me)Ph | - |
| **5j** | 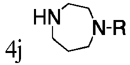 | (2-Me)Ph | - |
| **5k** | 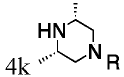 | (2-Me)Ph | - |
| **5l** | 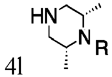 | Ph | - |
| **5m** | 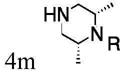 | (3-Me)Ph | - |
| **7** | 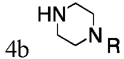 | (2-Me)Ph | CH₂SCH₂ |
| **8** | 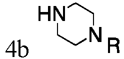 | (2-Me)Ph | (CH₂)₃ |
| **9** | 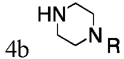 | (2-Me)Ph | SCH₂ |
| **10** | 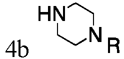 | (2-Me)Ph | C≡C |
| **11** | 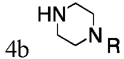 | (2-Me)Ph | (*E*)-HC=CH |
| **16** | 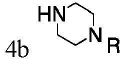 | (2-Me)Ph | (*E*)-*c-*C₃H₄ |

### Pharmaceutical Compositions and Method of Use

The agents disclosed herein may be for use in a method of treating prostate cancer in a subject, particularly castration-resistant prostate cancer. In certain embodiments a subject is identified as having castration-resistant prostate cancer that may be responsive to the agents disclosed herein. For example, patients that are offered any form of androgen deprivation therapy or anti-androgen therapy, including treatment with abiraterone or MDV3100, for the management of prostate cancer would be candidates for treatment with the agents disclosed herein.

Administration of the agent may reduce the nuclear level of androgen receptor in castration-resistant prostate cancer (CRPC) cells relative to the untreated control CRPC cells. Reducing nuclear androgen receptor levels is expected to inhibit its activation. Reduction of androgen receptor activation can be determined via measuring androgen-responsive genes, such as prostate-specific antigen (PSA).

In certain embodiments, the agent for use in the method of treatment may be co-administered with another therapeutic agent such as, for example, an immunostimulant, an anti-cancer agent, an antibiotic, or a combination thereof. In particular, the agents targeting AR nuclear localization could be used in combination with standard androgen deprivation therapy (ADT) or with abiratrone in the treatment of CRPC. In one embodiment, the agent for use in the method of treatment is co-administered with MDV3100 (enzalutamide), which may produce synergistic results since MDV3100 targets the ligand binding domain whereas the agent targets other domain(s) of the androgen receptor.

The agents disclosed herein can be included in a pharmaceutical composition suitable for administration to a subject. The pharmaceutical compositions suitable for administration to a subject can include at least one further pharmaceutically acceptable additive such as carriers, thickeners, diluents, buffers, preservatives, surface active agents and the like in addition to the molecule of choice. Pharmaceutical compositions can also include one or more additional active ingredients such as antimicrobial agents, anti-inflammatory agents, anesthetics, and the like. The pharmaceutically acceptable carriers useful for these formulations are conventional. Remington's Pharmaceutical Sciences, by E. W. Martin, Mack Publishing Co., Easton, PA, 19th Edition (1995), describes compositions and formulations suitable for pharmaceutical delivery of the compounds herein disclosed.

The pharmaceutical compositions may be in a dosage unit form such as an injectable fluid, an oral delivery fluid (e.g., a solution or suspension), a nasal delivery fluid (e.g., for delivery as an aerosol or vapor), a semisolid form (e.g., a topical cream), or a solid form such as powder, pill, tablet, or capsule forms.

In general, the nature of the carrier will depend on the particular mode of administration being employed. For instance, parenteral formulations usually contain injectable fluids that include pharmaceutically and physiologically acceptable fluids such as water, physiological saline, balanced salt solutions, aqueous dextrose, glycerol or the like as a vehicle. For solid compositions (for example, powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

The agents disclosed herein for use according to the claims can be administered to subjects by a variety of mucosal administration modes, including by oral, rectal, intranasal, intrapulmonary, or transdermal delivery, or by topical delivery to other surfaces. Optionally, the agents can be administered by non-mucosal routes, including by intramuscular, subcutaneous, intravenous, intra-arterial, intra-articular, intraperitoneal, intrathecal, intracerebroventricular, or parenteral routes. In other alternative embodiments, the agents can be administered *ex vivo* by direct exposure to cells, tissues or organs originating from a subject.

To formulate the pharmaceutical compositions, the agents can be combined with various pharmaceutically acceptable additives, as well as a base or vehicle for dispersion of the compound. Desired additives include, but are not limited to, pH control agents, such as arginine, sodium hydroxide, glycine, hydrochloric acid, citric acid, and the like. In addition, local anesthetics (for example, benzyl alcohol), isotonizing agents (for example, sodium chloride, mannitol, sorbitol), adsorption inhibitors (for example, Tween 80 or Miglyol 812), solubility enhancing agents (for example, cyclodextrins and derivatives thereof), stabilizers (for example, serum albumin), and reducing agents (for example, glutathione) can be included. Adjuvants, such as aluminum hydroxide (for example, Amphogel, Wyeth Laboratories, Madison, NJ), Freund's adjuvant, MPL^{™} (3-O-deacylated monophosphoryl lipid A; Corixa, Hamilton, IN) and IL-12 (Genetics Institute, Cambridge, MA), among many other suitable adjuvants well known in the art, can be included in the compositions. When the composition is a liquid, the tonicity of the formulation, as measured with reference to the tonicity of 0.9% (w/v) physiological saline solution taken as unity, is typically adjusted to a value at which no substantial, irreversible tissue damage will be induced at the site of administration. Generally, the tonicity of the solution is adjusted to a value of about 0.3 to about 3.0, such as about 0.5 to about 2.0, or about 0.8 to about 1.7.

The agents can be dispersed in a base or vehicle, which can include a hydrophilic compound having a capacity to disperse the compound, and any desired additives. The base can be selected from a wide range of suitable compounds, including but not limited to, copolymers of polycarboxylic acids or salts thereof, carboxylic anhydrides (for example, maleic anhydride) with other monomers (for example, methyl (meth)acrylate, acrylic acid and the like), hydrophilic vinyl polymers, such as polyvinyl acetate, polyvinyl alcohol, polyvinylpyrrolidone, cellulose derivatives, such as hydroxymethylcellulose, hydroxypropylcellulose and the like, and natural polymers, such as chitosan, collagen, sodium alginate, gelatin, hyaluronic acid, and nontoxic metal salts thereof. Often, a biodegradable polymer is selected as a base or vehicle, for example, polylactic acid, poly(lactic acid-glycolic acid) copolymer, polyhydroxybutyric acid, poly(hydroxybutyric acid-glycolic acid) copolymer and mixtures thereof. Alternatively or additionally, synthetic fatty acid esters such as polyglycerin fatty acid esters, sucrose fatty acid esters and the like can be employed as vehicles. Hydrophilic polymers and other vehicles can be used alone or in combination, and enhanced structural integrity can be imparted to the vehicle by partial crystallization, ionic bonding, crosslinking and the like. The vehicle can be provided in a variety of forms, including fluid or viscous solutions, gels, pastes, powders, microspheres and films for direct application to a mucosal surface.

The agents can be combined with the base or vehicle according to a variety of methods, and release of the agents can be by diffusion, disintegration of the vehicle, or associated formation of water channels. In some circumstances, the agent is dispersed in microcapsules (microspheres) or nanocapsules (nanospheres) prepared from a suitable polymer, for example, isobutyl 2-cyanoacrylate (see, for example, Michael et al., J. Pharmacy Pharmacol. 43:1-5, 1991), and dispersed in a biocompatible dispersing medium, which yields sustained delivery and biological activity over a protracted time.

The compositions of the disclosure can alternatively contain as pharmaceutically acceptable vehicles substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, and triethanolamine oleate. For solid compositions, conventional nontoxic pharmaceutically acceptable vehicles can be used which include, for example, pharmaceutical grades of mannitol, lactose, starch, magnesium stearate, sodium saccharin, talcum, cellulose, glucose, sucrose, magnesium carbonate, and the like.

Pharmaceutical compositions suitable for administering the agents can also be formulated as a solution, microemulsion, or other ordered structure suitable for high concentration of active ingredients. The vehicle can be a solvent or dispersion medium containing, for example, water, ethanol, polyol (for example, glycerol, propylene glycol, liquid polyethylene glycol, and the like), and suitable mixtures thereof. Proper fluidity for solutions can be maintained, for example, by the use of a coating such as lecithin, by the maintenance of a desired particle size in the case of dispersible formulations, and by the use of surfactants. In many cases, it will be desirable to include isotonic agents, for example, sugars, polyalcohols, such as mannitol and sorbitol, or sodium chloride in the composition. Prolonged absorption of the compound can be brought about by including in the composition an agent which delays absorption, for example, monostearate salts and gelatin.

In certain embodiments, the agents for use according to the claims can be administered in a time release formulation, for example in a composition which includes a slow release polymer. These compositions can be prepared with vehicles that will protect against rapid release, for example a controlled release vehicle such as a polymer, microencapsulated delivery system or bioadhesive gel. Prolonged delivery in various compositions of the disclosure can be brought about by including in the composition agents that delay absorption, for example, aluminum monostearate hydrogels and gelatin. When controlled release formulations are desired, controlled release binders suitable for use in accordance with the disclosure include any biocompatible controlled release material which is inert to the active agent and which is capable of incorporating the compound and/or other biologically active agent. Numerous such materials are known in the art. Useful controlled-release binders are materials that are metabolized slowly under physiological conditions following their delivery (for example, at a mucosal surface, or in the presence of bodily fluids). Appropriate binders include, but are not limited to, biocompatible polymers and copolymers well known in the art for use in sustained release formulations. Such biocompatible compounds are non-toxic and inert to surrounding tissues, and do not trigger significant adverse side effects, such as nasal irritation, immune response, inflammation, or the like. They are metabolized into metabolic products that are also biocompatible and easily eliminated from the body.

Exemplary polymeric materials for use in the present disclosure include, but are not limited to, polymeric matrices derived from copolymeric and homopolymeric polyesters having hydrolyzable ester linkages. A number of these are known in the art to be biodegradable and to lead to degradation products having no or low toxicity. Exemplary polymers include polyglycolic acids and polylactic acids, poly(DL-lactic acid-co-glycolic acid), poly(D-lactic acid-co-glycolic acid), and poly(L-lactic acid-co-glycolic acid). Other useful biodegradable or bioerodable polymers include, but are not limited to, such polymers as poly(epsilon-caprolactone), poly(epsilon-caprolactone-CO-lactic acid), poly(epsilon.-caprolactone-CO-glycolic acid), poly(beta-hydroxy butyric acid), poly(alkyl-2-cyanoacrilate), hydrogels, such as poly(hydroxyethyl methacrylate), polyamides, poly(amino acids) (for example, L-leucine, glutamic acid, L-aspartic acid and the like), poly(ester urea), poly(2-hydroxyethyl DL-aspartamide), polyacetal polymers, polyorthoesters, polycarbonate, polymaleamides, polysaccharides, and copolymers thereof. Many methods for preparing such formulations are well known to those skilled in the art (see, for example, Sustained and Controlled Release Drug Delivery Systems, J. R. Robinson, ed., Marcel Dekker, Inc., New York, 1978). Other useful formulations include controlled-release microcapsules (U.S. Patent Nos. 4,652,441 and 4,917,893), lactic acid-glycolic acid copolymers useful in making microcapsules and other formulations (U.S. Patent Nos. 4,677,191 and 4,728,721) and sustained-release compositions for water-soluble peptides (U.S. Patent No. 4,675,189).

The pharmaceutical compositions of the disclosure typically are sterile and stable under conditions of manufacture, storage and use. Sterile solutions can be prepared by incorporating the compound in the required amount in an appropriate solvent with one or a combination of ingredients enumerated herein, as required, followed by filtered sterilization. Generally, dispersions are prepared by incorporating the compound and/or other biologically active agent into a sterile vehicle that contains a basic dispersion medium and the required other ingredients from those enumerated herein. In the case of sterile powders, methods of preparation include vacuum drying and freeze-drying which yields a powder of the compound plus any additional desired ingredient from a previously sterile-filtered solution thereof. The prevention of the action of microorganisms can be accomplished by various antibacterial and antifungal agents, for example, parabens, chlorobutanol, phenol, sorbic acid, thimerosal, and the like.

The agent for use according to the claims can be delivered to a subject in a manner consistent with conventional methodologies associated with management of the disorder for which treatment or prevention is sought. In accordance with the disclosure herein, a prophylactically or therapeutically effective amount of the agent for use is administered to a subject in need of such treatment for a time and under conditions sufficient to prevent, inhibit, and/or ameliorate a selected disease or condition or one or more symptom(s) thereof as defined in the claims.

The administration of the agent can be for either prophylactic or therapeutic purpose. When provided prophylactically, the agent for use is provided in advance of any symptom. The prophylactic administration of the agents serves to prevent or ameliorate any subsequent disease process. When provided therapeutically, the compound for use is provided at (or shortly after) the onset of a symptom of disease or infection.

For prophylactic and therapeutic purposes, the agent for use can be administered to the subject by the oral route or in a single bolus delivery, via continuous delivery (for example, continuous transdermal, mucosal or intravenous delivery) over an extended time period, or in a repeated administration protocol (for example, by an hourly, daily or weekly, repeated administration protocol). The therapeutically effective dosage of the agent can be provided as repeated doses within a prolonged prophylaxis or treatment regimen that will yield clinically significant results to alleviate one or more symptoms or detectable conditions associated with a targeted disease or condition as set forth herein. Determination of effective dosages in this context is typically based on animal model studies followed up by human clinical trials and is guided by administration protocols that significantly reduce the occurrence or severity of targeted disease symptoms or conditions in the subject. Suitable models in this regard include, for example, murine, rat, avian, porcine, feline, non-human primate, and other accepted animal model subjects known in the art. Alternatively, effective dosages can be determined using *in vitro* models. Using such models, only ordinary calculations and adjustments are required to determine an appropriate concentration and dose to administer a therapeutically effective amount of the compound (for example, amounts that are effective to elicit a desired immune response or alleviate one or more symptoms of a targeted disease). **In** alternative embodiments, an effective amount or effective dose of the agents may simply inhibit or enhance one or more selected biological activities correlated with a disease or condition, as set forth herein, for either therapeutic or diagnostic purposes.

The actual dosage of the agents will vary according to factors such as the disease indication and particular status of the subject (for example, the subject's age, size, fitness, extent of symptoms, susceptibility factors, and the like), time and route of administration, other drugs or treatments being administered concurrently, as well as the specific pharmacology of the agent for eliciting the desired activity or biological response in the subject. Dosage regimens can be adjusted to provide an optimum prophylactic or therapeutic response. A therapeutically effective amount is also one in which any toxic or detrimental side effects of the agent is outweighed in clinical terms by therapeutically beneficial effects. A non-limiting range for a therapeutically effective amount of an agent within the methods and formulations of the disclosure is about 0.01 mg/kg body weight to about 20 mg/kg body weight, such as about 0.05 mg/kg to about 5 mg/kg body weight, or about 0.2 mg/kg to about 2 mg/kg body weight. Dosage can be varied by the attending clinician to maintain a desired concentration at a target site (for example, the lungs or systemic circulation). Higher or lower concentrations can be selected based on the mode of delivery, for example, trans-epidermal, rectal, oral, pulmonary, or intranasal delivery versus intravenous or subcutaneous delivery. Dosage can also be adjusted based on the release rate of the administered formulation, for example, of an intrapulmonary spray versus powder, sustained release oral versus injected particulate or transdermal delivery formulations, and so forth.

### Examples

Only compounds defined by the claims fall within the scope of the invention. The synthesis and/or activity of any compounds described in the Examples that fall outside the scope of the compounds defined by the claims are Reference compounds.

### 1. Biological Materials and Methods

### Materials

Phosphate buffered saline (PBS) solution was purchased from Fisher Scientific (MA, USA). Trypsin-EDTA solution, dimethyl sulfoxide (DMSO), Roswell Park Memorial Institute (RPMI) 1640 medium, ethanol (200 proof), puromycin powder, and G418 powder were purchased from Sigma-Aldrich (MO, USA). Fetal bovine Serum (FBS), penicillin-streptomycin solution were purchased from Invitrogen (NY, USA). Dual-Luciferase^{®} Reporter Assay System was purchased from Promega (WI, USA). PSA6.1-luc plasmid was a gift from Dr. Marianne Sadar at the University of British Columbia (BC, CA) and pRL-TK Renilla luciferase reporter plasmid was purchased from Promega (WI, USA). The C4-2 castration-resistant prostate cancer cell line was kindly provided by Dr. Leland W.K. Chung (Cedars-Sinai Medical Center).

### 2. Chemistry

### General

Moisture and air-sensitive reactions were performed under N₂ or Ar atmosphere and glassware used for these reactions was flamed dried and cooled under N₂ or Ar prior to use. THF and Et₂O were distilled from sodium/benzophenone ketyl. DMF and CH₂Cl₂ were distilled from CaH₂. 1,4-Dioxane was purchased from Acros (Sure/Seal bottle) and used as received. Et₃N was distilled from CaH₂ and stored over KOH. Toluene was purified by passage through an activated alumina filtration system. Melting points were determined using a Mel-Temp II instrument and are not corrected. Infrared spectra were determined using a Smiths Detection IdentifyIR FT-IR spectrometer. High-resolution mass spectra were obtained on a Micromass UK Limited, Q-TOF Ultima API, Thermo Scientific Exactive Orbitrap LC-MS. Automated column chromatography was done using an Isco Combiflash Rf. ¹H and ¹³C NMR spectra were obtained on Bruker Advance 300 MHz, 400 MHz, or 500 MHz instruments. Chemical shifts (δ) were reported in parts per million with the residual solvent peak used as an internal standard, δ ¹H/¹³C (Solvent): 7.26/77.00 (CDCl₃); 2.05/29.84 (acetone-d6); 2.50/39.52 (DMSO-d6), 3.31/49.00 (CD₃OD); and are tabulated as follows: chemical shift, multiplicity (s = singlet, brs = broad singlet, d = doublet, brd = broad doublet, t = triplet, app t = apparent triplet, q = quartet, m = multiplet), number of protons, and coupling constant(s). ¹³C NMR spectra were obtained at 75 MHz, 100 MHz, or 125 MHz using a proton-decoupled pulse sequence and are tabulated by observed peak. CDCl₃ was filtered through dried basic alumina prior to use. Thin-layer chromatography was performed using pre-coated silica gel 60 F₂₅₄ plates (EMD, 250 µm thickness) and visualization was accomplished with a 254 nm UV light and by staining with a PMA solution (5 g of phosphomolybdic acid in 100 mL of 95% EtOH), Vaughn's reagent (4.8 g of (NH₄)₆Mo₇O₂₄ •4H₂O and 0.2 g of Ce(SO₄)₂ in 100 mL of a 3.5 N H₂SO₄ solution) or a KMnO₄ solution (1.5 g of KMnO₄ and 1.5 g of K₂CO₃ in 100 mL of a 0.1% NaOH solution). Chromatography on SiO₂ (Silicycle, Silia-P Flash Silica Gel or SiliaFlash^{®} P60, 40-63 µm) was used to purify crude reaction mixtures. Final products were >95% purity as analyzed by RP (reverse phase) HPLC (Alltech Prevail C-18, 100 × 4.6 mm, 1 mL/min, CH₃CN, H₂O and 0.1% TFA) with UV (210, 220 and 254 nm), ELS (nebulizer 45 °C, evaporator 45 °C, N₂ flow 1.25 SLM), and MS detection using a Thermo Scientific Exactive Orbitrap LC-MS (ESI positive). All other materials were obtained from commercial sources and used as received.

### Example 1

### Synthesis and Characterization

Synthesis of several of the compounds is described in detail below:

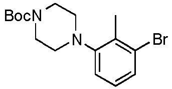

***tert*-Butyl** 4-(3-bromo-2-methylphenyl)piperazine-1-carboxylate (BRE454-64). A microwave vial under Ar was charged with *tert*-butyl 1-piperazinecarboxylate (154 mg, 0.825 mmol), NaO-t-Bu (0.0952 g, 0.990 mmol), (rac)-BINAP (0.0393 g, 0.0619 mmol, 7.5 mol%), Pd₂(dba)₃ (0.0192 g, 0.0206 mmol), and degassed toluene (2.1 mL). 2-Bromo-6-iodotoluene (121 µL, 0.825 mmol) was added, and the mixture was heated in sealed vial at 80 °C for 19 h, cooled to room temperature, diluted with CH₂Cl₂, filtered through Celite, and concentrated *in vacuo.* The mixture was purified by chromatography on SiO₂ (1:9, EtOAc/hexanes) to give the product (0.095 g, 0.27 mmol, 32%) as a yellow oil: ¹H NMR (500 MHz, CDCl₃) δ 7.30 (d, *J* = 8.0 Hz, 1 H), 7.02 (t, *J =* 8.0 Hz, 1 H), 6.95 (d, *J =* 7.5 Hz, 1 H), 3.57 (m, 4 H), 2.83 (t, *J =* 4.5 Hz, 4 H), 2.40 (s, 3 H), 1.49 (s, 9 H).

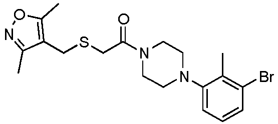

**1-(4-(3-Bromo-2-methylphenyl)piperazin-1-yl)-2-(((3,5-dimethylisoxazol-4-yl)methyl)thio) ethan-1-one (BRE454-75).** A solution of **BRE454-64** (0.0770 g, 0.22 mmol) in THF (0.3 mL) at 0 °C was treated with 4 M HCl in dioxane (1.3 mL) and stirred at 0 °C for 2 h. The yellow solid was collected by filtration, washed with Et₂O, dried under high vacuum and carried on to the next step without further purification.

To a solution of ([(3,5-dimethylisoxazol-4-yl)methyl]thio)acetic acid (0.0350 g, 0.174 mmol) in CH₂Cl₂ (1.7 mL) was added 4-(3-bromo-2-methylphenyl)piperazine hydrochloride and triethylamine (121 µL, 0.870 mmol). The mixture was cooled to 0 °C, treated with T3P (50% solution in EtOAc, 184 µL, 0.261 mmol), warmed to room temperature, stirred for 20 h, diluted with CH₂Cl₂, and washed with sat. NH₄Cl, sat. NaHCO₃, and brine. The organic layer was dried (Na₂SO₄), filtered, and concentrated *in vacuo.* The crude material was purified by chromatography on SiO₂ (3:2, EtOAc/hexanes, base washed with 0.1% Et₃N prior to use) to give the product (0.0762 g, 0.174 mmol, quant. 100% pure by ELSD) as a colorless oil: IR (ATR) 2921, 2820, 1637, 1587, 1562, 1460, 1428, 1282, 1237, 1195, 1136, 1038, 994, 913, 780, 731, 714 cm⁻¹; ¹H NMR (400 MHz, CDCl₃) δ 7.32 (dd, *J* = 0.8, 7.6 Hz, 1 H), 7.03 (t, *J* = 8.0 Hz, 1 H), 6.94 (dd, *J* = 0.8, 8.0 Hz, 1 H), 3.77 (br s, 2 H), 3.63 (s, 2 H), 3.63-3.57 (m, 2 H), 3.23 (s, 2 H), 2.90 (t, *J* = 4.4 Hz, 2 H), 2.88-2.83 (m, 2 H), 2.43 (s, 3 H), 2.40 (s, 3 H), 2.31 (s, 3 H); ¹³C-NMR (125 MHz, CDCl₃) δ 167.6, 166.8, 159.7, 152.2, 132.9, 128.1, 127.4, 126.6, 118.3, 109.7, 52.1, 51.8, 46.8, 42.2, 32.1, 23.8, 18.2, 11.1, 10.2; HRMS (ESI) *m*/*z* calcd for C₁₉H₂₅N₃O₂BrS ([M+H]⁺) 438.0845, found 438.0831.

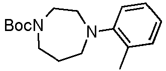

***tert-*Butyl 4-(*o*-tolyl)-1,4-diazepane-1-carboxylate (BRE454-66).** A microwave vial under Ar was charged with 1-Boc-homopiperazine (223 mg, 1.10 mmol), NaO-t-Bu (0.116 g, 1.20 mmol), (rac)-BINAP (0.0478 g, 0.0752 mmol, 7.5% mol), Pd₂(dba)₃ (0.0233 g, 0.0251 mmol, 2.5% mol in Pd), and degassed toluene (2.8 mL). 2-Bromotoluene (0.175 g, 1.00 mmol) was added, and the mixture was heated in a sealed vial at 80 °C for 19 h, cooled to room temperature,, diluted with CH₂Cl₂, filtered over Celite, and concentrated. The crude material was purified by chromatography on SiO₂ (1:9, EtOAc/hexanes) to give the product (0.139 g, 0.479 mmol, 48%) as a yellow oil: IR (ATR) 2973, 2828, 1689, 1598, 1491, 1457, 1411, 1364, 1233, 1215, 1156, 1122, 878, 761, 725 cm⁻¹; ¹H NMR (500 MHz, CDCl₃, rt, rotamers) δ 7.16 (d, *J =* 6.0 Hz, 1 H), 7.12 (d, *J =* 6.0 Hz, 1 H), 7.04 (d, *J =* 7.5 Hz, 1 H), 6.95 (t, *J =* 7.0 Hz, 1 H), 3.62-3.52 (m, 4 H), 3.12-3.04 (m, 4 H), 2.31 (s, 3 H), 2.00-1.88 (m, 2 H), 1.49 (s, 9 H); ¹³C-NMR (100 MHz, CDCl₃, rt, rotamers) δ 155.6, 155.5, 153.9, 153.8, 132.9, 130.9, 126.5, 123.1, 120.8 (2 C), 79.3, 56.2, 56.0, 55.5, 55.2, 48.4, 48.0, 46.2, 45.4, 29.0, 28.9, 28.5, 18.5; HRMS (ESI) *m*/*z* calcd for C₁₇H₂₇N₂O₂ ([M+H]⁺) 291.2067, found 291.2062.

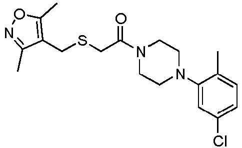

**1-(4-(5-Chloro-2-methylphenyl)piperazin-1-yl)-2-(((3,5-dimethylisoxazol-4-yl)methyl)thio)** ethan-1-one (BRE454-58). To a solution of ([(3,5-dimethylisoxazol-4-yl)methyl]thio)acetic acid (0.0450 g, 0.224 mmol) in CH₂Cl₂ (2.2 mL) was added 1-(5-chloro-2-methylphenyl)piperazine (0.0565 g, 0.268 mmol) and triethylamine (93 µL, 0.671 mmol). The mixture was cooled to 0 °C, treated with T3P (50% solution in EtOAc, 237 µL, 0.335 mmol), warmed to room temperature, stirred for 20 h, diluted with CH₂Cl₂, and washed with sat. NH₄Cl, sat. NaHCO₃, and brine. The organic layer was dried (Na₂SO₄), filtered, and concentrated *in vacuo.* The crude material was purified by chromatography on SiO₂ (1:1, EtOAc/hexanes, base washed with 0.1% Et₃N prior to use) to give the product (0.0881 g, 0.224 mmol, quant, 99.9% pure by ELSD) as a clear colorless oil: IR (ATR) 2921, 2818, 1635, 1592, 1489, 1438, 1270, 1224, 1195, 1148, 1039, 924, 910, 818, 728 cm⁻¹; ¹H NMR (400 MHz, CDCl₃) δ 7.11 (d, *J =* 8.0 Hz, 1 H), 6.99 (dd, *J* = 2.0, 8.0 Hz, 1 H), 6.94 (d, *J =* 2.4 Hz, 1 H), 3.76 (t, *J* = 4.8 Hz, 2 H), 3.63 (s, 2 H), 3.59 (t, *J =* 4.8 Hz, 2 H), 3.23 (s, 2 H), 2.91 (t, *J =* 4.8 Hz, 2 H), 2.86 (t, *J =* 4.8 Hz, 2 H), 2.43 (s, 3 H), 2.30 (s, 3 H), 2.27 (s, 3 H); ¹³C NMR (125 MHz, CDCl₃) δ 167.6, 166.8, 159.7, 151.7, 132.1, 131.8, 130.9, 123.7, 119.7, 109.7, 51.6, 51.5, 46.8, 42.2, 32.0, 23.7, 17.4, 11.1, 10.2; HRMS (ESI) *m*/*z* calcd for C₁₉H₂₅N₃O₂ClS ([M+H]⁺) 394.1351, found 394.1340.

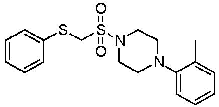

**1-(((Phenylthio)methyl)sulfonyl)-4-(o-tolyl)piperazine (BRE454-84).** A solution of 1-(2-methylphenyl)piperazine (0.500 g, 2.75 mmol) and triethylamine (0.39 mL, 2.75 mmol) in CH₂Cl₂ (9.8 mL) at 0 °C was treated with chloromethanesulfonyl chloride (0.460 g, 3.03 mmol), gradually warmed to room temperature, and stirred for 14 h. The reaction mixture was quenched with sat. NH₄Cl (3 mL) and extracted with EtOAc (3 × 20 mL). The combined organic portion was washed with water (2 × 10 mL) and brine (10 mL), dried (Na₂SO₄), filtered, and concentrated. The crude solid was filtered through a plug of SiO₂ (pretreated with 0.1% Et₃N in 30% EtOAc/hexanes) and washed thoroughly with 30% EtOAc/hexanes to give the product as an orange solid (0.676 g, 2.34 mmol, 85%): ¹H NMR (400 MHz, CDCl₃) δ 7.21-7.17 (m, 2 H), 7.05-7.00 (m, 2 H), 4.57 (s, 2 H), 3.63 (t, *J* = 4.8 Hz, 4 H), 2.98 (t, *J =* 5.2 Hz, 4 H), 2.31 (s, 3 H). A solution of this product (0.0400 g, 0.139 mmol), thiophenol (0.0610 g, 0.554 mmol), and Cs₂CO₃ (0.0903 g, 0.277 mmol) in DMF (0.28 mL) was stirred at 80 °C for 2 d. The reaction mixture was diluted with brine (10 mL) and extracted with EtOAc (20 mL). The organic layer was separated, washed with brine (2 × 10 mL), dried (Na₂SO₄), and concentrated *in vacuo.* The crude material was purified by chromatography on SiO₂ (1:4, EtOAc/hexanes) the product as a clear colorless oil (0.0257 g, 0.0709 mmol, 51%): IR (ATR) 3054, 2918, 2823, 1598, 1581, 1493, 1440, 1342, 1324, 1262, 1225, 1153, 1112, 1070, 954, 765, 744, 725, 691 cm⁻¹; ¹H NMR (500 MHz, CDCl₃) δ 7.59 (d, *J =* 7.5 Hz, 2 H), 7.39-7.30 (m, 3 H), 7.21-7.14 (m, 2 H), 7.02 (t, *J =* 7.5 Hz, 1 H), 6.98 (d, *J =* 8.0 Hz, 1 H), 4.33 (s, 2 H), 3.51 (t, *J =* 4.5 Hz, 4 H), 2.92 (t, *J =* 4.5 Hz, 4 H), 2.28 (s, 3 H); ¹³C NMR (100 MHz, CDCl₃) δ 150.7, 133.4, 132.7, 131.2, 131.1, 129.4, 128.1, 126.7, 123.9, 119.4, 54.2, 51.8, 46.8, 17.7; HRMS (+ESI) *m*/*z* calcd for C₁₈H₂₃N₂O₂S₂ ([M+H]⁺) 363.1195, found 363.1190.

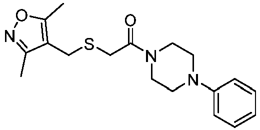

**2-(((3,5-Dimethylisoxazol-4-yl)methyl)thio)-1-(4-phenylpiperazin-1-yl)ethanone (5a).** To a solution of 2-(((3,5-dimethylisoxazol-4-yl)methyl)thio)acetic acid 3a (0.0200 g, 0.0994 mmol) in CH₂Cl₂ (1.25 mL) was added 1-phenylpiperazine **4a** (0.0190 g, 0.119 mmol) and Et₃N (41 µL, 0.298 mmol). The reaction mixture was cooled to 0 °C, treated with T3P (50 wt. % solution in EtOAc, 105 µL, 0.149 mmol), allowed to warm to room temperature, stirred for 2 d, diluted with CH₂Cl₂ and washed with satd. aqueous NH₄Cl, satd. aqueous NaHCO₃, brine, dried (Na₂SO₄), filtered, and concentrated *in vacuo.* The crude residue was purified by chromatography on SiO₂ (ISCO, 12 g column, liquid load in CH₂Cl₂, EtOAc/hexanes gradient (10-100%), product eluted at 60%) to give **5a** (0.0330 g, 0.0955 mmol, 96%, 100% pure by ELSD) as a colorless solid: Mp 74-75 °C; IR (ATR) 2856, 2802, 1627, 1599, 1496, 1440, 1416, 1229, 1141, 1034, 909, 765, 698 cm⁻¹; ¹H NMR (300 MHz, CDCl₃) δ 7.26-7.21 (m, 1 H), 6.89-6.83 (m, 3 H), 3.72 (app t, 2 H, *J =* 5.2 Hz), 3.56 (s, 2 H), 3.56-3.54 (m, 2 H), 3.18 (s, 2 H), 3.15-3.10 (m, 2 H), 2.34 (s, 3 H), 2.23 (s, 3 H); ¹³C NMR (75 MHz, CDCl₃) δ 166.5, 165.8, 158.6, 149.8, 128.2, 119.6, 115.6, 108.7, 48.5, 48.3, 45.3, 40.7, 31.0, 22.7, 10.0, 9.1; HRMS (ESI) *m*/*z* calcd for C₁₈H₂₄N₃O₂S ([M+H]⁺) 346.1584, found: 346.1571.

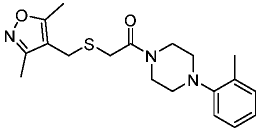

**2-(((3,5-Dimethylisoxazol-4-yl)methyl)thio)-1-(4-(*o*-tolyl)piperazin-1-yl)ethanone (5b).** To a solution of 2-(((3,5-dimethylisoxazol-4-yl)methyl)thio)acetic acid **(3a,** 0.0200 g, 0.0994 mmol) in CH₂Cl₂ (1.25 mL) was added 1-(o-tolyl)piperazine **4b** (0.0210 g, 0.119 mmol) and Et₃N (41 µL, 0.298 mmol). The reaction mixture was cooled to 0 °C, treated with T3P (50 wt.% solution in EtOAc, 105 µL, 0.149 mmol), allowed to warm to room temperature, stirred for 2 d, diluted with CH₂Cl₂ and washed with satd. aqueous NH₄Cl, satd. aqueous NaHCO₃, brine, dried (Na₂SO₄), filtered, and concentrated *in vacuo.* The crude residue was purified by chromatography on SiO₂ (ISCO, 12 g column, liquid load in CH₂Cl₂, EtOAc/hexanes gradient (10-100%), product eluted at 40%) to give 5b (0.0348 g, 0.0968 mmol, 97%, 100% pure by ELSD) as a colorless solid: Mp 89-91 °C; IR (ATR) 2959, 2828, 1631, 1492, 1430, 1261, 1226, 1138, 1036, 979, 959, 776, 726 cm⁻¹; ¹H NMR (300 MHz, CDCl₃) δ 7.18 (dd, 2 H, *J =* 9.0, 7.5 Hz), 7.01 (dd, 2 H, *J =* 14.1, 9.0 Hz), 3.76 (app t, 2 H, *J =* 4.9 Hz), 3.63 (s, 2 H), 3.59 (app t, 2 H, *J =* 4.9 Hz), 3.24 (s, 2 H), 2.93 (app t, 2 H, *J =* 4.9 Hz), 2.88 (app t, 2 H, *J* = 4.9 Hz), 2.43 (s, 3 H), 2.32 (s, 3 H), 2.30 (s, 3 H); ¹³C NMR (75 MHz, CDCl₃) δ 166.5, 165.8, 158.7, 149.6, 131.7, 130.2, 125.7, 122.8, 118.1, 108.7, 50.8, 50.6, 46.0, 41.3, 31.1, 22.7, 16.7, 10.0, 9.1; HRMS (ESI) *m*/*z* calcd for C₁₉H₂₆N₃O₂S ([M+H]⁺) 360.1740, found 360.1725.

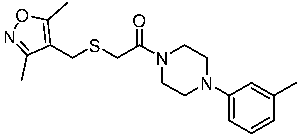

**2-(((3,5-Dimethylisoxazol-4-yl)methyl)thio)-1-(4-(*m*-tolyl)piperazin-1-yl)ethanone (5c).** A solution of 2-(((3,5-dimethylisoxazol-4-yl)methyl)thio)acetic acid **(3a,** 0.0200 g, 0.0994 mmol) in CH₂Cl₂ (1.25 mL) was added 1-(*m*-tolyl)piperazine **(4c,** 21 µL, 0.119 mmol), Et₃N (41 µL, 0.298 mmol). The reaction mixture was cooled to 0 °C, treated with T3P (50 wt.% solution in EtOAc, 105 µL, 0.149 mmol), allowed to warm to room temperature, stirred for 2 d, diluted with CH₂Cl₂ and washed with satd. aqueous NH₄Cl, satd. aqueous NaHCO₃, brine, dried (Na₂SO₄), filtered, and concentrated *in vacuo.* The crude residue was purified by chromatography on SiO₂ (ISCO, 12 g column, liquid load in CH₂Cl₂, EtOAc/hexanes gradient (10-100%), eluted at 60%) to give **5c** (0.0343 g, 0.954 mmol, 96%, 99.5% pure by ELSD) as a yellow oil: IR (ATR) 2918, 2819, 1635, 1600, 1493, 1424, 1244, 1192, 1145, 995, 957, 775, 729, 694 cm⁻¹; ¹H NMR (300 MHz, CDCl₃) δ 7.17 (app t, 1 H, *J =* 7.8 Hz), 6.75-6.72 (m, 3 H), 3.76 (app t, 2 H, *J =* 5.2 Hz), 3.61 (s, 2 H), 3.60-3.58 (m, 2 H), 3.23 (s, 2 H), 3.17 (ddd, 4 H, *J =* 5.5, 5.2, 5.0 Hz), 2.41 (s, 3 H), 2.32 (s, 3 H), 2.28 (s, 3 H); ¹³C NMR (75 MHz, CDCl₃) δ 166.5, 165.8, 158.6, 149.8, 138.0, 128.1, 120.5, 116.5, 112.8, 108.7, 48.6, 48.5, 45.3, 40.8, 31.0, 22.7, 20.7, 10.0, 9.1; HRMS (ESI) *m*/*z* calcd for C₁₉H₂₆N₃O₂S ([M+H]⁺) 360.1740, found: 360.1725.

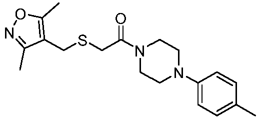

**2-(((3,5-Dimethylisoxazol-4-yl)methyl)thio)-1-(4-(*p*-tolyl)piperazin-1-yl)ethanone (5d).** A solution of 2-(((3,5-dimethylisoxazol-4-yl)methyl)thio)acetic acid **(3a,** 0.0200 g, 0.0994 mmol) in CH₂Cl₂ (1.25 mL) was added 1-(*p*-tolyl)piperazine **(4d,** 21 µL, 0.119 mmol), Et₃N (41 µL, 0.298 mmol). The reaction mixture was cooled to 0 °C, treated with T3P (50 wt.% solution in EtOAc, 105 µL, 0.149 mmol), allowed to warm to room temperature, stirred for 2 d, diluted with CH₂Cl₂, washed with satd. aqueous NH₄Cl, satd. aqueous NaHCO₃, and brine, dried (Na₂SO₄), filtered, and concentrated *in vacuo.* The crude residue was purified by chromatography on SiO₂ (ISCO, 12 g column, liquid load in CH₂Cl₂, EtOAc/hexanes gradient ((10-100%), eluted at 60%) to give **5d** (0.0266 g, 0.0740 mmol, 74%, 100% pure by ELSD) as a red solid: Mp 83-85 °C; IR (ATR) 2855, 2801, 1627, 1514, 1440, 1416, 1261, 1230, 1142, 1043, 960, 815, 724 cm⁻¹; ¹H NMR (300 MHz, CDCl₃) δ 7.10 (d, 2 H, *J =* 8.1 Hz), 6.85 (d, 2 H, *J =* 8.1 Hz), 3.77 (app t, 2 H, *J =* 4.7 Hz), 3.61-3.58 (m, 4 H), 3.23 (s, 2 H), 3.13 (ddd, 4 H, *J =* 5.6, 5.5, 4.7 Hz), 2.41 (s, 3 H), 2.28, (s, 6 H); ¹³C NMR (75 MHz, CDCl₃) δ 167.5, 166.8, 159.7, 148.7, 130.3, 129.8, 117.0, 109.7, 50.1, 49.9, 46.4, 41.8, 32.1, 23.7, 20.4, 11.0, 10.1; HRMS (ESI) *m*/*z* calcd for C₁₉H₂₆N₃O₂S ([M+H]⁺) 360.1740, found 360.1725.

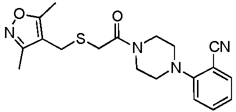

**2-(4-(2-(((3,5-Dimethylisoxazol-4-yl)methyl)thio)acetyl)piperazin-1-yl)benzonitrile (MK415-62; 5e).** To a solution of ([(3,5-dimethylisoxazol-4-yl)methyl]thio)acetic acid **(3a,** 0.0280 g, 0.132 mmol) in CH₂Cl₂ (1.3 mL) was added 2-(piperazin-1-yl)benzonitrile **(4e,** 0.0253 g, 0.132 mmol) and Et₃N (56 µL, 0.400 mmol). The reaction mixture was cooled to 0 °C, treated with T3P (50 wt.% solution in EtOAc, 140 µL, 0.200 mmol), allowed to warm to room temperature, stirred for 20 h, diluted with CH₂Cl₂, washed with satd. aqueous NH₄Cl, satd. aqueous NaHCO₃, and brine, dried (Na₂SO₄), filtered, and concentrated *in vacuo.* The crude residue was purified by chromatography on SiO₂ (95:5, CH₂Cl₂/MeOH) to give **5e** (0.0390 g, 0.105 mmol, 80%, 99.9% pure by ELSD) as a yellow solid: Mp 142-143 °C; IR (neat) 2919, 2216, 1637, 1593, 1420, 1232 cm⁻¹; ¹H NMR (400 MHz, CDCl₃) δ 7.61 (dd, 1 *H, J =* 7.6, 1.6 Hz), 7.51 (ddd, 1 *H, J =* 8.4, 7.6, 1.6 Hz), 7.09 (dt, 1 H, *J =* 7.6, 0.9 Hz), 7.02 (d, 1 H, *J =* 8.4 Hz), 3.82 (app t, 2 H, *J =* 4.8 Hz), 3.67 (app t, 2 H, *J =* 4.8 Hz), 3.62 (s, 2 H), 3.24 (s, 2 H), 3.24-3.21 (m, 2 H) 3.15 (app t, 2 H, *J =* 5.4 Hz), 2.41 (s, 3 H), 2.28 (s, 3 H); ¹³C NMR (100 MHz, CDCl₃) δ 167.6, 166.7, 159.6, 154.9, 134.3, 133.9, 122.7, 118.9, 118.0, 109.7, 106.7, 51.9, 51.1, 46.6, 41.8, 32.1, 23.7, 11.0, 10.1; HRMS (ESI) *m*/*z* calcd for C₁₉H₂₃N₄O₂S ([M+H]⁺) 371.1542, found 371.1536.

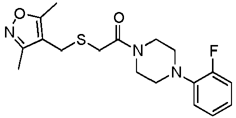

**2-(((3,5-Dimethylisoxazol-4-yl)methyl)thio)-1-(4-(2-fluorophenyl)piperazin-1-yl)ethan-1-one (BRE454-54; 5f).** To a solution of 2-(((3,5-dimethylisoxazol-4-yl)methyl)thio)acetic acid **(3a,** 0.0758 g, 0.377 mmol) in CH₂Cl₂ (3.8 mL) was added 1-(2-fluorophenyl)-piperazine **(4f,** 0.0814 g, 0.452 mmol) and Et₃N (262 µL, 1.88 mmol). The reaction mixture was cooled to 0 °C, treated with T3P (50 wt. % solution in EtOAc, 399 µL, 0.565 mmol), allowed to warm to room temperature, stirred for 20 h, diluted with CH₂Cl₂, and washed with satd. aqueous NH₄Cl solution, satd. aqueous NaHCO₃ solution, and brine, dried (Na₂SO₄), filtered, and concentrated *in vacuo.* The crude residue was purified by chromatography on SiO₂ (3:2, EtOAc/hexanes, base washed with 0.1% Et₃N prior to use) to give **5f** (0.134 g, 0.369 mmol, 98%, 100% pure by ELSD) as a light yellow oil: IR (ATR) 2918, 2827, 1636, 1613, 1500, 1439, 1237, 1195, 1147, 1031, 909, 811, 753, 725 cm⁻¹; ¹H NMR (400 MHz, CDCl₃) δ 7.10-6.90 (m, 4 H), 3.79 (app t, 2 H, *J =* 5.2 Hz), 3.63-3.59 (m, 4 H), 3.23 (s, 2 H), 3.10 (app t, 2 H, *J* = 4.8 Hz), 3.05 (app t, 2 H, *J =* 5.2 Hz), 2.28 (s, 3 H), 2.42 (s, 3 H); ¹³C NMR (125 MHz, CDCl₃) δ 167.5, 166.8, 159.7, 155.7 (d, *J_{C-F} =* 245.0 Hz), 139.4 (d, *J_{C-F} =* 8.8 Hz), 124.5 (d*, J_{C-F} = 3.8* Hz), 123.3 (d, *J_{C-F} =* 8.8 Hz), 119.2 (d, *J_{C-F} =* 2.5 Hz), 116.3 (d, *J_{C-F} =* 20.0 Hz), 109.7, 50.7 (d, *J_{C-F} =* 2.5 Hz), 50.3 (d, *J_{C-F} =* 2.5 Hz), 46.6, 41.9, 32.1, 23.7, 11.1, 10.2; HRMS (ESI) *m*/*z* calcd for C₁₈H₂₃N₃O₂FS ([M+H]⁺) 364.1490, found 364.1474.

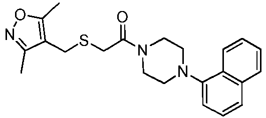

**2-(((3,5-Dimethylisoxazol-4-yl)methyl)thio)-1-(4-(naphthalen-1-yl)piperazin-1-yl)ethanone (5g).** A Schlenk flask was charged under N₂ with piperazine (0.0500 g, 0.580 mmol), NaO-*t*-Bu (0.100 g, 1.06 mmol), (*rac*)-BINAP (0.0051 g, 0.0079 mmol), Pd₂(dba)₃ (0.0050 g, 0.0053 mmol), and degassed toluene (5 mL). After addition of 1-bromonaphthalene (75 µL, 0.530 mmol), the reaction mixture was heated at 110 °C for 24 h, cooled to room temperature, diluted with CH₂Cl₂, filtered through Celite, and concentrated *in vacuo.* The resulting 1-(naphthalen-1-yl)piperazine **(4g)** was used without further purification for the next reaction.

To a solution of (((3,5-dimethylisoxazol-4-yl)methyl)thio)acetic acid **(3a,** 0.0580 g, 0.272 mmol) in CH₂Cl₂ (4 mL) was added 1-(naphthalen-1-yl)piperazine **4g** (0.0750 g, 0.353 mmol) and Et₃N (114 µL, 0.815 mmol). The reaction mixture was cooled to 0 °C, treated with T3P (50 wt.% solution in EtOAc, 288 µL, 0.408 mmol), allowed to warm to room temperature, stirred for 20 h, diluted with CH₂Cl₂, and washed with satd. aqueous NH₄Cl, satd. aqueous NaHCO₃, and brine, dried (Na₂SO₄), filtered, and concentrated *in vacuo.* The crude residue was purified by chromatography on SiO₂ (95:5 CH₂Cl₂/MeOH) to give **5g** (0.0700 g, 0.177 mmol, 65% 2 steps, 99.9% pure by ELSD) as a yellow oil: IR (neat) 2919, 1637, 1435, 1398, 1215, 1192 cm⁻¹; ¹H NMR (500 MHz, CDCl₃) δ 8.21 (d, 1 H, *J* = 7.5 Hz), 7.85 (d, 1 *H, J =* 7.5 Hz), 7.61 (d, 1 *H, J* = 8.0 Hz), 7.54-7.49 (m, 2 H), 7.42 (d, 1 H, *J =* 8.0 Hz), 7.08 (d, 1 H*, J =* 7.5 Hz), 3.73-3.66 (m, 4 H), 3.64 (s, 2 H), 3.28 (s, 2 H), 3.27-2.85 (m, 4 H), 2.45 (s, 3 H), 2.32 (s, 3 H); ¹³C NMR (125 MHz, CDCl₃) δ 167.6, 166.8, 159.7, 148.7, 134.7, 128.7, 128.5, 126.0, 125.7 (2 C), 124.2, 123.0, 115.0, 109.7, 52.9, 52.7, 47.0, 42.4, 32.1, 23.7, 11.1, 10.2; HRMS (ESI) *m*/*z* calcd for C₂₂H₂₆N₃O₂S ([M+H]⁺) 396.1746, found 396.1740.

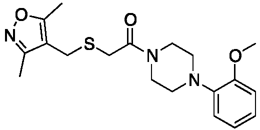

**2-(((3,5-Dimethylisoxazol-4-yl)methyl)thio)-1-(4-(2-methoxyphenyl)piperazin-1-yl)ethanone (5h)**. To a solution of 2-(((3,5-dimethylisoxazol-4-yl)methyl)thio)acetic acid (3a, 0.0200 g, 0.0994 mmol) in CH₂Cl₂ (1.25 mL) was added 1-(*o-*methoxyphenyl)piperazine **(4h,** 0.0230 g, 0.119 mmol) and Et₃N (41 µL, 0.298 mmol). The reaction mixture was cooled to 0 °C, treated with T3P (50 wt.% solution in EtOAc, 105 µL, 0.149 mmol), warmed to room temperature, stirred for 2 d, diluted with CH₂Cl₂ and washed with satd. aqueous NH₄Cl, satd. aqueous NaHCO₃, and brine, dried (Na₂SO₄), filtered, and concentrated *in vacuo.* The crude residue was purified by chromatography on SiO₂ (ISCO, 12 g column, liquid load in CH₂Cl₂, EtOAc/hexanes gradient (10-100%, eluted at 50-70%) to give **5h** (0.0195 g, 0.0519 mmol, 52%, 100% pure by ELSD) as a colorless solid: Mp 91-93 °C; IR (ATR) 2997, 2926, 2812, 1626, 1500, 1447, 1243, 1223, 1143, 1023, 979, 751, 741, 726 cm⁻¹; ¹H NMR (300 MHz, CDCl₃) δ 7.06-7.01 (m, 1 H), 6.95-6.87 (m, 3 H), 3.87 (s, 3 H), 3.80 (app t, 2 H, *J =* 5.0 Hz), 3.64-3.62 (m, 4 H), 3.23 (s, 2 H), 3.07 (app t, 2 H, *J* = 5.0 Hz), 3.03 (app t, 2 H, *J =* 5.0 Hz), 2.41 (s, 3 H), 2.28 (s, 3 H); ¹³C NMR (75 MHz, CDCl₃) δ 167.4, 166.7, 159.8, 152.2, 140.4, 123.6, 121.0, 118.4, 111.3, 109.7, 55.4, 50.7, 50.5, 46.7, 42.0, 32.1, 23.7, 11.0, 10.1; HRMS (ESI) *m*/*z* calcd for C₁₉H₂₆N₃O₂₃S ([M+H]⁺) 376.1689, found 376.1673.

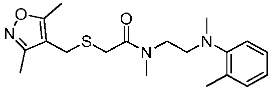

**2-(((3,5-Dimethylisoxazol-4-yl)methyl)thio)-N-methyl-N-(2-(methyl(*o*-tolyl)amino)ethyl) acetamide (5i).** To a solution of 2-(((3,5-dimethylisoxazol-4-yl)methyl)thio)acetic acid **(3a,** 0.0608 g, 0.302 mmol) in CH₂Cl₂ (3.0 mL) was added *N,N*'-dimethyl-*N*-(*o*-tolyl)ethane-1,2-diamine **(4i,** 0.0500 g, 0.275 mmol) and Et₃N (115 µL, 0.825 mmol). The reaction mixture was cooled to 0 °C, treated with T3P (50 wt.% solution in EtOAc, 292 µL, 0.412 mmol), warmed to room temperature, stirred for 20 h, diluted with CH₂Cl₂, and washed with satd. aqueous NH₄Cl solution, satd. aqueous NaHCO₃ solution, and brine, dried (Na₂SO₄), filtered, and concentrated *in vacuo.* The crude residue was purified by chromatography on SiO₂ (3:2, EtOAc/hexanes, base washed with 0.1% Et₃N prior to use) to give **5i** (0.0752 g, 0.207 mmol, 75%, 99.6% pure by ELSD) as a light yellow oil: IR (ATR) 2932, 2795, 1640, 1598, 1493, 1451, 1421, 1393, 1196, 1108, 1047, 766, 738 cm⁻¹; ¹H NMR (400 MHz, CDCl₃, room temperature, mixture of rotamers coalescing in DMSO-d₆ at 357 K) δ 7.20-7.12 (m, 2 H), 7.07-6.95 (m, 2 H), 3.59, 3.58 (2s, 2 H), 3.54 (t, 1 H, *J =* 6.6 Hz), 3.39 (t, 1 H, *J =* 6.6 Hz), 3.16-3.08 (m, 3 H), 2.97, 2.95 (2s, 4 H), 2.71, 2.67 (2s, 3 H), 2.38 (s, 3 H), 2.30 (s, 2 H), 2.27, 2.26 (3s, 4 H); ¹³C NMR (125 MHz, CDCl₃, room temperature, mixture of rotamers coalescing in DMSO-d₆ at 357 K) δ 169.2, 168.8, 166.7 (2 C), 159.7, 151.7, 150.8, 133.8, 132.9, 131.4, 131.2, 126.7, 126.5, 124.0, 123.2, 120.2, 119.9, 109.8, 53.9, 53.2, 48.4, 46.4, 43.3, 42.3, 36.7, 33.8, 32.4, 31.6, 23.7, 23.4, 18.2, 18.0, 11.0 (2 C), 10.1; HRMS (ESI) *m*/*z* calcd for C₁₉H₂₈N₃O₂S ([M+H]⁺) 362.1897, found 362.1890.

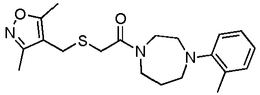

**2-(((3,5-Dimethylisoxazol-4-yl)methyl)thio)-1-(4-(*o*-tolyl)-1,4-diazepan-1-yl)ethan-1-one (BRE454-76; 5j).** A solution of *tert-*butyl 4-(o-tolyl)-1,4-diazepane-1-carboxylate **(29a,** 0.0750 g, 0.258 mmol) in THF (0.3 mL) was cooled to 0 °C, treated with 4 M HCl in dioxane (1.6 mL) and stirred at 0 °C for 2 h. The reaction mixture was concentrated *in vacuo* and the yellow solid **4j** was precipitated in Et₂O, filtered off from the solution, washed with Et₂O, dried under high vacuum, and used without further purification for the next step.

To a solution of 2-(((3,5-dimethylisoxazol-4-yl)methyl)thio)acetic acid **(3a,** 0.0460 g, 0.229 mmol) in CH₂Cl₂ (2.3 mL) was added 4-(o-tolyl)-1,4-diazepane hydrochloride **(4j,** 0.258 mmol) and Et₃N (159 µL, 1.14 mmol). The reaction mixture was cooled to 0 °C, treated with T3P (50 wt.% solution in EtOAc, 242 µL, 0.343 mmol), warmed to room temperature, stirred for 20 h, diluted with CH₂Cl₂, and washed with satd. aqueous NH₄Cl solution, satd. aqueous NaHCO₃ solution, and brine, dried (Na₂SO₄), filtered, and concentrated *in vacuo.* The crude residue was purified by chromatography on SiO₂ (3:2, EtOAc/hexanes, base washed with 0.1% Et₃N) to give **5j** (0.0854 g, 0.229 mmol, quant. 100% pure by ELSD) as a clear colorless oil: IR (ATR) 2945, 2825, 1634, 1598, 1491, 1447, 1423, 1215, 1194, 1136, 915, 762, 726 cm⁻¹; ¹H NMR (400 MHz, CDCl₃, room temperature, mixture of rotamers) δ 7.20 (app d, 1 H, *J* = 7.6 Hz), 7.17 (app t, 1 H, *J =* 7.6 Hz), 7.05 (app d, 1 H, *J =* 7.6 Hz), 7.01 (app dt, 1 H, *J =* 7.2, 2.0 Hz), 3.82-3.78 (m, 2 H), 3.71-3.65 (m, 4 H), 3.24-3.20 (m, 3 H), 3.15 (t, 1 H, *J =* 5.2 Hz), 3.12-3.07 (m, 2 H), 2.46 (app s, 3 H), 2.32 (2s, 6 H), 2.04 (sept, 2 H, *J =* 6.0 Hz); ¹³C NMR (125 MHz, CDCl₃, room temperature, mixture of rotamers) δ 168.9, 168.8, 166.9, 166.8, 159.8 (2 C), 153.4, 153.3, 132.9 (2 C), 131.1 (2 C), 126.7, 126.6, 123.6, 123.4, 120.8, 120.7, 109.9, 56.4, 55.8, 55.5, 54.9, 50.1, 47.6, 47.2, 44.9, 32.2, 32.0, 29.5, 28.2, 23.7, 18.5 (2 C), 11.1, 10.2 (2 C); HRMS (ESI) *m*/*z* calcd for C₂₀H₂₈N₃O₂S ([M+H]⁺) 374.1897, found 374.1883.

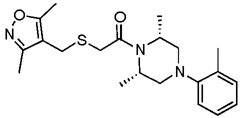

**1-(2,6-Dimethyl-4-(o-tolyl)piperazin-1-yl)-2-(((3,5-dimethylisoxazol-4-yl)methyl)thio)ethanone (5k).** A solution of (((3,5-dimethylisoxazol-4-yl)methyl)thio)acetic acid **(3a,** 0.0300 g, 0.142 mmol) in CH₂Cl₂ (2 mL) was treated with 3,5-dimethyl-1-(o-tolyl)piperazine **(4k,** 0.0350 g, 0.170 mmol) and Et₃N (59 µL, 0.425 mmol). The reaction mixture was cooled to 0 °C, treated with T3P (50 wt.% solution in EtOAc, 150 µL, 0.212 mmol), warmed to room temperature, stirred for 20 h, diluted with CH₂Cl₂, and washed with satd. aqueous NH₄Cl, satd. aqueous NaHCO₃, and brine, dried (Na₂SO₄), filtered, and concentrated *in vacuo.* The crude residue was purified by chromatography on SiO₂ (95:5 CH₂Cl₂/MeOH) to give **5k** (0.0450 g, 0.116 mmol, 82%, 99.8% pure by ELSD) as a light yellow oil: IR (neat) 2975, 1629, 1491, 1422, 1327, 1127 cm⁻¹; ¹H NMR (500 MHz, CDCl₃) δ 7.22-7.19 (m, 2 H), 7.06-7.02 (m, 2 H), 4.68 (brs, 1 H), 4.05 (brs, 1 H), 3.73-3.70 (m, 1 H), 3.66-3.61 (m, 1 H), 3.30-3.19 (m, 2 H), 2.98-2.96 (m, 2 H), 2.94-2.89 (m, 1 H), 2.81-2.78 (m, 1 H), 2.44 (s, 3 H), 2.41 (s, 3 H), 2.31 (s, 3 H), 1.55 (d, 3 H, *J =* 6.0 Hz), 1.48 (d, 3 H, *J =* 6.0 Hz); ¹³C NMR (125 MHz, CDCl₃) δ 168.2, 166.7, 151.2, 133.3, 131.2, 126.8, 124.1, 119.6, 109.8, 57.0, 56.8, 49.8, 45.8, 32.0, 23.6, 21.6, 20.3, 18.2, 11.0, 10.1; HRMS (ESI) *m*/*z* calcd for C₂₁H₃₀N₃O₂S ([M+H]⁺) 388.2059, found 388.2053.

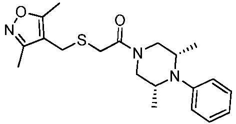

**1-(3,5-Dimethyl-4-phenylpiperazin-1-yl)-2-(((3,5-dimethylisoxazol-4-yl)methyl)thio)ethan-1-one (5l)**. A solution of *tert-*butyl 3,5-dimethyl-4-phenylpiperazine-1-carboxylate **(29b,** 0.0330 g, 0.114 mmol) in THF (0.1 mL) at 0 °C was treated with 4 M HCl in dioxane (0.70 mL) and stirred at 0 °C for 1.5 h and at room temperature for 1.5 h. The yellow solid was filtered off, washed with Et₂O, dried under high vacuum and the resulting crude **4l** was directly used for the next step.

To a solution of 2-(((3,5-dimethylisoxazol-4-yl)methyl)thio)acetic acid **3a** (0.0229 g, 0.114 mmol) in CH₂Cl₂ (1.1 mL) was added 2,6-dimethyl-1-phenylpiperazine hydrochloride (**4l**, 0.0258 g, 0.114 mmol) and Et₃N (79 µL, 0.569 mmol). The reaction mixture was cooled to 0 °C, treated with T3P (50 wt.% solution in EtOAc, 121 µL, 0.171 mmol), warmed to room temperature, stirred for 20 h, diluted with CH₂Cl₂, and washed with satd. aqueous NH₄Cl solution, satd. aqueous NaHCO₃ solution, and brine, dried (Na₂SO₄), filtered, and concentrated *in vacuo.* The crude residue was purified by chromatography on SiO₂ (1:1, acetone/hexanes, base washed with 0.1% Et₃N prior to use) to give **5l** (0.0322 g, 0.0862 mmol, 76%, 100% pure by ELSD) as a colorless oil: IR (ATR) 2967, 2931, 1639, 1597, 1493, 1449, 1377, 1319, 1272, 1238, 1151, 1091, 886, 771, 731, 703 cm⁻¹; ¹H NMR (400 MHz, CDCl₃) δ 7.31 (t, 2 H, *J =* 7.6 Hz), 7.18 (t, 1 H, *J* = 7.2 Hz), 7.10 (d, 2 H, *J =* 7.6 Hz), 4.42 (ddd, 1 H, *J =* 12.8, 4.0, 2.4 Hz), 3.70-3.60 (m, 3 H), 3.29-3.18 (m, 2 H), 3.10-2.93 (m, 3 H), 2.67 (dd, 1 H, *J =* 13.2, 10.4 Hz), 2.43 (s, 3 H), 2.30 (s, 3 H), 0.77 (d, 3 H, *J =* 6.4 Hz), 0.76 (d, 3 H, *J =* 5.6 Hz); ¹³C NMR (100 MHz, CDCl₃) δ 167.1, 166.8, 159.7, 148.5, 128.9, 126.4, 125.6, 109.8, 56.0, 55.6, 53.4, 48.7, 31.9, 23.7, 18.2, 18.2, 11.1, 10.2; HRMS (ESI) *m*/*z* calcd for C₂₀H₂₈N₃O₂S ([M+H]⁺) 374.1897, found 374.1887.

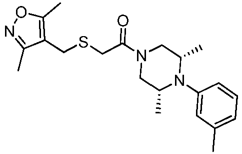

**1-(3,5-Dimethyl-4-(*m*-tolyl)piperazin-1-yl)-2-(((3,5-dimethylisoxazol-4-yl)methyl)thio)ethan-1-one (5m).** A solution of *tert-*butyl 3,5-dimethyl-4-(m-tolyl)piperazine-1-carboxylate (29c, 0.0400 g, 0.131 mmol) in THF (0.1 mL) at 0 °C was treated with 4 M HCl in dioxane (0.80 mL), and stirred at 0 °C for 1.5 h and at room temperature for 1.5 h. A yellow precipitate formed and the solid was filtered off, washed with Et₂O, and dried under high vacuum and the resulting crude 4m was used directly for the next step.

To a solution of 2-(((3,5-dimethylisoxazol-4-yl)methyl)thio)acetic acid (3a, 0.0264 g, 0.131 mmol) in CH₂Cl₂ (1.3 mL) was added 2,6-dimethyl-1-(m-tolyl)piperazine hydrochloride (4m, 0.0316 g, 0.131 mmol) and Et₃N (91 µL, 0.656 mmol). The reaction mixture was cooled to 0 °C, treated with T3P (50 wt. % solution in EtOAc, 139 µL, 0.197 mmol), warmed to room temperature, stirred for 20 h, diluted with CH₂Cl₂, washed with satd. aqueous NH₄Cl solution, satd. aqueous NaHCO₃ solution, and brine, dried (Na₂SO₄), filtered, and concentrated *in vacuo.* The crude residue was purified by chromatography on SiO₂ (3:2, EtOAc/hexanes, base washed with 0.1% Et₃N prior to use) to give 5m (0.0400 g, 0.103 mmol, 79%, 100% pure by ELSD) as a clear colorless oil: IR (ATR) 2966, 2929, 1637, 1602, 1451, 1376, 1319, 1271, 1194, 1149, 1108, 1088, 911, 889, 788, 730, 709 cm⁻¹; ¹H NMR (400 MHz, CDCl₃) δ 7.18 (t, 1 H, *J =* 7.6 Hz), 6.97 (d, 1 H, *J =* 7.6 Hz), 6.90-6.88 (m, 2 H), 4.41 (app d, 1 H, *J =* 12.8 Hz), 3.64 (brs, 3 H), 3.27-3.19 (m, 2 H), 3.15-2.91 (m, 3 H), 2.67 (t, 1 H, *J =* 9.2 Hz), 2.43 (s, 3 H), 2.32 (s, 3 H), 2.30, (s, 3 H), 0.77 (br app s, 6 H); ¹³C NMR (100 MHz, CDCl₃) δ 167.1, 166.8, 159.7, 148.4, 138.7, 128.7, 127.1, 126.4, 123.4, 109.8, 56.0, 55.6, 53.4, 48.7, 32.0, 23.7, 21.4, 18.3, 18.2, 11.1, 10.2; HRMS (ESI) *m*/*z* calcd for C₂₁H₃₀N₃O₂S ([M+H]⁺) 388.2053, found 388.2046.

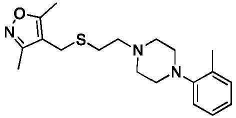

**3,5-Dimethyl-4-(((2-(4-(*o*-tolyl)piperazin-1-yl)ethyl)thio)methyl)isoxazole (6).** A solution of 2-(((3,5-dimethylisoxazol-4-yl)methyl)thio)-1-(4-(o-tolyl)piperazin-1-yl)ethanone **(5b,** 0.0387 g, 0.108 mmol) in THF (1 mL) at 0 °C was treated with LiAlH₄ (1 M solution in Et₂O, 120 µL, 0.118 mmol), stirred at 0 °C for 1 h, and then quenched with Rochelle's salt (NaKC₄H₄O₆, satd. aqueous solution, 1 mL). The mixture was stirred for an additional 1 h at 0 °C, diluted with EtOAc, extracted with EtOAc (2 x 15 mL), dried (Na₂SO₄), filtered, and concentrated *in vacuo.* The crude residue was purified by chromatography on SiO₂ (ISCO, 4 g column, liquid load in CH₂Cl₂, 0-20% MeOH/CH₂Cl₂, product eluted at 5% MeOH) to give a colorless oil. This oil was further purified by chromatography on SiO₂ (CH₂Cl₂ to 5:95, MeOH/CH₂Cl₂) on a pipette column to give 6 (0.0155 g, 0.0449 mmol, 42%, 100% pure by ELSD) as a colorless oil: IR (neat) 3393, 2925, 2814, 1637, 1599, 1493, 1448, 1424, 1372, 1227, 1195, 1130, 1041, 1006, 931, 763, 723 cm⁻¹; ¹H NMR (300 MHz, CDCl₃) δ 7.16 (app t, 2 H, *J =* 7.4 Hz), 7.03-6.95 (m, 2 H), 3.75 (t, 1 H, *J =* 5.7 Hz), 3.50 (s, 2 H), 2.93 (app t, 4 H, *J =* 4.5 Hz), 2.63 (brs, 8 H), 2.38 (s, 3 H), 2.30 (s, 3 H), 2.29 (s, 3 H); ¹³C NMR (75 MHz, CDCl₃) δ 165.9, 159.6, 151.4, 132.6, 131.0, 126.6, 123.2, 119.0, 110.5, 77.2, 58.1, 53.6, 51.6, 29.1, 24.0, 23.5, 17.8, 11.1, 10.2; HRMS (ESI) *m*/*z* calcd for C₁₉H₂₈ON₃S ([M+H]⁺) 346.1948, found 346.1946.

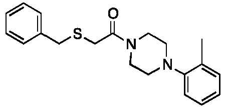

**2-(Benzylthio)-1-(4-(*o*-tolyl)piperazin-1-yl)ethanone (7).** A solution of 2-(benzylthio)acetic acid **3b** (0.0440 g, 0.241 mmol) in CH₂Cl₂ (3.05 mL) was treated with 1-(o-tolyl)piperazine **4b** (0.0521 g, 0.290 mmol) and Et₃N (101 µL, 0.724 mmol). The reaction mixture was cooled to 0 °C, treated with T3P (50 wt.% solution in EtOAc, 256 µL, 0.362 mmol), warmed to room temperature and stirred for 2 d. The solution was diluted with CH₂Cl₂ and washed with satd. aqueous NH₄Cl, satd. aqueous NaHCO₃, and brine, dried (Na₂SO₄), filtered, and concentrated *in vacuo.* The crude residue was purified by chromatography on SiO₂ (ISCO, 12 g column, liquid load in CH₂Cl₂, EtOAc/hexanes gradient (10-100%)) to give 7 (0.0635 g, 0.187 mmol, 77%, 100% pure by ELSD) as a yellow oil: IR (ATR) 2917, 1815, 1634, 1598, 1492, 1437, 1223, 1150, 1031, 975, 761, 700 cm⁻¹; ¹H NMR (300 MHz, CDCl₃) δ 7.45-7.23 (m, 7 H), 7.06-7.01 (m, 2 H), 3.89 (s, 2 H), 3.79 (app t, 2 H, *J =* 4.9 Hz), 3.59 (app t, 2 H, *J* = 4.9 Hz), 3.30 (s, 2 H), 2.95-2.90 (m, 4 H), 2.37 (s, 3 H); ¹³C NMR (75 MHz, CDCl₃) δ 167.7, 150.9, 137.7, 132.8, 131.2, 129.3, 128.5, 127.2, 126.7, 123.8, 119.3, 51.9, 51.7, 46.9, 42.4, 36.3, 32.4, 17.8; HRMS (ESI) *m*/*z* calcd for C₂₀H₂₅N₂OS ([M+H]⁺) 341.1682, found: 341.1674.

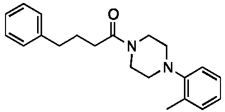

**4-Phenyl-1-(4-(*o*-tolyl)piperazin-1-yl)butan-1-one (8).** To a solution of phenyl butanoic acid (3c, 0.0500 g, 0.305 mmol) in CH₂Cl₂ (3.05 mL) was added 1-(*o*-tolyl)piperazine **(4b,** 0.0657 g, 0.365 mmol) and Et₃N (85 µL, 0.609 mmol). The reaction mixture was cooled to 0 °C, treated with T3P (50 wt. % solution in EtOAc, 322 µL, 0.457 mmol), warmed to room temperature, stirred overnight, diluted with CH₂Cl₂ and washed with satd. aqueous NH₄Cl, satd. aqueous NaHCO₃, and brine, dried (Na₂SO₄), filtered, and concentrated *in vacuo.* The crude residue was purified by chromatography on SiO₂ (ISCO, 12 g column, liquid load in CH₂Cl₂, EtOAc/hexanes gradient (10-100%), eluted at 30%) to give 8 (0.0863 g, 0.268 mmol, 88%, 100% pure by ELSD) as a colorless oil: IR (ATR) 3024, 2917, 2813, 1641, 1492, 1432, 1223, 1150, 1025, 761, 722 cm⁻¹; ¹H NMR (300 MHz, CDCl₃) δ 7.36-7.31 (m, 2 H), 7.27-7.18 (m, 5 H), 7.07-6.99 (m, 2 H), 3.80 (app t, 2 H, *J* = 4.8 Hz), 3.55 (app t, 2 H, *J* = 4.8 Hz), 2.88 (app t, 4 H, *J =* 4.8 Hz), 2.75 (t, 2 H, *J* =7.5 Hz), 2.41 (t, 2 H, *J =* 7.5 Hz), 2.36 (s, 3 H), 2.06 (ddd, 2 H, *J =* 7.9, 7.7, 7.3 Hz); ¹³C NMR (75 MHz, CDCl₃) δ 171.2, 150.8, 141.6, 132.6, 131.0, 128.4, 128.3, 126.6, 125.8, 123.6, 119.0, 51.9, 51.6, 45.9, 41.9, 35.2, 32.3, 26.6, 17.7; HRMS (ESI) *m*/*z* calcd for C₂₁H₂₇N₂O ([M+H]⁺) 323.2118, found: 323.2110.

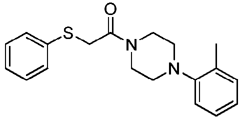

**2-(Phenylthio)-1-(4-(o-tolyl)piperazin-1-yl)ethan-1-one (9).** To a solution of 2-(phenylthio)acetic acid (3d, 0.0500 g, 0.297 mmol) in CH₂Cl₂ (3.0 mL) was added 1-(o-tolyl)piperazine **(4b,** 0.0642 g, 0.357 mmol) and Et₃N (83 µL, 0.594 mmol). The mixture was cooled to 0 °C, treated with T3P (50 wt.% solution in EtOAc, 315 µL, 0.446 mmol), warmed to room temperature, stirred for 3 d, diluted with CH₂Cl₂ and washed with satd. aqueous NH₄Cl, satd. aqueous NaHCO₃, and brine. The organic layer was dried (Na₂SO₄), filtered, and concentrated *in vacuo.* The crude residue was purified by chromatography on SiO₂ (ISCO, 4 g column, liquid load in CH₂Cl₂, EtOAc/hexanes gradient (0-30%), eluted at 20-30%) to give 9 (0.0746 g, 0.229 mmol, 77%, 100% pure by ELSD) as a clear colorless oil: IR (ATR) 3057, 2947, 2911, 2856, 2815, 1639, 1598, 1492, 1482, 1382, 1275, 1223, 1203, 1149, 1115, 1032, 974, 950, 909, 762, 738, 723, 690 cm⁻¹; ¹H NMR (400 MHz, CDCl₃) δ 7.48 (dd, 2 H, *J* = 7.6, 1.2 Hz), 7.34 (app t, 2 H, *J =* 7.6 Hz), 7.26-7.17 (m, 3 H), 7.02 (app t, 1 H, *J* = 7.6 Hz), 6.98 (app d, 1 H, *J =* 7.6 Hz), 3.81 (s, 2 H), 3.76 (app t, 2 H, *J =* 4.8 Hz), 3.63 (app t, 2 H, *J =* 4.8 Hz), 2.91 (app t, 2 H, *J =* 4.8 Hz), 2.86 (t, 2 H, *J =* 4.8 Hz), 2.33 (s, 3 H); ¹³C NMR (100 MHz, CDCl₃) δ 167.1, 150.7, 134.9, 132.7, 131.2, 130.3, 129.1, 127.0, 126.7, 123.8, 119.2, 51.9, 51.6, 47.0, 42.5, 36.7, 17.8; HRMS (ESI) *m*/*z* calcd for C₁₉H₂₃N₂OS ([M+H]⁺) 327.1526, found 327.1514.

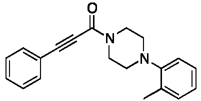

**3-Phenyl-1-(4-(*o*-tolyl)piperazin-1-yl)prop-2-yn-1-one (10).** To a solution of phenyl propiolic acid (3e, 0.200 g, 1.37 mmol) in CH₂Cl₂ (12 mL) was added 1-(o-tolyl)piperazine **(4b,** 0.290 g, 1.64 mmol) and Et₃N (570 µL, 4.11 mmol). The reaction mixture was cooled to 0 °C, treated with T3P (50 wt. % solution in EtOAc, 1.45 mL, 2.05 mmol), warmed to room temperature, stirred for 3 d, diluted with CH₂Cl₂ (30 mL), and washed with satd. aqueous NH₄Cl (5 mL), satd. aqueous NaHCO₃ (5 mL), and brine (5 mL), dried (Na₂SO₄), filtered, and concentrated *in vacuo.* The crude residue was purified by chromatography on SiO₂ (ISCO, 24 g column, liquid load in CH₂Cl₂, EtOAc/hexanes gradient (10-100%), product eluted at 40% EtOAc/hexanes) to give **10** (0.401 g, 1.32 mmol, 96%, >99.9% pure by ELSD) as a colorless solid: Mp 127-129 °C; IR (neat) 3037, 2907, 2857, 2206, 1616, 1491, 1424, 1279, 1226, 1207, 1035, 923, 758, 726, 686 cm⁻¹; ¹H NMR (300 MHz, CDCl₃) δ 7.59-7.56 (m, 2 H), 7.43-7.34 (m, 3 H), 7.22-7.16 (m, 2 H), 7.05-6.99 (m, 2 H), 3.99 (app t, 2 H, *J =* 5.0 Hz), 3.85 (app t, 2 H, *J =* 5.0 Hz), 2.99 (app t, 2 H, *J* = 5.0 Hz), 2.92 (app t, 2 H, *J =* 5.0 Hz), 2.35 (s, 3 H); ¹³C NMR (75 MHz, CDCl₃) δ 153.2, 150.8, 132.8, 132.4, 131.2, 130.1, 128.6, 126.8, 123.9, 120.5, 119.3, 90.9, 81.2, 52.2, 51.5, 47.7, 42.1, 17.8; HRMS (ESI) *m*/*z* calcd for C₂₀H₂₁ON₂ ([M+H]⁺) 305.1648, found 305.1643.

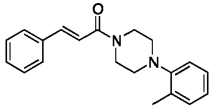

**(*E*)-3-Phenyl-1-(4-(*o*-tolyl)piperazin-1-yl)prop-2-en-1-one (11).** A solution of *trans-*cinnamic acid **(3f,** 0.0400 g, 0.270 mmol) in CH₂Cl₂ (2.5 mL) was treated with 1-(*o*-tolyl)piperazine **(4b,** 0.0570 g, 0.320 mmol), Et₃N (113 µL, 0.810 mmol). The reaction mixture was cooled to 0 °C, treated with T3P (50 wt.% solution in EtOAc, 290 µL, 0.405 mmol), warmed to room temperature, stirred for 3 d, diluted with CH₂Cl₂ (10 mL), and washed with satd. aqueous NH₄Cl (2 mL), satd. aqueous NaHCO₃ (2 mL), and brine (2 mL), dried (Na₂SO₄), filtered, and concentrated *in vacuo.* The crude residue was purified by chromatography on SiO₂ (ISCO, 12 g column, liquid load in CH₂Cl₂, EtOAc/hexanes gradient (10-100%), product eluted at 35%, EtOAc/hexanes) to give 11 (0.0520 g, 0.168 mmol, 62%, >99% purity by ELSD) as a yellow solid: Mp 110-111 °C; IR (neat) 3045, 2920, 2840, 1643, 1595, 1423, 1327, 1225, 1152, 986, 765, 710, 682 cm⁻¹; ¹H NMR (400 MHz, CDCl₃) δ 7.72 (d, 1 H, *J =* 11.4 Hz), 7.55 (dd, 2 H, *J =* 6.8, 1.4 Hz), 7.41-7.36 (m, 3 H), 7.20 (dd, 2 H, *J =* 14.6, 7.4 Hz), 7.02 (ddd, 2 H, *J =* 14.6, 7.4, 0.6 Hz), 6.95 (d, 1 H, *J =* 15.6 Hz), 3.90 (brs, 2 H), 3.81 (brs, 2 H), 2.96 (brs, 4 H), 2.36 (s, 3 H); ¹³C NMR (75 MHz, CDCl₃) δ 165.5, 150.8, 142.8, 135.2, 132.7, 131.1, 129.6, 128.8, 127.7, 126.6, 123.7, 119.2, 117.1, 52.1, 51.6, 46.4, 42.6, 17.8; HRMS (ESI) *m*/*z* calcd for C₂₀H₂₃ON₂ ([M+H]⁺) 307.1805, found 307.1796.

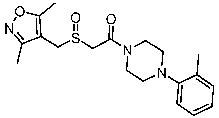

**2-(((3,5-Dimethylisoxazol-4-yl)methyl)sulfinyl)-1-(4-(*o*-tolyl)piperazin-1-yl)ethan-1-one (12).** To a solution of 2-(((3,5-dimethylisoxazol-4-yl)methyl)thio)-1-(4-(o-tolyl)piperazin-1-yl)ethanone **(5b,** 0.0500 g, 0.139 mmol) in MeOH (0.30 mL) at 0 °C was added dropwise a solution of sodium metaperiodate (0.0301 g, 0.139 mmol) in water (0.14 mL). The resulting heterogeneous mixture was allowed to warm to room temperature and stirred for 15 h. The reaction mixture was filtered through a plug of Celite (MeOH), concentrated, dissolved in CH₂Cl₂, dried (MgSO₄), filtered and concentrated *in vacuo.* The crude residue was purified by chromatography on SiO₂ (100% EtOAc) to give **12** (0.0356 g, 0.0948 mmol, 68%, 100% pure by ELSD) as a colorless foam: IR (ATR) 2917, 2818, 1631, 1599, 1493, 1441, 1384, 1275, 1224, 1195, 1151, 1053, 1028, 911, 764, 727 cm⁻¹; ¹H NMR (400 MHz, CDCl₃) δ 7.21-7.15 (m, 2 H), 7.02 (app t, 1 H, *J* = 7.2 Hz), 6.97 (app d, 1 H, *J =* 8.0 Hz), 4.18 (d, 1 H, *J =* 14.0 Hz), 3.90-3.84 (m, 5 H), 3.64 (app t, 2 H, *J =* 4.4 Hz), 2.95 (app t, 2 H, *J* = 4.4 Hz), 2.85 (brs, 2 H), 2.45 (s, 3 H), 2.32 (s, 3 H), 2.31 (s, 3 H); ¹³C NMR (100 MHz, CDCl₃) δ 169.2, 162.9, 159.9, 150.4, 132.7, 131.2, 126.7, 124.0, 119.2, 104.5, 53.7, 52.0, 51.5, 47.0, 46.8, 42.5, 17.7, 11.6, 10.3; HRMS (ESI) *m*/*z* calcd for C₁₉H₂₆N₃O₃S ([M+H]⁺) 376.1689, found 376.1684.

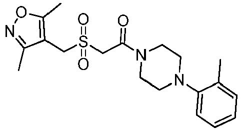

**2-(((3,5-Dimethylisoxazol-4-yl)methyl)sulfonyl)-1-(4-(*o*-tolyl)piperazin-1-yl)ethan-1-one (13).** A solution of 2-(((3,5-dimethylisoxazol-4-yl)methyl)thio)-1-(4-(o-tolyl)piperazin-1-yl)ethanone **(5b,** 0.0429 g, 0.117 mmol) in CH₂Cl₂ (0.65 mL) was treated with 3-chloroperoxybenzoic acid (70 wt.%, 0.0576 g, 0.234 mmol) in 2 portions. The reaction mixture was stirred at room temperature for 15 h, quenched with 10% aqueous sodium metabisulfite solution (2 mL), diluted with aqueous 1M NaOH (10 mL) and extracted with CH₂Cl₂ (2 × 15 mL). The combined organic layers were washed with 1 M NaOH (10 mL), dried (MgSO₄), filtered, and concentrated *in vacuo.* The crude residue was purified by chromatography on SiO₂ (70-100% EtOAc/hexanes) to give 13 (0.0203 g, 0.0519 mmol, 44%, 100% pure by ELSD) as a colorless foam: IR (ATR) 2919, 2819, 1641, 1599, 1493, 1445, 1318, 1225, 1150, 1126, 1030, 911, 765, 728 cm⁻¹; ¹H NMR (400 MHz, CDCl₃) δ 7.21-7.16 (m, 2 H), 7.05-6.98 (m, 2 H), 4.36 (s, 2 H), 4.09 (s, 2 H), 3.85 (app brs, 2 H), 3.72 (brt, 2 H, *J =* 4.0 Hz), 3.00 (brt, 2 H, *J =* 4.0 Hz), 2.93 (brt, 2 H, *J* = 4.4 Hz), 2.50 (s, 3 H), 2.35 (s, 3 H), 2.33 (s, 3 H); ¹³C NMR (100 MHz, CDCl₃) δ 170.1, 160.7, 160.3, 150.3, 132.7, 131.2, 126.7, 124.0, 119.2, 101.8, 54.9, 51.7, 51.4, 48.3, 47.8, 43.0, 17.8, 11.5, 10.2; HRMS (ESI) *m*/*z* calcd for C₁₉H₂₆N₃O₄S ([M+H]⁺) 392.1639, found 392.1633.

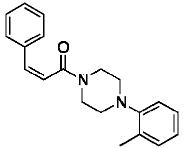

**(*Z*)-3-Phenyl-1-(4-(*o*-tolyl)piperazin-1-yl)prop-2-en-1-one (14).** To a solution of 3-phenyl-1-(4-(o-tolyl)piperazin-1-yl)prop-2-yn-1-one (10, 0.103 g, 0.337 mmol) in MeOH (2 mL) and EtOAc (1 mL) was added Lindlar's catalyst (5% Pd on CaCO₃, lead poisoned, 0.120 g) and quinoline (15 µL, 0.130 mmol). The reaction mixture was purged and backfilled with H₂ (balloon, 2 x), allowed to stir for 45 min, filtered through SiO₂, and concentrated *in vacuo.* The crude residue was purified by chromatography on SiO₂ (ISCO, modified dry load in CH₂Cl₂, 0-90% EtOAc/hexanes gradient, product eluted at 25% EtOAc/hexanes) to give 14 (0.104 g, 0.339 mmol, quant., 99.6% purity by ELSD) as a yellow oil: IR (neat) 3022, 2914, 2815, 1513, 1597, 1493, 1434, 1364, 1223, 1149, 1115, 1034, 973, 913, 855, 762, 722, 698 cm⁻¹; ¹H NMR (300 MHz, CDCl₃) δ 7.41-7.30 (m, 5 H), 7.17-7.11 (m, 2 H), 6.98 (t, 1 H, *J =* 7.1 Hz), 6.81 (d, 1 H, *J =* 7.8 Hz), 6.71 (d, 1 H*, J* = 12.6 Hz), 6.07 (d, 1 H, *J =* 12.6 Hz), 3.81 (app brt, 2 H, *J* = 4.8 Hz), 3.48 (app t, 2 H, *J*= 4.8 Hz), 2.81 (app t, 2 H, *J* = 4.8 Hz), 2.44 (app t, 2 H, *J =* 4.8 Hz), 2.25 (s, 3 H); ¹³C NMR (75 MHz, CDCl₃) δ 167.6, 150.9, 135.6, 133.5, 132.7, 131.1, 128.7, 128.6, 128.5, 126.6, 123.7, 123.2, 119.1, 51.5, 51.3, 46.8, 41.7, 17.7; HRMS (ESI) *m*/*z* calcd for C₂₀H₂₃ON₂ ([M+H]⁺) 307.1805, found 307.1800.

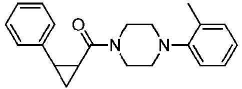

**(2-Phenylcyclopropyl)(4-(*o*-tolyl)piperazin-1-yl)methanone (15).** A solution of anhydrous CrCl₂ (0.0486 g, 0.392 mmol) in THF (0.6 mL) at room temperature under N₂ was treated with a solution of (Z)-3-phenyl-1-(4-(*o*-tolyl)piperazin-1-yl)prop-2-en-1-one **(14,** 0.0200 g, 0.0653 mmol) in THF (0.5 mL) and CH₂ICl (20 µL, 0.261 mmol). The reaction mixture was stirred for 18 h at reflux, quenched by addition of 1 M aqueous HCl (6 mL) and extracted with EtOAc. The combined organic layers were dried (Na₂SO₄), filtered, and concentrated *in vacuo.* The crude residue was purified by chromatography on SiO₂ (4:1, EtOAc/hexanes) to give 15 (0.0120 g, 0.0375 mmol, 57%, 100% pure by ELSD) as a brown oil: IR (neat) 2920, 1638, 1491, 1457, 1340, 1223, 1028 cm⁻¹; ¹H NMR (400 MHz, CDCl₃) δ 7.30-7.27 (m, 2 H), 7.22-7.11 (m, 5 H), 6.98 (dt, 1 H, *J =* 7.2, 1.2 Hz), 6.72 (dd, 1 H, *J =* 7.9, 0.8 Hz), 3.93-3.90 (m, 1 H), 3.77-3.73 (m, 1 H), 3.60-3.53 (m, 1H), 3.30-3.22 (m, 1 H), 2.75-2.72 (m, 2 H), 2.50-2.41 (m, 1 H), 2.26 (s, 3 H), 2.24-2.16 (m, 1 H), 2.10-2.00 (m, 1 H), 1.87 (dd, 1 *H, J =* 12.4, 5.8 Hz), 1.40-1.33 (m, 1 H), 0.92-0.80 (m, 1 H); ¹³C NMR (100 MHz, CDCl₃) δ 167.2, 150.9, 137.6, 132.7, 131.0, 128.2, 127.4, 126.5, 126.4, 123.6, 119.2, 51.9, 51.6, 45.7, 42.3, 24.4, 24.1, 17.7, 10.60 HRMS (ESI) *m*/*z* calcd for C₂₁H₂₅ON₂ ([M+H]⁺) 321.1967, found 321.1961.

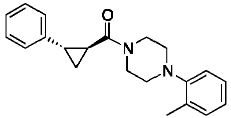

**((1*SR*,2*SR*)-2-Phenylcyclopropyl)(4-(*o*-tolyl)piperazin-1-yl)methanone (16).** To a solution of *trans*-2-phenylcyclopropanecarboxylic acid **(3g,** 0.0400 g, 0.247 mmol) in CH₂Cl₂ (2.5 mL) was treated with 1-(o-tolyl)piperazine **(4b,** 0.0540 g, 0.296 mmol), Et₃N (100 µL, 0.740 mmol). The reaction mixture was cooled to 0 °C, treated with T3P (50 wt.% solution in EtOAc, 260 µL, 0.370 mmol, 1.5 equiv), warmed to room temperature, stirred for 3 d, diluted with EtOAc (10 mL), and washed with satd. aqueous NH₄Cl (2 mL), satd. aqueous NaHCO₃ (2 mL), and brine (2 mL), dried (Na₂SO₄), filtered, and concentrated *in vacuo.* The crude residue was purified by chromatography on SiO₂ (ISCO, 12 g column, liquid load in CH₂Cl₂, EtOAc/hexanes gradient (10-90%), product eluted at 20%) to give 16 (0.0676 g, 0.211 mmol, 86%, >99.9% pure by ELSD) as a yellow oil: IR (neat) 3026, 2912, 2814, 1631, 1600, 1493, 1440, 1381, 1223, 1150, 1033, 919, 910, 760, 723, 696 cm⁻¹; ¹H NMR (300 MHz, CDCl₃) δ 7.32-7.26 (m, 2 H), 7.23-7.12 (m, 5 H), 7.01 (dd, 2 H, *J =* 11.1, 7.5 Hz), 3.79 (brs, 4 H), 2.90 (brs, 4 H), 2.52 (brpent, 1 H, *J* = 4.6 Hz), 2.33 (s, 3 H), 2.02 (pent, 1 H, *J* = 4.6 Hz), 1.71 (pent, 1 H, *J* = 4.6 Hz), 1.34-1.26 (m, 2 H); ¹³C NMR (100 MHz, CDCl₃) δ 170.6, 150.9, 141.0, 132.7, 131.2, 128.6, 126.7, 126.3, 126.1, 123.8, 119.2, 52.2, 51.7, 46.2, 25.6, 23.3, 17.9, 16.2; HRMS (ESI) *m*/*z* calcd for C₂₁H₂₅ON₂ ([M+H]⁺) 321.1961, found 321.1957.

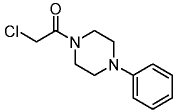

**2-Chloro-1-(4-(*o*-tolyl)piperazin-1-yl)ethanone (17a)** (Glennon et al., J. Med. Chem. 1986, 29, 2375-2380; Jorand-Lebrun et al., J. Med. Chem. 1997, 40, 3974-3978.). To a solution of chloroacetyl chloride (0.698 g, 6.05 mmol) and potassium carbonate (1.14 g, 8.25 mmol) in THF (7.0 mL) was added 1-(o-tolyl)piperazine **(4b,** 1.00 g, 5.50 mmol) in THF (12.6 mL) at 0 °C. The reaction mixture was gradually warmed to room temperature, stirred for 16 h, diluted with water, and extracted with EtOAc (3 x 20 mL). The combined organic extracts were washed sequentially with satd. aqueous NaHCO₃, 0.1 M aqueous HCl, and brine, dried (Na₂SO₄), filtered and concentrated *in vacuo.* The crude solid was filtered through a plug of SiO₂ (3:7, EtOAc/hexanes v/v 1% Et₃N) and washed thoroughly with EtOAc/hexanes (3:7) to give 17a (1.37 g, 5.42 mmol, 99%) as an off white solid: ¹H NMR (400 MHz, CDCl₃) δ 7.22-7.16 (m, 2 H), 7.05-6.99 (m, 2 H), 4.12 (s, 2 H), 3.78 (app t, 2 H, *J =* 4.8 Hz), 3.67 (app t, 2 H, *J* = 4.8 Hz), 2.97 (app t, 2 H, *J =* 4.8 Hz), 2.91 (app t, 2 H, *J* = 4.8 Hz), 2.33 (s, 3 H).

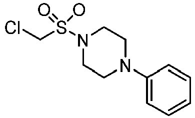

**1-((Chloromethyl)sulfonyl)-4-(o-tolyl)piperazine (17b)** (Zhou et al., J. Org. Lett. 2008, 10, 2517-2520.). To a solution of 1-(*o*-tolyl)piperazine **(4b,** 0.500 g, 2.75 mmol) in CH₂Cl₂ (9.8 mL) and Et₃N (0.390 mL, 2.75 mmol) at 0 °C was added chloromethanesulfonyl chloride (0.460 g, 3.03 mmol). The reaction mixture was stirred at 0 °C, gradually warmed to room temperature quenched after 14 h with satd. aqueous NH₄Cl solution (3 mL), and extracted with EtOAc (3 x 20 mL). The combined organic extracts were washed water (2 x 10 mL) and brine (10 mL), dried (Na₂SO₄), filtered and concentrated *in vacuo.* The crude solid was filtered through a plug of SiO₂ (3:7, EtOAc/hexanes containing 1% Et₃N) and washed thoroughly with EtOAc/hexanes (3:7). The combined filtrates were concentrated *in vacuo* to give **17b** (0.676 g, 2.34 mmol, 85%) as an orange solid: ¹H NMR (300 MHz, CDCl₃) δ 7.19 (t, 2 H, *J =* 8.1 Hz), 7.03 (t, 2 H, *J =* 8.1 Hz), 4.56 (s, 2 H), 3.63 (app t, 4 H, *J* = 5.0 Hz), 2.99 (app t, 4 H, *J* = 5.0 Hz), 2.32 (s, 3 H); ¹³C NMR (75 MHz, CDCl₃) δ 150.6, 132.7, 131.2, 126.8, 124.1, 119.4, 54.5, 51.9, 47.1, 17.7.

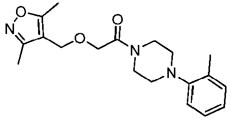

**2-((3,5-Dimethylisoxazol-4-yl)methoxy)-1-(4-(o-tolyl)piperazin-1-yl)ethan-1-one (18a).** A solution of (3,5-dimethylisoxazol-4-yl)methanol **(27,** 0.0302 g, 0.237 mmol) in THF (0.48 mL) was cooled to 0 °C and NaH (60% dispersion in mineral oil, 0.0190 g, 0.475 mmol) was added. The reaction mixture was stirred at 0 °C for 30 min, treated with 2-chloro-1-(4-(o-tolyl)piperazin-1-yl)ethanone **(17a,** 0.0600 g, 0.237 mmol), warmed to room temperature, stirred for 20 h, quenched with brine (1 mL), diluted with EtOAc (15 mL) and brine (5 mL), and extracted with EtOAc (2 × 15 mL). The combined organic layers were dried (Na₂SO₄) and concentrated *in vacuo.* The crude residue was purified by chromatography on SiO₂ (3:2, EtOAc/hexanes) to give **18a** (0.0735 g, 0.214 mmol, 90%, 100% pure by ELSD) as a light yellow oil: IR (ATR) 2918, 2817, 1645, 1599, 1493, 1443, 1369, 1273, 1225, 1116, 1030, 977, 764, 725 cm⁻¹; ¹H NMR (400 MHz, CDCl₃) δ 7.21-7.16 (m, 2 H), 7.02 (dt, 1 H, *J =* 7.6, 1.2 Hz), 6.97 (app d, 1 H, *J* = 8.0 Hz), 4.41 (s, 2 H), 4.17 (s, 2 H), 3.77 (brs, 2 H), 3.59 (app t, 2 H, *J* = 4.8 Hz), 2.89 (app t, 4 H, *J* = 3.6 Hz), 2.41 (s, 3 H), 2.32 (s, 3 H), 2.30 (s, 3 H); ¹³C NMR (125 MHz, CDCl₃) δ 167.8, 167.5, 159.8, 150.7, 132.7, 131.2, 126.7, 123.9, 119.2, 110.5, 68.7, 61.7, 52.1, 51.7, 45.6, 42.3, 17.8, 11.1, 10.1; HRMS (ESI) *m*/*z* calcd for C₁₉H₂₆N₃O₃ ([M+H]⁺) 344.1969, found 344.1960.

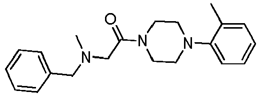

**2-(Benzyl(methyl)amino)-1-(4-(o-tolyl)piperazin-1-yl)ethanone (18b).** A solution of 2-chloro-1-(4-(o-tolyl)piperazin-1-yl)ethanone **(17a,** 0.0534 g, 0.211 mmol), in CH₃CN (4 mL) was treated with *N-*methylbenzylamine (23 µL, 0.176 mmol) and K₂CO₃ (0.730 g, 0.528 mmol). The reaction mixture was heated at reflux for 5 h, cooled to room temperature, filtered, and concentrated *in vacuo.* The crude residue was purified by chromatography on SiO₂ (2:3, EtOAc/hexanes) to give **18b** (0.0590 g, 0.175 mmol, 99%, >95% pure by LCMS) as a light yellow oil: IR (neat) 2933, 2816, 1640, 1450, 1491, 1222 cm⁻¹; ¹H NMR (500 MHz, CDCl₃) δ 7.39-7.33 (m, 4 H), 7.31-7.27 (m, 1 H), 7.23-7.19 (m, 2 H), 7.04 (t, 1 H, *J =* 7.5 Hz), 7.01 (d, 1 H, *J =* 8.0 Hz), 3.77 (brs, 2 H), 3.71-3.69 (m, 2 H), 3.61 (s, 2 H), 3.27 (s, 2 H), 2.91-2.87 (m, 4 H), 2.35 (s, 3 H) 2.34 (s, 3 H); ¹³C NMR (125 MHz, CDCl₃) δ 150.9, 138.1, 132.6, 131.1, 129.1, 128.2, 127.2, 126.6, 123.6, 119.1, 62.0, 60.3, 52.1, 51.7, 46.1, 42.4, 42.2, 17.8; HRMS (ESI) *m*/*z* calcd for C₂₁H₂₈N₃O ([M+H]⁺) 338.2238, found 338.2211.

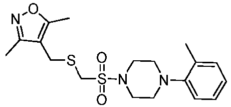

**3,5-Dimethyl-4-(((((4-(*o*-tolyl)piperazin-1-yl)sulfonyl)methyl)thio)methyl)isoxazole (18c).** A suspension of NaH (60% dispersion in mineral oil, 0.0200 g, 0.499 mmol) in THF (0.6 mL) was treated under an atmosphere of N₂ at 0 °C with a solution of (3,5-dimethylisoxazol-4-yl)methanethiol **(25,** 0.0536 g, 0.374 mmol) in THF (0.4 mL). The reaction mixture was stirred for 10 min, treated with 1-((chloromethyl)sulfonyl)-4-(*o*-tolyl)piperazine **(17b,** 0.0360 g, 0.125 mmol), stirred for 2 d at room temperature, quenched (water) and extracted (EtOAc). The combined organic layers were dried (Na₂SO₄), filtered, and concentrated *in vacuo.* The residue was purified by chromatography on SiO₂ (1:4, EtOAc/hexanes) to give crude **18c** that was further purified by preparative TLC (2:3, Et₂O/hexanes) to give **18c** (2.0 mg, 0.00506 mmol, 4%, 100% pure by ELSD) as a colorless oil: IR (neat) 2924, 1636, 1450, 1420, 1320, 1152 cm⁻¹; ¹H NMR (400 MHz, CDCl₃) δ 7.20 (t, 2 H, *J =* 7.7 Hz), 7.06-7.00 (m, 2 H), 3.87 (s, 2 H), 3.76 (s, 2 H), 3.58 (app t, 4 H, *J* = 4.8 Hz), 2.99 (app t, 4 H, *J* = 4.8 Hz), 2.44 (s, 3 H), 2.32 (s, 3 H), 2.31 (s, 3 H); ¹³C NMR (100 MHz, CDCl₃) δ 167.5, 159.7, 150.6, 132.7, 131.2, 126.8, 124.0, 119.4, 108.7, 51.8, 48.6, 47.0, 24.1, 17.8, 11.1, 10.2; HRMS (ESI) *m*/*z* calcd for C₁₈H₂₆O₃N₃S₂ ([M+H]⁺) 396.1416, found 396.1410.

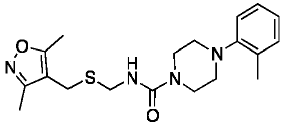

***N*-((((3,5-Dimethylisoxazol-4-yl)methyl)thio)methyl)-4-(*o*-tolyl)piperazine-1-carboxamide (20a).** To a solution of 2-(((3,5-dimethylisoxazol-4-yl)methyl)thio)acetic acid **(3a,** 0.0500 g, 0.248 mmol) in toluene (4.0 mL) was added DPPA (57 µL, 0.261 mmol) and Et₃N (37 µL, 0.261 mmol). The reaction mixture was heated at 110 °C for 60 min, cooled and washed with satd. aqueous NaHCO₃, dried (MgSO₄), filtered and concentrated to give the isocyanate **19** as a pink oil that was used without further purification.

A solution of 1-(*o*-tolyl)piperazine **(4b,** 0.460 g, 0.261 mmol) and Et₃N (37 µL, 0.261 mmol) in CH₂Cl₂ (0.5 mL) was cooled to 0 °C and treated with a solution of the isocyanate **19** in CH₂Cl₂ (0.5 mL). The reaction mixture was stirred overnight at room temperature, then diluted with EtOAc and satd. aqueous NH₄Cl. The organic layer was washed with satd. aqueous NaHCO₃ and brine, dried (Na₂SO₄), filtered, and concentrated *in vacuo.* The residue was purified by chromatography on SiO₂ (ISCO, 4 g column, gradient hexanes to 1:1, EtOAc/hexanes, with an initial base wash of the column using hexanes containing 1% Et₃N) to give **20a** (0.0606 g, 0.162 mmol, 65%, 98% pure by ELSD) as a clear oil that turns to a red oil upon standing: IR (CH₂Cl₂) 3336, 2941, 2891, 2850, 1629, 1523, 1491, 1495, 1420, 1254, 1223, 1193, 997, 907, 761, 731 cm⁻¹; ¹H NMR (300 MHz, CDCl₃) δ 7.18 (dd, 2 H, *J* = 8.7, 7.5 Hz), 7.04-6.98 (m, 2 H), 4.88 (brt, 1 H, *J =* 6.0 Hz), 4.44 (d, 2 H, *J* = 6.0 Hz), 3.67 (s, 2 H), 3.50 (app t, 4 H, *J =* 5.0 Hz), 2.89 (app t, 4 H, *J* = 5.0 Hz), 2.39 (s, 3 H), 2.32 (s, 3 H), 2.29 (s, 3 H); ¹³C NMR (75 MHz, CDCl₃) δ 166.0, 159.5, 156.9, 150.9, 132.7, 131.2, 126.7, 123.7, 119.1, 110.8, 51.6, 44.4, 43.9, 23.6, 17.8, 11.0, 10.2; HRMS (ESI) *m*/*z* calcd for C₁₉H₂₇N₄O₂S ([M+H]⁺) 375.1849, found 375.1845.

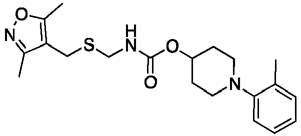

**1-(*o*-Tolyl)piperidin-4-yl((((3,5-dimethylisoxazol-4-yl)methyl)thio)methyl)-carbamate (20b).** To a solution of 2-(((3,5-dimethylisoxazol-4-yl)methyl)thio)acetic acid (3a, 0.0500 g, 0.248 mmol) in toluene (4.0 mL) was added DPPA (0.06 mL, 0.261 mmol) and Et₃N (37 µL, 0.261 mmol). The reaction mixture was heated at 110 °C for 60 min, cooled to room temperature and treated with a solution of 1-(o-tolyl)piperidin-4-ol **(4n,** 0.0427 g, 0.224 mmol) in CH₂Cl₂ (0.5 mL). The reaction mixture was stirred overnight at 80 °C, and diluted with EtOAc and satd. aqueous NH₄Cl. The organic layer was washed with satd. aqueous NaHCO₃ and brine, dried (Na₂SO₄), filtered, and concentrated *in vacuo.* The residue was purified by chromatography on SiO₂ (ISCO, 4 g column, gradient hexanes to 3:7, EtOAc/hexanes, with an initial base wash of the column with hexanes w/ 1% Et₃N) to give **20b** (0.0168 g, 0.0431 mmol, 17%, 100% pure by ELSD) as a clear oil that eventually turned to a light yellow oil upon standing: IR (CH₂Cl₂) 3323, 2947, 2924, 2848, 2811, 1711, 1491, 1450, 1422, 1228, 1195, 1027, 762, 723 cm⁻¹; ¹H NMR (400 MHz, DMSO-d6) δ 7.98 (t, 1 H, *J* = 6.4 Hz), 7.16-7.11 (m, 2 H), 7.02 (d, 1 H, *J* = 7.2 Hz), 6.94 (dt, 1 H, *J* = 7.2, 1.2 Hz), 4.72-4.69 (m, 1 H), 4.15 (d, 2 H, *J* = 6.4 Hz), 3.66 (s, 2 H), 3.01-2.98 (m, 2 H), 2.78-2.72 (m, 2 H), 2.36 (s, 3 H), 2.24 (s, 3 H), 2.18 (s, 3 H), 2.04-1.94 (m, 2 H), 1.77-1.67 (m, 2 H); ¹³C NMR (100 MHz, DMSO-d6) δ 165.7, 159.2, 155.5, 151.5, 131.8, 130.7, 126.5, 122.8, 118.9, 110.9, 69.9, 49.2, 42.9, 31.6, 21.9, 17.4, 10.5, 9.7; HRMS (ESI) *m*/*z* calcd for C₂₀H₂₈N₃O₃S ([M+H]⁺) 390.1846, found 390.1846.

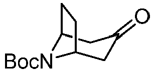

***tert*-Butyl 3-oxo-8-azabicyclo[3.2.1]octane-8-carboxylate (21a)** (WO 2012/152854 A1). A solution of nortropinone•HCl **(21,** 2.00 g, 12.4 mmol) in a minimum amount of water (6.0 mL) was cooled to 0 °C, treated dropwise with 1 M NaOH (14.8 mL, 14.8 mmol, 1.2 equiv), warmed to room temperature over 20 min, extracted with CH₂Cl₂ (3 x 40 mL), dried (MgSO₄), filtered, and concentrated *in vacuo* (water bath at 23 °C) to give nortropinone **21** as the free base (1.54 g, quant.). The colorless oil was used without further purification.

To a solution of nortropinone **21** (1.54 g, 12.3 mmol) in CH₂Cl₂ (50 mL) cooled to 0 °C was added Boc anhydride (4.26 mL, 18.6 mmol), DMAP (0.302 g, 2.47 mmol), and Et₃N (7.0 mL, 50.2 mmol). The reaction mixture was allowed to warm to room temperature and stirred overnight. After 19 h, the reaction mixture was concentrated *in vacuo,* and the residue was diluted with water, extracted with EtOAc (3x), washed with brine, dried (MgSO₄), filtered, and concentrated *in vacuo* to give a red sticky solid which was purified by chromatography on SiO₂ (CH₂Cl₂) to give **21a** (2.18 g, 9.68 mmol, 78% over two steps) as a pale yellow oil that solidified to an off-white solid upon standing at room temperature: ¹H NMR (300 MHz, DMSO-d6) δ 4.34-4.30 (m, 2 H), 2.55 (dt, 2 H, *J* = 15.6, 4.2 Hz), 2.23 (d, 2 H, *J* = 15.6 Hz), 2.20 (app s, 1 H), 2.03-1.94 (m, 2 H), 1.60-1.52 (m, 2 H), 1.44 (s, 9 H); ¹³C NMR (75 MHz, DMSO-d6) δ 207.4, 152.6, 79.2, 52.7, 48.1, 28.0 (2 C).

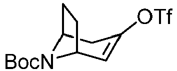

***tert*-Butyl 3-(((trifluoromethyl)sulfonyl)oxy)-8-azabicyclo[3.2.1]oct-2-ene-8-carboxylate (22)** (WO 2012/152854 A1). A solution of NaHMDS (0.895 g, 4.88 mmol) in THF (12 mL) was added dropwise (over 10 min) at -78 °C to a solution of *tert*-butyl 3-oxo-8-azabicyclo[3.2.1]octane-8-carboxylate **(21a,** 1.00 g, 4.44 mmol) in THF (12 mL). The reaction mixture was stirred at -78 °C for 2 h, treated dropwise (over 20 min) with a solution of PhN(Tf)₂ (1.90 g, 5.33 mmol) in THF (12 mL), stirred for an additional 30 min at -78 °C and then allowed to warm to room temperature and stirred for 2 h. After addition of 10% aqueous Na₂CO₃ (50 mL), the solution was extracted with Et₂O (2 x 75 mL). The combined organic layers were washed with 10% aqueous Na₂CO₃ solution, dried (MgSO₄), and concentrated *in vacuo.* The crude residue was purified by chromatography on SiO₂ (1:19, EtOAc/hexanes with 1% Et₃N) to give **22** (1.24 g, 3.47 mmol, 78%) as a clear oil that solidified to a wax upon storage at -20 °C: ¹H NMR (400 MHz, CDCl₃) δ 6.09 (brs, 1 H), 4.54-4.38 (m, 2 H), 3.07-3.02 (m, 1 H), 2.30-2.20 (m, 1 H), 2.11-1.99 (m, 3 H), 2.00-1.97 (m, 2 H), 1.79-1.70 (m, 1 H), 1.45 (s, 9 H).

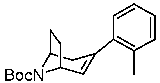

***tert*-Butyl 3-(*o*-tolyl)-8-azabicyclo[3.2.1]oct-2-ene-8-carboxylate (23a).** A solution of Na₂CO₃ (0.330 g, 3.11 mmol), lithium chloride (0.0600 g, 1.41 mmol), *tert*-butyl 3-(((trifluoromethyl)sulfonyl)oxy)-8-azabicyclo[3.2.1]oct-2-ene-8-carboxylate **(22,** 0.460 g, 1.41 mmol) and o-tolylboronic acid (0.235 g, 1.70 mmol) in DME (11 mL) and H₂O (3 mL) was sparged with N₂ for 1 h, and treated with Pd(PPh₃)₄ (0.0376 g, 0.0325 mmol). The flask was evacuated and backfilled with nitrogen (3x) and the mixture was heated at 60 °C for 3 h. The mixture was allowed to cool to room temperature, diluted with brine, extracted with EtOAc (3x), dried (Na₂SO₄), and concentrated *in vacuo.* The resulting brown oil was dry loaded onto SiO₂ and purified by chromatography on SiO₂ (hexanes to 15:1, hexanes/EtOAc) to give **23a** (0.330 g, 1.10 mmol, 78%) as a colorless solid: Mp 67.5-68.4 °C; IR (neat) 2975, 2934, 1685, 1420, 1364, 1329, 1169, 1094 cm⁻¹; ¹H NMR (400 MHz, CDCl₃, mixture of rotamers) δ 7.20-7.12 (m, 3 H), 7.02-7.00 (m, 1 H), 5.94-5.86 (m, 1 H), 4.50-4.30 (m, 2 H), 3.11-2.91 (m, 1 H), 2.27 (app s, 4 H), 2.10-1.90 (m, 3 H), 1.90-1.80 (m, 1 H), 1.50 (s, 9 H); ¹³C NMR (100 MHz, CDCl₃, 1:1 mixture of rotamers) δ 154.4, 141.6, 136.2, 135.5, 134.9, 131.3, 130.8, 130.7, 130.1, 129.3, 128.1, 126.9, 126.8, 125.6, 123.5, 120.0, 114.8, 79.3, 53.6, 52.9, 52.7, 52.0, 39.2, 38.4, 34.9, 34.3, 30.4, 29.6, 28.4, 19.5, 15.8; HRMS (ESI) *m*/*z* calcd for C₁₄H₁₇N ([M+H-C₅H₉O₂]⁺) 200.1439, found 200.1435.

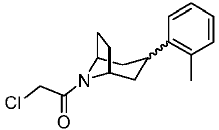

**2-Chloro-1-(3-(*o*-tolyl)-8-azabicyclo[3.2.1]octan-8-yl)ethan-1-one (24).** A solution of *tert*-butyl 3-(o-tolyl)-8-azabicyclo[3.2.1]oct-2-ene-8-carboxylate **(23a,** 0.196 g, 0.655 mmol) in EtOH (5.0 mL) was treated with Pd/C (5%, 0.0480 g). The flask was evacuated and flushed with H₂ (balloon, 3x). The reaction mixture was stirred under H₂ (1 atm, balloon) overnight, filtered through Celite, rinsed with EtOH and concentrated *in vacuo* to give **(23,** 0.160 g, 0.531 mmol, 81%) as a yellow liquid that was used without further purification.

A solution of **23** (0.200 g, 0.664 mmol) in CH₂Cl₂ (5 mL) was treated at room temperature with TFA (0.30 mL, 3.98 mmol). After 16 h, the solution was concentrated *in vacuo.* The oily residue was extracted with CH₂Cl₂, washed with satd. aqueous NaHCO₃ and brine, dried (Na₂SO₄), filtered, and concentrated *in vacuo* to give 3-(o-tolyl)-8-azabicyclo[3.2.1]octane **(23b,** 0.133 g, 0.661 quant) as a light yellow oil that was used without further purification.

A solution of **23b** (0.130 g, 0.646 mmol) and Et₃N (0.10 mL, 0.710 mmol) in THF (3 mL) was cooled to 0 °C and treated with chloroacetyl chloride (60 µL, 0.710 mmol) dropwise over 1 min. The reaction mixture was stirred at 0 °C for 1 h and then at room temperature for 20 h. The solution was filtered, concentrated *in vacuo* and the residue was dissolved in EtOAc, washed with water, dried (Na₂SO₄), filtered, and concentrated *in vacuo.* The crude residue was purified by chromatography on SiO₂ (1:1, hexanes/EtOAc) to give **24** (0.141 g, 0.508 mmol, 79%) as a brown oil. ¹H NMR analysis indicated an approximately 4:3 ratio of *endo*/*exo* isomers: ¹H NMR (400 MHz, CDCl₃) δ 7.22-7.09 (m, 6.8 H), 4.85-4.80 (m, 1 H), 4.80-4.74 (m, 0.7 H), 4.38-4.30 (m, 1.7 H), 4.14-4.04 (m, 3.6 H), 3.49-3.39 (m, 1 H), 2.99-2.88 (m, 0.7 H), 2.58-2.49 (m, 1 H), 2.38 (s, 3 H), 2.32 (s, 2 H), 2.22-1.70 (m, 11 H), 1.55-1.48 (m, 1 H); ¹³C NMR (100 MHz, CDCl₃) δ 163.4, 162.1, 141.8, 141.7, 135.9, 135.0, 130.4 (2 C), 126.5, 126.4, 126.2, 126.1 (2 C), 126.0, 55.7, 55.4, 52.6, 49.6, 41.5, 41.4, 39.5, 39.1, 37.9, 37.5, 32.8, 30.9, 30.3, 29.7, 28.9, 27.1, 19.4, 19.3; HRMS (ESI) *m*/*z* calcd for C₁₆H₂₁ClNO ([M+H]⁺), 298.1312, found 298.1301.

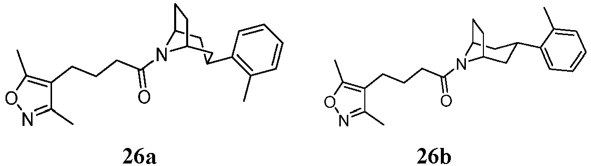

**2-(((3,5-Dimethylisoxazol-4-yl)methyl)thio)-1-(3-*endo*-(*o*-tolyl)-8-azabicyclo[3.2.1]octan-8-yl)ethanone (26a)** and **2-(((3,5-dimethylisoxazol-4-yl)methyl)thio)-1-(3-*exo*-(*o*-tolyl)-8-azabicyclo[3.2.1]octan-8-yl)ethanone (26b).** A solution of (3,5-dimethylisoxazol-4-yl)methanethiol (25, 0.0247 g, 0.172 mmol) in THF (0.4 mL) was added to a suspension of NaH (60% dispersion in mineral oil, 0.0115 g, 0. mmol) in THF (1.0 mL) at 0 °C. The resultant slurry was stirred at 0 °C for 30 min and a solution of 2-chloro-1-(3-(o-tolyl)-8-azabicyclo[3.2.1]octan-8-yl)ethanone **(24,** 0.0400 g, 0.144 mmol) in THF (0.4 mL) was added. The reaction mixture was allowed to warm to room temperature, stirred for 24 h, quenched with brine (1 mL), diluted with EtOAc (15 mL) and brine (5 mL), and extracted with EtOAc (2 x 15 mL). The combined organic layers were dried (Na₂SO₄) and concentrated *in vacuo.* The crude residue was purified by chromatography on SiO₂ (3:7, EtOAc/hexanes) to give **26a** (16.2 mg, 0.0421 mmol, 29%, 99.8% pure by ELSD) and **26b** (16.6 mg, 0.0432 mmol, 30%, 100% pure by ELSD) as light yellow oils.

**26a** *(dr* 82:18 by ¹HNMR): IR (neat) 2952, 2933, 1629, 1446, 1424, 1195 cm⁻¹; ¹H NMR (400 MHz, CDCl₃) δ 7.17-7.11 (m, 4 H), 4.81-4.80 (m, 1 H), 4.25-4.24 (m, 1 H), 3.72 (s, 2 H), 3.46-3.40 (m, 1 H), 3.19 (s, 2 H), 2.44 (brs, 4 H), 2.37 (s, 3 H), 2.31 (s, 3 H), 2.19-2.09 (m, 1 H), 2.08-1.84 (m, 5 H), 1.80-1.66 (m, 2 H); ¹³C NMR (100 MHz, CDCl₃) δ 166.8, 164.8, 159.8, 141.9, 135.1, 130.5, 126.5, 126.2, 126.0, 109.9, 55.8, 52.2, 39.2, 37.6, 32.5, 30.4, 28.9, 27.3, 23.8, 19.3, 11.0, 10.1; HRMS (ESI) *m*/*z* calcd for C₂₂H₂₉O₂N₂S ([M+H]⁺) 385.1950, found 385.1946.

**26b** (*dr* 92:8 by ¹HNMR): IR (neat) 2952, 2934, 1629, 1489, 1446, 1193, 1163 cm⁻¹; ¹H NMR (400 MHz, CDCl₃) δ 7.20-7.12 (m, 4 H), 4.76 (t, 1 H, J = 7.6 Hz), 4.20 (t, 1 H, J = 7.6 Hz), 3.80 (d, 1 H, J = 14.0 Hz), 3.62 (d, 1 H, J = 14.0 Hz), 3.20 (d, 1 H, J = 12.8 Hz), 3.10 (d, 1 H, J = 13.6 Hz), 3.01-2.90 (m, 1 H), 2.60-2.45 (m, 5 H), 2.40 (s, 6 H), 2.22-2.11 (m, 1 H), 2.10-2.00 (m, 1 H), 1.85-1.69 (m, 2 H), 1.55-1.40 (m, 2 H); ¹³C NMR (100 MHz, CDCl₃) δ 166.9, 166.3, 159.8, 142.0, 135.7, 130.4, 126.5, 126.2, 126.0, 109.9, 53.3, 49.3, 39.0, 38.0, 32.8, 31.9, 31.1, 29.9, 23.9, 19.5, 11.1, 10.2; HRMS (ESI) *m*/*z* calcd for C₂₂H₂₉O₂N₂S ([M+H]⁺) 385.1950, found 385.1944.

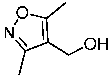

**(3,5-Dimethylisoxazol-4-yl)methanol (27)** (Natale et al., Synth. Commun. 1983, 13, 817-822.) To a solution of 3,5-dimethylisoxazole-4-carboxylic acid (1.60 g, 11.3 mmol) in THF (69 mL) at 0 °C was added dropwise a 2 M solution of LiAlH₄ in THF (5.6 mL, 11.2 mmol). The reaction mixture was allowed to warm to room temperature, stirred overnight, transferred to a 500-mL Erlenmeyer flask and treated with sodium sulfate decahydrate until the foaming subsided. Celite (2.3 g) was added and the slurry was filtered and washed with CH₂Cl₂ (75 mL). The filtrate was concentrated *in vacuo* to give **27** (1.14 g, 8.97 mmol, 79%) as a clear colorless oil: ¹H NMR (400 MHz, CDCl₃) δ 4.46 (s, 2 H), 2.38 (s, 3 H), 2.29 (s, 3 H).

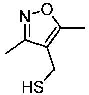

**(3,5-Dimethylisoxazol-4-yl)methanethiol (25)** (Moreno-Mañas et al., J. Heterocycl. Chem. 1992, 29, 1557-1560.) A solution of (3,5-dimethylisoxazol-4-yl)methanol **(27,** 0.500 g, 3.90 mmol) in toluene (13 mL) was treated with Lawesson's reagent (0.890 g, 2.15 mmol) at room temperature, heated to 80 °C and stirred for 1 d. The crude mixture was loaded directly onto SiO₂ and purified by chromatography on SiO₂ (4:1, hexanes/EtOAc) to give **25** (0.115 g, 0.803 mmol, 21%) as a light yellow oil: ¹H NMR (300 MHz, CDCl₃) δ 3.49 (d, 2 H, *J* = 6.6 Hz), 2.36 (s, 3 H), 2.30 (s, 3 H), 1.64 (t, 1 H, *J =* 6.6 Hz); ¹³C NMR (75 MHz, CDCl₃) δ 165.2, 159.0, 113.3, 15.9, 10.9, 10.0.

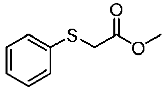

**Methyl 2-(phenylthio)acetate (28)** (Bahrami et al., J. Org. Chem. 2010, 75, 6208-6213.) A solution of thiophenol (0.10 mL, 0.977 mmol), and methyl bromoacetate (0.164 g, 1.07 mmol) in THF (13 mL) was treated with Et₃N (0.17 mL, 1.17 mmol), stirred at room temperature for 4 h, and diluted with Et₂O and satd. aqueous NaHCO₃. The aqueous layer was extracted with Et₂O (2 x 5 mL). The combined organic layers were dried (MgSO₄), filtered and concentrated *in vacuo* to give **28** (0.176 g, 0.966 mmol, 99%) as a clear oil: ¹H NMR (300 MHz, CDCl₃) δ 7.42-7.38 (m, 2 H), 7.33-7.20 (m, 3 H), 3.71 (s, 3 H), 3.65 (s, 2 H); ¹³C NMR (75 MHz, CDCl₃) δ 170.1, 134.9, 129.9, 129.0, 127.0, 52.5, 36.5.

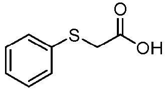

**2-(Phenylthio)acetic acid (3d)** (Bahrami et al., J. Org. Chem. 2010, 75, 6208-6213; Miura et al., Org. Lett. 2001, 3, 2591-2594.) To a solution of methyl 2-(phenylthio)acetate **(28,** 0.176 g, 0.966 mmol) in MeOH (2 mL) was added 2 M LiOH (1 mL). The reaction mixture was stirred at room temperature for 1 h and TLC analysis (4:1, hexanes/EtOAc) indicated that **28** had been consumed. The solution was concentrated *in vacuo,* diluted with water (3 mL) and acidified to pH 2 with 1 M HCl at 0 °C. The aqueous layer was extracted with EtOAc (3 x 10 mL). The combined organic layers were dried (MgSO₄), filtered and concentrated *in vacuo* to give **3d** (0.144 g, 0.857 mmol, 89%) as a colorless solid: ¹H NMR (300 MHz, CDCl₃) δ 11.27 (brs, 1 H), 7.43 (d, 2 H, *J =* 7.6 Hz), 7.36-7.24 (m, 3 H), 3.69 (s, 2 H); ¹³C NMR (75 MHz, CDCl₃) δ 175.9, 134.4, 130.1, 129.2, 127.2, 36.6.

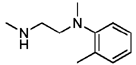

**N,N'-Dimethyl-N-(o-tolyl)ethane-1,2-diamine (4i)** (Gruseck et al., Tet. Lett. 1987, 28, 6027-6030.). A microwave vial was flushed with argon and charged with the N,N'-dimethylethylene-diamine (0.180 g, 2.04 mmol), NaO-t-Bu (0.202 g, 2.04 mmol), (rac)-BINAP (0.0162 g, 0.0260 mmol), Pd₂(dba)₃ (0.0078 g, 0.0085 mmol), degassed toluene (10.2 mL), and 2-bromotoluene (0.297 g, 1.70 mmol). The reaction mixture was heated in the sealed vial under argon at 110 °C for 24 h, cooled to room temperature, diluted with CH₂Cl₂, filtered through Celite, and concentrated *in vacuo.* The residue was purified by chromatography on basic Al₂O₃ (95:5, CH₂Cl₂/MeOH) to give **4i** (0.0508 g, 0.285 mmol, 17%) as a brown oil: ¹H NMR (400 MHz, CDCl₃) δ 7.16 (t, 2 H, *J* = 7.6 Hz), 7.08 (d, 1 H, *J* = 7.6 Hz), 6.98 (d, 1 H, *J* = 7.2 Hz), 3.05 (t, 2 H, *J* = 6.4 Hz), 2.71 (t, 2 H, *J* = 6.4 Hz), 2.65 (s, 3 H), 2.43 (s, 3 H), 2.32 (s, 3 H), 1.36 (brs, 1 H); HRMS (ESI) *m*/*z* calcd for C₁₁H₁₉N₂ ([M+H]⁺) 179.1543, found 179.1541.

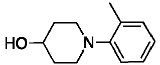

**1-(*o*-Tolyl)piperidin-4-ol (4n)** (Harris et al., Org. Lett. 2002, 4, 2885-2888.) An oven-dried microwave tube was charged with Pd₂(dba)₃ (0.0606 g, 0.0653 mmol), CyJohnphos (0.0292 g, 0.0816 mmol), and 4-piperidinol (0.330 g, 3.26 mmol). The microwave tube was evacuated and back-filled with argon. A 1 M solution of LiN(TMS)₂ (1.21 g, 7.17 mmol) in degassed THF (7.2 mL) was added via syringe along with 2-bromotoluene (0.600 g, 3.26 mmol). The reaction vessel was sealed and heated at 65 °C with stirring for 22 h. The reaction mixture was cooled to room temperature, quenched with 1 M HCl (10 mL), stirred at room temperature for 5 min, neutralized with a satd. aqueous NaHCO₃ solution, and diluted with EtOAc. The

organic layer was dried (MgSO₄), filtered through Celite, and concentrated *in vacuo.* The crude residue was purified by chromatography on SiO₂ (ISCO, 12 g column, gradient hexanes to 3:7, EtOAc/hexanes) to give **4n** (0.372 g, 1.94 mmol, 60%) as a brown oil: ¹H NMR (300 MHz, CDCl₃) δ 7.17 (dd, 2 H, *J* = 9.3, 7.2 Hz), 7.04-6.96 (m, 2 H), 3.87-3.81 (m, 1 H), 3.15-3.08 (m, 2 H), 2.74 (dt, 2 H, *J* = 9.6, 2.7 Hz), 2.32 (s, 3 H), 2.06-2.00 (m, 2 H), 1.80-1.69 (m, 3 H); ¹³C NMR (75 MHz, CDCl₃) δ 151.9, 132.7, 130.9, 126.4, 123.0, 119.0, 68.0, 49.8, 35.2, 17.7; HRMS (ESI) *m*/*z* calcd for C₁₂H₁₈NO ([M+H]⁺) 192.1383, found 192.1307.

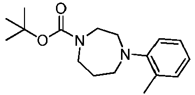

***tert*-Butyl 4-(*o*-tolyl)-1,4-diazepane-1-carboxylate (29a).** A microwave vial was flushed with argon and charged with Boc-homopiperazine (0.223 g, 1.10 mmol), NaO-*t*-Bu (0.116 g, 1.20 mmol), (rac)-BINAP (0.0478 g, 0.0752 mmol, 7.5 mol%), Pd₂(dba)₃ (0.0233 g, 0.0251 mmol), degassed toluene (2.8 mL), and 2-bromotoluene (0.175 g, 1.00 mmol). The reaction mixture was heated in the sealed vial under argon at 80 °C for 19 h, cooled to room temperature, diluted with CH₂Cl₂, filtered through Celite, and concentrated *in vacuo.* The residue was purified by chromatography on SiO₂ (1:9, EtOAc/hexanes) to give **29a** (0.139 g, 0.479 mmol, 48%) as a yellow oil: IR (ATR) 2973, 2828, 1689, 1598, 1491, 1457, 1411, 1364, 1233, 1215, 1156, 1122, 878, 761, 725 cm⁻¹; ¹H NMR (500 MHz, CDCl₃, room temperature, mixture of rotamers) δ 7.16 (d, 1 H, *J* = 6.0 Hz), 7.12 (d, 1 H, *J* = 6.0 Hz), 7.04 (d, 1 H, *J* = 7.5 Hz), 6.95 (t, 1 H, *J* = 7.0 Hz), 3.61-3.56 (m, 4 H), 3.11-3.04 (m, 4 H), 2.31 (s, 3 H), 1.96-1.91 (m, 2 H), 1.49 (s, 9 H); ¹³C NMR (100 MHz, CDCl₃, room temperature, mixture of rotamers) δ 155.6, 155.5, 153.9, 153.8, 132.9, 130.9, 126.5, 123.1, 120.8 (2 C), 79.3, 56.2, 56.0, 55.5, 55.2, 48.4, 48.0, 46.2, 45.4, 29.0, 28.9, 28.5, 18.5; HRMS (ESI) *m*/*z* calcd for C₁₇H₂₇N₂O₂ ([M+H]⁺) 291.2067, found 291.2062.

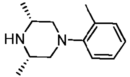

**(3*S*,5*R*)-3,5-Dimethyl-1-(*o*-tolyl)piperazine (4k)** (WO 2015/042297 A1). A Schlenk flask was flushed with N₂ and charged with *cis*-2,6-dimethylpiperazine (0.110 g, 0.963 mmol), NaO-*t*-Bu (0.170 g, 1.75 mmol), (*rac*)-BINAP (0.0084 g, 0.0130 mmol), Pd₂(dba)₃ (0.0083 g, 0.0087 mmol), degassed toluene (4 mL), and 2-bromotoluene (0.150 g, 0.880 mmol). The reaction mixture was heated under N₂ at 110 °C for 24 h, cooled to room temperature, diluted with CH₂Cl₂, filtered through Celite, and concentrated *in vacuo.* The crude residue was purified by chromatography on SiO₂ (1:19, MeOH/CH₂Cl₂) to give **4k** (0.140 g, 0.685 mmol, 78%) as clear, yellow oil: ¹H NMR (500 MHz, CDCl₃) δ 7.19-7.15 (m, 2 H), 7.02-6.98 (m, 2 H), 3.13-3.10 (m, 2 H), 3.01 (app d, 2 H, *J* = 10.5 Hz), 2.35-2.31 (m, 5 H), 1.12 (d, 6 H, *J =* 6.5 Hz).

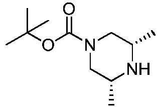

***tert*-Butyl (3*R*,5*S*)-3,5-dimethylpiperazine-1-carboxylate (30)** (Jacobsen et al., J. Med. Chem. 1999, 42, 1123-1144.) To a solution of *cis-*2,6-dimethylpiperazine (0.500 g, 4.38 mmol) in CH₂Cl₂ (11 mL) at 0 °C was added dropwise a solution of Boc-anhydride (0.946 g, 4.33 mmol) in CH₂Cl₂ (2.6 mL). The reaction mixture was allowed to warm to room temperature, stirred overnight, diluted with CH₂Cl₂ and washed with satd. aqueous Na₂CO₃ solution. The aqueous layer was extracted with CH₂Cl₂. The combined organic layers were washed with brine, dried (MgSO₄), filtered, and concentrated *in vacuo* to give 30 (0.813 g, 3.79 mmol, 87%) as an off-white solid: ¹H NMR (300 MHz, CDCl₃) δ 4.10-3.80 (m, 2 H), 2.85-2.70 (m, 2 H), 2.40-2.20 (m, 2 H), 1.46 (s, 9 H), 1.05 (d, 6 H, *J =* 6.3 Hz).

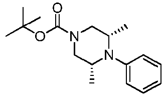

***tert*-Butyl (3*R*,5*S*)-3,5-dimethyl-4-phenylpiperazine-1-carboxylate (29b).** To a sealed tube under an argon atmosphere was added a solution of KHMDS (0.241 g, 1.15 mmol) in dry 1,4-dioxane (2.0 mL), a solution of *tert*-butyl 3,5-dimethylpiperazine-1-carboxylate **(30,** 0.246 g, 1.15 mmol) in dry 1,4-dioxane (0.9 mL) and bromobenzene (100 µL, 0.955 mmol). The reaction mixture was stirred at 100 °C for 18 h, cooled to room temperature, quenched with water (5 mL), diluted with Et₂O (15 mL) and the aqueous layer was extracted with Et₂O (2 × 15 mL). The combined organic layers were washed with brine, dried (Na₂SO₄), filtered, and concentrated *in vacuo.* The crude residue was purified by chromatography on SiO₂ (1:9, EtOAc/hexanes) to give **29b** (0.0970 g, 0.334 mmol, 35%) as a colorless oil: ¹H NMR (300 MHz, CDCl₃) δ 7.33-7.27 (m, 2 H), 7.15-7.09 (m, 3 H), 4.00-3.80 (m, 2 H), 3.07-3.03 (m, 2 H), 2.82 (brt, 2 H, *J* = 11.7 Hz), 1.50 (s, 9 H), 0.77 (d, 6 H, *J =* 6.3 Hz).

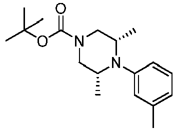

***tert*-Butyl 3,5-dimethyl-4-(m-tolyl)piperazine-1-carboxylate (29c).** A sealed tube under an argon atmosphere was treated with KHMDS (0.221 g, 1.05 mmol) in dry 1,4-dioxane (2.0 mL), a solution of*tert-*butyl 3,5-dimethylpiperazine-1-carboxylate **(30,** 0.226 g, 1.05 mmol) in dry 1,4-dioxane (0.7 mL) and bromotoluene (105 µL, 0.877 mmol). The reaction mixture was stirred at 100 °C for 18 h, cooled to room temperature, quenched with water (5 mL), diluted with Et₂O (15 mL) and the aqueous layer was extracted with Et₂O (2 × 15 mL). The combined organic layers were washed with brine, dried (Na₂SO₄), filtered, and concentrated *in vacuo.* The crude residue was purified by chromatography on SiO₂ (1:9, EtOAc/hexanes) to give 29c (0.0441 g, 0.145 mmol, 17%) as a colorless oil: ¹H NMR (300 MHz, CDCl₃) δ 7.18 (t, 1 H, *J* = 7.5 Hz), 6.96-6.89 (m, 3 H), 4.00-3.80 (m, 2 H), 3.06-3.00 (m, 2 H), 2.81 (brt, 2 *H, J =* 11.7 Hz), 2.32 (s, 3 H), 1.50 (s, 9 H), 0.77 (d, 6 H, *J =* 6.3 Hz).

### Example 2

### Synthesis and Characterization of (4-(5-Chloro-2-methylphenyl)piperazin-1-yl)((1RS,2SR)-2-(4-fluorophenyl)cyclopropyl)methanone (JJ-450)

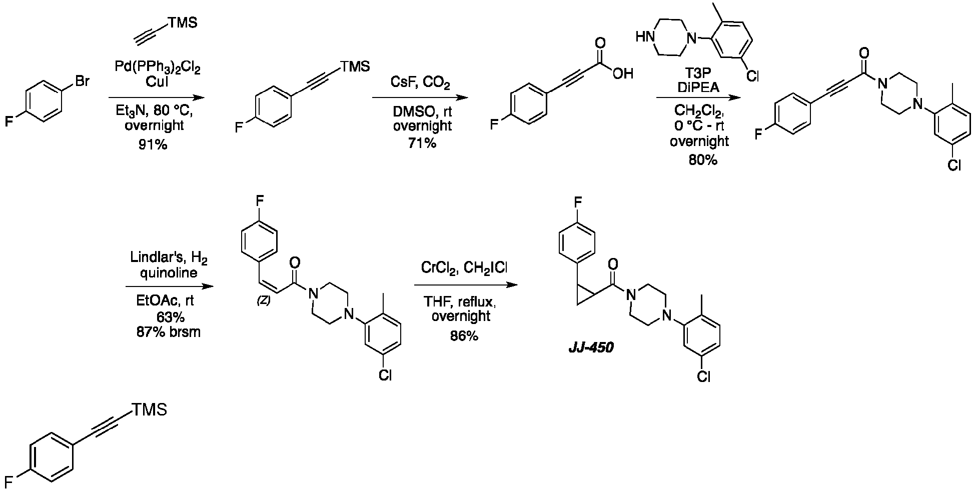

**((4-Fluorophenyl)ethynyl)trimethylsilane** (Everett et al., Org. Lett. 2013, 15, 2926-2929; Yonemoto-Kobayashi et al., Org. Biomol. Chem. 2013, 11, 3773-3775). A flame-dried flask under Ar was charged with Pd(PPh)₂Cl₂ (0.361 g, 0.514 mmol), CuI (0.0979 g, 0.514 mmol), and 4-fluorobromobenzene (5.66 mL, 51.4 mmol). Et₃N (110 mL) and (trimethylsilyl)acetylene (10.9 mL, 77.1 mmol) were added via syringe and the solution was sparged with Ar for 30 min. The reaction mixture was heated to 80 °C overnight and analysis by TLC (4: 1, hexanes/EtOAc) indicated that 4-fluorobromobenzene had been consumed. The solution was cooled to room temperature and filtered through celite. The celite was washed (Et₂O) until the washes appeared colorless. The combined filtrates were concentrated in vacuo. The crude residue was purified by chromatography on SiO₂ (hexanes) to afford 4-fluorophenyl)ethynyl)trimethylsilane (9.03 g, 47.0 mmol, 91%) as a pale orange oil: ¹H NMR (300 MHz, CDCl₃) δ 7.47-7.42 (m, 2 H), 6.99 (t, 2 H, *J =* 8.7 Hz), 0.25 (s, 9 H); ¹³C NMR (75 MHz, CDCl₃) δ 162.6 (d, *J _{C-F} =* 248 Hz), 133.9 (d, *J _{C-F} =* 8 Hz), 119.3 (d, *J_{C-F} =* 4 Hz), 115.5 (d, *J _{C-F} =* 22 Hz), 104.0, 93.8, -0.07.

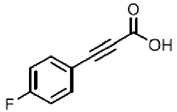

**3-(4-Fluorophenyl)propiolic acid** (Yonemoto-Kobayashi et al., Org. Biomol. Chem. 2013, 11, 3773-3775). CsF (4.74 g, 31.2 mmol) was loaded into an oven-dried 250-mL round bottom flask in a glovebox. The flask was removed from the glovebox, attached to a CO₂ balloon, equipped with a magnetic stirrer and a septum, and filled with anhydrous DMSO (60 mL). Neat ((4-fluorophenyl)ethynyl)trimethylsilane (5.00 g, 26.0 mmol) was added dropwise. The reaction mixture was stirred under CO₂ at room temperature overnight, diluted with water (600 mL) and washed with CH₂Cl₂ (2 × 150 mL). The aqueous layer was acidified at 0 °C to pH 1 with 6 M HCl and then extracted with Et₂O (3 × 200 mL). The combined organic layers were dried (MgSO₄), filtered, and concentrated in vacuo to afford 3-(4-fluorophenyl)propiolic acid (3.02 g, 18.4 mmol, 71%) as an orange solid: ¹H NMR (400 MHz, Acetone-d₆) δ 11.74 (brs, 1 H), 7.71 (dd, 2 H, *J =* 8.6, 5.6 Hz), 7.26 (t, 2 H, *J =* 8.6 Hz); ¹³C NMR (100 MHz, Acetone-d₆) δ 164.8 (d, *J*_{C-F} = 249 Hz), 154.7, 136.1 (d, *J*_{C-F} = 9 Hz), 117.1 (d, *J*_{C-F} = 23 Hz), 84.6, 81.8.

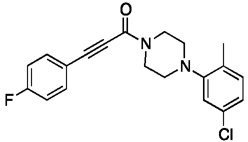

**1-(4-(5-Chloro-2-methylphenyl)piperazin-1-yl)-3-(4-fluorophenyl)prop-2-yn-1-one.** To a solution of 3-(4-fluorophenyl)propiolic acid (3.00 g, 18.3 mmol) in anhydrous CH₂Cl₂ (180 mL) at 0 °C was added 1-(5-chloro-2-methylphenyl)piperazine (4.62 g, 21.9 mmol), and Et₃N (6.35 mL, 45.7 mmol), followed by dropwise addition of T3P (50 wt.% solution in EtOAc, 19.4 mL, 27.4 mmol). The reaction mixture was stirred at 0 °C for 30 min, warmed to room temperature overnight, diluted with CH₂Cl₂ (200 mL), washed with 1 M HCl (150 mL), dried (MgSO₄), filtered, and concentrated in vacuo. The residue was purified by chromatography on SiO₂ (2:1, hexanes/EtOAc) to give 1-(4-(5-chloro-2-methylphenyl)piperazin-1-yl)-3-(4-fluorophenyl)prop-2-yn-1-one (5.22 g, 14.6 mmol, 80%) as an off white solid: Mp 138.7-140.4 °C; IR (neat) 2924, 2216, 1625, 1596, 1504, 1443, 1431, 1219, 1038, 837 cm⁻¹; ¹H NMR (300 MHz, CDCl₃) δ 7.55 (dd, 2 H, *J* = 7.5, 5.4 Hz), 7.12-6.94 (m, 5 H), 3.96 (app t, 2 H, *J =* 4.8 Hz), 3.82 (app t, 2 H, *J =* 4.8 Hz), 2.95 (app t, 2 H, *J* = 4.8 Hz), 2.87 (app t, 2 H, *J* = 4.8 Hz), 2.28 (s, 3 H); ¹³C NMR (75 MHz, CDCl₃) δ 163.5 (d, *J*_{C-F} *=* 251 Hz), 153.0, 151.7, 134.5 (d, *J*_{C-F} = 9 Hz), 132.1, 131.8, 130.9, 123.7, 119.8, 116.4 (d, *J*_{C-F} = 4 Hz), 116.0 (d, *J*_{C-F} = 23 Hz), 89.9, 80.9, 51.9, 51.3, 47.4, 41.8, 17.3; HRMS (ESI) *m*/*z* calcd for C₂₀H₁₉ClFON₂ ([M+H]⁺) 357.1164, found 357.1165.

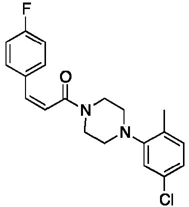

**(*Z*)-1-(4-(5-Chloro-2-methylphenyl)piperazin-1-yl)-3-(4-fluorophenyl)prop-2-en-1-one.** To a solution of 1-(4-(5-chloro-2-methylphenyl)piperazin-1-yl)-3-(4-fluorophenyl)prop-2-yn-1-one (5.00 g, 14.0 mmol) in dry EtOAc (140 mL) was added Lindlar's catalyst (5% Pd on CaCO₃, lead poisoned, 0.298 g, equivalent to 1 mol% Pd) and quinoline (0.83 mL, 7.01 mmol). The reaction vessel was placed under vacuum, backfilled with H₂ (balloon, 2x) and allowed to stir at room temperature for 6 h. Analysis by TLC (2: 1, hexanes/EtOAc) indicated that 1-(4-(5-chloro-2-methylphenyl)piperazin-1-yl)-3-(4-fluorophenyl)prop-2-yn-1-one had been mostly consumed. The reaction mixture was filtered through Celite, washed with EtOAc, and concentrated under vacuum. The combined organic layers were washed with 1 M HCl, dried (MgSO₄), filtered, and concentrated in vacuo. The crude material was purified by chromatography on SiO₂ (1: 1, hexanes/EtOAc) to afford (*Z*)-1-(4-(5-chloro-2-methylphenyl)piperazin-1-yl)-3-(4-fluorophenyl)prop-2-en-1-one (3.15 g, 8.78 mmol, 63%, 87% brsm) as a colorless solid: IR (neat) 2913, 2239, 1616, 1506, 1437, 1223, 837, 725 cm⁻¹; ¹H NMR (400 MHz, CDCl₃) δ 7.41-7.36 (m, 2 H), 7.08-7.02 (m, 3 H), 6.96 (dd, 1 H, *J* = 8.1, 2.1 Hz), 6.80 (d, 1 H, *J* = 2.1 Hz), 6.66 (d, 1 H *J =* 12.5 Hz), 6.05 (d, 1 H, *J* = 12.5 Hz), 3.80 (m, 2 H, *J* = 5.0 Hz), 3.49 (t, 2 H, *J* = 5.0 Hz), 2.80 (t, 2 H, *J* = 5.0 Hz), 2.53 (t, 2 H, *J* = 5.0 Hz), 2.21 (s, 3 H); ¹³C NMR (100 MHz, CDCl₃) δ 167.3, 162.7 (d, *J*_{C-F} = 248 Hz), 151.7, 132.6, 132.0, 131.8, 131.5 (d, *J*_{C-F} = 3 Hz), 132.1, 131.8, 130.9, 130.2 (d, *J*_{C-F} = 8 Hz), 123.6, 122.7, 119.6, 115.6 (d, *J*_{C-F} = 21 Hz), 51.4, 51.2, 46.5, 41.5, 17.3; HRMS (ESI) *m*/*z* calcd for C₂₀H₂₁ClFON₂ ([M+H]⁺) 359.1321, found 359.1329.

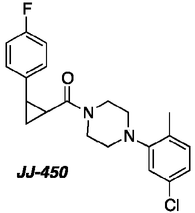

**(4-(5-Chloro-2-methylphenyl)piperazin-1-yl)((1*RS*,2*SR*)-2-(4-fluorophenyl)cyclopropyl)-methanone (JJ-450).** THF (90 mL) was degassed by sparging with Ar for 60 min and treated at room temperature under Ar atmosphere with anhydrous CrCl₂ (6.43 g, 51.8 mmol) followed by (Z)-1-(4-(5-chloro-2-methylphenyl)piperazin-1-yl)-3-(4-fluorophenyl)prop-2-en-1-one (3.10 g, 8.64 mmol) and CH₂ICl (3.36 mL, 43.2 mmol). The reaction mixture was stirred for 20 h at 80 °C, cooled to room temperature, quenched by the addition of 1.0 M aqueous HCl (300 mL) and extracted with EtOAc (3 x 300 mL). The combined organic layers were filtered through a plug of basic Al₂O₃, and concentrated in vacuo. The residue was purified by chromatography on SiO₂ (1: 1, hexanes/EtOAc) to afford an oil that was further purified twice by chromatography on basic Al₂O₃ (1: 1, hexanes/EtOAc) to give (4-(5-chloro-2-methylphenyl)piperazin-1-yl)((1*RS*,2*SR*)-2-(4-fluorophenyl)cyclopropyl)methanone (2.76 g, 7.41 mmol, 86%) as a clear oil that solidified after storage on high vacuum overnight: Mp 78.2-80.4 °C (hexanes); IR (CH₂Cl₂) 2936, 1637, 1592, 1510, 1487, 1435, 1223, 1033, 837, 815 cm⁻¹; ¹H NMR (400 MHz, CDCl₃) δ 7.16-7.11 (m, 2 H), 7.07 (dd, 1 H, *J* = 8.1, 0.5 Hz), 7.00-6.94 (m, 3 H), 6.73 (d, 1 H, *J* = 2.1 Hz), 3.81-3.76 (m, 1 H), 3.71-3.60 (m, 2 H), 3.36 (ddd, 1 H, *J =* 12.4, 8.8, 3.1 Hz), 2.79-2.71 (m, 2 H), 2.45 (td, 1 H, *J* = 8.8, 7.0 Hz), 2.35-2.29 (m, 1 H), 2.26-2.16 (m, 5 H), 1.83 (dt, 1 H, *J* = 7.0, 5.6 Hz), 1.35 (td, 1 H, *J =* 8.8, 5.6 Hz); ¹³C NMR (100 MHz, CDCl₃) δ 167.2, 161.7 (d, *J*_{C-F} = 244 Hz), 151.9, 133.1 (d, *J*_{C-F} = 3 Hz), 131.9 (d, *J*_{C-F} = 14 Hz), 130.9, 129.1 (d, *J*_{C-F} = 8 Hz), 123.6, 119.7, 115.0 (d, *J*_{C-F} = 21 Hz), 51.8, 51.6, 45.6, 42.2, 23.8, 23.5, 17.3, 10.7; HRMS (ESI) *m*/*z* calcd for C₂₁H₂₃ClFON₂ ([M+H]⁺) 373.1477, found 373.1478.

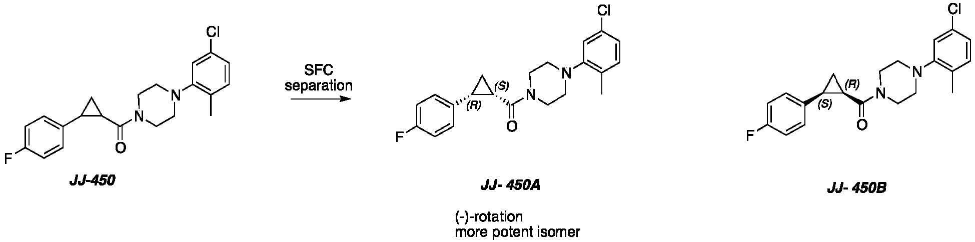

Racemic **JJ-450** was separated on a SFC Chiralpak-IC semiprep (250 x 10 mm) column (20% MeOH, 6 mL/min, 220 nM, P=100) to afford (4-(5-chloro-2-methylphenyl)piperazin-1-yl)((1*S*,2*R*)-2-(4-fluorophenyl)cyclopropyl)methanone **JJ-450A** (retention time 13.1 min) as a colorless viscous oil (100% purity by ELSD): [α]²⁰_{D} -118.7 (c 0.39, CHCl₃); ¹H NMR (300 MHz, CDCl₃) δ 7.17-7.10 (m, 2 H), 7.07 (d, 1 H, *J* = 8.1 Hz), 7.02-6.94 (m, 3 H), 6.72 (d, 1 H *J* = 2.1 Hz), 3.83-3.75 (m, 1 H), 3.72-3.58 (m, 2 H), 3.39-3.31 (m, 1 H), 2.81-2.69 (m, 2 H), 2.45 (td, 1 H, *J* = 8.7, 6.9 Hz), 2.36-2.25 (m, 1 H), 2.25-2.15 (m, 5 H), 1.83 (dt, 1 H, *J* = 6.9, 5.5 Hz), 1.35 (td, 1 H, *J* = 8.7, 5.5 Hz); HRMS (ESI) *m*/*z* calcd for C₂₁H₂₃ClFON₂ ([M+H]⁺) 373.1477, found 373.1476. The enantiomeric excess was 100% ee (SFC Chiralpak-IC (250 x 4.6 mm); 20% MeOH, 220 nM, 2 mL/min; retention time: 9.8 min).

(4-(5-Chloro-2-methylphenyl)piperazin-1-yl)((1*R*,2*S*)-2-(4-fluorophenyl)cyclopropyl)-methanone **JJ-450B** (retention time 16.5 min) was obtained as a colorless viscous oil (100% purity by ELSD): [α]²⁰_{D} +117.4 (c 0.38, CHCl₃); ¹H NMR (300 MHz, CDCl₃) δ 7.17-7.10 (m, 2 H), 7.07 (d, 1 H, *J* = 8.1Hz), 7.01-6.94 (m, 3 H), 6.72 (d, 1 H, *J* = 2.1 Hz), 3.82-3.74 (m, 1 H), 3.71-3.60 (m, 2 H), 3.39-3.30 (m, 1 H), 2.81-2.68 (m, 2 H), 2.45 (td, 1 H, *J* = 8.6, 7.0 Hz), 2.35-2.26 (m, 1 H), 2.25-2.15 (m, 5 H), 1.83 (dt, 1 H, *J* = 7.0, 5.6 Hz), 1.35 (td, 1 H, *J =* 8.6, 5.6 Hz); HRMS (ESI) *m*/*z* calcd for C₂₁H₂₃ClFON₂ ([M+H]⁺) 373.1477, found 373.1476. The enantiomeric excess was 100% ee (SFC Chiralpak-IC (250 x 4.6 mm); 20% MeOH, 220 nM, 2 mL/min; retention time: 12 min).

### Example 3

### Activity of Compounds in PSA Luciferase Assay

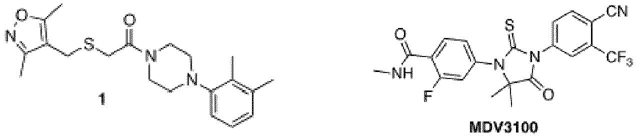

The biological activity of analogs **5-16, 18, 20, 26, JJ-450,** and the resolved enantiomers **JJ-450A** and **J-450B** was determined and compared to HTS hit 1 (IC₅₀ 7.3 µM) and MDV3100 (IC₅₀ 1.1 µM) using the Dual-Glo luciferase system (Promega, WI, USA) in C4-2-PSA-rl cells, which were generated by transfection with PSA6.1--luc and pRL-TK followed by stable selection using G418 and puromycin. C4-2-PSA-rl stable cells were cultured in RPMI 1640 medium with 10% FBS, 1% penicillin-streptomycin, 1% L-glutamine, 10 mg/mL puromycin, and 50 mg/mL G418. C4-2-PSA-rl cells were seeded in 24-well plates such that they reached 75-80% cell monolayer density after 24 h. C4-2-PSA-rl cells were then treated for 24 h with 0, 0.2, 0.8, 3.2, 12.8, or 25 µM of each compound dissolved in DMSO (0.8% DMSO/well) in the presence of 1 nM synthetic androgen R1881, with each experimental condition in triplicate. The cells were also treated in parallel with 12.8 µM compound 1 and 12.8 µM MDV3100 as positive controls. Each compound was tested in at least two independent experiments. Luciferase activity was assayed using the Dual-Luciferase^{®} Reporter Assay System (Promega) using LMax II Microplate Reader (Molecular Devices). The luciferase assay results were acquired using SoftMax Pro5.45 software (Molecular Devices) and analyzed using GraphPad Prism. PSA6.1-luc activity was normalized to the Renilla luciferase activity. Relative luciferase activity was calculated as the quotient of androgen-induced PSA-firefly/Renilla luciferase activity. Since PSA promoter activity correlates to AR transcriptional activity, inhibition of AR will result in decreased PSA-luciferase activity. IC₅₀ values were calculated using GraphPad Prism and data represent the mean and SD of 2-6 independent experiments (Table 2).

**Table 2. In vitro activity of analogs in the PSA luciferase assay in C4-2-PSA-rl cells.**

| **Entry** | **Compound** | **IC₅₀ (µM)** | **Entry** | **Compound** | **IC₅₀ (µM)** |
|---|---|---|---|---|---|
| 1 | **1** | 7.3±2.5^{c} | 19 | **10** | 20.3±11.6^{a} |
| 2 | **5a** | >25^{a} | 20 | **11** | >25^{a} |
| 3 | **5b** | 14.5±3.2^{b} | 21 | **12** | >25^{b} |
| 4 | **5c** | >25^{a} | 22 | **13** | 16.1±3.3^{b} |
| 5 | **5d** | >25^{a} | 23 | **14** | 12.7±0.8^{a} |
| 6 | **5e** | 12.0±1.6^{b} | 24 | **15** | 2.9±1.0^{b} |
| 7 | **5f** | 12.6±7.7^{b} | 25 | **16** | >25^{b} |
| 8 | **5g** | 11.1±5.3^{b} | 26 | **18a** | >25^{b} |
| 9 | **5h** | >25^{a} | 27 | **18b** | >25^{b} |
| 10 | **5i** | 18.4±9.2^{b} | 28 | **18c** | 7.2±2.7^{c} |
| 11 | **5j** | 11.1±3.3^{a} | 29 | **20a** | >25^{a} |
| 12 | **5k** | 3.1±1.1^{a} | 30 | **20b** | >25^{c} |
| 13 | **5l** | 14.7±4.4^{a} | 31 | **26a** | 7.7±1.6^{b} |
| 14 | **5m** | 16.6±4.8^{b} | 32 | **26b** | 7.9±2.8^{a} |
| 15 | **6** | 10.8±5.7^{b} | 33 | **JJ-450** | 2.7±1.1 |
| 16 | **7** | 13.7±0.8^{b} | 34 | **JJ-450A** | 1.6±0.1 |
| 17 | **8** | 14.4±3.7^{b} | 35 | **JJ-450B** | 13.1±1.8 |
| 18 | **9** | >25^{a} | 36 | **MDV3100** | 1.1±0.5^{e} |

| | | | | | |
|---|---|---|---|---|---|
| Assay repeats: ^{a}n=2; ^{b}n=3; ^{c}n=4; ^{d}n=5; ^{e}n=6. | | | | | |

Modifications of the substituents on the benzene ring in zone 1 revealed that methyl groups in the 3- and 4-positions **(5c, 5d)** led to loss of activity, while the 2-methyl analog **5b** (IC₅₀ 14.5 µM) retained about half of the activity of the 2,3-dimethylated 1 (Table 2). Removal of the 2-methyl group in **5a** deleted activity. In agreement with this trend in zone 1, the bulky 1-naphthyl substituent **(5g)** recovered activity (IC₅₀ 11.1 µM). Analogs with electron-withdrawing substituents at the benzene 2-position (2-NC, **5e**, and 2-F, 5f) also maintained or slightly increased activity (IC₅₀ 12-13 µM); however, the electron-donating 2-methoxy substituted **5h** was not tolerated and resulted in a complete loss of activity, possibly due to an increase in the pKa of the aniline and/or an unfavorable increase in the π-electron density of the aromatic ring.

The piperazine core (zone 2) was queried through substitutions with flexible as well as constrained acyclic and cyclic diamines. The flexible *N*,*N*'-dimethylethylenediamine linker in **5i** (IC₅₀ 18.4 µM) and the 7-membered diazepane **5j** (IC₅₀ 11.1 µM) both conserved activity. The dimethylated piperazines **5l** and **5m** (IC₅₀ 15-17 µM) were both also almost as active as the initial hit. In contrast, the conformationally more highly constraint 2,6-dimethylpiperazine **5k** was considerably more active with an IC₅₀ of 3.1 µM. Installment of an ethylene bridge and a carbon-linked (2-Me)Ph group decreased activity again, since both diastereomers of the bicyclo[3.2.1] ring systems **26a** and **26b,** showed an IC₅₀ of 8 µM.

Reduction of amide **5b** to amine 6 resulted in a 1.3-fold increase in activity to an IC₅₀ of 10.8 µM. Sulfonamide **18c** (IC₅₀ 7.2 µM) was twice as active as the initial hit **1,** but urea **20a** and carbamate **20b** were inactive.

The replacement of the thioether linkage in zone 2 with an ether group abolished activity in **18a.** Substituting the thioether with the *N*-methylated amine in **18b** also abolished activity. In contrast, in an analogous system with a phenyl group in place of the isoxazole, both thioether 7 as well as the all-carbon chain containing **8** showed constant activity (IC₅₀ ~14 µM).

In order to verify that the biological effect in the thioether series was not a result of *S*-oxidation in the cellular assay, common products of thioether oxidation, i.e. sulfoxide **12** and sulfone **13,** were tested. While sulfone **13** retained some activity (IC₅₀ 16.1 µM), sulfoxide **12** was inactive. Shortening the three-atom chain to afford the two-atom thioether-linked **9** also abolished activity. The rigidified alkyne **10** and the corresponding (*E*)-alkene **11** and its cyclopropane isostere **16** were also found to be essentially inactive. In contrast, the (*Z*)-alkene **14** surprisingly showed an IC₅₀ of 12.7 µM, and the corresponding cis-fused cyclopropane isostere **15** was even more potent than analog **1,** showing an IC₅₀ of 2.9 µM (Table 2).

In summary, zone 1 modifications showed that the ortho-substituent on the phenyl ring was important for activity. In zone 2, the sterically encumbered 2,6-dimethylpiperazine proved superior to flexible, unsubstituted, and bridged analogs. In zone 3, a carbonyl group was not required, and a sulfonamide and even the reduced amine were well tolerated. In zone 4, thioether oxidation reduces activity, and only the cis-cyclopropane successfully and significantly improves the IC₅₀. Limited substitutions were performed in zone 5, but in general analogs with a phenyl group were equipotent with their 3,5-dimethylisoxazole congeners (see, for example, **7** vs **5b).** Compounds **5k, 15,** and **JJ-450** (particularly **JJ-450A)** were found to be significantly more potent than **1.** Compounds **15** and **JJ-450** are of particular interest due to the isosteric replacement of the thioether linker with the metabolically more stable cyclopropane.

Compounds 559, 562, 475, 476, 484, and 458 are all also active in the PSA luciferase assay at sub-micromolar EC50s (450-900 nM), and they are inactive against androgen receptor (AR) negative cell lines in cell proliferation assays.

Additional compounds are shown below:

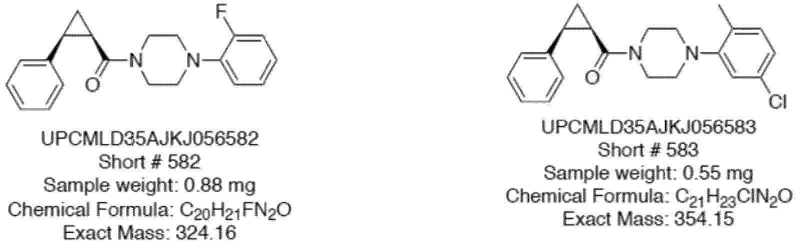

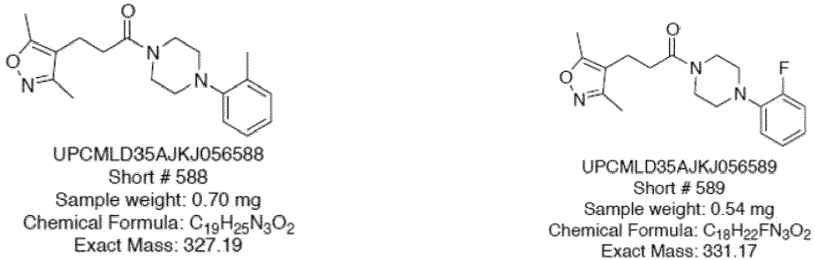

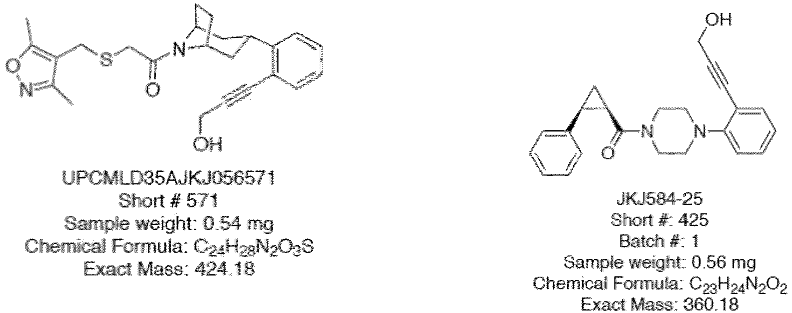

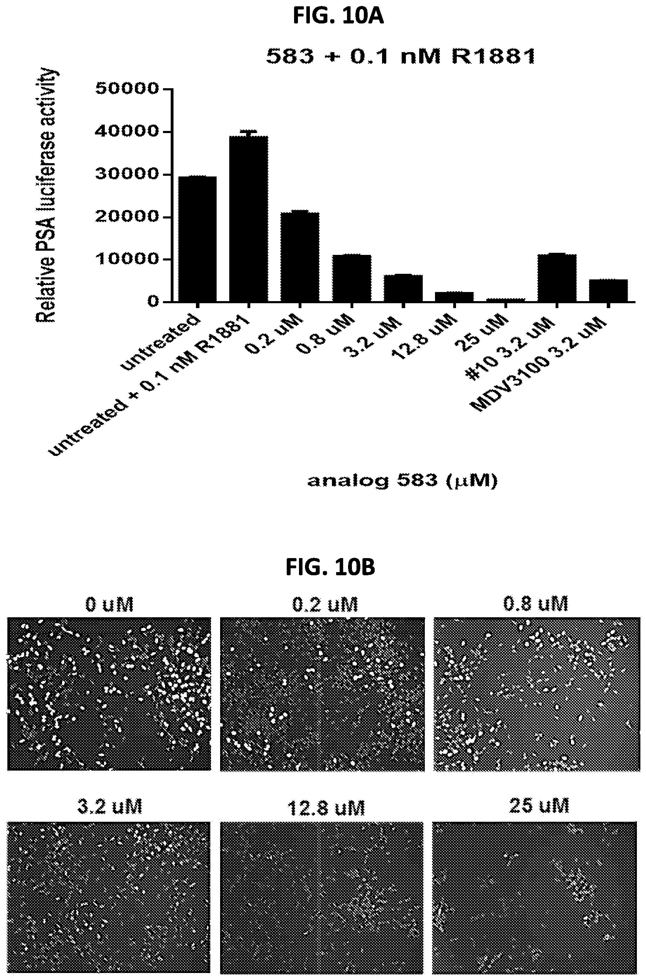

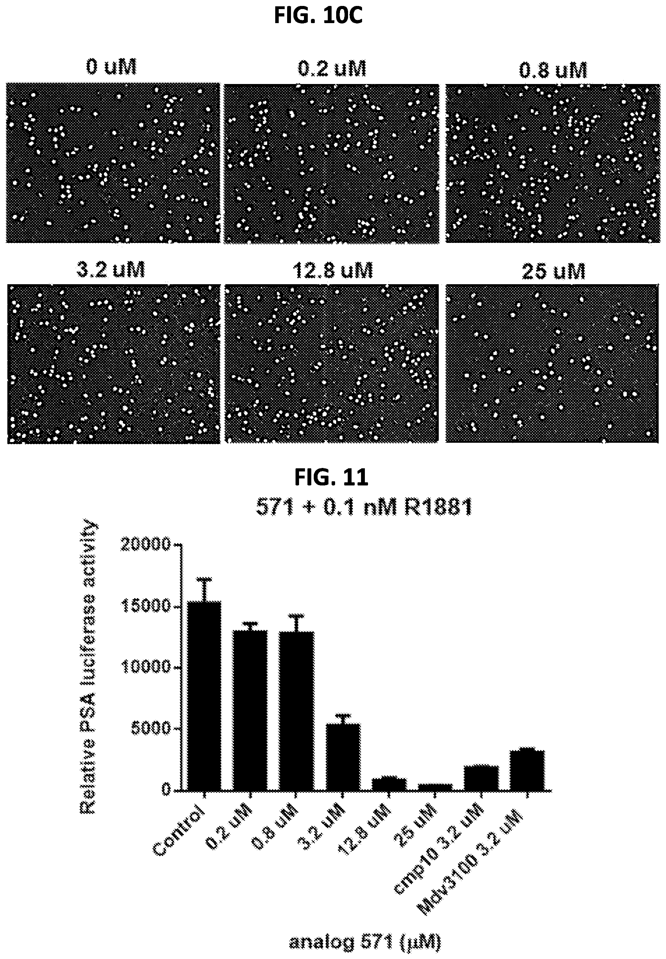

Compound #583 is very potent, with an IC₅₀ >1 uM in inhibiting AR-dependent PSA promoter activity (Fig. 10A). As expected, #583 inhibited proliferation of AR-positive C4-2 (Fig. 10B), but not AR-negative PC3 (Fig. 10C), prostate cancer cells. Also, #583 does not contain a sulfur atom in the structure and should therefore be more resistant to oxidative metabolic degradation than the sulfur-containing compounds.

Compounds #571 and #425 were developed for conjugation to agarose matrix. #571 is quite active, with an IC50 of ~3 uM in the inhibition of AR activation of PSA promoter in a luciferase assay (Fig. 11).

### Example 4

### Inhibition of Xenograft Tumor Growth by JJ-450

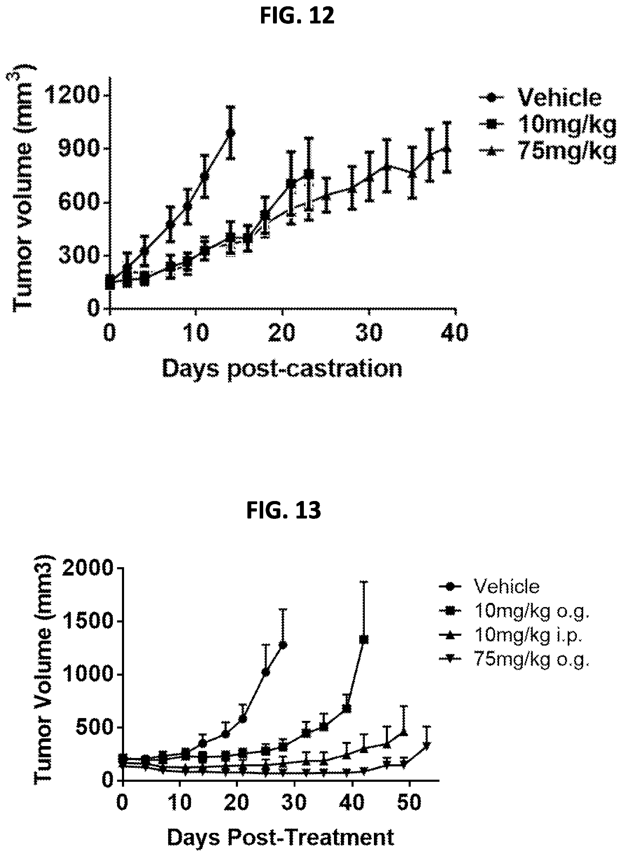

22Rv1 xenograft tumors were established in SCID mice by subcutaneous injection of 2x10⁶ cells in Matrigel. Once the tumors reached ~150 µL in volume, the mice were castrated and randomized into 3 groups for daily IP injection of vehicle (n=11), 10 mg/kg (n=11) and 75 mg/kg (n=11) groups. Injection of JJ-450 was initiated at time of castration. Tumor volumes were measured 3 times every week. As shown in Fig. 12, compound JJ-450 significantly inhibited tumor growth. Error bars, SEM.

LNCaP xenograft tumors were established in SCID mice by subcutaneous injection of 2x10⁶ cells in Matrigel. Once the tumors reached ~200 ul in volume, the mice were castrated and randomized into 4 groups: oral gavage of vehicle (n=6), oral gavage at 10 mg/kg (n=6), IP injection at 10 mg/kg (n=8), and oral gavage at 75 mg/kg (n=7) groups. Administration of JJ-450 was started 2 weeks after castration. Tumor volumes were measured twice every week. As shown in Fig. 13, compound JJ-450 significantly inhibited tumor growth. Error bars, SEM.

### Example 5

### Synthesis and Characterization of Additional Analogs

### General:

All glassware was flame-dried or dried in an oven at 120 °C for more than two hours prior to use. All air- and moisture-sensitive reactions were performed under N₂ or Ar atmosphere. Reactions carried out at 0 °C or -78 °C employed an ice bath or an acetone/dry ice bath. Tetrahydrofuran and diethyl ether were either distilled over sodium/benzophenone ketyl, CH₂Cl₂ and toluene were distilled from CaH₂. All other materials were obtained from commercial sources and used as received. Infrared spectra were determined neat on a Smiths Detection Identify IR FT-IR spectrometer. ¹H and ¹³C NMR spectra were obtained on a Bruker Advance 300 MHz, 400 MHz or 500 MHz NMR in CDCl₃ unless otherwise specified. Chemical shifts (δ) were reported in parts per million, with the residual solvent peak used as an internal standard δ ¹H/¹³C (Solvent); 7.26/77.00 (CDCl₃), 2.50/39.50 (DMSO-d6); they are tabulated as follows: chemical shift, multiplicity (s = singlet, brs = broad singlet, d = doublet, brd = broad doublet, t = triplet, brt = broad triplet, q = quartet, m = multiplet), number of protons, and coupling constant(s). ¹³C NMR spectra were obtained at 75 MHz, 100 MHz or 125 MHz unless otherwise specified using a proton-decoupled pulse sequence and are tabulated by observed peak. ¹⁹F spectra were obtained at 471MHz or 376 MHz unless otherwise specified using a proton-decoupled pulse sequence and are tabulated by observed peak. Reactions were monitored by thin-layer chromatography analysis using pre-coated silica gel 60 F254 plates (EMD, 250 µm thickness), and visualization was accomplished with a 254 nm UV light. Flash chromatography was performed using SiO₂ (Silicycle, Silia-P Flash Silica Gel, 40-63 µm).

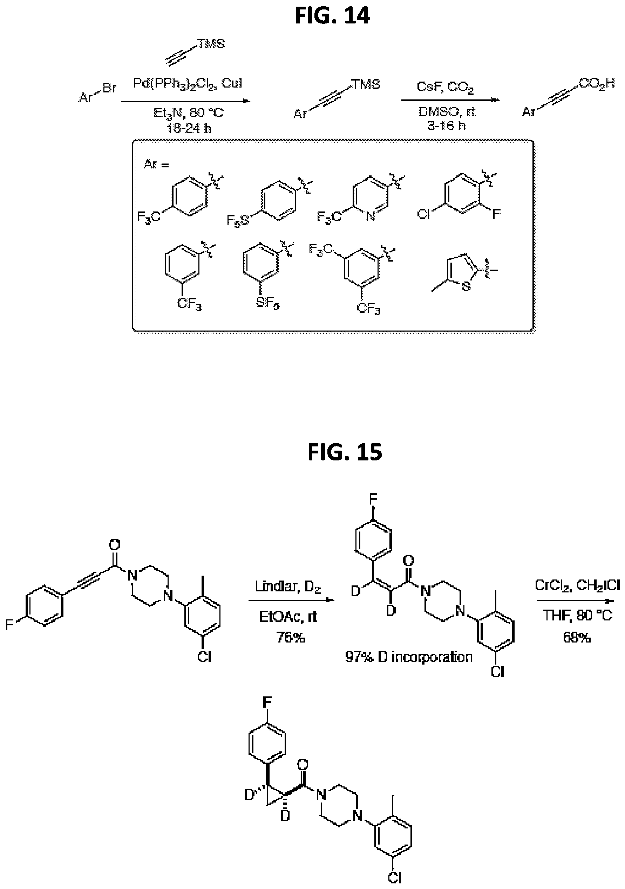

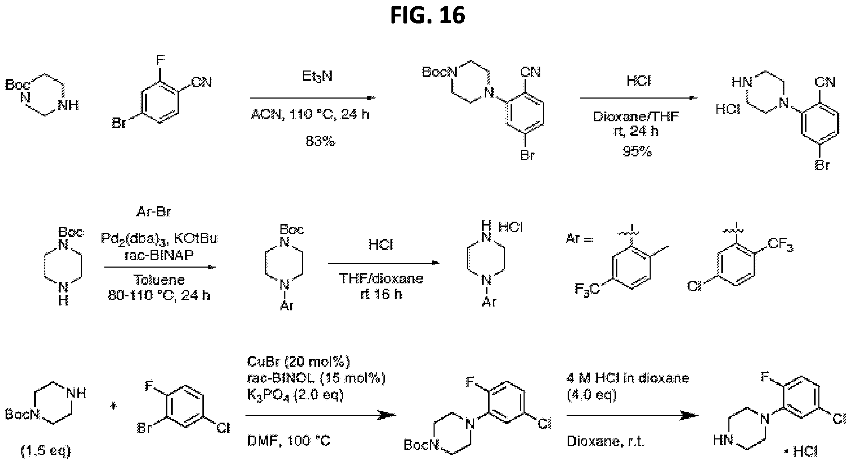

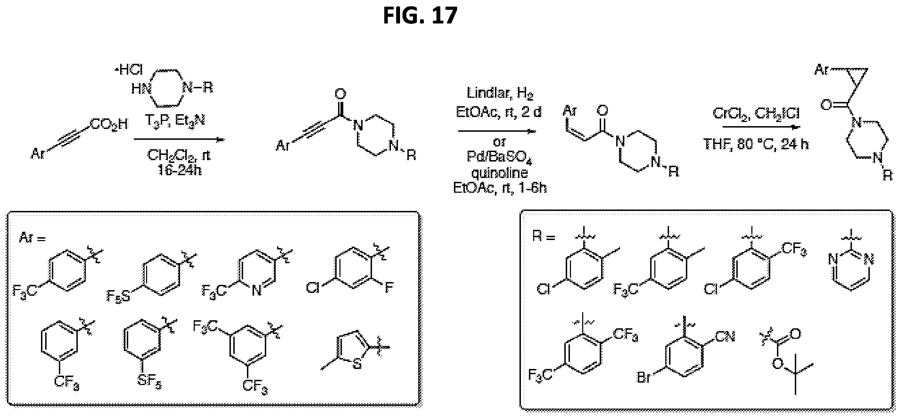

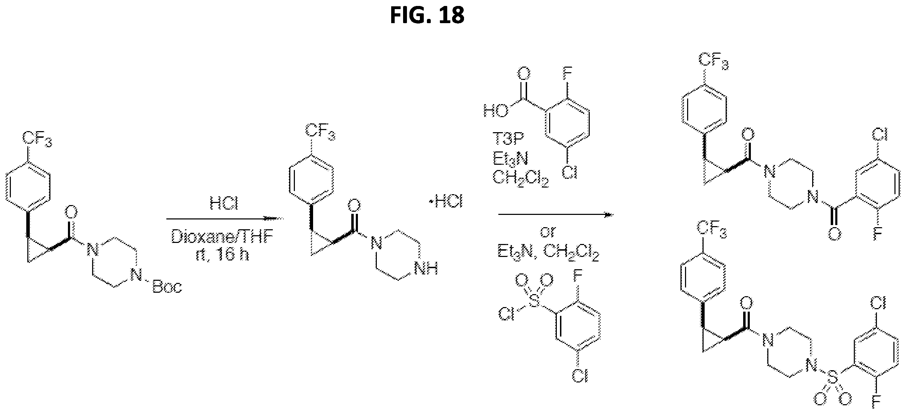

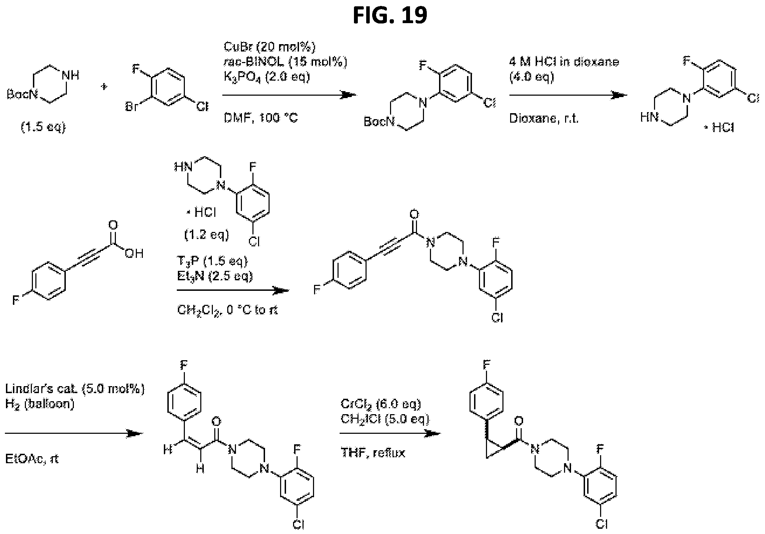

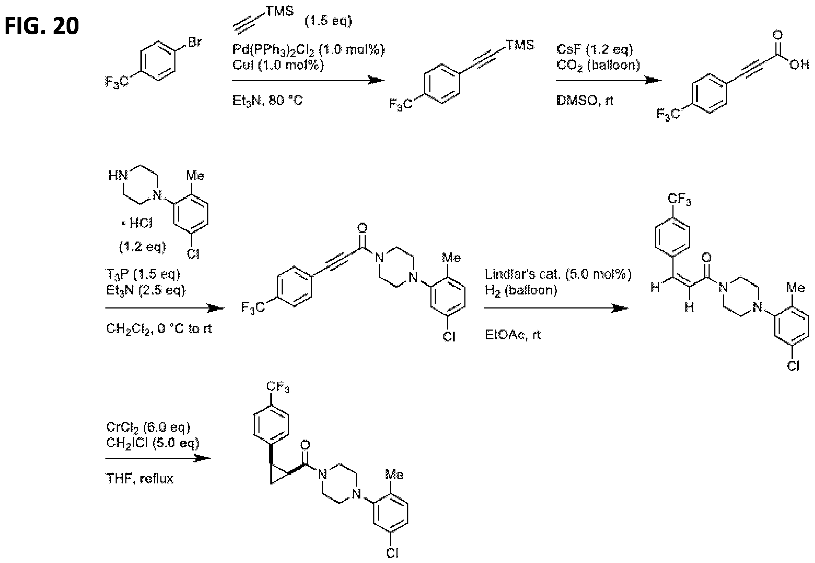

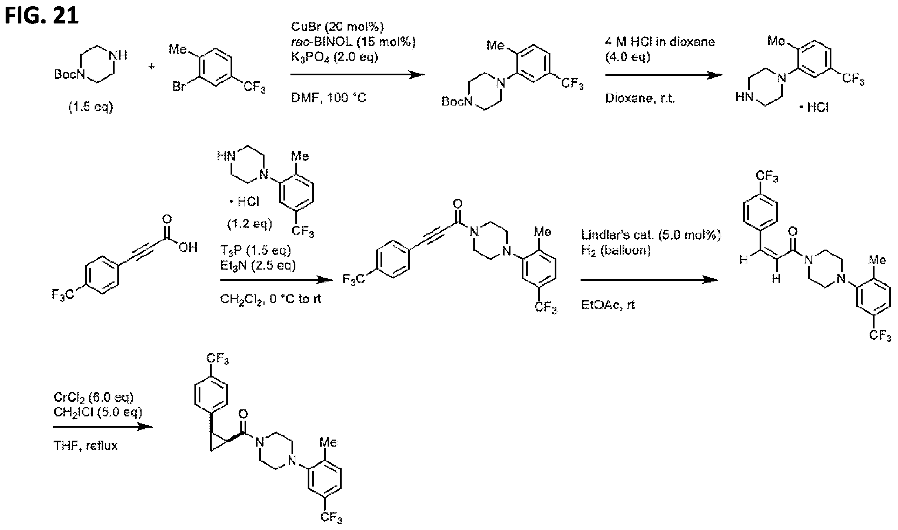

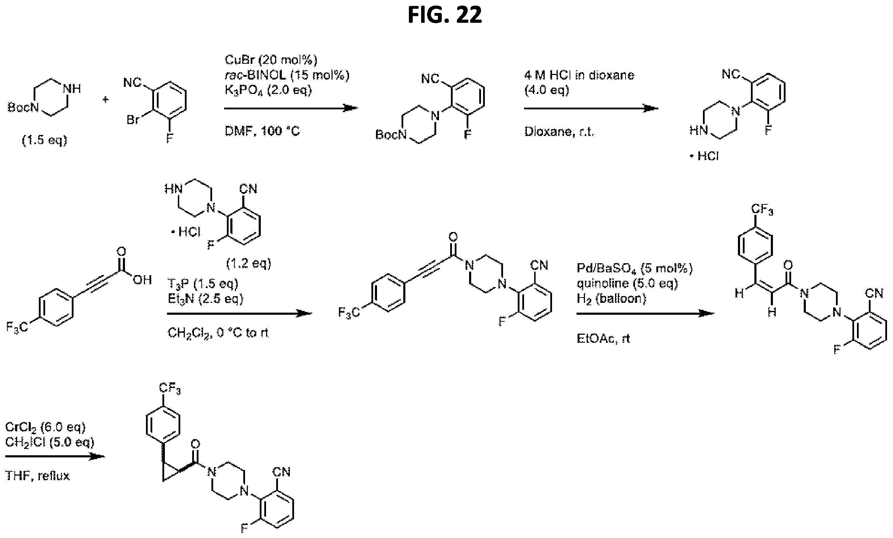

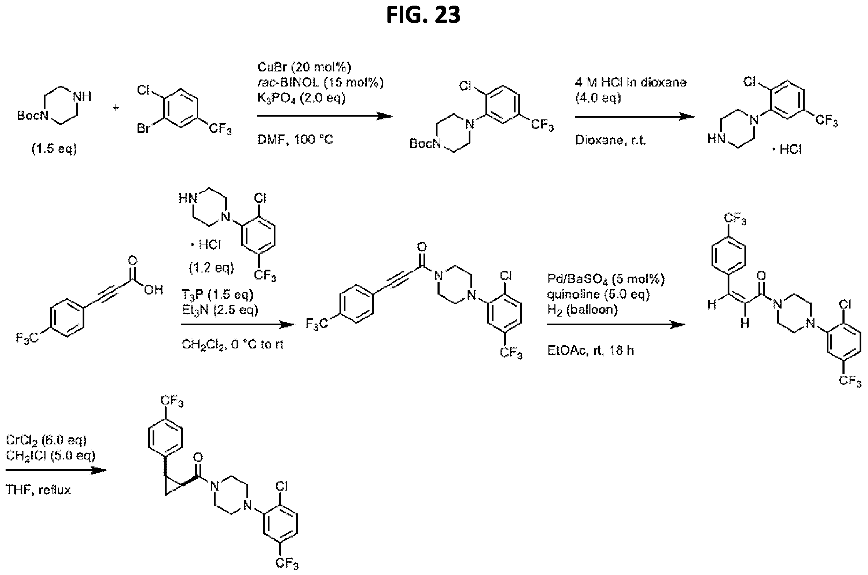

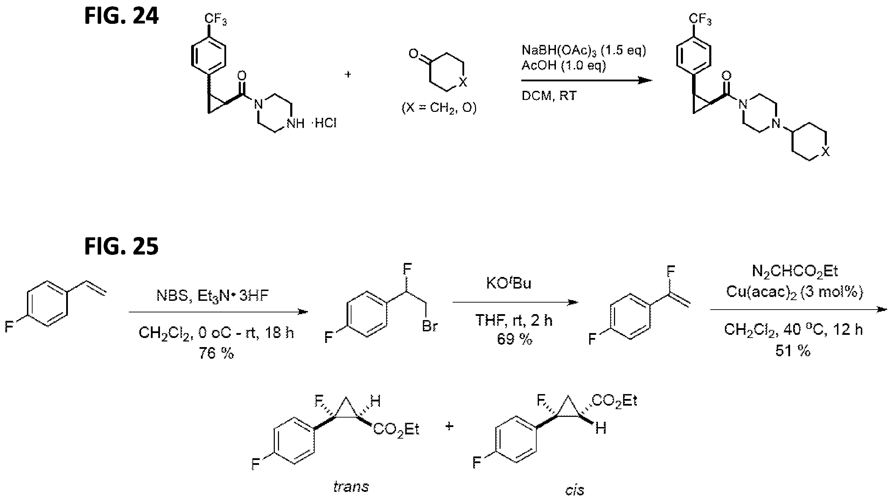

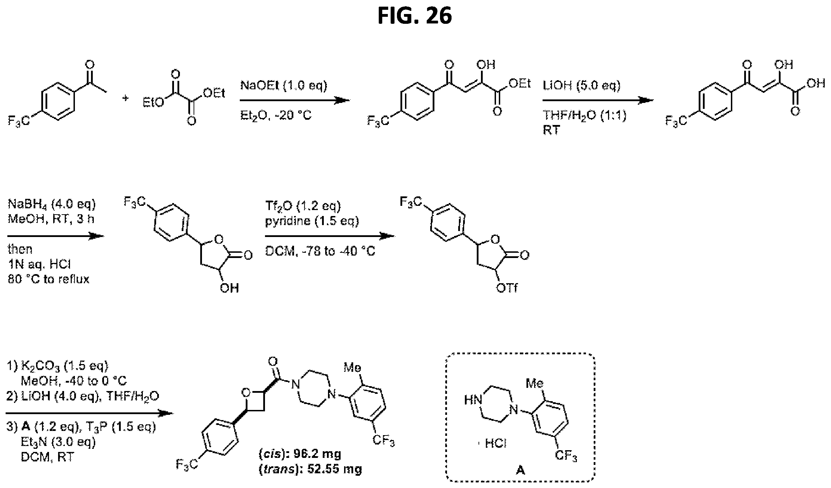

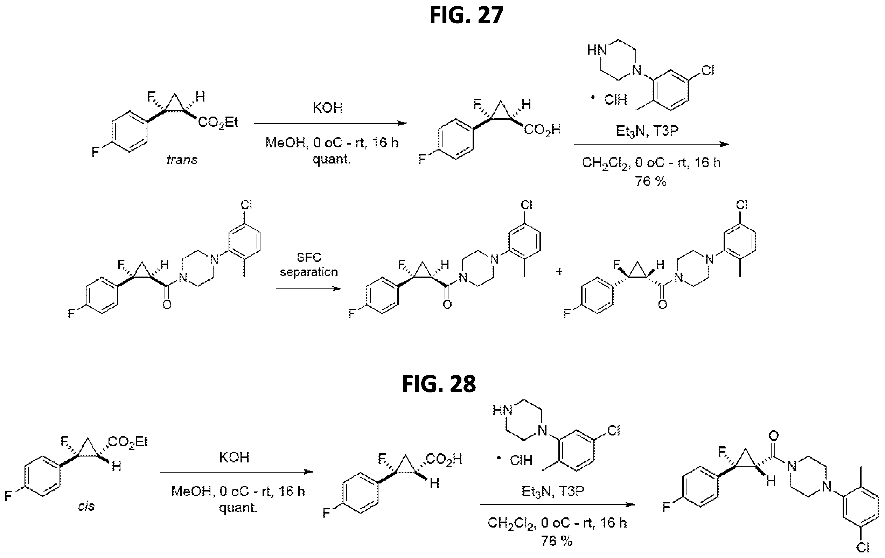

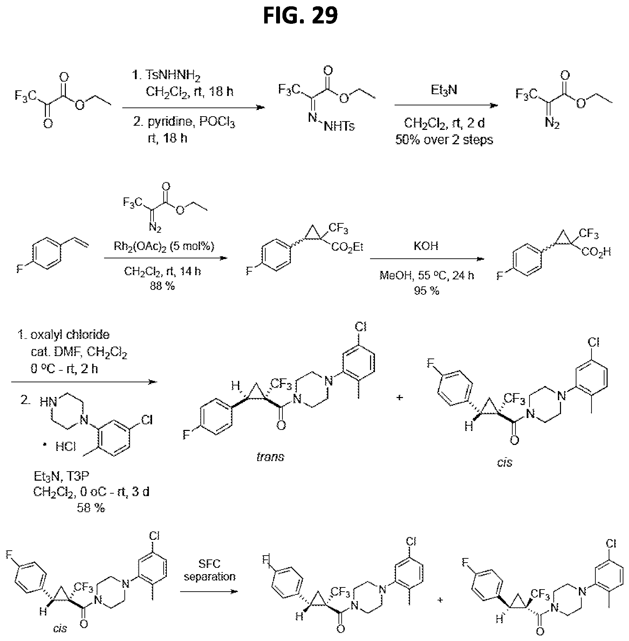

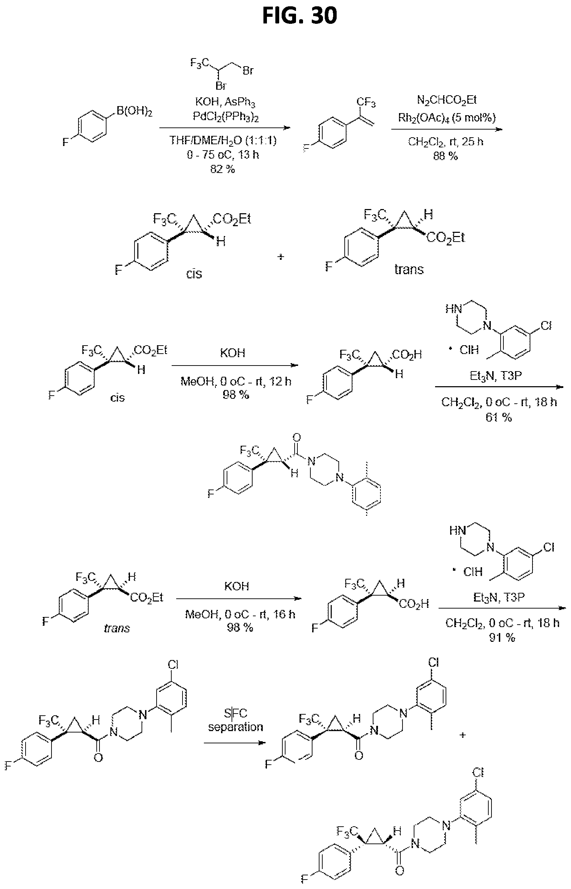

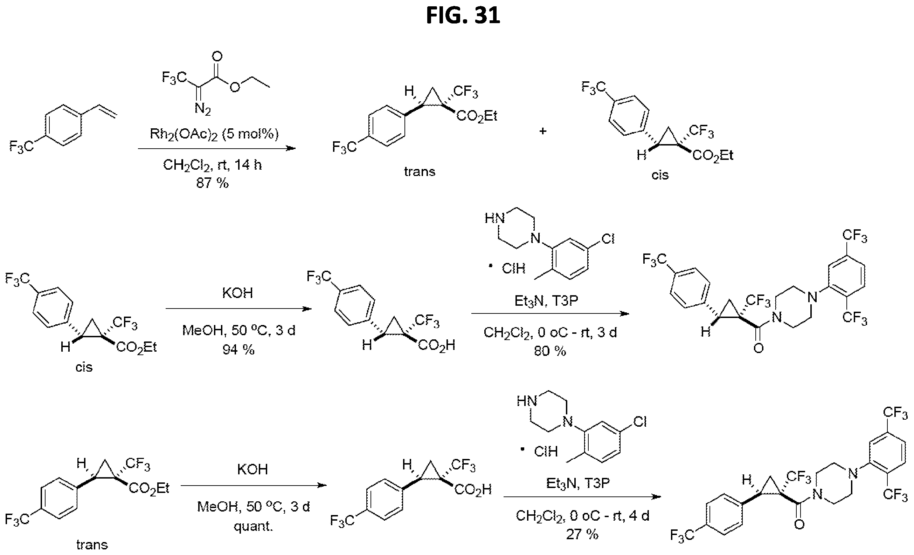

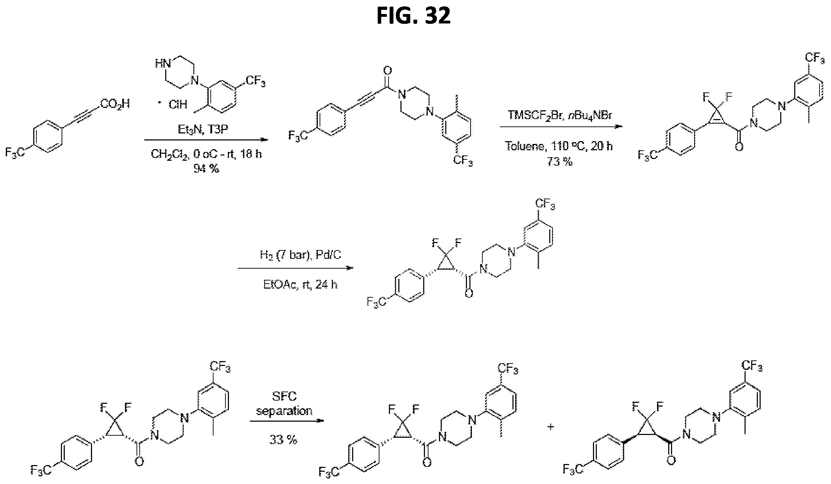

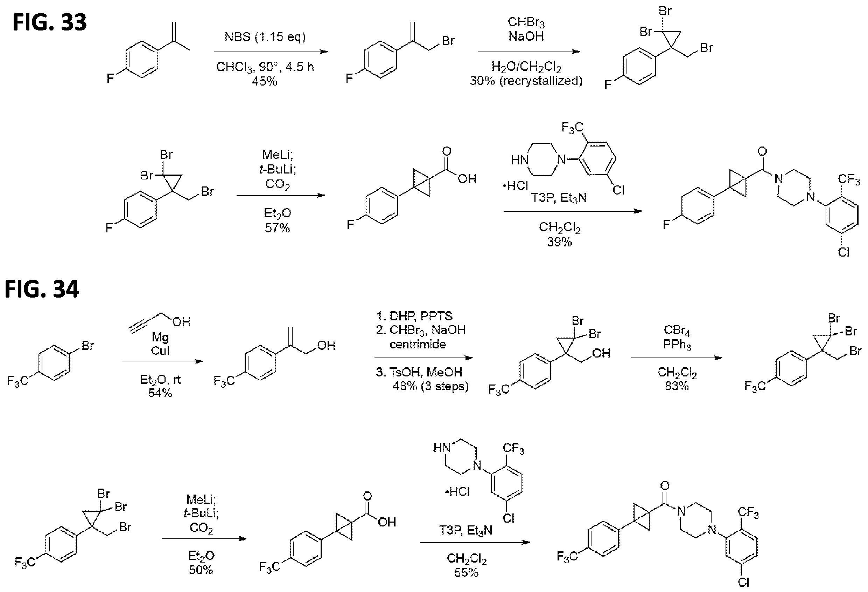

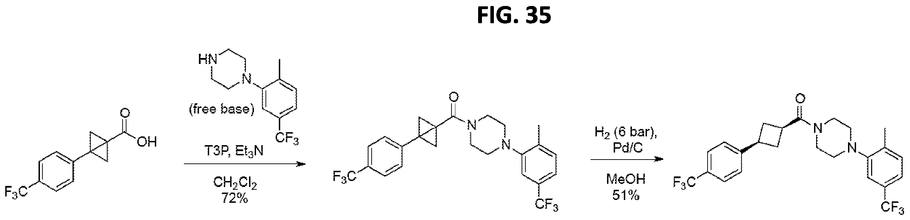

Figs. 14-35 provide exemplary reaction schemes for several of the analogs described in detail below. Fig. 14 is a general reaction scheme for propiolic acid precursor compounds. Fig. 15 is a reaction scheme for (4-(5-chloro-2-methylphenyl)piperazin-1-yl)((1*RS*,2*SR*)-2-(4-fluorophenyl)-cyclopropyl-1,2-*d*2)-methanone. FIG. 16 shows three general reaction schemes for several precursor compounds including aryl and piperazinyl moieties. Fig. 17 is a general reaction scheme for several analogs comprising cyclopropyl and piperazinyl moieties. Fig. 18 is a reaction scheme for (4-(5-chloro-2-fluorobenzoyl)piperazin-1-yl)((1S,2R)-2-(4-(trifluoromethyl)phenyl)cyclopropyl)-methanone and (4-((5-chloro-2-fluorophenyl)-sulfonyl)piperazin-1-yl)((1*S*,2*R*)-2-(4-(trifluoromethyl)phenyl)-cyclopropyl)methanone. Fig. 19 is a reaction scheme for (4-(5-chloro-2-fluorophenyl)piperazin-1-yl)(2-(4-fluorophenyl)cyclopropyl)-methanone. Fig. 20 is a reaction scheme for (4-(5-chloro-2-methylphenyl)piperazin-1-yl)(2-(4-(trifluoromethyl)phenyl)-cyclopropyl)methanone. Fig. 21 is a reaction scheme for (4-(2-methyl-5-(trifluoromethyl)phenyl)piperazin-1-yl)(2-(4-(trifluoromethyl)phenyl)cyclopropyl)-methanone. Fig. 22 is a reaction scheme for 3-fluoro-2-(4-(2-(4-(trifluoromethyl)phenyl)cyclopropane-1-carbonyl)piperazin-1-yl)benzonitrile. Fig. 23 is a reaction scheme for (4-(2-chloro-5-(trifluoromethyl)phenyl)piperazin-1-yl)(2-(4-(trifluoromethyl)phenyl)-cyclopropyl)methanone. Fig. 24 is a reaction scheme for (4-cyclohexyl-piperazin-1-yl)(2-(4-(trifluoromethyl)phenyl)cyclopropyl)methanone and (4-(Tetrahydro-2H-pyran-4-yl)piperazin-1-yl)(2-(4-(trifluoromethyl)phenyl)cyclopropyl)methanone. Fig. 26 is a reaction scheme for (4-(2-methyl-5-(trifluoromethyl)phenyl)piperazin-1-yl)(4-(4-(trifluoromethyl)phenyl)oxetan-2-yl)methanone. Fig. 27 is a reaction scheme for *trans*-(4-(5-chloro-2-methylphenyl)piperazin-1-yl)((1SR,2RS)-2-fluoro-2-(4-fluorophenyl)-cyclopropyl)methanone. Fig. 28 is a reaction scheme for cis-(4-(5-chloro-2-methylphenyl)piperazin-1-yl)((1SR,2RS)-2-fluoro-2-(4-fluorophenyl)cyclopropyl)-methanone. Fig. 29 is a reaction scheme for *cis-* and *trans-*(4-(5-chloro-2-methylphenyl)piperazin-1-yl)(2-(4-fluorophenyl)-1-(trifluoromethyl)-cyclopropyl)methanone. Fig. 30 is a reaction scheme for *trans*-(4-(5-chloro-2-methylphenyl)piperazin-1-yl)((1RS,2RS)-2-(4-fluorophenyl)-2-(trifluoromethyl)cyclopropyl)-methanone. Fig. 31 is a reaction scheme for *trans*-(4-(2,5-bis(trifluoromethyl)phenyl)-piperazin-1-yl)(1-(trifluoromethyl)-2-(4-(trifluoromethyl)-phenyl)cyclopropyl)methanone. Fig. 32 is a reaction scheme for *cis-*((1SR,3RS)-2,2-difluoro-3-(4-(trifluoromethyl)phenyl)cyclopropyl)(4-(2-methyl-5-(trifluoromethyl)-phenyl)piperazin-1-yl)methanone. Fig. 33 is a reaction scheme for (4-(5-chloro-2-(trifluoromethyl)-phenyl)piperazin-1-yl)(3-(4-fluorophenyl)bicyclo[1.1.0]butan-1-yl)methanone. Fig. 34 is a reaction scheme for (4-(5-chloro-2-(trifluoromethyl)phenyl)piperazin-1-yl)(3-(4-(trifluoromethyl)phenyl)bicyclo[1.1.0]butan-1-yl)methanone. Fig. 35 is a reaction scheme for (4-(2-methyl-5-(trifluoromethyl)-phenyl)piperazin-1-yl)(3-(4-(trifluoromethyl)phenyl)cyclobutyl)-methanone.

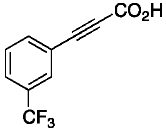

**3-(3-(Trifluoromethyl)phenyl)propiolic acid** (Yonemoto-Kobayashi et al., Org. & Biomolec. Chem. 2013, 11:3773-3775; Solomon et al., JACS 1963, 85:3492-3496; Austin et al., J. Org. Chem. 1981, 46:2280-2286. A solution of Pd(PPh₃)₂Cl₂ (0.0134 g, 0.0436 mmol), CuI (0.00829 g, 0.0436 mmol), and 3-bromobenzo-triflouride (0.62 mL, 4.36 mol) in Et₃N (8.7 mL) was sparged with Ar for 15 min and treated with (trimethylsilyl)acetylene (0.93 mL, 6.53 mmol) and sparged for an additional 2 min. The resulting mixture was heated to 80 °C overnight, cooled to rt, filtered through Celite, washed (Et₂O) until the washes appeared colorless and the filtrate was concentrated under reduced pressure. The crude residue was purified by chromatography on SiO₂ (hexanes) to give the trimethyl((3-(trifluoromethyl)phenyl)ethynyl)silane (1.03 g, 4.24 mmol, 97 %) as a pale yellow oil.

A solution of CsF (0.775 g, 5.10 mmol) in dry DMSO (6.5 mL) under an atmosphere of CO₂ (balloon) at rt was treated with a solution of trimethyl((3-(trifluoromethyl)phenyl)ethynyl)silane (1.03 g, 4.25 mmol) dropwise and the reaction was stirred under CO₂ at rt overnight. The reaction mixture was diluted with H₂O (80 mL) and extracted with CH₂Cl₂ (2 × 25 mL). The aqueous layer was acidified (> pH 1) with 6 M aqueous HCl at 0 °C and extracted with Et₂O (3 × 25 mL). The combined organic layers were dried (MgSO₄), concentrated under reduced pressure, and dried under high vacuum to give 3-(3-(trifluoromethyl)phenyl)propiolic acid (0.774 g, 3.62 mmol, 85%) as an pale tan orange waxy solid: ¹H NMR (400 MHz, Acetone-d₆) δ 11.85 (bs, 1 H), 7.95-7.87 (m, 3 H), 7.36 (t, *J* = 7.4 Hz, 1 H); ¹³C NMR (100 MHz, Acetone-d₆) δ 154.3, 137.1, 131.7 (q, *J_{CF} =* 33.0 Hz), 130.0 (q, *J_{CF} =* 3.8 Hz), 128.1 (q, *J_{CF} =* 3.4 Hz), 124.5 (q, *J_{CF} =* 272.0 Hz), 121.7, 83.6, 82.9.

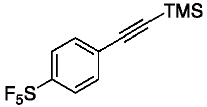

**Trimethyl((4-(pentafluoro-λ⁶-sulfaneyl)phenyl)ethynyl)silane.** A solution of Pd(PPh₃)₂Cl₂ (0.0365 g, 0.0519 mmol), CuI (0.0100 g, 0.0519 mmol), and (4-bromophenyl)pentafluoro-λ6-sulfane (1.50 g, 5.19 mmol) in Et₃N (11 mL) was sparged with Ar for 10 min, treated with (trimethylsilyl)acetylene (1.10 mL, 7.79 mmol), sparged with Ar for 5 min, heated to 80 °C for 22 h, cooled to rt, filtered through Celite, washed (Et₂O) until the washes appeared colorless, and the combined filtrates were concentrated under reduced pressure. The crude residue was purified by chromatography on SiO₂ (hexanes) to give trimethyl((4-(pentafluoro-λ6-sulfaneyl)phenyl)ethynyl)silane (1.32 g, 4.40 mmol, 85 %) as a pale yellow oil: IR (CH₂Cl₂) 2963, 2165, 1599, 1493, 1401, 1251, 1095, 826, 802, 759 cm⁻¹; ¹H NMR (400 MHz, CDCl₃) δ 7.68 (dt, *J* = 9.0, 2.0 Hz, 2 H), 7.52 (d, *J* = 9.0 Hz, 2 H), 0.28 (s, 9 H); ¹³C NMR (100 MHz, CDCl₃) δ 153.2 (quint, *J_{CF} =* 18.0 Hz), 126.9, 125.9 (quint, *J_{CF} =* 5.0 Hz), 102.7, 98.0, -0.3; ¹⁹F NMR (376 MHz, CDCl₃) δ 84.0 (quint, *J* = 150.2 Hz, 1 F), 62.6 (d, *J* = 150.2 Hz, 4 F); HRMS (ESI) *m*/*z* calcd for C₁₁H₁₃F₅SiS ([M]⁺) 300.0427, found 300.0400.

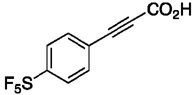

**3-(4-(Pentafluoro-λ6-sulfaneyl)phenyl)propiolic acid.** A solution of CsF (0.801 g, 5.27 mmol) in dry DMSO (3 mL) under CO₂ (balloon) at rt was treated with a solution of trimethyl((4-(pentafluoro-16-sulfaneyl)phenyl)ethynyl)silane (1.32 g, 4.40 mmol) in DMSO (5.8 mL) dropwise and the reaction was stirred under CO₂ at rt overnight, diluted with H₂O (90 mL) and extracted with CH₂Cl₂ (2 × 50 mL). The aqueous layer was acidified (> pH 1) with 6 M aqueous HCl at 0 °C and extracted with Et₂O (3 × 50 mL). The combined organic layers were washed with H₂O (50 mL), dried (MgSO₄), concentrated under reduced pressure, and dried under high vacuum to give 3-(4-(pentafluoro-λ6-sulfaneyl)phenyl)propiolic acid (0.338 g, 1.24 mmol, 28%) as brown solid. Mp 156.5-159.6 °C; IR (CHCl₃) 2979, 2876, 2577, 2235, 1677, 1416, 1298, 1217, 886, 829, 751 cm⁻¹; ¹H NMR (400 MHz, CDCl₃) δ 10.00 (bs, 1 H), 7.82 (dt, *J* = 9.0, 2.0 Hz, 2 H), 7.73 (d, *J* = 9.0 Hz, 2 H); ¹³C NMR (100 MHz, CDCl₃) δ 157.7, 155.2 (quint, *J_{CF} =* 19.0 Hz), 133.3, 126.5 (quint, *J_{CF} =* 4.0 Hz), 122.7, 85.9, 81.7; ¹⁹F NMR (376 MHz, CDCl₃) δ 82.6 (quint, *J* = 150.5 Hz, 1 F), 62.3 (d, *J=* 150.3 Hz, 4 F); HRMS (ESI) *m*/*z* calcd for C₉H₄O₂F₅S ([M-H]⁻) 270.9858, found 270.9858.

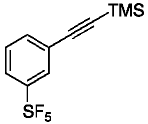

**Trimethyl((3-(pentafluoro-λ6-sulfaneyl)phenyl)ethynyl)silane.** A solution of Pd(PPh₃)₂Cl₂ (0.0365 g, 0.0519 mmol), CuI (0.0100 g, 0.0519 mmol), and (3-bromophenyl)pentafluoro-λ6-sulfane (1.50 g, 5.19 mmol) in Et₃N (11 mL) was sparged with Ar for 10 min, treated with (trimethylsilyl)acetylene (1.10 mL, 7.79 mmol), sparged with Ar for 5 min heated to 80 °C for 22 h, cooled to rt, filtered through Celite, washed (Et₂O) until the washes appeared colorless. The combined filtrates were concentrated under reduced pressure. The crude residue was purified by chromatography on SiO₂ (hexanes) to give trimethyl((3-(pentafluoro-λ6-sulfaneyl)phenyl)ethynyl)silane (1.29 g, 4.29 mmol, 83%) as a yellow oil: IR (CH₂Cl₂) 2963, 2902, 2168, 1601, 1479, 1421, 1251, 1109, 831, 803, 789, 759, 683 cm⁻¹; ¹H NMR (400 MHz, CDCl₃) δ 7.87 (t, *J* = 2.0 Hz, 1 H), 7.68 (ddd, *J* = 8.0, 2.0, 0.8 Hz, 1 H), 7.58 (d, *J* = 8.0 Hz, 1 H), 7.38 (t, *J =* 8.0 Hz, 1 H), 0.29 (s, 9 H); ¹³C NMR (100 MHz, CDCl₃) δ 153.7 (quint, *J_{CF} =* 17.8 Hz), 134.8, 129.5 (quint, *J_{CF} =* 4.6 Hz), 128.6, 125.8 (quint, *J_{CF}* = 4.8 Hz), 124.4, 102.8, 96.7, -0.3; ¹⁹F NMR (376 MHz, CDCl₃) δ 83.6 (quint, *J =* 150.4 Hz, 1 F), 62.5 (d, *J =* 150.3 Hz, 4 F); HRMS (ESI) *m*/*z* calcd for C₁₁H₁₃F₅SiS ([M]⁺) 300.0427, found 300.0405.

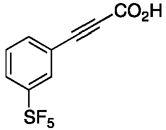

**3-(3-(Pentafluoro-λ6-sulfaneyl)phenyl)propiolic acid.** A solution of CsF (0.783 g, 5.15 mmol) in dry DMSO (3 mL) under CO₂ (balloon) at rt was treated a solution of trimethyl((3-(pentafluoro-λ6-sulfaneyl)phenyl)ethynyl)silane (1.29 g, 4.30 mmol) in DMSO (5.6 mL) dropwise and the reaction was stirred under CO₂ at rt overnight. The reaction mixture was diluted with H₂O (90 mL) and extracted with CH₂Cl₂ (2 × 50 mL). The aqueous layer was acidified (> pH 1) with 6M aqueous HCl at 0 °C and then extracted with Et₂O (3 × 50 mL). The combined organic layers were washed with H₂O (50 mL), dried (MgSO₄), concentrated under reduced pressure, and further dried under high vacuum to give 3-(3-(pentafluoro-λ6-sulfaneyl)phenyl)propiolic acid (0.935 g, 3.43 mmol, 80%) as tan solid: Mp 121.1-124.4 °C; IR (CHCl₃) 2831, 2218, 1688, 1479, 1426, 1214, 831, 791, 768, 680 cm⁻¹; ¹H NMR (400 MHz, CDCl₃) δ 10.92 (s, 1 H), 8.01 (t, *J* = 2.0 Hz, 1 H), 7.87 (ddd, *J* = 8.0, 2.0, 0.9 Hz, 1H), 7.75 (d, *J* = 8.0 Hz, 1 H), 7.54 (t, *J =* 8.0 Hz, 1 H); ¹³C NMR (100 MHz, CDCl₃) δ 158.1, 153.8 (quint, *J_{CF} =* 19.0 Hz), 135.9, 130.7 (quint, *J_{CF} =* 5.0 Hz), 129.3, 128.4 (quint, *J_{CF}* = 4.0 Hz), 120.2, 86.2, 81.0; ¹⁹F NMR (376 MHz, CDCl₃) δ 82.5 (quint, *J* = 150.4 Hz, 1 F), 62.5 (d, *J* = 150.4 Hz, 4 F); HRMS (ESI) *m*/*z* calcd for C₉H₄O₂F₅S ([M-H]⁻) 270.9858, found 270.9856.

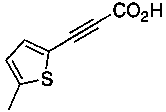

**3-(5-Methylthiophen-2-yl)propiolic acid** (He et al., Chem. Sci. 2013, 4:3478-3483; Paegle et al., Euro. J. Org. Chem. 2015; 2015:4389-4399; Kub et al., Macromolecules 2010, 34:2124-2129). A solution of Pd(PPh₃)₂Cl₂ (0.0269 g, 0.0877 mmol), CuI (0.0167 g, 0.0877 mmol), and 2-bromo-5-methyl thiophene (1.00 mL, 8.77 mmol) in Et₃N (17.5 mL) sparged with Ar for 15 min and treated with (trimethylsilyl)acetylene (1.9 mL, 13.2 mmol) and the mixture was further sparged for 2 min, heated to 80 °C overnight, cooled to rt, filtered through Celite, washed (Et₂O) until the washes appeared colorless and the filtrate was concentrated under reduced pressure. The crude residue was purified by chromatography on SiO₂ (hexanes) to give the trimethyl((5-methylthiophen-2-yl)ethynyl)silane 1.06 g, 5.47 mmol, 62%) as a pale yellow oil.

To a solution of CsF (0.994 g, 6.54 mmol) in dry DMSO (8 mL) under an atmosphere of CO₂ (balloon) at rt was added a solution of trimethyl((5-methylthiophen-2-yl)ethynyl)silane (1.06 g, 5.45 mmol) dropwise and the reaction was stirred under CO₂ at rt overnight. The reaction mixture was diluted with H₂O (100 mL) and extracted with CH₂Cl₂ (2 × 25 mL). The aqueous layer was acidified (> pH 1) with 6 M aqueous HCl at 0 °C and extracted with Et₂O (3 × 25 mL). The combined organic layers were washed with brine (50 mL), dried (MgSO₄), filtered, and concentrated under reduced pressure to give the product (0.571 g, 3.44 mmol, 63%) as a brown solid: ¹H NMR (300 MHz, CDCl₃) δ 10.14 (bs, 1 H), 7.36 (d, *J* = 3.3 Hz, 1 H), 6.73 (dd, *J =* 3.3 Hz, 1 H), 2.52 (s, 3 H).

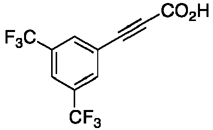

**3-(3,5-Bis(trifluoromethyl)phenyl)propiolic acid.** A solution of Pd(PPh₃)₂Cl₂ (0.239 g, 0.341 mmol), CuI (0.0650 g, 0.341 mmol), and 1-bromo-3,5-bis(trifluoromethyl)benzene (2.00 g, 6.83 mmol) in Et₃N (13 mL) was sparged with Ar for 10 min and treated with (trimethylsilyl)acetylene (1.42 mL, 10.2 mmol) and the solution was sparged with Ar for 2 min. The resulting mixture was heated to 80 °C for 22 h, cooled to rt, and filtered through Celite, which was washed with Et₂O until the washes appeared colorless. The filtrate was concentrated under reduced pressure and the crude residue was purified by chromatography on SiO₂ (hexanes) to give the desired product (1.79 g, 5.78 mmol) as a light yellow solid.

A solution of CsF (1.05 g, 6.92 mmol) in DMSO (4.6 mL) under CO₂ at rt was treated with a solution of ((3,5-bis(trifluoromethyl)phenyl)ethynyl)trimethylsilane (1.79 g, 5.77 mmol) in DMSO (7 mL) dropwise and the reaction was stirred under CO₂ at rt overnight. The reaction mixture was diluted with H₂O (90 mL) and extracted with CH₂Cl₂ (2 × 50 mL). The aqueous layer was acidified (> pH 1) with 6 M aqueous HCl at 0 °C and then extracted with Et₂O (3 × 100 mL). The combined organic layers were washed with H₂O (50 mL), dried (MgSO₄). The solvent was concentrated under reduced pressure and further dried under high vacuum to give 3-(3,5-bis(trifluoromethyl)phenyl)propiolic acid (0.859 g, 3.04 mmol, 45% (2 steps)) as brown solid: Mp 124.5-128.2 °C; IR (CH₂Cl₂) 2915, 2226, 1688, 1377, 1278, 1131, 972, 903, 683 cm⁻¹; ¹H NMR (400 MHz, CDCl₃) δ 11.25 (s, 1 H), 8.06 (s, 2 H), 7.98 (s, 1 H); ¹³C NMR (100 MHz, CDCl₃) δ 157.7, 132.9 (q, *J_{CF} =* 3.4 Hz), 132.6 (q, *J_{CF} =* 34.4 Hz), 124.5 (q, *J_{CF} =* 3.5 Hz), 122.5 (q, *J_{CF} =* 273.2 Hz), 121.5, 84.5, 82.0; ¹⁹F NMR (376 MHz, CDCl₃) δ -63.3 (s, 6 F); HRMS (ESI) *m*/*z* calcd for C₁₁H₃F₆O₂ ([M-H]⁻) 281.0043, found 281.0039

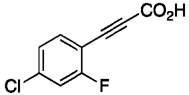

**3-(4-Chloro-2-fluorophenyl)propiolic acid.** A solution of Pd(PPh₃)₂Cl₂ (0.197 g, 0.281 mmol), CuI (0.0535 g, 0.281 mmol), and 4-chloro-2-fluoro-1-iodobenzene (1.80 g, 7.02 mmol) in Et₃N (14 mL) was sparged with Ar for 10 min followed by addition of (trimethylsilyl)acetylene (1.46 mL, 10.5 mmol) and the solution was further sparged with Ar for 2 min. The resulting mixture was heated to 80 °C for 22 h. After cooling the reaction to rt, the solution was filtered through Celite, which was washed with Et₂O until the washes appeared colorless. The filtrate was concentrated under reduced pressure. The crude residue was purified by chromatography on SiO₂ (hexanes) to give ((4-chloro-2-fluorophenyl)ethynyl)trimethylsilane (1.42 g, 6.26 mmol) as a yellow oil.

A solution of CsF (1.14 g, 7.51 mmol) in DMSO (5 mL) under CO₂ at rt was treated with a solution of ((4-chloro-2-fluorophenyl)ethynyl)trimethylsilane (1.42 g, 6.26 mmol) in DMSO (7 mL) dropwise and the reaction was stirred under CO₂ at rt overnight. The reaction mixture was diluted with H₂O (90 mL) and extracted with CH₂Cl₂ (2 × 50 mL). The aqueous layer was acidified (> pH 1) with 6 M aqueous HCl at 0 °C and then extracted with Et₂O (3 × 100 mL). The combined organic layers were washed with H₂O (50 mL), dried (MgSO₄), concentrated under reduced pressure, and dried under high vacuum to give 3-(4-chloro-2-fluorophenyl)propiolic acid (0.828 g, 4.17 mmol, 60% (2 steps)) as tan solid: Mp 174.1-176.4 °C; IR (CH₂Cl₂) 2972, 2233, 1720, 1603, 1486, 1387, 1298, 1189, 1072, 883, 827 cm⁻¹; ¹H NMR (400 MHz, CDCl₃) δ 9.09 (s, 1 H), 7.53 (t, *J* = 7.4 Hz, 1 H), 7.20 (d, *J =* 8.2 Hz, 2 H); ¹³C NMR (100 MHz, CDCl₃) δ 163.6 (d, *J_{CF}* = 259.9 Hz), 156.9, 138.7 (d, *J_{CF} =* 10.0 Hz), 135.2 (d, *J_{CF} =* 0.9 Hz), 125.1 (d, *J_{CF} =* 3.7 Hz), 117.0 (d, *J_{CF}* = 23.6 Hz), 106.8 (d, *J_{CF} =* 15.5 Hz), 85.1, 81.1; ¹⁹F NMR (376 MHz, CDCl₃) δ -104.3 (s, 1 F); HRMS (ESI) *m*/*z* calcd for C₉H₃ClFO₂ ([M-H]⁻) 196.9811, found 196.9831.

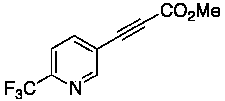

**Methyl 3-(6-(trifluoromethyl)pyridin-3-yl)propiolate.** To two flasks each containing a solution of Pd(PPh₃)₂Cl₂ (0.0466 g, 0.0664 mmol), CuI (0.0126 g, 0.0664 mmol), and 5-bromo-2-trifluoromethyl pyridine (1.50 g, 6.64 mmol) in Et₃N (13 mL) sparged with Ar for 10 min followed by addition of (trimethylsilyl)acetylene (1.4 mL, 9.96 mmol) and sparged with Ar for 2 min. The resulting mixtures were heated to 80 °C overnight where by TLC (hexanes/EtOAc, 4:1) the SM had been consumed. After cooling the reaction to rt, the reactions were combined, the solution was filtered through Celite, which was washed with Et₂O (100 mL) until the washes appeared colorless. The filtrate was concentrated under reduced pressure. The crude residue was purified by chromatography on SiO₂ (hexanes/EtOAc, 9: 1) to give 2-(trifluoromethyl)-5-((trimethylsilyl)ethynyl)pyridine (3.37 g, 13.9 mmol) as orange/brown waxy solid that was taken on to the carboxylation.

A solution of CsF (2.52 g, 16.6 mmol) in DMSO (20 mL) under CO₂ at rt was treated with a solution of 2-(trifluoromethyl)-5-((trimethylsilyl)ethynyl)pyridine (3.37 g, 13.9 mmol) in DMSO (7 mL) dropwise and the reaction was stirred under CO₂ (balloon) at rt for 5 h, treated with MeI (0.95 mL, 15.2 mmol) was added and the solution was stirred for 1 h at rt. The reaction mixture was diluted with H₂O (200 mL), brine (100 mL) and extracted with Et₂O (3 × 150 mL). The combined organic layers were washed with H₂O (100 mL), dried (MgSO₄), and concentrated under reduced pressure. The crude product was purified by chromatography on SiO₂ (hexanes/EtOAc, 4: 1) to give methyl 3-(6-(trifluoromethyl)pyridin-3-yl)propiolate (1.87 g, 8.17 mmol, 59% (2 steps)) as tan solid: Mp 98.2-99.7 °C; IR (neat) 2962, 2233, 1712, 1433, 1337, 1242, 1127, 1085, 864, 745 cm⁻¹; ¹H NMR (400 MHz, CDCl₃) δ 8.87 (s, 1 H), 8.04 (ddd, *J =* 8.0, 1.2, 0.6 Hz, 1 H), 7.71 (d, *J* = 8.0 Hz, 1 H), 3.86 (s, 3 H); ¹³C NMR (100 MHz, CDCl₃) δ 153.4, 153.1, 148.6 (q, *J_{CF}* = 35.5 Hz), 141.2, 121.0 (q, *J_{CF}* = 274.4 Hz), 120.1 (q, *J_{CF} =* 2.8 Hz), 120.0, 84.7, 80.7, 53.2; ¹⁹F NMR (376 MHz, CDCl₃) δ -68.3 (s, 3 F); HRMS (ESI) *m*/*z* calcd for C₁₀H₇F₃NO₂ ([M+H]⁺) 230.0423, found 230.0422.

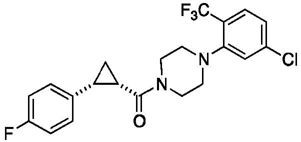

**(4-(5-Chloro-2-(trifluoromethyl)phenyl)piperazin-1-yl)((1S,2R)-2-(4-fluorophenyl)cyclopropyl)-methanone..** A solution of 3-(4-fluorophenyl)propiolic acid (0.0800 g, 0.487 mmol) and 1-(5-chloro-2-(trifluoromethyl)phenyl)piperazinehydrochloride (0.142 g, 0.536 mmol) in CH₂Cl₂ (4.9 mL) at 0 °C was treated Et₃N (0.27 mL, 1.95 mmol). The cooled solution was treated with T3P (50% solution in EtOAc) (0.52 mL, 0.73 mmol) dropwise and the reaction was stirred at 0 °C for 30 min, warmed to rt overnight, diluted with CH₂Cl₂ (30 mL), washed with H₂O (20 mL), satd. aqueous NaHCO₃ (20 mL), dried (MgSO₄), filtered, and concentrated under reduced pressure. The crude material was purified by automated chromatography on SiO₂ (4g column, liquid load CH₂Cl₂, 100% hexanes to 30% EtOAc/hexanes), to give 1-(4-(5-chloro-2-(trifluoromethyl)phenyl)piperazin-1-yl)-3-(4-fluorophenyl)prop-2-yn-1-one (0.140 g, 0.341 mmol) as a colorless solid.

A solution of 1-(4-(5-chloro-2-(trifluoromethyl)phenyl)piperazin-1-yl)-3-(4-fluorophenyl)prop-2-yn-1-one (0.140 g, 0.341 mmol) in EtOAc (3.4 mL) was treated with Lindlar's catalyst (5% Pd on CaCO₃, lead poisoned, 0.0363 g, equivalent to 5 mol% Pd). The reaction was placed under a balloon of H₂ (3 vacuum/backfill cycles) and stirred at rt for 2 d. TLC (hexanes/EtOAc, 2:1) indicated that the SM had been mostly consumed. The reaction was filtered through Celite (eluting with EtOAc (10 mL)) and the combined filtrates were concentrated under reduced pressure. The crude residue was purified by automated chromatography on SiO₂ (4g column, 100% hexanes to 40% EtOAc/hexanes, product eluted at 20% EtOAc/hexanes) to give (Z)-1-(4-(5-chloro-2-(trifluoromethyl)phenyl)piperazin-1-yl)-3-(4-fluorophenyl)prop-2-en-1-one (0.0510 g, 0.124 mmol) as a colorless solid.

A solution of CrCl₂ (0.0911 g, 0.741 mmol) and (Z)-1-(4-(5-chloro-2-(trifluoromethyl)phenyl)-piperazin-1-yl)-3-(4-fluorophenyl)prop-2-en-1-one (0.0510 g, 0.124 mmol) in dry degassed THF (1.2 mL) was sparged with Ar for 5 min and treated with CH₂ICl (0.071 mL, 0.618 mmol) at rt, stirred for 2 d at 80 °C, cooled to rt, diluted with EtOAc (50 mL) and washed with 1 M aqueous HCl (3 x 20 mL). The organic layer was dried (MgSO₄), filtered and concentrated under reduced pressure. The crude residue was purified by automated chromatography on SiO₂ (4g column, liquid load CH₂Cl₂, 100% hexanes to 30% EtOAc/hexanes, product eluted at 20% EtOAc/hexanes), filtered through basic Al₂O₃ (CH₂Cl₂/EtOAc, 1:1) and concentrated under reduced pressure. The resulting oil was recrystallized from a mixture of hexanes/cyclohexane (1:1), the crystals were washed with hexanes and dried under high vacuum to give (4-(5-chloro-2-(trifluoromethyl)phenyl)piperazin-1-yl)((1*S*,2*R*)-2-(4-fluorophenyl)cyclopropyl)-methanone. (0.0238 g, 0.0558 mmol, 11% (3 steps) (100% purity by ELSD)) as a colorless solid: Mp 101.0-103.2 °C; IR (CH₂Cl₂) 2917, 1639, 1513, 1418, 1308, 1225, 1126, 1085, 1031, 838 cm⁻¹; ¹H NMR (400 MHz, CDCl₃) δ 7.45 (dd, *J* = 8.4, 0.6 Hz, 1 H), 7.25-7.22 (m, 1 H), 7.16-7.11 (m, 2 H), 7.00 (d, *J* = 1.8 Hz, 1 H), 6.99-6.93 (m, 2 H), 3.92-3.86 (m, 1 H), 3.79-3.73 (m, 1 H), 3.67 (ddd, *J* = 12.6, 8.6, 3.2 Hz, 1 H), 3.37 (ddd, *J =* 12.6, 9.0, 3.2 Hz, 1 H), 3.01-2.95 (m, 2 H), 2.49-2.39 (m, 2 H), 2.31-2.25 (m, 1 H), 2.19 (ddd, *J* = 9.0, 8.6, 5.8 Hz, 1 H), 1.83 (dt, *J* = 6.8, 5.8 Hz, 1 H), 1.36 (td, *J =* 8.6, 5.6 Hz, 1 H); ¹³C NMR (100 MHz, CDCl₃) δ 167.2, 161.6 (d, *J_{CF} =* 245.1 Hz), 149.1, 132.5, 131.1, 130.1 (q, *J_{CF} =* 32.7 Hz), 129.1, 129.0, 123.6 (q, *J_{CF} =* 272.2 Hz), 120.8 (q, *J_{CF} =* 3.8 Hz), 117.2 (q, *J_{CF} =* 3.8 Hz), 115.0 *(d, J_{CF} =* 21.3 Hz), 51.3, 51.0, 45.3, 41.9, 23.9, 23.5 10.7; ¹⁹F NMR (376 MHz, CDCl₃) δ -62.6 (s, 3 F), -116.4 (s, 1 F); HRMS (ESI) *m*/*z* calcd for C₂₁H₂₀ClF₄N₂O ([M+H]⁺) 427.1195, found 427.1192.

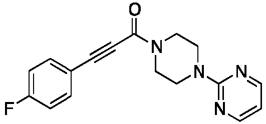

**3-(4-Fluorophenyl)-1-(4-(pyrimidin-2-yl)piperazin-1-yl)prop-2-yn-1-one.** A solution of 3-(4-fluorolphenyl)propionic acid (, 0.150 g, 0.914 mmol) and 2-(1-piperazinyl)pyrimidine (, 0.188 g, 0.841 mmol) in CH₂Cl₂ (9.1 mL) at 0 °C was treated Et₃N (0.51 mL, 3.66 mmol). The cooled solution was treated with T3P (50% solution in EtOAc) (1.0 mL, 1.37 mmol) dropwise and the reaction was stirred at 0 °C for 30 min and allowed to warm to rt overnight. The reaction was diluted with CH₂Cl₂ (30 mL) and washed with H₂O (20 mL), dried (MgSO₄), filtered, and concentrated under reduced pressure. The crude material was purified by automated chromatography on SiO₂ (4g column, gradient hexanes to EtOAc), to give 3-(4-fluorophenyl)-1-(4-(pyrimidin-2-yl)piperazin-1-yl)prop-2-yn-1-one (0.223 g, 0.719 mmol, 79%) as a colorless solid: Mp 168.4-170.8 °C; IR (CH₂Cl₂) 2859, 2217, 1618, 1585, 1506, 1435, 1355, 1261, 1227, 980, 838, 732 cm⁻¹; ¹H NMR (400 MHz, CDCl₃) δ 8.34 (d, *J* = 4.6 Hz, 2 H), 7.56 (dd, *J* = 8.8, 5.3 Hz, 2 H), 7.08 (t, *J* = 8.8 Hz, 2 H), 6.56 (t, *J* = 4.6 Hz, 1 H), 3.95-3.93 (m, 2 H), 3.88 (app dd, *J* = 6.6, 3.4 Hz, 4 H), 3.76 (app dd, *J =* 6.6, 4.1 Hz, 2 H); ¹³C NMR (100 MHz, CDCl₃) δ 163.6 (d, *J* = 253.0 Hz), 161.4, 157.8, 153.1, 134.6 (d, *J* = 8.7 Hz), 116.4 (d, *J* = 3.6 Hz), 116.1 (d, *J* = 22.1 Hz), 110.6, 90.0, 80.8, 46.8, 44.0, 43.3 41.5.; ¹⁹F NMR (376 MHz, CDCl₃) δ -107.3 (s, 1 F); HRMS (ESI) *m*/*z* calcd for C₁₇H₁₆FN₄O ([M+H]⁺) 311.1303, found 311.1301.

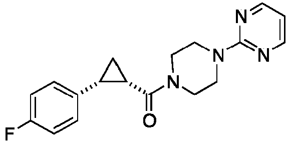

**((1*RS*,2*SR*)-2-(4-Fluorophenyl)cyclopropyl)(4-(pyrimidin-2-yl)piperazin-1-yl)methanone.** A solution of 3-(4-fluorophenyl)-1-(4-(pyrimidin-2-yl)piperazin-1-yl)prop-2-yn-1-one (0.200 g, 0.644 mmol) in EtOAc (6.4 mL) was treated with Lindlar's catalyst (5% Pd on CaCO₃, lead poisoned, 0.0685 g, equivalent to 5 mol% Pd). The reaction vessel was placed under vacuum and backfilled with H₂ (balloon, 4x) and stirred for 18 h at rt, filtered through Celite, washed (EtOAc), and the combined filtrates were concentrated under reduced pressure. The crude residue was purified by automated chromatography on SiO₂ (4g column, hexanes to 60:40 hexanes:EtOAc) to give (Z)-3-(4-fluorophenyl)-1-(4-(pyrimidin-2-yl)piperazin-1-yl)prop-2-en-1-one (0.211 g, 0.676 mmol) as a colorless solid that was taken on to the cyclopropanation. A solution of CrCl₂ (0.472 g, 3.84 mmol) and (Z)-3-(4-fluorophenyl)-1-(4-(pyrimidin-2-yl)piperazin-1-yl)prop-2-en-1-one (0.200 g, 0.640 mmol) in dry degassed THF (6.4 mL) (previously sparged with Ar for 15 min) was treated with CH₂ICl (0.37 mL, 3.20 mmol) and further sparged with Ar for 2 min. The reaction mixture was stirred for 20 h at 80 °C, cooled to rt, diluted with EtOAc (10 mL), filtered through a plug of basic Al₂O₃ (EtOAc), and concentrated under reduced pressure. The crude material was purified by automated chromatography on SiO₂ (4g column, hexanes to 60% EtOAc:hexanes) to give ((1RS,2SR)-2-(4-fluorophenyl)cyclopropyl)(4-(pyrimidin-2-yl)piperazin-1-yl)methanone (0.128 g, 0.392 mmol, 61% (2 steps) (100% purity by ELSD)) as a colorless solid: Mp 141.9-145.3 °C; IR (CH₂Cl₂) 2922, 1636, 1583, 1510, 1432, 1359, 1224, 1027, 981, 837, 797 cm⁻¹; ¹H NMR (400 MHz, CDCl₃) δ 8.28 (d, *J* = 4.6 Hz, 2 H), 7.12 (dd, *J* = 8.7, 5.3 Hz, 2 H), 6.92 (t, *J* = 8.7 Hz, 2 H), 6.50 (t, *J* = 4.6 Hz, 1 H), 4.00 (dt, *J* = 13.3, 4.4 Hz, 1 H), 3.93 (dt, *J* = 13.0, 4.4 Hz, 1 H), 3.76 (ddd, *J* = 13.0, 5.1, 3.6 Hz, 1 H), 3.66 (dt, *J =* 13.0, 4.4 Hz, 1 H), 3.53 (ddd, *J* = 13.0, 8.8, 3.6 Hz, 1 H), 3.26 (ddd, *J* = 13.0, 8.8, 3.4 Hz, 1 H), 3.13 (ddd, *J* = 13.0, 8.8, 3.6 Hz, 1 H), 3.00 (ddd, *J* = 13.0, 8.8, 3.6 Hz, 1 H), 2.49-2.43 (m, 1 H), 2.18 (ddd, *J* = 9.2, 8.4, 5.8 Hz, 1 H), 1.83 (q, *J* = 5.8 Hz, 1 H), 1.34 (td, *J =* 8.4, 5.8 Hz, 1 H); ¹³C NMR (100 MHz, CDCl₃) δ 167.3, 161.7 (d, *J_{CF} =* 244.9 Hz), 161.3, 157.7, 133.0, 129.0 (d, *J_{CF} =* 8.0 Hz), 115.0 (d, *J_{CF} =* 21.3 Hz), 110.3, 45.0, 43.8, 43.4, 41.6, 23.9, 23.5, 10.6; ¹⁹F NMR (470 MHz, CDCl₃) δ -116.4 (s, 1 F); HRMS (ESI) *m*/*z* calcd for C₁₈H₂₀FN₄O ([M+H]⁺) 327.1616, found 327.1616.

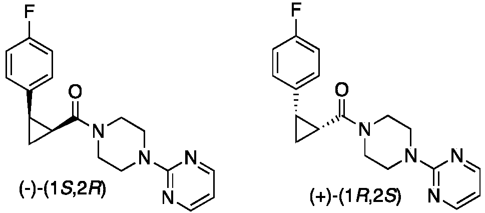

Racemic ((1RS,2SR)-2-(4-fluorophenyl)cyclopropyl)(4-(pyrimidin-2-yl)piperazin-1-yl)methanone was separated on a SFC Chiralpak-IC semiprep (250 x 10 mm) column (30% Methanol/CO₂, 7 mL/min, 100 bar, 90µL injection, 20 mg/mL in MeOH) to give ((1S,2R)-2-(4-fluorophenyl)cyclopropyl)(4-(pyrimidin-2-yl)piperazin-1-yl)methanone (retention time 7.63 min) as a colorless solid (100% purity by ELSD): [α]¹⁷_{D} - 207.0 (c 0.53, MeOH); ¹H NMR (300 MHz, CDCl₃) δ 8.28 (d, *J* = 4.6 Hz, 1 H), 7.12 (dd, *J* = 8.6, 5.3 Hz, 1 H), 6.92 (t, *J* = 8.6 Hz, 1 H), 6.50 (t, *J* = 4.6 Hz, 1 H), 4.02-3.91 (m, 2 H), 3.79-3.73 (m, 1 H), 3.69-3.64 (m, 1 H), 3.56-3.48 (m, 2 H), 3.29-3.21 (m, 1 H), 3.16-3.08 (m, 1 H), 2.98 (ddd, *J =* 12.6, 9.0, 3.7 Hz, 1 H), 2.51-2.42 (m, 1 H), 2.18 (ddd, *J* = 9.0, 8.4, 6.0, 1 H), 1.83 (q, *J* = 5.6 Hz, 1 H), 1.35 (td, *J* = 8.4, 5.6 Hz, 1 H). The enantiomeric excess was >99.9% ee (SFC Chiralpak-IC (250 x 10 mm); 30% Methanol:CO₂, 7 mL/min, p = 100 bar, 220 nm; retention time: 8.0 min).

((1*R*,2*S*)-2-(4-Fluorophenyl)cyclopropyl)(4-(pyrimidin-2-yl)piperazin-1-yl)methanone (retention time 8.61 min) was obtained as a colorless solid (100% purity by ELSD): [α]¹⁷_{D} +213.2 (c 0.54, MeOH); ¹H NMR (300 MHz, CDCl₃) δ 8.28 (d, *J* = 4.6 Hz, 2 H), 7.12 (dd, *J* = 8.6, 5.3 Hz, 2 H), 6.92 (t, *J* = 8.6 Hz, 2 H), 6.50 (t, *J* = 4.6 Hz, 1 H), 4.02-3.90 (m, 2 H), 3.79-3.73 (m, 1 H), 3.69-3.62 (m, 1 H), 3.57-3.48 (m, 2 H), 3.30-3.21 (m, 1 H), 3.16-3.08 (m, 1 H), 2.98 (ddd, *J* = 13.0, 9.0, 3.5 Hz, 1 H), 2.47 (td, *J* = 9.0, 6.0 Hz, 1 H), 2.18 (ddd, *J* = 9.0, 8.4, 6.0 Hz, 1 H), 1.83 (q, *J* = 5.6 Hz, 1 H), 1.35 (td, *J* = 8.4, 5.6 Hz, 1 H). The enantiomeric excess was >99.9% ee (SFC Chiralpak-IC (250 x 10 mm); 30% Methanol:CO₂, 7 mL/min, p = 100 bar, 220 nm; retention time: 9.0 min).

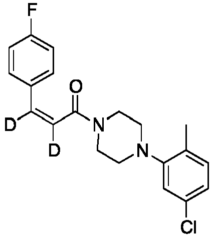

**(*Z*)-1-(4-(5-Chloro-2-methylphenyl)piperazin-1-yl)-3-(4-fluorophenyl)prop-2-en-1-one-2,3-*d*2.** A solution of 1-(4-(5-chloro-2-methylphenyl)piperazin-1-yl)-3-(4-fluorophenyl)prop-2-yn-1-one (0.150 g, 0.140 mmol) in anhydrous EtOAc (4.2 mL) was treated with Lindlar's catalyst (5% Pd on CaCO₃, lead poisoned, 0.0447 g, 0.0210 mmol, equivalent to 5 mol% Pd). The reaction was placed under a balloon of D₂ (3 vacuum/backfill cycles) and stirred vigorously at rt for 24 h, filtered through Celite, washed (EtOAc), and concentrated under reduced pressure. The crude residue was purified by automated chromatography on SiO₂ (4 g column, liquid load with CH₂Cl₂, hexanes to 30% EtOAc:hexanes, product eluted at 25% EtOAc:hexanes) to give (Z)-1-(4-(5-chloro-2-methylphenyl)piperazin-1-yl)-3-(4-fluorophenyl)prop-2-en-1-one-2,3-d2 (0.0919 g, 0.255 mmol, 61%, 97% deuterium incorporation) as a colorless solid: Mp 122.1-124.8 °C; IR (CH₂Cl₂) 2918, 2819, 1628, 1595, 1505, 1432, 1222, 1022, 852, 817, 735 cm⁻¹; ¹H NMR (400 MHz, CDCl₃) δ 7.39 (dd, *J=* 8.6, 5.4 Hz, 2 H), 7.07-7.01 (m, 3 H), 6.95 (dd, *J=* 8.2, 2.0 Hz, 1 H), 6.79 (d, *J=* 2.0 Hz, 1 H), 3.79 (bs, 2 H), 3.48 (t, *J=* 5.0 Hz, 2 H), 2.80 (t, *J=* 5.0 Hz, 2 H), 2.52 (t, *J=* 5.0 Hz, 2 H), 2.20 (s, 3 H); ¹³C NMR (100 MHz, CDCl₃) δ 167.2, 162.6 (d, *J =* 249.3 Hz), 151.7, 132.1 (t, *J_{CD}* = 23.6 Hz), 132.0, 131.7, 131.4 (d, *J_{CF} =* 3.2 Hz), 130.8, 130.2 (d, *J_{CF} =* 8.1 Hz), 123.6, 122.2 (t, *J_{CD}* = 24.2 Hz), 119.5, 115.6 *(d, J_{CF} =* 21.4 Hz), 51.4, 51.1, 46.5, 41.4 17.3; ¹⁹F NMR (376 MHz, CDCl₃) δ -112.0 (s, 1 F); HRMS (ESI) *m*/*z* calcd for C₂₀H₁₉D₂ClFN₂O ([M+H]⁺) 361.1446, found 361.1446

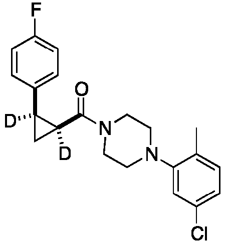

***(4-(5-Chloro-2-methylphenyl)piperazin-1-yl)((1RS,2SR)-2-(4-fluorophenyl)cyclopropyl-1,2-***d2)methanone. A solution of CrCl₂ (0.225 g, 1.83 mmol) and (4-(5-chloro-2-methylphenyl)piperazin-1-yl)(2-(4-fluorophenyl)cyclopropyl-1,2-d2)methadone (0.110 g, 0.305 mmol) in dry degassed THF (3 mL) (previously sparged with Ar for 15 min) was treated with CH₂ICl (0.18 mL, 1.52 mmol) and sparged with Ar for 2 min. The reaction mixture was stirred for 24 h at 80 °C, cooled to rt, diluted with EtOAc (50 mL), and washed with 1 M HCl (3 x 20 mL). The organic layer was dried (MgSO₄), filtered and concentrated under reduced pressure. The crude residue was purified by automated chromatography on SiO₂ (4g column, liquid load CH₂Cl₂, hexanes to 40% EtOAc/hexanes) to give a yellow oil that was filtered through basic Al₂O₃ (1:1 CH₂Cl₂/EtOAc) concentrated under reduced pressure to a clear oil that was triturated in minimal cyclohexane to give a colorless solid that was dried under high vacuum to give (4-(5-chloro-2-methylphenyl)piperazin-1-yl)((1*RS*,2*SR*)-2-(4-fluorophenyl)cyclopropyl-1,2-*d*2)methanone (0.0799 g, 0.213 mmol, 70% (100% purity by ELSD)) as a colorless solid: Mp 100.2-102.7 °C; IR (CH₂Cl₂) 2917, 1632, 1512, 1431, 1221, 1032, 818, 729 cm⁻¹; ¹H NMR (400 MHz, CDCl₃) δ 7.16-7.11 (m, 2 H), 7.06 (d, *J = 8.2* Hz, 1 H), 7.00-6.94 (m, 3 H), 6.71 (d, *J* = 2.0 Hz, 1 H), 3.81-3.77 (m, 1 H), 3.72-3.59 (m, 2 H), 3.37-3.31 (m, 1 H), 2.79-2.69 (m, 2 H), 2.33-2.28 (m, 1 H), 2.24-2.20 (m, 4 H), 1.81 (d, *J=* 5.4 Hz, 1 H), 1.32 (d, *J =* 5.4 Hz, 1 H); ¹³C NMR (100 MHz, CDCl₃) δ 167.2, 161.5 (d, *J_{CF}* = 244.7 Hz), 151.8, 133.0 (d, *J_{CF} =* 3.1 Hz), 131.9, 131.7, 130.9, 129.0 (d, *J_{CF} =* 7.7 Hz), 123.5, 119.6, 114.9 (d, *J_{CF}* = 21.3 Hz), 51.7, 51.5, 45.5, 42.1, 23.4 (t, *J_{CD}* = 25.4 Hz), 23.0 (t, *J_{CD}* = 24.6 Hz) 17.3, 10.4; ¹⁹F NMR (376 MHz, CDCl₃) δ -116.3 (s, 1 F); HRMS (ESI) *m*/*z* calcd for C₂₁H₂₁D₂ClFN₂O ([M+H]⁺) 375.1603, found 375.1602.

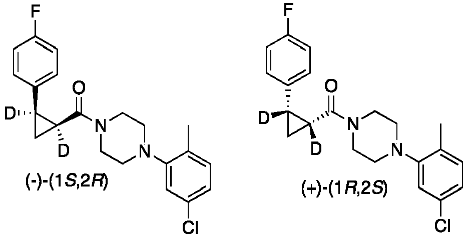

Racemic (4-(5-chloro-2-methylphenyl)piperazin-1-yl)((1*RS*,2*SR*)-2-(4-fluorophenyl)cyclopropyl-1,2-*d*2)methanone was separated on a SFC Chiralpak-IC semiprep (250 x 10 mm) column (30% Methanol:CO₂, 7 mL/min, p = 100 bar, 220 nm) injection volume 90 µL, 20 mg/mL) to give (4-(5-chloro-2-methylphenyl)piperazin-1-yl)((1*S*,2*R*)-2-(4-fluorophenyl)cyclopropyl-1,2-*d*2)methanone (retention time 7.58 min) as a colorless viscous oil (100% purity by ELSD): [α]¹⁸_{D} -152.0 (c 0.70, MeOH); ¹H NMR (300 MHz, CDCl₃) δ 7.16-7.12 (m, 2 H), 7.07 (d, *J* = 8.1 Hz, 1 H), 7.01-6.94 (m, 3 H), 6.72 *(d, J=* 1.8 Hz, 1 H), 3.82-3.76 (m, 1 H), 3.74-3.58 (m, 2 H), 3.39-3.31 (m, 1 H), 2.80-2.69 (m, 2 H), 2.80-2.69 (m, 2 H), 2.35-2.17 (m, 5 H), 1.82 (d, *J=* 5.4 Hz, 1 H), 1.33 (d, *J=* 5.4 Hz, 1 H). The enantiomeric excess was >99.9% ee (SFC Chiralpak-IC (250 x 10 mm); 30% Methanol:CO₂, 7 mL/min, p = 100 bar, 220 nm; retention time: 7.8 min). (4-(5-Chloro-2-methylphenyl)piperazin-1-yl)((1*R*,2*S*)-2-(4-fluorophenyl)cyclopropyl-1,2-*d*2)methanone (retention time 9.28 min) was obtained as a colorless viscous oil (100% purity by ELSD): [α]¹⁹_{D} +154.0 (c 0.66, MeOH); ¹H NMR (300 MHz, CDCl₃) δ 7.17-7.10 (m, 2 H), 7.07 (d, *J=* 8.2 Hz, 1 H), 7.02-6.94 (m, 3 H), 6.72 (d, *J=* 2.0 Hz, 1 H), 3.83-3.74 (m, 1 H), 3.73-3.58 (m, 2 H), 3.39-3.31 (m, 1 H), 2.80-2.69 (m, 2 H), 2.36-2.18 (m, 5 H), 1.82 (d, *J=* 5.4 Hz, 1 H), 1.33 (d, *J* = 5.4 Hz, 1 H). The enantiomeric excess was >99.9% ee (SFC Chiralpak-IC (250 x 10 mm); 30% Methanol: CO₂, 7 mL/min, p = 100 bar, 220 nm; retention time: 9.6 min).

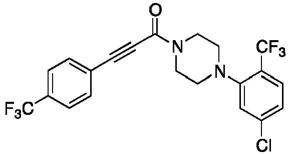

**1-(4-(5-Chloro-2-(trifluoromethyl)phenyl)piperazin-1-yl)-3-(4-(trifluoromethyl)phenyl)prop-2-yn-1-one.** A solution of 3-(4-(trifluoromethyl)phenyl)propiolic acid (0.100 g, 0.467 mmol) and 1-(5-chloro-2-(trifluoromethyl)phenyl)piperazinehydrochloride (0.155 g, 0.514 mmol) in CH₂Cl₂ (4.7 mL) cooled to 0 °C was treated with Et₃N (0.26 mL, 1.87 mmol). The cooled solution was treated with T3P (50 wt. % solution in EtOAc, 0.49 mL, 0.701 mmol) dropwise and the reaction was stirred at 0 °C for 30 min and allowed to warm to rt overnight, diluted with CH₂Cl₂ (30 mL) and washed with H₂O (20 mL), satd. aqueous NaHCO₃ (20 mL), dried (MgSO₄), filtered, and concentrated under reduced pressure. The crude material was purified by automated chromatography on SiO₂ (4g column, liquid load CH₂Cl₂, hexanes to 30% EtOAc/hexanes), to give 1-(4-(5-chloro-2-(trifluoromethyl)phenyl)piperazin-1-yl)-3-(4-(trifluoromethyl)phenyl)prop-2-yn-1-one (0.161 g, 0.350 mmol, 75%) as a colorless solid: Mp 145.8-148.8 °C; IR (CH₂Cl₂) 2928, 2827, 2223, 1634, 1596, 1432, 1322, 1310, 1122, 1107, 1033, 844 cm⁻¹; ¹H NMR (400 MHz, CDCl₃) δ 7.65 (app q, *J* = 7.3 Hz, 4 H), 7.58 (d, *J=* 8.4 Hz, 1 H), 7.28-7.24 (m, 2 H), 3.96 (app t, *J=* 5.0 Hz, 2 H), 3.84 (app t, *J=* 5.0 Hz, 2 H), 3.00 (app t, *J =* 5.0 Hz, 2 H), 2.94 (app t, *J =* 5.0 Hz, 2 H); ¹³C NMR (100 MHz, CDCl₃) δ 152.5, 138.8, 132.5, 131.6 (q, *J_{CF} =* 33.0 Hz), 128.5 (q, *J_{CF} =* 5.4 Hz), 125.7 (q, *J_{CF} =* 29.4 Hz), 125.7, 125.4 (q, *J_{CF}* = 3.7 Hz), 124.7, 124.1, 123.5 (q, *J_{CF} =* 270.8 Hz, 2 C), 89.0, 82.7, 53.6, 52.7, 47.3, 41.8; ¹⁹F NMR (376 MHz, CDCl₃) δ -60.3 (s, 3 F), -63.1 (s, 3 F); HRMS (ESI) *m*/*z* calcd for C₂₁H₁₆ClF₆N₂O ([M+H]⁺) 461.0850, found 461.0847.

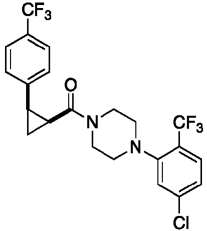

**(4-(5-Chloro-2-(trifluoromethyl)phenyl)piperazin-1-yl)((1RS,2SR)-2-(4-(trifluoromethyl)phenyl)-cyclopropyl)methanone.** A solution of 1-(4-(5-chloro-2-(trifluoromethyl)phenyl)piperazin-1-yl)-3-(4-(trifluoromethyl)phenyl)prop-2-yn-1-one (0.127 g, 0.276 mmol) in EtOAc (2.8 mL) was treated with quinoline (0.16 mL, 1.38 mmol) and 5% Pd/BaSO₄ (0.0059 g, equivalent to 1 mol% Pd). The reaction was placed under and atmosphere of H₂ (balloon) (3 vacuum/backfill cycles) and stirred at rt for 1.5 h, filtered through Celite, washed (EtOAc), and the combined filtrates were washed with 1 M aqueous HCl (10 mL), dried (MgSO₄), filtered, and concentrated under reduced pressure. The crude residue was purified by automated chromatography on SiO₂ (4 g column, liquid load CH₂Cl₂, hexanes to 30% EtOAc/hexanes; product eluted at 20% EtOAc) to give the product (0.114 g, 0.246 mmol) as a colorless solid.

To a flame dried 5 mL microwave vial containing anhydrous CrCl₂ (0.182 g, 0.148 mmol) was added a solution of (Z)-1-(4-(5-chloro-2-(trifluoromethyl)phenyl)piperazin-1-yl)-3-(4-(trifluoromethyl)-phenyl)prop-2-en-1-one (0.114 g, 0.246 mmol) in anhydrous THF (2.5 mL) and the mixture was sparged with Ar for 15 min and added CH₂ICl (0.14 mL, 1.23 mmol) at rt and under Ar atmosphere. The reaction mixture was stirred for 2 d at 80 °C. The reaction was cooled to rt, combined, quenched by the addition of EtOAc (50 mL) and washed with 1 M aqueous HCl (3 x 20 mL). The organic layer was dried (MgSO₄), filtered and concentrated under reduced pressure. The crude material was purified by automated chromatography on SiO₂ (4g column, liquid load CH₂Cl₂, 100% hexanes to 30% EtOAc/hexanes, product eluted at 20% EtOAc/hexanes) to give a clear oil that was filtered through basic Al₂O₃ (1:1 CH₂Cl₂/EtOAc) concentrated, and dried under high vacuum to give (4-(5-chloro-2-(trifluoromethyl)phenyl)piperazin-1-yl)((1RS,2SR)-2-(4-(trifluoromethyl)phenyl)cyclopropyl)methanone (0.0681 g, 0.143 mmol, 52% (2 steps) (100% purity by ELSD)) as a colorless solid: Mp 138.0-139.9 °C; IR (CH₂Cl₂) 3014, 2825, 1641, 1596, 1326, 1309, 1116, 1031, 844 cm⁻¹; ¹H NMR (400 MHz, CDCl₃) δ 7.56 (d, *J =* 8.2 Hz, 2 H), 7.51 (d, *J=* 8.5 Hz, 1 H), 7.29 (d, *J=* 8.2 Hz, 2 H), 7.19 (dd, *J=* 8.5, 1.4 Hz, 1 H), 6.89 (d, *J=* 1.4 Hz, 1 H), 3.95 (bd, *J=* 12.4 Hz, 1 H), 3.73 (bd, *J =* 12.4 Hz, 1 H), 3.54 (bt, *J* = 10.0 Hz, 1 H), 3.21 (bt, *J* = 10.0 Hz, 1 H), 2.74 (bt, *J* = 10.0 Hz, 2 H), 2.50 (td, *J=* 8.9, 7.1 Hz, 1 H), 2.29-2.19 (m, 2 H), 1.98 (bt, *J* = 8.9 Hz, 1 H), 1.92 (q, *J* = 6.2 Hz, 1 H), 1.43 (td, *J =* 8.4, 5.6 Hz, 1 H); ¹³C NMR (100 MHz, CDCl₃) δ 166.5, 152.7, 142.1, 138.9, 128.8 (q, *J_{CF}* = 32.6 Hz), 128.4 (q, *J_{CF}* = 5.8 Hz), 127.9, 125.7 (q, *J_{CF} =* 30.2 Hz), 125.6, 125.0 (q, *J_{CF}* = 3.7 Hz), 124.6 (q, *J_{CF} =* 273.0 Hz), 124.4, 123.6 (q, *J_{CF}* = 272.9 Hz), 53.7, 52.9, 45.5, 42.1, 24.7, 23.9, 11.2; ¹⁹F NMR (376 MHz, CDCl₃) δ -60.4 (s, 3 F), -62.2 (s, 3 F); HRMS (ESI) *m*/*z* calcd for C₂₂H₂₀ClF₆N₂O ([M+H]⁺) 477.1163, found 477.1160.

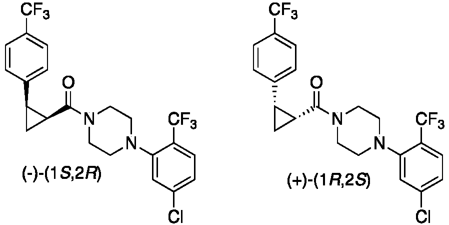

Racemic (4-(5-chloro-2-(trifluoromethyl)phenyl)piperazin-1-yl)((1*RS*,2*SR*)-2-(4-(trifluoromethyl)phenyl)-cyclopropyl)methanone was separated on a SFC Chiralpak-IC semiprep (250 x 10 mm) column (25% Methanol:CO₂, 7 mL/min, p = 100 bar, 220 nm) injection volume 90 µL, 20 mg/mL) to give (4-(5-chloro-2-(trifluoromethyl)phenyl)piperazin-1-yl)((1S,2R)-2-(4-(trifluoromethyl)phenyl)cyclopropyl)methanone (retention time 3.57 min) as a colorless viscous oil (100% purity by ELSD): [α]¹⁹_{D} -106.6 (c 0.68, MeOH); ¹H NMR (300 MHz, CDCl₃) δ 7.57 (d, *J=* 8.1 Hz, 2 H), 7.52 (d, *J= 8.4* Hz, 1 H), 7.29 *(d, J=* 8.1 Hz, 2 H), 7.20 (d, *J=* 8.4 Hz, 1 H), 6.88 (d, *J=* 0.9 Hz, 1 H), 3.98-3.93 (m, 1 H), 3.76-3.70 (m, 1 H), 3.54 (ddd, *J =* 12.3, 9.3, 3.0 Hz, 2 H), 3.21 (ddd, *J=* 12.3, 9.3, 3.0 Hz, 2 H), 2.76-2.71 (m, 2 H), 2.51 (td, *J* = 9.3, 7.2 Hz, 1 H), 2.30-2.18 (m, 2 H), 2.00-1.89 (m, 2 H), 1.44 (td, *J =* 8.4, 5.7 Hz, 1 H). The enantiomeric excess was >99.9% ee (SFC Chiralpak-IC (250 x 10 mm); 25% Methanol: CO₂, 7 mL/min, p = 100 bar, 220 nm; retention time: 3.8 min).

(4-(5-Chloro-2-(trifluoromethyl)phenyl)piperazin-1-yl)((1*R*,2*S*)-2-(4-(trifluoromethyl)phenyl)-cyclopropyl)methanone (retention time 4.20 min) was obtained as a colorless viscous oil (100% purity by ELSD): [α]¹⁹_{D} +111.1 (c 0.71, MeOH); ¹H NMR (300 MHz, CDCl₃) δ 7.57 (d, *J=* 8.1 Hz, 2 H), 7.52 (d, *J =* 8.4 Hz, 1 H), 7.29 (d, *J=* 8.1 Hz, 2 H), 7.20 (d, *J=* 8.4 Hz, 1 H), 6.88 (s, 1 H), 3.99-3.93 (m, 1 H), 3.76-3.70 (m, 1 H), 3.58-3.49 (m, 1 H), 3.25-3.16 (m, 1 H), 2.76-2.71 (m, 2 H), 2.51 (q, *J* = 9.6 Hz, 1 H), 2.30-2.18 (m, 2 H), 2.00-1.89 (m, 2 H), 1.44 (td, *J =* 8.4, 5.6 Hz, 1 H). The enantiomeric excess was >99.9% ee (SFC Chiralpak-IC (250 x 10 mm); 25% Methanol:CO₂, 7 mL/min, p = 100 bar, 220 nm; retention time: 4.1 min).

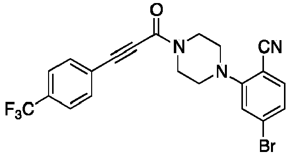

**4-Bromo-2-(4-(3-(4-(trifluoromethyl)phenyl)propioloyl)piperazin-1-yl)benzonitrile.** A solution of 3-(4-trifluoromethylphenyl)propiolic acid (0.400 g, 1.87 mmol) and 4-bromo-2-(piperazin-1-yl)benzonitrile hydrochloride (0.735 g, 2.43 mmol) in CH₂Cl₂ (19 mL) at 0 °C was treated Et₃N (1.0 mL, 7.47 mmol). The cooled solution was treated with T3P (50% solution in EtOAc) (2.0 mL, 2.80 mmol) dropwise and the reaction was stirred at 0 °C for 30 min, warmed to rt overnight, diluted with EtOAc (80 mL) and washed with H₂O (20 mL), satd. aqueous NaHCO₃ (20 mL), dried (MgSO₄), filtered, and concentrated under reduced pressure. The crude residue was purified by chromatography on SiO₂ (hexanes/EtOAc, 1:1), to give 4-bromo-2-(4-(3-(4-(trifluoromethyl)phenyl)propioloyl)piperazin-1-yl)benzonitrile (0.784 g, 1.70 mmol, 91%) as a pale yellow solid: Mp 176.3-179.1 °C; IR (CH₂Cl₂) 2824, 2222, 1626, 1583, 1432, 1324, 1163, 1129, 1107, 1035, 949, 928, 846, 816 cm⁻¹; ¹H NMR (500 MHz, CDCl₃) δ 7.66 (dd, *J* = 7.8 Hz, 4 H), 7.45 (d, *J=* 8.0 Hz, 1 H), 7.22 (dd, *J=* 8.0, 1.6 Hz, 1 H), 7.15 (d, *J=* 1.6 Hz, 1 H), 4.05 (t, *J=* 5.0 Hz, 2 H), 3.91 (t, *J=* 5.0 Hz, 2 H), 3.31 *(t, J=* 5.0 Hz, 2 H), 3.22 (t, *J=* 5.0 Hz, 2 H); ¹³C NMR (125 MHz, CDCl₃) δ 155.6, 152.6, 135.1, 132.6, 131.8 (q, *J_{CF} =* 32.8 Hz), 128.8, 125.9, 125.5 (q, *J_{CF} =* 3.8 Hz), 124.0, 123.5 (q, *J_{CF} =* 272.5 Hz), 122.7, 117.4, 105.1, 89.3, 82.5, 52.0, 50.7, 47.0, 41.5; ¹⁹F NMR (470 MHz, CDCl₃) δ -63.1 (s, 3 F); HRMS (ESI) *m*/*z* calcd for C₂₁H₁₆BrF₃N₃O ([M+H]⁺) 462.0423, found 462.0423.

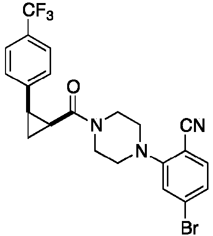

***4-Bromo-2-(4-((1RS,2SR)-2-(4-(trifluoromethyl)phenyl)cyclopropane-1-carbonyl)piperazin-1-*yl)benzonitrile.** A solution of 4-bromo-2-(4-(3-(4-(trifluoromethyl)phenyl)propioloyl)piperazin-1-yl)benzonitrile (0.750 g, 1.62 mmol) in EtOAc (16 mL) was treated with Lindlar's catalyst (5% Pd on CaCO₃, lead poisoned, 0.691 g, equivalent to 20 mol% Pd). The reaction was placed under a balloon of H₂ (3 vacuum/backfill cycles) and stirred at rt for 3 d, filtered through Celite, washed (EtOAc), and the combined filtrates were concentrated under reduced pressure. The crude residue was purified by chromatography on SiO₂ (hexanes/EtOAc, 1:4) to give (Z)-4-bromo-2-(4-(3-(4-(trifluoromethyl)phenyl)acryloyl)piperazin-1-yl)benzonitrile (0.318 g, 0.685 mmol) as a tan foam.

A solution of CrCl₂ (0.477 g, 0.899 mmol) and (Z)-4-bromo-2-(4-(3-(4-(trifluoromethyl)phenyl)-acryloyl)piperazin-1-yl)benzonitrile (0.300 g, 0.646 mmol) in dry degassed THF (6.5 mL) was sparged with Ar for 5 min and treated with CH₂ICl (0.37 mL, 3.23 mmol) at rt, stirred for 2 d at 80 °C, cooled to rt, diluted with EtOAc (150 mL) and washed with 1 M aqueous HCl (3 x 50 mL). The organic layer was dried (MgSO₄), filtered and concentrated under reduced pressure. The crude residue was purified by chromatography on SiO₂ (hexanes/EtOAc, 1:9) to give a colorless oil that was filtered through basic Al₂O₃ (EtOAc) concentrated and further dried under high vacuum to give 4-bromo-2-(4-((1*RS*,2*SR*)-2-(4-(trifluoromethyl)phenyl)cyclopropane-1-carbonyl)piperazin-1-yl)benzonitrile (0.148 g, 0.309 mmol, 20% (2 steps) (100% purity by ELSD)) as a colorless oil: IR (CH₂Cl₂) 2833, 2222, 1642, 1583, 1484, 1436, 1326, 1228, 1116, 1069, 844 cm⁻¹; ¹H NMR (400 MHz, CDCl₃) δ 7.52 (d, *J=* 8.5 Hz, 2 H), 7.38 (d, *J=* 8.0 Hz, 1 H), 7.27 (d, *J=* 8.5 Hz, 2 H), 7.17 (dd, *J=* 8.0, 1.5 Hz, 1 H), 6.90 (d, *J=* 1.5 Hz, 1 H), 3.93 (dt, *J=* 13.0, 3.5 Hz, 1 H), 3.78 (dt, *J=* 13.0, 3.5 Hz, 1 H), 3.67 (ddd, *J =* 13.0, 9.0, 3.0 Hz, 1H), 3.35 (ddd, *J=* 13.0, 9.0, 3.0 Hz, 1 H), 3.18-3.15 (m, 1 H), 3.08-3.06 (m, 1 H), 2.55-2.45 (m, 2 H), 2.29-2.24 (m, 2 H), 1.92 (q, *J=* 6.0 Hz, 1 H), 1.43 (td, *J =* 8.0, 6.0 Hz, 1 H); ¹³C NMR (125 MHz, CDCl₃) δ 166.8, 155.7, 141.8, 135.0, 128.8, 128.8 (q, *J_{CF} =* 32.6 Hz), 127.9, 125.7, 125.1 (q, *J_{CF} =* 3.6 Hz), 124.1 (q, *J_{CF} =* 271.8 Hz), 122.4, 117.5, 105.0, 52.1, 50.8, 45.2, 41.7, 24.5, 24.1, 11.2; ¹⁹F NMR (376 MHz, CDCl₃) δ -62.3 (s, 3 F); HRMS (ESI) *m*/*z* calcd for C₂₂H₂₀BrF₃N₃O ([M+H]⁺) 478.0736, found 478.0734.

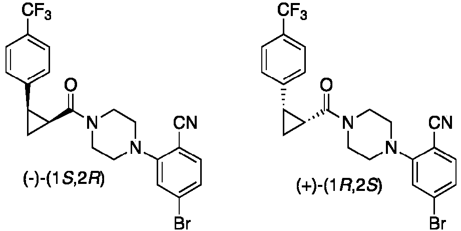

Racemic 4-bromo-2-(4-((1*RS,*2*SR*)-2-(4-(trifluoromethyl)phenyl)cyclopropane-1-carbonyl)piperazin-1-yl)benzonitrile was separated on a SFC Chiralpak-IC semiprep (250 x 10 mm) column (25% Methanol/CO₂, 7 mL/min, 220nm, p =100 bar, 20 mg/mL in MeOH) to give 4-bromo-2-(4-((1S,2R)-2-(4-(trifluoromethyl)-phenyl)cyclopropane-1-carbonyl)piperazin-1-yl)benzonitrile (retention time 10.5 min) as a colorless solid (100% purity by ELSD): [α]¹⁹_{D} -144.1 (c 1.31, MeOH); ¹H NMR (300 MHz, CDCl₃) δ 7.52 (d, *J=* 8.1 Hz, 2 H), 7.39 (d, *J=* 8.1 Hz, 1 H), 7.27 (d, *J=* 8.1 Hz, 2 H), 7.17 (dd, *J=* 8.1, 1.5 Hz, 1 H), 6.90 (d, *J=* 1.5 Hz, 1 H), 3.95-3.89 (m, 1 H), 3.76-3.67 (m, 2 H), 3.38-3.33 (m, 1 H), 3.16-3.04 (m, 2 H), 2.56-2.45 (m, 2 H), 2.27 (td, *J=* 8.7, 6.0 Hz, 2 H), 1.92 (q, *J=* 6.0 Hz, 1 H), 1.44 (td, *J=* 8.4, 5.5 Hz, 1 H). The enantiomeric excess was >99.9% ee (SFC Chiralpak-IC (250 x 10 mm); 25% MeOH, 7 mL/min, 220nm, p =100 bar; retention time: 10.8 min).

4-Bromo-2-(4-((1*R*,2*S*)-2-(4-(trifluoromethyl)phenyl)cyclopropane-1-carbonyl)piperazin-1-yl)benzonitrile (retention time 11.6 min) was obtained as a colorless solid (100% purity by ELSD): [α]¹⁹_{D} +135.8 (c 1.43, MeOH); ¹H NMR (300 MHz, CDCl₃) δ 7.52 *(d, J=* 8.1 Hz, 2 H), 7.39 *(d, J=* 8.1 Hz, 1 H), 7.29 (s, 2 H), 7.17 (dd, *J=* 8.1, 1.5 Hz, 1 H), 6.90 (d, *J=* 1.5 Hz, 1 H), 3.93-3.90 (m, 1 H), 3.78-3.67 (m, 2 H), 3.38-3.33 (m, 1 H), 3.17-3.04 (m, 2 H), 2.56-2.43 (m, 2 H), 2.27 (td, *J=* 8.7, 6.0 Hz, 2 H), 1.92 (q, *J =* 6.0 Hz, 1 H), 1.44 (td, *J =* 8.4, 5.6 Hz, 1 H). The enantiomeric excess was >99.9% ee (SFC Chiralpak-IC (250 x 10 mm); 25% MeOH, 7 mL/min, 220nm, p =100 bar; retention time: 11.8 min).

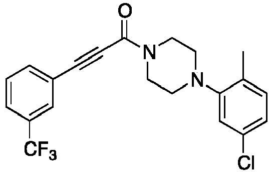

**1-(4-(5-Chloro-2-methylphenyl)piperazin-1-yl)-3-(3-(trifluoromethyl)phenyl)prop-2-yn-1-one.** A solution of 3-(3-(trifluoromethyl)phenyl)propionic acid (0.0750 g, 0.350 mmol) and 1-(5-chloro-2-methylphenyl)piperazine hydrochloride (0.104 g, 0.420 mmol) in CH₂Cl₂ (3.5 mL) cooled to 0 °C was treated with Et₃N (0.15 mL, 1.05 mmol). The cooled solution was treated with T3P (50 wt. % solution in EtOAc 0.37 mL, 0.525 mmol) dropwise and the reaction was stirred at 0 °C for 30 min, warmed to rt overnight, diluted with CH₂Cl₂ (30 mL), washed with 1 M aqueous HCl (20 mL), dried (MgSO₄), filtered, and concentrated under reduced pressure. The crude material was purified by automated chromatography on SiO₂ (4g column, liquid load CH₂Cl₂, hexanes to 40% EtOAc/hexanes), to give 1-(4-(5-chloro-2-methylphenyl)piperazin-1-yl)-3-(3-(trifluoromethyl)phenyl)prop-2-yn-1-one (0.106 g, 0.261 mmol, 75%) as a colorless solid: Mp 130.3-132.8 °C; IR (CH₂Cl₂) 2820, 2218, 2161, 1631, 1489, 1435, 1201, 1128, 1041, 805, 695 cm⁻¹; ¹H NMR (400 MHz, CDCl₃) δ 7.81 (s, 1 H), 7.74 *(d, J=* 7.8 Hz, 1 H), 7.68 *(d, J=* 7.8 Hz, 1 H), 7.52 (t, *J=* 7.8 Hz, 1 H), 7.12 (d, *J=* 8.1 Hz, 1 H), 7.00 (dd, *J=* 8.1, 2.1 Hz, 1 H), 6.96 (d, *J=* 2.1 Hz, 1 H), 3.98 (app t, *J =* 5.0 Hz, 2 H), 3.84 (app t, *J=* 5.0 Hz, 2 H), 2.98 (app t, *J* = 5.0 Hz, 2 H), 2.90 (app t, *J =* 5.0 Hz, 2 H), 2.29 (s, 3 H); ¹³C NMR (100 MHz, CDCl₃) δ 152.6, 151.6, 135.4, 132.1, 131.8, 131.2 (q, *J_{CF} =* 33.0 Hz), 130.9, 129.17, 129.0 (q, *J_{CF} =* 4.1 Hz), 126.6 (q, *J_{CF} =* 3.6 Hz), 123.8, 123.5 (q, *J_{CF} =* 272.5 Hz), 121.4, 119.8, 88.9, 82.1, 51.9, 51.3, 47.4, 41.9; HRMS (ESI) *m*/*z* calcd for C₂₁H₁₉ClF₃N₂O ([M+H]⁺) 407.1133, found 407.1130.

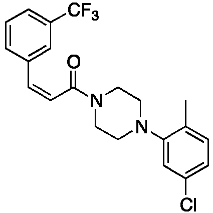

**(Z)-1-(4-(5-Chloro-2-methylphenyl)piperazin-1-yl)-3-(3-(trifluoromethyl)phenyl)prop-2-en-1-one.** To a solution of 1-(4-(5-chloro-2-methylphenyl)piperazin-1-yl)-3-(3-(trifluoromethyl)phenyl)prop-2-yn-1-one (0.100 g, 0.246 mmol) in EtOAc (2.5 mL) was added Lindlar's catalyst (5% Pd on CaCO₃, lead poisoned, 0.0105 g, equivalent to 2 mol% Pd) and quinoline (0.015 mL, 0.123 mmol). The reaction vessel was placed under vacuum and backfilled with H₂ (balloon, 4x) stirred for 3.5 h at rt, filtered through Celite, washed (EtOAc), and the combined filtrates were washed with 1 M aqueous HCl, dried (Na₂SO₄), filtered, and concentrated under reduced pressure. The crude residue was purified by automated chromatography on SiO₂ (4g column, liquid load CH₂Cl₂, 10% EtOAc/hexanes to 40% EtOAc/hexanes) to give (Z)-1-(4-(5-chloro-2-methylphenyl)piperazin-1-yl)-3-(3-(trifluoromethyl)phenyl)prop-2-en-1-one (0.0932 g, 0.228 mmol, 93%) as a clear colorless solid: Mp 130.3-132.8 °C; IR (CH₂Cl₂) 2918, 1619, 1489, 1440, 1327, 1161, 1096, 1074, 805, 737, 696 cm⁻¹; ¹H NMR (400 MHz, CDCl₃) δ 7.66 (s, 1 H), 7.59 (t, *J=* 7.6 Hz, 2 H), 7.49 *(t, J=* 7.6 Hz, 1 H), 7.06 (d, *J=* 8.1 Hz, 1 H), 6.96 (dd, *J=* 8.1, 2.1 Hz, 1 H), 6.77 (d, *J=* 2.1 Hz, 1 H), 6.73 (d, *J=* 12.5 Hz, 1 H), 6.19 (d, *J=* 12.5 Hz, 1 H), 3.80 (app t, *J=* 5.0 Hz, 2 H), 3.48 (app t, *J=* 5.0 Hz, 2 H), 2.80 (app t, *J* = 5.0 Hz, 2 H), 2.49 (app t, *J =* 5.0 Hz, 2 H), 2.20 (s, 3 H); ¹³C NMR (100 MHz, CDCl₃) δ 166.7, 151.7, 136.1, 132.0 (2 C), 131.8, 131.6, 131.4 (q, *J_{CF} =* 30.4 Hz), 130.9, 129.2, 125.2 (q, *J_{CF} =* 4.0 Hz), 125.0 (q, *J_{CF}* = 3.8 Hz), 124.9, 123.8 (q, *J_{CF} =* 272.3 Hz), 123.7, 119.7, 51.4, 51.1, 46.6, 41.5, 17.3; HRMS (ESI) *m*/*z* calcd for C₂₁H₂₁ClF₃N₂O ([M+H]⁺) 409.1289, found 409.1289.

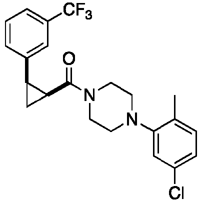

**(4-(5-Chloro-2-methylphenyl)piperazin-1-yl)((1*RS*,2*SR*)-2-(3-(trifluoromethyl)phenyl)cyclopropyl)-methanone.** A solution of CrCl₂ (0.105 g, 0.851 mmol) and (Z)-1-(4-(5-chloro-2-methylphenyl)piperazin-1-yl)-3-(3-(trifluoromethyl)phenyl)prop-2-en-1-one (0.0580 g, 0.142 mmol) in dry degassed THF (1.4 mL)(previously sparged with Ar for 15 min) was treated with CH₂ICl (82 uL, 0.709 mmol) and the mixture was sparged with Ar for 2 min, stirred for 20 h at 80 °C, cooled to rt, diluted with Et₂O (50 mL) and washed with 1 M HCl (3 x 20 mL). The organic layer was dried (MgSO₄), filtered and concentrated under reduced pressure. The crude material was purified by automated chromatography on SiO₂ (4g column, liquid load CH₂Cl₂, 100% hexanes to 40% EtOAc/hexanes) to give a clear oil that filtered through basic Al₂O₃ (CH₂Cl₂/EtOAc, 1:1), recrystallized from hot cyclohexane the mother liquor was decanted and the crystals were washed with hexanes (2 x 1 mL) and dried under high vacuum to give (4-(5-chloro-2-methylphenyl)-piperazin-1-yl)((1*RS*,2*SR*)-2-(3-(trifluoromethyl)phenyl)cyclopropyl)methanone (0.0356 g, 0.0842 mmol, 59% (100% purity by ELSD)) as colorless needles: Mp 115.6-117.4 °C; IR (CH₂Cl₂) 2914, 2820, 1639, 1593, 1490, 1437, 1225, 1161, 1123, 1074, 806 cm⁻¹; ¹H NMR (400 MHz, CDCl₃) δ 7.49-7.48 (m, 2 H), 7.40 (t, *J=* 8.0 Hz, 1 H), 7.32 (d, *J=* 8.0 Hz, 1 H), 7.06 (d, *J=* 8.0 Hz, 1 H), 6.95 (dd, *J=* 8.0, 2.1 Hz, 1 H), 6.67 (d, *J=* 2.1 Hz, 1 H), 3.86 (dt, *J=* 13.0, 2.9 Hz, 1 H), 3.74 (dt, *J=* 13.0, 3.2 Hz, 1 H), 3.62-3.56 (m, 1 H), 3.26 (ddd, *J =* 12.4, 9.0, 3.2 Hz, 1 H), 2.79-2.70 (m, 2 H), 2.53 (td, *J =* 9.0, 6.9 Hz, 1 H), 2.26 (ddd, *J =* 9.0, 8.4, 5.9 Hz, 1 H), 2.20 (s, 3 H), 2.10 (ddd, *J =* 11.2, 9.0, 2.4 Hz, 1 H), 1.91 (app q, *J =* 5.8 Hz, 1 H), 1.41 (td, *J* = 8.4, 5.8 Hz, 1 H); ¹³C NMR (100 MHz, CDCl₃) δ 166.7, 151.7, 138.7, 131.9, 131.8, 130.9, 130.5, 130.5 (d, *J_{CF} =* 32.2 Hz) 128.7, 125.1 (d, *J_{CF} =* 4.0 Hz), 124.2 (d, *J_{CF} =* 272.2 Hz), 123.6, 123.3 (d, *J_{CF} =* 3.7 Hz), 119.6, 51.7, 51.6, 45.5, 42.2, 24.1, 23.9, 17.3, 10.8; ¹⁹F NMR (376 MHz, CDCl₃) δ -62.4 (s, 3 F); HRMS (ESI) *m*/*z* calcd for C₂₂H₂₃ClF₃N₂O ([M+H]⁺) 423.1446, found 423.1443.

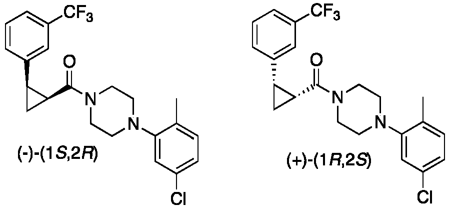

Racemic (4-(5-chloro-2-methylphenyl)piperazin-1-yl)((1*RS*,2*SR*)-2-(3-(trifluoromethyl)phenyl)-cyclopropyl)methanone was separated on a SFC Chiralpak-IC semiprep (250 x 10 mm) column (30% Methanol:CO₂, 7 mL/min, p = 100 bar, 220 nm) injection volume 90 µL, 20 mg/mL) to give (4-(5-chloro-2-methylphenyl)piperazin-1-yl)((1*S*,2*R*)-2-(3-(trifluoromethyl)phenyl)cyclopropyl)methanone (retention time 4.82 min) as a colorless viscous oil (100% purity by ELSD): [α]¹⁹_{D} -133.5 (c 0.70, MeOH); ¹H NMR (300 MHz, CDCl₃) δ 7.48 (bs, 2 H), 7.41 (t, *J=* 8.0 Hz, 1H), 7.33 (d, *J=* 7.4 Hz, 1 H), 7.06 (d, *J=* 8.1 Hz, 1H), 6.95 (dd, *J=* 8.1, 2.0 Hz, 1 H), 6.67 (d, *J=* 2.0 Hz, 1 H), 3.89-3.82 (m, 1 H), 3.78-3.71 (m, 1 H), 3.63-3.55 (m, 1 H), 3.31-3.23 (m, 1 H), 2.79-2.69 (m, 2H), 2.53 (td, *J* = 8.4*,* 7.2 Hz, 1 H), 2.30-2.07 (m, 6 H), 1.92 (q, *J=* 5.4 Hz, 1 H), 1.41 (td, *J=* 8.4, 5.4 Hz, 1 H). The enantiomeric excess was >99.9% ee (SFC Chiralpak-IC (250 x 10 mm); 30% Methanol:CO₂, 7 mL/min, p = 100 bar, 220 nm; retention time: 5.0 min).

(4-(5-Chloro-2-methylphenyl)piperazin-1-yl)((1*R*,2*S*)-2-(3-(trifluoromethyl)phenyl)cyclopropyl)methanone (retention time 5.57 min) was obtained as a colorless viscous oil (100% purity by ELSD): [α]¹⁹_{D} +133.1 (c 0.65, MeOH); ¹H NMR (300 MHz, CDCl₃) δ 7.48 (bs, 2 H), 7.41 (t, *J=* 8.0 Hz, 1 H), 7.33 (d, *J =* 8.0 Hz, 1H), 7.06 (d, *J =* 8.0 Hz, 1H), 6.95 (dd, *J=* 8.0, 2.0 Hz, 1 H), 6.67 (d, *J* = 2.0 Hz, 1 H), 3.90-3.82 (m, 1 H), 3.78-3.70 (m, 1 H), 3.64-3.55 (m, 1 H), 3.31-3.23 (m, 1 H), 2.80-2.69 (m, 2 H), 2.53 (td, *J=* 8.4, 6.8 Hz, 1 H), 2.30-2.07 (m, 6 H), 1.92 (q, *J=* 5.4 Hz, 1 H), 1.41 (td, *J=* 8.4, 5.4 Hz, 1 H). The enantiomeric excess was >99.9% ee (SFC Chiralpak-IC (250 x 10 mm); 30% Methanol:CO₂, 7 mL/min, p = 100 bar, 220 nm; retention time: 5.7 min).

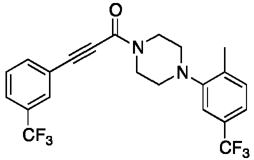

**1-(4-(2-Methyl-5-(trifluoromethyl)phenyl)piperazin-1-yl)-3-(3-(trifluoromethyl)phenyl)prop-2-yn-1-one.** A solution of 3-(3-(trifluoromethyl)phenyl)propionic acid (0.0763 g, 0.356 mmol) and 1-(2-methyl-5-(trifluoromethyl)phenyl)piperazine hydrochloride (0.100 g, 0.356 mmol) in CH₂Cl₂ (3.6 mL) cooled to 0 °C was treated with Et₃N (0.20 mL, 1.42 mmol). The cooled solution was treated with T3P (50 wt. % solution in EtOAc, 0.38 mL, 0.534 mmol) dropwise and the reaction was stirred at 0 °C for 30 min, warmed to rt overnight, diluted with CH₂Cl₂ (30 mL), washed with 1 M aqueous HCl (20 mL), dried (MgSO₄), filtered, and concentrated under reduced pressure. The crude residue was purified by automated chromatography on SiO₂ (4g column, liquid load CH₂Cl₂, 100% hexanes to 40% EtOAc/hexanes), to give 1-(4-(2-methyl-5-(trifluoromethyl)phenyl)piperazin-1-yl)-3-(3-(trifluoromethyl)phenyl)prop-2-yn-1-one (0.0819 g, 0.186 mmol, 52%) as a pink solid: Mp 121.8-125.2 °C; IR (CH₂Cl₂) 2924, 2217, 1633, 1436, 1333, 1310, 1166, 1076, 1042, 695 cm⁻¹; ¹H NMR (400 MHz, CDCl₃) 6 7.81 (s, 1 H), 7.74 *(d, J=* 7.8 Hz, 1 H), 7.67 *(d, J=* 7.8 Hz, 1 H), 7.52 (t, *J=* 7.8 Hz, 1 H), 7.30 (d, *J=* 8.1 Hz, 1 H), 7.26 (d, *J* = 8.1 Hz, 1 H), 7.22 (s, 1 H), 4.00 (app t, *J=* 5.0 Hz, 2 H), 3.86 (app t, *J* = 4.8 Hz, 2 H), 3.03 (app t, *J=* 5.0 Hz, 2 H), 2.93 (app t, *J=* 5.0 Hz, 2 H), 2.39 (s, 3 H); ¹³C NMR (100 MHz, CDCl₃) δ 152.6, 150.9, 136.8, 135.4, 135.4, 131.5, 131.2 (q, *J_{CF} =* 33.0 Hz), 129.2 (s), 129.0 (q, *J_{CF} =* 32.2 Hz), 129.0 (q, *J_{CF} =* 3.9 Hz), 126.6 (q, *J_{CF} =* 3.7 Hz), 124.0 (q, *J_{CF} =* 272.0 Hz), 123.4 (q, *J_{CF} =* 272.3 Hz), 121.3, 120.5 (q, *J_{CF} =* 3.8 Hz), 88.9, 82.0, 51.8, 51.3, 47.4, 41.9, 17.9; ¹⁹F NMR (376 MHz, CDCl₃) δ -62.3 (s, 3 F), -63.0 (s, 3 F); HRMS (ESI) *m*/*z* calcd for C₂₂H₁₉F₆N₂O ([M+H]⁺) 441.1396, found 441.1391.

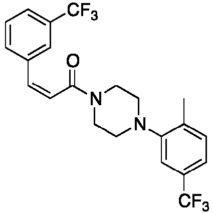

**(Z)-1-(4-(2-Methyl-5-(trifluoromethyl)phenyl)piperazin-1-yl)-3-(3-(trifluoromethyl)phenyl)prop-2-en-1-one.** A solution of 1-(4-(2-methyl-5-(trifluoromethyl)phenyl)piperazin-1-yl)-3-(3-(trifluoromethyl)-phenyl)prop-2-yn-1-one (0.0800 g, 0.182 mmol) in EtOAc (1.8 mL) was treated with Lindlar's catalyst (5% Pd on CaCO₃, lead poisoned, 0.0193 g, equivalent to 5 mol% Pd). The reaction was placed under a balloon of H₂ (3 vacuum/backfill cycles) and stirred at rt for 2 d, filtered through Celite, washed (EtOAc), and concentrated under reduced pressure. The crude residue was purified by automated chromatography on SiO₂ (4g column, liquid load CH₂Cl₂, 100% hexanes to 40% EtOAc/hexanes, product eluted at 10% EtOAc/hexanes) to give the (Z)-1-(4-(2-methyl-5-(trifluoromethyl)phenyl)piperazin-1-yl)-3-(3-(trifluoromethyl)phenyl)prop-2-en-1-one (0.0572 g, 0.129 mmol, 71%) as clear/tan viscous oil: IR (CH₂Cl₂) 2922, 1621, 1443, 1418, 1329, 1309, 1163, 1076, 1044, 823 cm⁻¹; ¹H NMR (400 MHz, CDCl₃) δ 7.68 (s, 1 H), 7.59 (t, *J=* 7.7 Hz, 2 H), 7.49 (t, *J=* 7.7 Hz, 1 H), 7.27-7.22 (m, 2 H), 7.01 (s, 1 H), 6.74 (d, *J =* 12.5 Hz, 1 H), 6.20 (d, *J=* 12.5 Hz, 1 H), 3.82 (bt, *J=* 4.1 Hz, 2 H), 3.50 (app t, *J=* 5.0 Hz, 2 H), 2.82 (app t, *J=* 5.0 Hz, 2 H), 2.47 (app t, *J=* 5.0 Hz, 2 H), 2.30 (s, 3 H); ¹³C NMR (100 MHz, CDCl₃) δ 166.7, 151.0, 136.8, 136.2, 132.0, 131.6, 131.4, 131.2 (q, *J_{CF} =* 32.1 Hz), 129.2, 129.0 (q, *J_{CF} =* 32.3 Hz), 125.2 (q, *J_{CF} =* 3.7 Hz), 125.0 (q, *J_{CF} = 3.8* Hz), 124.9, 124.1 (q, *J_{CF}* = 271.9 Hz), 123.8 (q, *J_{CF} =* 272.5 Hz), 120.4 (q, *J_{CF} =* 3.9 Hz), 116.0 (q, *J_{CF} =* 3.7 Hz), 51.3, 51.1, 46.6, 41.5, 17.8; ¹⁹F NMR (376 MHz, CDCl₃) δ -62.4 (s, 3 F), -62.7 (s, 3 F); HRMS (ESI) *m*/*z* calcd for C₂₂H₂₁F₆N₂O ([M+H]⁺) 443.1553, found 443.1551.

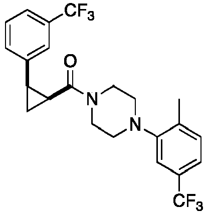

**(4-(2-Methyl-5-(trifluoromethyl)phenyl)piperazin-1-yl)((1RS,2SR)-2-(3-(trifluoromethyl)phenyl)-cyclopropyl)methanone.** A solution of CrCl₂ (0.0955 g, 0.777 mmol) and (Z)-1-(4-(2-methyl-5-(trifluoromethyl)phenyl)piperazin-1-yl)-3-(3-(trifluoromethyl)phenyl)prop-2-en-1-one (0.0573 g, 0.130 mmol) in dry degassed THF (1.3 mL) was sparged with Ar for 5 min and treated with CH₂ICl (75 uL, 0.648 mmol) at rt and under Ar atmosphere, heated for 20 h at 80 °C, cooled to rt, diluted with Et₂O (50 mL), and washed with 1 M aqueous HCl (3 x 20 mL). The organic layer was dried (MgSO₄), filtered and concentrated under reduced pressure. The crude material was purified by automated chromatography on SiO₂ (4g column, liquid load CH₂Cl₂, 100% hexanes to 40% EtOAc/hexanes) to give a clear oil that was filtered through basic Al₂O₃ (CH₂Cl₂/EtOAc, 1:1), recrystallized from CH₂Cl₂/hexanes (ca. 1:5) the mother liquor was decanted and the clear colorless cubes were washed with hexanes (2 x 1 mL) and dried under high vacuum to give (4-(2-methyl-5-(trifluoromethyl)phenyl)piperazin-1-yl)((1RS,2SR)-2-(3-(trifluoromethyl)phenyl)cyclopropyl)methanone (0.0115 g, 0.0252 mmol, 20% (100% purity by ELSD)) as a colorless solid: Mp 113.4-116.8 °C; IR (CH₂Cl₂) 2914, 2824, 1641, 1418, 1328, 1309, 1163, 1121, 1073 cm⁻¹; ¹H NMR (400 MHz, CDCl₃) δ 7.47 (bd, *J =* 7.1 Hz, 2 H), 7.41 *(t, J=* 7.8 Hz, 1 H), 7.33 *(d, J=* 7.8 Hz, 1 H), 7.24 (s, 2 H), 6.92 (s, 1 H), 3.94 (d, *J=* 12.2 Hz, 1 H), 3.79 (d, *J* = 12.2 Hz, 1 H), 3.58 (ddd, *J =* 12.2, 9.2, 3.0 Hz, 1 H), 3.24 (ddd, *J=* 12.2, 9.2, 2.6 Hz, 1 H), 2.77 (td, *J=* 12.2, 3.7 Hz, 2 H), 2.53 (td, *J=* 9.2, 7.0 Hz, 1 H), 2.29 (s, 3 H), 2.28-2.18 (m, 2 H), 2.11-2.05 (m, 1 H), 1.92 (q, *J=* 6.0 Hz, 1 H), 1.42 (td, *J =* 8.5, 6.0 Hz, 1 H); ¹³C NMR (100 MHz, CDCl₃) δ 166.7, 151.1, 138.8, 136.9, 131.4, 130.6 (q, *J_{CF} =* 31.2 Hz), 130.5, 129.1 (q, *J_{CF} =* 32.1 Hz), 128.7, 125.1 (q, *J_{CF} =* 3.7 Hz), 124.2 (q, *J_{CF} =* 272.3 Hz, 2 C), 123.2 (q, *J_{CF}* = 3.8 Hz), 120.4 (q, *J_{CF}* = 3.8 Hz), 116.1 (q, *J_{CF}* = 3.7 Hz), 51.7 (2 C), 45.6, 42.2, 24.2, 24.0, 17.8, 10.9; ¹⁹F NMR (376 MHz, CDCl₃) δ -62.4 (s, 3 H), -62.6 (s, 3 H); HRMS (ESI) *m*/*z* calcd for C₂₂H₂₃F₆N₂O ([M+H]⁺) 457.1709, found 457.1704.

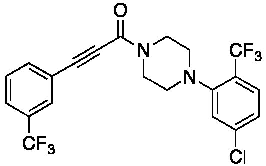

**1-(4-(5-Chloro-2-(trifluoromethyl)phenyl)piperazin-1-yl)-3-(3-(trifluoromethyl)phenyl)prop-2-yn-1-one.** A solution of 3-(3-(trifluoromethyl)phenyl)propiolic acid (0.100 g, 0.467 mmol) and 1-(5-chloro-2-(trifluoromethyl)phenyl)piperazinehydrochloride (0.155 g, 0.514 mmol) in CH₂Cl₂ (4.7 mL) cooled to 0 °C was treated with Et₃N (0.26 mL, 1.87 mmol). The cooled solution was treated with T3P (50 wt. % solution in EtOAc, 0.49 mL, 0.701 mmol) dropwise and the reaction was stirred at 0 °C for 30 min, warmed to rt overnight, diluted with CH₂Cl₂ (30 mL) and washed with H₂O (20 mL), satd. aqueous NaHCO₃ (20 mL), dried (MgSO₄), filtered, and concentrated under reduced pressure. The crude residue was purified by automated chromatography on SiO₂ (4g column, liquid load CH₂Cl₂, 100% hexanes to 30% EtOAc/ hexanes), to give the crude product that was contaminated with the piperazine starting material (ca 10%). The product was dissolved in hot absolute EtOH then stored overnight in the freezer (-20 °C) to facilitate crystallization. The supernatant was removed and the crystals were washed with hexanes and then dried under high vacuum overnight to give 1-(4-(5-chloro-2-(trifluoromethyl)phenyl)piperazin-1-yl)-3-(3-(trifluoromethyl)phenyl)prop-2-yn-1-one (0.168 g, 0.365 mmol, 78%) as a colorless solid: Mp 87.6-91.2 °C; IR (CH₂Cl₂) 2921, 2827, 2222, 1633, 1596, 1436, 1332, 1309, 1121, 1034, 695 cm⁻¹; ¹H NMR (400 MHz, CDCl₃) δ 7.78 (s, 1 H), 7.71 (d, *J=* 7.8 Hz, 1 H), 7.65 (d, *J=* 8.5 Hz, 1 H), 7.56 (d, *J=* 8.5 Hz, 1 H), 7.50 (t, *J=* 7.8 Hz, 1 H), 7.26-7.22 (m, 2 H), 3.95 (app t, *J=* 5.0 Hz, 2 H), 3.81 (app t, *J=* 5.0 Hz, 2 H), 2.99 (app t, *J=* 5.0 Hz, 2 H), 2.92 (app t, *J=* 5.0 Hz, 2 H); ¹³C NMR (100 MHz, CDCl₃) δ 152.6, 138.8, 135.4, 131.2 (q, *J_{CF} =* 33.0 Hz), 129.2, 129.0 (q, *J_{CF} =* 3.7 Hz), 128.6 (q, *J_{CF} =* 5.4 Hz), 126.6 (q, *J_{CF} =* 3.7 Hz), 125.8 (q, *J_{CF}* = 29.5 Hz), 125.7, 124.7, 123.5 (q, *J_{CF} =* 273.0 Hz), 123.2 (q, *J_{CF} =* 272.5 Hz), 121.3, 88.9, 82.0, 53.6, 52.7, 47.4, 41.8; ¹⁹F NMR (376 MHz, CDCl₃) δ -60.3 (s, 3 F), -63.0 (s, 3 F); HRMS (ESI) *m*/*z* calcd for C₂₁H₁₆ClF₆N₂O ([M+H]⁺) 461.0850, found 461.0849.

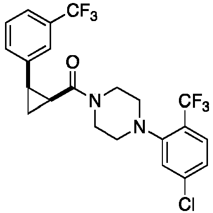

**(4-(5-Chloro-2-(trifluoromethyl)phenyl)piperazin-1-yl)(2-(3-(trifluoromethyl)phenyl)cyclopropyl)-methanone.** A solution of 1-(4-(5-chloro-2-(trifluoromethyl)phenyl)piperazin-1-yl)-3-(3-(trifluoromethyl)-phenyl)prop-2-yn-1-one (0.0684 g, 0.148 mmol) in EtOAc (1.5 mL) was treated with Lindlar's catalyst (5% Pd on CaCO₃, lead poisoned, 0.0158 g, equivalent to 5 mol% Pd). The reaction was placed under a balloon of H₂ (3 vacuum/backfill cycles) and stirred at rt for 3 d, filtered through Celite, washed (EtOAc), and the combined filtrates were concentrated under reduced pressure. The crude residue was purified by automated chromatography on SiO₂ (4g column, liquid load CH₂Cl₂, 100% hexanes to 40% EtOAc:hexanes) to give (Z)-1-(4-(5-chloro-2-(trifluoromethyl)phenyl)piperazin-1-yl)-3-(3-(trifluoromethyl)phenyl)prop-2-en-1-one (0.0512 g, 0.111 mmol) as a colorless solid.

A solution of CrCl₂ (0.816 g, 0.940 mmol) and (Z)-1-(4-(5-chloro-2-(trifluoromethyl)phenyl)-piperazin-1-yl)-3-(3-(trifluoromethyl)phenyl)prop-2-en-1-one (0.0512 g, 0.111 mmol) in dry degassed THF (1.6 mL) was sparged with Ar for 5 min and treated with CH₂ICl (0.064 mL, 0.553 mmol) at rt, heated for 2 d at 80 °C, cooled to rt, diluted with EtOAc (50 mL), and washed with 1 M aqueous HCl (3 x 20 mL). The organic layer was dried (MgSO₄), filtered and concentrated under reduced pressure. The crude residue was purified by automated chromatography on SiO₂ (4g column, liquid load CH₂Cl₂, hexanes to 30% EtOAc/hexanes, product eluted at 20% EtOAc/hexanes) to give a clear oil that was filtered through basic Al₂O₃ (eluting with 1:1 CH₂Cl₂/EtOAc), concentrated, and dried under high vacuum to give (4-(5-chloro-2-(trifluoromethyl)phenyl)piperazin-1-yl)(2-(3-(trifluoromethyl)phenyl)cyclopropyl)methanone (0.0409 g, 0.0858 mmol, 59% (2 steps) (100% purity by ELSD)) as a clear colorless oil: IR (CH₂Cl₂) 2918, 1641, 1596, 1437, 1327, 1120, 1032, 809, 702 cm⁻¹; ¹H NMR (400 MHz, CDCl₃) δ 7.54-7.50 (m, 3 H), 7.44 *(t, J=* 8.0 Hz, 1 H), 7.34 (d, *J=* 8.0 Hz, 1 H), 7.20 (dd, *J=* 8.5, 1.4 Hz, 1 H), 6.87 (d, *J=* 1.6 Hz, 1 H), 4.02 *(d, J=* 13.0 Hz, 1 H), 3.80 (d, *J=* 13.0 Hz, 1 H), 3.51 (ddd, *J=* 12.8, 9.8, 3.0 Hz, 1 H), 3.13 (ddd, *J=* 12.5, 9.8, 2.7 Hz, 1 H), 2.73 (td, *J=* 7.7, 3.6 Hz, 2 H), 2.52 (td, *J =* 9.0, 7.0 Hz, 1 H), 2.25 (ddd, *J* = 9.0, 8.4, 6.0 Hz, 1 H), 2.21-2.15 (m, 1 H), 1.97-1.90 (m, 2 H), 1.42 (td, *J =* 8.4, 6.0 Hz, 1 H); ¹³C NMR (100 MHz, CDCl₃) δ 166.6, 152.7, 138.9, 138.7, 130.6 (q, *J_{CF} =* 32.1 Hz), 130.5, 128.7, 128.3 (q, *J_{CF} =* 5.4 Hz), 125.8 (q, *J_{CF} =* 29.4 Hz), 125.6, 125.1 (q, *J_{CF}* = 3.8 Hz), 124.6, 124.2 (q, *J_{CF}* = 272.4 Hz), 123.5 (q, *J_{CF}* = 272.9 Hz), 123.2 (q, *J_{CF} =* 3.7 Hz), 53.5, 53.0, 45.5, 42.1, 24.3, 23.9, 10.9; ¹⁹F NMR (376 MHz, CDCl₃) δ -60.4 (s, 3 F), -62.4 (s, 3 F); HRMS (ESI) *m*/*z* calcd for C₂₂H₂₀ClF₆N₂O ([M+H]⁺) 477.1163, found 477.1166.

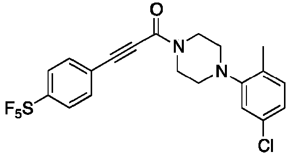

**1-(4-(5-Chloro-2-methylphenyl)piperazin-1-yl)-3-(4-(pentafluoro-λ6-sulfaneyl)phenyl)prop-2-yn-1-one.** A solution of 3-(4-(pentafluoro-λ6-sulfaneyl)phenyl)propiolic acid (0.100 g, 0.367 mmol) and 1-(1-(5-chloro-2-methylphenyl)piperazine hydrochloride (0.0999 g, 0.404 mmol) in CH₂Cl₂ (3.7 mL) cooled to 0 °C was treated with Et₃N (0.20 mL, 1.47 mmol). The cooled solution was treated with T3P (50 wt. % solution in EtOAc, 0.39 mL, 0.551 mmol) dropwise and the reaction was stirred at 0 °C for 30 min, warmed to rt overnight, diluted with EtOAc (30 mL), washed with H₂O (20 mL), satd. aqueous NaHCO₃ (20 mL), dried (MgSO₄), filtered, and concentrated under reduced pressure. The crude residue was purified by automated chromatography on SiO₂ (4g column, liquid load CH₂Cl₂, 100% hexanes to 30% EtOAc/hexanes), to give 1-(4-(5-chloro-2-methylphenyl)piperazin-1-yl)-3-(4-(pentafluoro-λ6-sulfaneyl)phenyl)prop-2-yn-1-one (0.127 g, 0.272 mmol, 74%) as a pale yellow foam: IR (CHCl₃) 2981, 2222, 1627, 1490, 1432, 1275, 832, 792, 727 cm⁻¹; ¹H NMR (400 MHz, CDCl₃) δ 7.76 (d, *J* = 8.8 Hz, 2 H), 7.64 (d, *J=* 8.8 Hz, 2 H), 7.11 (d, *J =* 8.1 Hz, 1 H), 6.99 (dd, *J=* 8.1, 2.1 Hz, 1 H), 6.95 (d, *J=* 2.1 Hz, 1 H), 3.96 (app t, *J=* 5.0 Hz, 2 H), 3.84 (app t, *J =* 5.0 Hz, 2 H), 2.97 (app t, *J=* 5.0 Hz, 2 H), 2.89 (app t, *J=* 5.0 Hz, 2 H), 2.29 (s, 3 H); ¹³C NMR (100 MHz, CDCl₃) δ 154.4 (quint, *J_{CF} =* 18.3 Hz), 152.4, 151.6, 132.4, 132.1, 131.9, 131.0, 126.2 (quint, *J_{CF} =* 4.6 Hz), 124.1, 123.8, 119.8, 88.2, 83.2, 51.9, 51.3, 47.4, 42.0, 17.3; ¹⁹F NMR (376 MHz, CDCl₃) δ 83.0 (quint, *J =* 150.3 Hz, 1 F), 62.4 (d, *J =* 150.3 Hz, 4 F); HRMS (ESI) *m*/*z* calcd for C₂₀H₁₉ClF₅N₂OS ([M+H]⁺) 465.0821, found 465.0819.

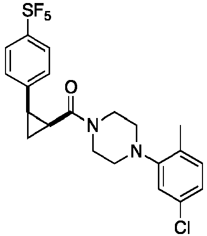

**(4-(5-Chloro-2-methylphenyl)piperazin-1-yl)((1*RS*,2*SR*)-2-(4-(pentafluoro-λ6-sulfaneyl)phenyl)-cyclopropyl)methanone.** A solution of 1-(4-(5-chloro-2-methylphenyl)piperazin-1-yl)-3-(4-(pentafluoro-λ6-sulfaneyl)phenyl)prop-2-yn-1-one (0.0850 g, 0.183 mmol) in EtOAc (1.8 mL) was treated with Lindlar's catalyst (5% Pd on CaCO₃, lead poisoned, 0.0195 g, equivalent to 5 mol% Pd). The reaction was placed under a balloon of H₂ (3 vacuum/backfill cycles) and stirred at rt for 2 d, filtered through Celite, washed (EtOAc), and the combined filtrates were concentrated under reduced pressure. The crude residue was purified by automated chromatography on SiO₂ (4g column, liquid load CH₂Cl₂, 100% hexanes to 40% EtOAc/hexanes, product eluted at 35% EtOAc/hexanes) to give (Z)-1-(4-(5-chloro-2-methylphenyl)-piperazin-1-yl)-3-(4-(pentafluoro-λ6-sulfaneyl)phenyl)prop-2-en-1-one (0.0811 g, 0.174 mmol) as a colorless solid.

A solution of CrCl₂ (0.126 g, 1.03 mmol) and (Z)-1-(4-(5-chloro-2-methylphenyl)piperazin-1-yl)-3-(4-(pentafluoro-λ6-sulfaneyl)phenyl)prop-2-en-1-one (0.0800 g, 0.171 mmol) in dry degassed THF (1.7 mL) was sparged with Ar for 5 min and added CH₂ICl (0.10 mL, 0.857 mmol) at rt, heated for 2 d at 80 °C, cooled to rt, diluted with EtOAc (50 mL) and washed with 1 M aqueous HCl (3 x 20 mL). The organic layer was dried (MgSO₄), filtered and concentrated under reduced pressure. The crude residue was purified by automated chromatography on SiO₂ (4g column, liquid load CH₂Cl₂, 100% hexanes to 30% EtOAc/hexanes, product eluted at 30% EtOAc/hexanes) to give a clear oil that was filtered through basic Al₂O₃ (CH₂Cl₂/EtOAc, 1:1), concentrated under reduced pressure, and dried under high vacuum to give (4-(5-chloro-2-methylphenyl)piperazin-1-yl)((1*RS*,2*SR*)-2-(4-(pentafluoro-λ6-sulfaneyl)phenyl)cyclopropyl)-methanone (0.0451 g, 0.0938 mmol, 52% (2 steps) (100% purity by ELSD)) as a colorless solid: Mp 169.8-172.0 °C; IR (CH₂Cl₂) 2919, 1639, 1490, 1466, 1438, 1225, 1035, 835, 750 cm⁻¹; ¹H NMR (400 MHz, CDCl₃) δ 7.66 (d, *J=* 8.6 Hz, 2 H), 7.25 (d, *J=* 8.6 Hz, 2 H), 7.06 (dd, *J =* 8.1, 0.4 Hz, 1 H), 6.95 (dd, *J =* 8.1, 2.1 Hz, 1 H), 6.69 (d, *J=* 2.1 Hz, 1 H), 3.82 (bd, *J =* 14.0 Hz, 1 H), 3.70-3.58 (m, 2 H), 3.33 (ddd, *J* = 12.4, 8.9, 3.0 Hz, 1 H), 2.75 (tdd, *J =* 14.0, 7.7, 3.4 Hz, 2 H), 2.50 (td, *J =* 8.9, 7.0 Hz, 1 H), 2.32-2.23 (m, 2 H), 2.20 (s, 3 H), 2.10 (ddd, *J* = 11.1, 8.2, 3.0 Hz, 1 H), 1.91 *(q, J=* 6.3 Hz, 1 H), 1.44 (td*, J =* 8.4, 5.6 Hz, 1 H); ¹³C NMR (100 MHz, CDCl₃) δ 166.5, 152.1 (quint, *J_{CF} =* 17.3 Hz), 151.6, 141.9, 131.9 (2 C), 130.9, 127.7, 125.7 (quint, *J_{CF} =* 4.6 Hz), 123.7, 119.5, 51.9, 51.5, 45.5, 42.2, 24.6, 23.7, 17.3, 11.3; ¹⁹F NMR (376 MHz, CDCl₃) δ 84.8 (quint, *J* = 150.4 Hz, 1 F), 63.2 *(d, J=* 150.4 Hz, 4 F); HRMS (ESI) *m*/*z* calcd for C₂₁H₂₃ClF₅N₂OS ([M+H]⁺) 481.1134, found 481.1134.

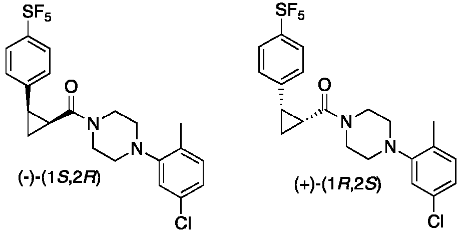

Racemic (4-(5-chloro-2-methylphenyl)piperazin-1-yl)((1*RS*,2*SR*)-2-(4-(pentafluoro-λ6-sulfaneyl)phenyl)-cyclopropyl)methanone was separated on a SFC Chiralpak-IC semiprep (250 x 10 mm) column (30% Methanol:CO₂, 7 mL/min, p = 100 bar, 220 nm) injection volume 90 µL, 20 mg/mL) to give (4-(5-chloro-2-methylphenyl)piperazin-1-yl)((1*S*,2*R*)-2-(4-(pentafluoro-λ6-sulfaneyl)phenyl)cyclopropyl)methanone (retention time 5.14 min) as a colorless viscous oil (100% purity by ELSD): [α]¹⁷_{D} -134.2 (c 0.60, MeOH); ¹H NMR (300 MHz, CDCl₃) δ 7.66 (d, *J =* 8.7 Hz, 2 H), 7.26 (overlap, 2 H), 7.06 (d, *J =* 8.1 Hz, 1 H), 6.96 (dd, *J=* 8.1, 1.8 Hz, 1 H), 6.70 *(d, J=* 1.8 Hz, 1 H), 3.87-3.79 (m, 1 H), 3.73-3.58 (m, 3 H), 3.38-3.31 (m, 1 H), 2.81-2.70 (m, 2 H), 2.50 *(q, J=* 8.7 Hz, 1 H), 2.33-2.24 (m, 2 H), 2.20 (s, 3 H), 2.16-2.08 (m, 1 H), 1.91 (q, *J=* 5.7 Hz, 1 H), 1.44 (td, *J* = 8.1*,* 5.7 Hz, 1 H). The enantiomeric excess was >99.9% ee (SFC Chiralpak-IC (250 x 10 mm); 30% Methanol:CO₂, 7 mL/min, p = 100 bar, 220 nm; retention time: 5.1 min). (4-(5-Chloro-2-methylphenyl)piperazin-1-yl)((1*S*,2*R*)-2-(4-(pentafluoro-λ6-sulfaneyl)phenyl)cyclopropyl)-methanone (retention time 6.57 min) was obtained as a colorless viscous oil (100% purity by ELSD): [α]¹⁷_{D} +136.3 (c 0.59, MeOH); ¹H NMR (300 MHz, CDCl₃) δ 7.66 (d, *J=* 8.7 Hz, 2 H), 7.26-7.24 (overlap, 2 H), 7.06 (d, *J=* 8.4 Hz, 1 H), 6.96 (dd, *J=* 8.4, 1.8 Hz, 1 H), 6.70 (d, *J=* 1.8 Hz, 1 H), 3.85-3.78 (m, 1 H), 3.67-3.61 (m, 2 H), 3.39-3.30 (m, 1 H), 2.81-2.70 (m, 2 H), 2.50 (q, *J =* 8.7 Hz, 1 H), 2.33-2.24 (m, 2 H), 2.20 (s, 3 H), 2.16-2.10 (m, 1 H), 1.91 (q, *J=* 5.7 Hz, 1 H), 1.44 (td, *J=* 8.4, 5.7 Hz, 2 H). The enantiomeric excess was >99.9% ee (SFC Chiralpak-IC (250 x 10 mm); 30% Methanol:CO₂, 7 mL/min, p = 100 bar, 220 nm; retention time: 6.5 min).

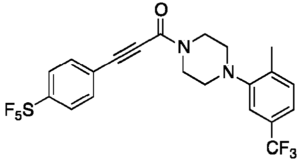

**1-(4-(2-Methyl-5-(trifluoromethyl)phenyl)piperazin-1-yl)-3-(4-(pentafluoro-λ6-sulfaneyl)phenyl)prop-2-yn-1-one.** A solution of 3-(4-(pentafluoro-16-sulfaneyl)phenyl)propiolic acid (0.100 g, 0.367 mmol) and 1-(2-methyl-5-(trifluoromethyl)phenyl)piperazine hydrochloride (0.113 g, 0.404 mmol) in CH₂Cl₂ (3.7 mL) cooled to 0 °C was treated with Et₃N (0.20 mL, 1.47 mmol). The cooled solution was treated with T3P (50 wt. % solution in EtOAc, 0.39 mL, 0.551 mmol) dropwise and the reaction was stirred at 0 °C for 30 min, warmed to rt overnight, diluted with EtOAc (30 mL), washed with H₂O (20 mL), satd. aqueous NaHCO₃ (20 mL), dried (MgSO₄), filtered, and concentrated under reduced pressure. The crude residue was purified by automated chromatography on SiO₂ (4g column, liquid load CH₂Cl₂, 100% hexanes to 40% EtOAc/hexanes), to give 1-(4-(2-methyl-5-(trifluoromethyl)phenyl)piperazin-1-yl)-3-(4-(pentafluoro-λ6-sulfaneyl)phenyl)prop-2-yn-1-one (0.139 g, 0.278 mmol, 76%) as a tan foam: IR (CH₂Cl₂) 2920, 2222, 1632, 1418, 1340, 1310, 1279, 1122, 839, 793 cm⁻¹; ¹H NMR (400 MHz, CDCl₃) δ 7.75 (d, *J* = 8.8 Hz, 2 H), 7.63 (d, *J* = 8.8 Hz, 2 H), 7.31-7.25 (m, 2 H), 7.21 (s, 1 H), 3.98 (t, *J* = 5.0 Hz, 2 H), 3.85 (app t, *J* = 5.0 Hz, 2 H), 3.02 (app t, *J=* 5.0 Hz, 2 H), 2.92 (app t, *J =* 5.0 Hz, 2 H), 2.38 (s, 3 H); ¹³C NMR (100 MHz, CDCl₃) δ 154.3 (quint, *J_{CF} =* 18.1 Hz), 152.4, 150.9, 136.8, 132.4, 131.5, 129.0 (q, *J_{CF} =* 32.1 Hz), 126.2 (quint, *J_{CF}* = 4.6 Hz), 124.1 (q, *J_{CF} =* 272.1 Hz), 124.0, 120.5 (q, *J_{CF} =* 3.8 Hz), 116.0 (q, *J_{CF} =* 3.6 Hz), 88.2, 83.1, 51.8, 51.3, 47.4, 41.9, 17.8; ¹⁹F NMR (376 MHz, CDCl₃) δ 83.0 (quint, *J* = 150.4 Hz, 1 F), 62.4 *(d, J=* 150.2 Hz, 4 F), -62.3 (s, 3 F); HRMS (ESI) *m*/*z* calcd for C₂₁H₁₉F₈N₂OS ([M+H]⁺) 499.1085, found 499.1086.

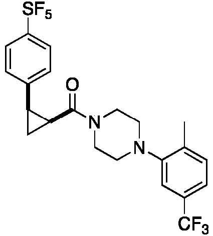

**(4-(2-Methyl-5-(trifluoromethyl)phenyl)piperazin-1-yl)((1*RS*,2*SR*)-2-(4-(pentafluoro-λ6-sulfaneyl)-phenyl)cyclopropyl)methanone (JKJ741.040).** A solution of 1-(4-(2-methyl-5-(trifluoromethyl)phenyl)-piperazin-1-yl)-3-(4-(pentafluorosulfanyl)phenyl)prop-2-yn-1-one (0.100 g, 0.201 mmol) in EtOAc (2 mL) was treated with Lindlar's catalyst (5% Pd on CaCO₃, lead poisoned, 0.0214 g, equivalent to 5 mol% Pd). The reaction was placed under a balloon of H₂ (3 vacuum/backfill cycles) and stirred at rt for 2 d, filtered through Celite, washed (EtOAc), and the combined filtrates were concentrated under reduced pressure. The crude residue was purified by automated chromatography on SiO₂ (4g column, 100% hexanes to 40% EtOAc/hexanes, product eluted at 30% EtOAc/hexanes) to give (Z)-1-(4-(2-methyl-5-(trifluoromethyl)-phenyl)piperazin-1-yl)-3-(4-(pentafluoro-λ6-sulfaneyl)phenyl)prop-2-en-1-one (0.0765 g, 0.153 mmol, 76% (92% brsm) as a colorless foam.

To a flame dried 5 mL microwave vial containing anhydrous CrCl₂ (0.111 g, 0.899 mmol) was treated with solution of (*Z*)-1-(4-(2-methyl-5-(trifluoromethyl)phenyl)piperazin-1-yl)-3-(4-(pentafluoro-16-sulfaneyl)phenyl)prop-2-en-1-one (0.0750 g, 0.150 mmol) in anhydrous THF (1.5 mL) and the mixture was sparged with Ar for 15 min and added CH₂ICl (0.087 mL, 0.749 mmol) at rt and under Ar atmosphere. The reaction mixture was stirred for 2 d at 80 °C. The reaction was cooled to rt, quenched by the addition of EtOAc (50 mL) and washed with 1 M aqueous HCl (3 x 20 mL). The organic layer was dried (MgSO₄), filtered and concentrated under reduced pressure. The crude material was purified by automated chromatography on SiO₂ (4g column, liquid load CH₂Cl₂, hexanes to 30% EtOAc/hexanes, product eluted at 30% EtOAc/hexanes) to give the product as a clear oil. The product was filtered through basic Al₂O₃ (eluting with 1:1 CH₂Cl₂/EtOAc) concentrated to a colorless solid. The solid was recrystallized from hot cyclohexane (2x). The supernatant was removed and the crystals were washed with rt cyclohexane (rt, 2 x 1 mL) and hexanes (2 x 1 mL) and dried under high vacuum to give the product (0.0176 g, 0.0342 mmol, 18% (2 steps) (100% purity by ELSD)) as a colorless solid: Mp 122.1-125.8 °C; IR (CH₂Cl₂) 2919, 1638, 1417, 1338, 1308, 1120, 824, 749 cm⁻¹; ¹H NMR (400 MHz, CDCl₃) δ 7.65 (d, *J =* 8.8 Hz, 2 H), 7.26-7.24 (m, 5 H), 6.96 (s, 1 H), 3.85 (bd, *J=* 13.0 Hz, 1 H), 3.72-3.60 (m, 2 H), 3.38-3.32 (m, 1 H), 2.86-2.75 (m, 2 H), 2.51 (td, *J=* 8.9, 7.0 Hz, 1 H), 2.32-2.26 (m, 5 H), 2.14 (ddd, *J=* 11.0, 8.6, 2.6 Hz, 1 H), 1.92 (q, *J=* 6.2 Hz, 1 H), 1.45 (td, *J =* 8.4, 5.6 Hz, 1 H); ¹³C NMR (100 MHz, CDCl₃) δ 166.5, 152.2 (quint, *J_{CF} =* 17.4 Hz), 150.9, 141.8, 136.7, 131.4, 129.1 (q, *J_{CF} =* 32.1 Hz), 127.7, 125.7, 125.7 (quint, *J_{CF} =* 4.6 Hz), 124.0 (q, *J_{CF}* = 271.9 Hz), 120.4 (q, *J_{CF} = 3.9* Hz), 115.7 (q, *J_{CF}* = 3.8 Hz), 51.9, 51.4, 45.5, 42.2, 24.6, 23.7, 17.9, 11.3; ¹⁹F NMR (376 MHz, CDCl₃) δ 84.6 (quint, *J =* 149.9 Hz, 1 F), 63.0 (d, *J =* 149.8 Hz, 4 F), -62.4 (s, 3 F); HRMS (ESI) *m*/*z* calcd for C₂₂H₂₃F₈N₂OS ([M+H]⁺) 515.1398, found 515.1378.

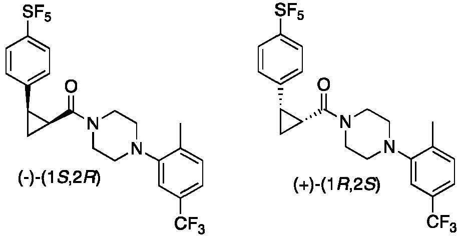

Racemic (4-(2-methyl-5-(trifluoromethyl)phenyl)piperazin-1-yl)((1*RS*,2*SR*)-2-(4-(pentafluoro-λ6-sulfaneyl)phenyl)cyclopropyl)methanone was separated on a SFC Chiralpak-IC semiprep (250 x 10 mm) column (30% Methanol: CO₂, 7 mL/min, p = 100 bar, 220 nm) injection volume (90 µL, 20 mg/mL) to give (4-(2-methyl-5-(trifluoromethyl)phenyl)piperazine-1-yl)((1*S*,2*R*)-2-(4-(pentafluoro-λ6-sulfaneyl)phenyl)-cyclopropyl)methanone (retention time 3.40 min) as a colorless viscous oil (100% purity by ELSD): [α]¹⁷_{D} - 119.1 (c 0.79, MeOH); ¹H NMR (300 MHz, CDCl₃) δ 7.66 (d, *J=* 8.7 Hz, 2 H), 7.25 (d, *J=* 8.7 Hz, 3 H), 6.97 (s, 1 H), 3.89-3.80 (m, 1 H), 3.72-3.60 (m, 2 H), 3.40-3.31 (m, 1 H), 2.86-2.74 (m, 2 H), 2.50 (q, *J* = 8.4 Hz, 1 H), 2.33-2.25 (m, 5 H), 2.18-2.12 (m, 1 H), 1.92 (q, *J=* 6.0 Hz, 1 H), 1.44 (td, *J= 8.4,* 6.0 Hz, 1 H). The enantiomeric excess was >99.9% ee (SFC Chiralpak-IC (250 x 10 mm); 30% Methanol:CO₂, 7 mL/min, p = 100 bar, 220 nm; retention time: 3.3 min).

(4-(2-Methyl-5-(trifluoromethyl)phenyl)piperazine-1-yl)((1*R*,2*S*)-2-(4-(pentafluoro-λ6-sulfaneyl)-phenyl)cyclopropyl)methanone (retention time 3.65 min) was obtained as a colorless viscous oil (100% purity by ELSD): [α]¹⁷_{D} +117.0 (c 0.87, MeOH); ¹H NMR (300 MHz, CDCl₃) δ 7.66 (d, *J =* 8.7 Hz, 2 H), 7.26 (d, *J=* 8.7 Hz, 3 H), 6.97 (s, 1 H), 3.88-3.82 (m, 2 H), 3.69-3.63 (m, 3 H), 3.40-3.31 (m, 2 H), 2.89-2.70 (m, 3 H), 2.50 (q, *J= 8.4* Hz, 2 H), 2.34-2.25 (m, 5 H), 2.20-2.12 (m, 1 H), 1.92 (q, *J=* 6.0 Hz, 1 H), 1.44 (td, *J=* 8.4, 6.0 Hz, 1 H). The enantiomeric excess was >99.9% ee (SFC Chiralpak-IC (250 x 10 mm); 30% Methanol:CO₂, 7 mL/min, p = 100 bar, 220 nm; retention time: 3.7 min).

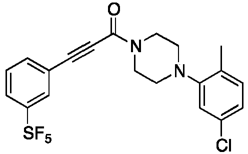

**1-(4-(5-Chloro-2-methylphenyl)piperazine-1-yl)-3-(3-(pentafluoro-a,6-sulfaneyl)phenyl)prop-2-yn-1-one.** A solution of 3-(3-(pentafluoro-λ6-sulfaneyl)phenyl)propiolic acid (0.100 g, 0.367 mmol) and 1-(1-(5-chloro-2-methylphenyl)piperazine hydrochloride (0.0999 g, 0.404 mmol) in CH₂Cl₂ (3.7 mL) cooled to 0 °C was treated with Et₃N (0.20 mL, 1.47 mmol). The cooled solution was treated with T3P (50 wt. % solution in EtOAc, 0.39 mL, 0.551 mmol) dropwise and the reaction was stirred at 0 °C for 30 min and allowed to warm to rt overnight. The reaction was diluted with EtOAc (30 mL) and washed with H₂O (20 mL), satd. aqueous NaHCO₃ (20 mL), dried (MgSO₄), filtered, and concentrated under reduced pressure. The crude residue was purified by automated chromatography on SiO₂ (4g column, liquid load CH₂Cl₂, 100% hexanes to 30% EtOAc/hexanes), to give 1-(4-(5-chloro-2-methylphenyl)piperazine-1-yl)-3-(3-(pentafluoro-λ6-sulfaneyl)phenyl)prop-2-yn-1-one (0.113 g, 0.244 mmol, 66%) as a pale yellow solid: Mp 127.9-133.0 °C; IR (CHCl₃) 2921, 2219, 1627, 1432, 1288, 1223, 1041, 838, 797, 728 cm⁻¹; ¹H NMR (400 MHz, CDCl₃) δ 7.94 (t, *J=* 2.0 Hz, 1 H), 7.80 (ddd, *J=* 8.4, 2.0, 0.8 Hz, 1 H), 7.69 (d, *J=* 7.7 Hz, 1 H), 7.49 (t, *J=* 8.1 Hz, 1 H), 7.11 (d, *J=* 8.1 Hz, 1 H), 6.99 (dd, *J=* 8.1, 2.1 Hz, 1 H), 6.96 (d, *J* = 2.1 Hz, 1 H), 3.97 (app t, *J=* 5.0 Hz, 2 H), 3.84 (app t, *J=* 5.0 Hz, 2 H), 2.98 (app t, *J=* 5.0 Hz, 2 H), 2.89 (app t, *J=* 5.0 Hz, 2 H), 2.29 (s, 3 H); ¹³C NMR (100 MHz, CDCl₃) δ 153.8 (quint, *J_{CF} =* 18.5 Hz), 152.4, 151.6, 135.1, 132.1, 131.9, 131.0, 129.7 (quint, *J_{CF} =* 7.1 Hz), 129.0, 127.3 (quint, *J_{CF} = 4.5* Hz), 123.8, 121.6, 119.8, 88.4, 82.3, 51.9, 51.3, 47.4, 42.0, 17.3; ¹⁹F NMR (376 MHz, CDCl₃) δ 82.9 (quint, *J =* 150.6 Hz, 1 F), 62.6 (d, *J=* 150.5 Hz, 4 F); HRMS (ESI) *m*/*z* calcd for C₂₀H₁₉ClF₅N₂OS ([M+H]⁺) 465.0821, found 465.0821.

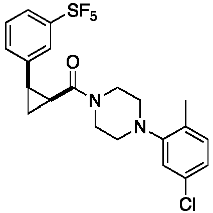

**(4-(5-Chloro-2-methylphenyl)piperazine-1-yl)((1*RS*,2*SR*)-2-(3-(pentafluoro-λ6-sulfaneyl)phenyl)cyclopropyl)methanone.** A solution of 1-(4-(5-chloro-2-methylphenyl)piperazine-1-yl)-3-(4-(pentafluorosulfanyl)phenyl)prop-2-yn-1-one (0.0930 g, 0.200 mmol) in EtOAc (2 mL) was treated with Lindlar's catalyst (5% Pd on CaCO₃, lead poisoned, 0.0213 g, equivalent to 5 mol% Pd). The reaction was placed under a balloon of H₂ (3 vacuum/backfill cycles) and stirred at rt for 3 d, filtered through Celite, washed (EtOAc) and the combined filtrates were concentrated under reduced pressure. The crude residue was purified by automated chromatography on SiO₂ (4g column, liquid load CH₂Cl₂, 100% hexanes to 30% EtOAc/hexanes, product eluted at 20% EtOAc/hexanes) to give (Z)-1-(4-(5-chloro-2-methylphenyl)-piperazine-1-yl)-3-(3-(pentafluoro-λ6-sulfaneyl)phenyl)prop-2-en-1-one (0.0470 g, 0.101 mmol) as a colorless solid.

To a flame dried 5 mL microwave vial containing CrCl₂ (0.0742 g, 0.604 mmol) was added a solution of (*Z*)-1-(4-(5-chloro-2-methylphenyl)piperazine-1-yl)-3-(3-(pentafluoro-λ6-sulfaneyl)phenyl)prop-2-en-1-one (0.0470 g, 0.101 mmol) in dry degassed THF (1 mL) and the mixture was sparged with Ar for 15 min and added CH₂Icl (0.058 mL, 0.503 mmol) at rt, heated for 2 d at 80 °C. The reaction was cooled to rt, diluted with EtOAc (50 mL) and washed with 1 M aqueous HCl (3 x 20 mL). The organic layer was dried (MgSO₄), filtered and concentrated under reduced pressure. The crude residue was purified by automated chromatography on SiO₂ (4g column, liquid load CH₂Cl₂, 100% hexanes to 30% EtOAc/hexanes, product eluted at 20% EtOAc/hexanes) to give the product as a clear oil. The product was filtered through basic Al₂O₃ (CH₂Cl₂/EtOAc, 1:1) concentrated and dried under high vacuum to give (4-(5-chloro-2-methylphenyl)piperazine-1-yl)((1*RS*,2*SR*)-2-(3-(pentafluoro-λ6-sulfaneyl)phenyl)cyclopropyl)methanone (0.0182 g, 0.0378 mmol, 19% (2 steps) (100% purity by ELSD)) as a colorless solid: Mp 112.9-116.1 °C; IR (CH₂Cl₂) 2854, 1640, 1490, 1439, 1225, 1036, 841 cm⁻¹; ¹H NMR (400 MHz, CDCl₃) δ 7.62-7.59 (m, 2 H), 7.38 (t, *J=* 8.1 Hz, 1 H), 7.29 (d, *J=* 7.8 Hz, 1 H), 7.06 (d, *J=* 8.5 Hz, 1 H), 6.95 (dd, *J=* 8.1, 2.1 Hz, 1 H), 6.70 (d, *J=* 2.1 Hz, 1 H), 3.84 (bd, *J =* 12.8 Hz, 1 H), 3.76-3.71 (m, 1 H), 3.61 (ddd, *J=* 12.8, 8.9, 3.2 Hz, 1 H), 3.29 (ddd, *J=* 12.8, 9.0, 3.1 Hz, 1 H), 2.80-2.71 (m, 2 H), 2.53 (td, *J =* 8.9, 6.9 Hz, 1 H), 2.31-2.23 (m, 2 H), 2.20 (s, 3 H), 2.15-2.09 (m, 1 H), 1.91 (q, *J=* 6.2 Hz, 1 H), 1.43 (td, *J* = 8.4*,* 5.6 Hz, 1 H); ¹³C NMR (100 MHz, CDCl₃) δ 166.6, 153.9 (quint, *J_{CF} =* 16.8 Hz), 151.8, 139.0, 132.0, 131.8, 131.0, 130.2, 128.6, 125.9 (quint, *J_{CF} =* 4.5 Hz), 124.0 (quint, *J_{CF} =* 4.6 Hz), 123.7, 119.7, 51.8, 51.6, 45.6, 42.2, 24.1, 24.0, 17.3, 11.0; ¹⁹F NMR (376 MHz, CDCl₃) δ 84.7 (quint, *J =* 150.1 Hz, 1 F), 62.9 (d, *J =* 149.9 Hz, 4 F); HRMS (ESI) *m*/*z* calcd for C₂₁H₂₃ClF₅N₂OS ([M+H]⁺) 481.1134, found 481.1133.

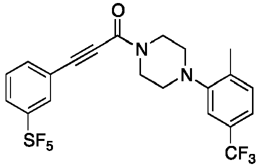

**1-(4-(2-Methyl-5-(trifluoromethyl)phenyl)piperazine-1-yl)-3-(3-(pentafluoro-λ6-sulfaneyl)phenyl)-prop-2-yn-1-one**. A solution of 3-(3-(pentafluoro-16-sulfaneyl)phenyl)propiolic acid (0.100 g, 0.367 mmol) and 1-(2-methyl-5-(trifluoromethyl)phenyl)piperazine hydrochloride (0.113 g, 0.404 mmol) in CH₂Cl₂ (3.7 mL) cooled to 0 °C was treated with Et₃N (0.20 mL, 1.47 mmol). The cooled solution was treated with T3P (50 wt. % solution in EtOAc, 0.39 mL, 0.551 mmol) dropwise and the reaction was stirred at 0 °C for 30 min, warmed to rt overnight, diluted with EtOAc (30 mL), washed with H₂O (20 mL), satd. aqueous NaHCO₃ (20 mL), dried (MgSO₄), filtered, and concentrated under reduced pressure. The crude residue was purified by automated chromatography on SiO₂ (4g column, liquid load CH₂Cl₂, 100% hexanes to 40% EtOAc/hexanes), to give 1-(4-(2-methyl-5-(trifluoromethyl)phenyl)piperazine-1-yl)-3-(3-(pentafluoro-16-sulfaneyl)phenyl)prop-2-yn-1-one (0.150 g, 0.300 mmol, 82%) as a tan foam: IR (CH₂Cl₂) 2919, 2825, 2219, 1629, 1418, 1309, 1152, 1119, 840, 797, 730 cm⁻¹; ¹H NMR (400 MHz, CDCl₃) δ 7.94 *(t, J=* 1.8 Hz, 1 H), 7.81 (dt, *J=* 8.2, 1.1 Hz, 1 H), 7.70 (d, *J=* 7.8 Hz, 1 H), 7.50 (t, *J* = 8.2 Hz, 1 H), 7.29 (q, *J=* 7.8 Hz, 2 H), 7.22 (s, 1 H), 4.00 (app t, *J=* 5.0 Hz, 2 H), 3.87 (app t, *J=* 5.0 Hz, 2 H), 3.04 (app t*, J =* 5.0 Hz, 2 H), 2.94 (app t, *J =* 5.0 Hz, 2 H), 2.39 (s, 3 H); ¹³C NMR (100 MHz, CDCl₃) δ 153.8 (quint, *J_{CF}* = 18.2 Hz), 152.5, 150.9, 136.8, 135.1, 131.6, 129.7 (quint, *J_{CF} =* 4.7 Hz), 129.1 (q, *J_{CF} =* 32.3 Hz), 129.1, 127.4 (quint, *J_{CF}* = 4.7 Hz), 124.1 (q, *J_{CF} =* 271.8 Hz), 121.5, 120.6 (q, *J_{CF}* = 3.9 Hz), 116.1 (q, *J_{CF} =* 3.6 Hz), 88.4, 82.3, 51.9, 51.3, 47.4, 42.0, 17.9; ¹⁹F NMR (376 MHz, CDCl₃) δ 82.9 (quint, *J =* 150.6 Hz, 1 F), 62.6 (d, *J =* 150.4 Hz, 4 F), -62.3 (s, 3 F); HRMS (ESI) *m*/*z* calcd for C₂₁H₁₉F₈N₂OS ([M+H]⁺) 499.1085, found 499.1086.

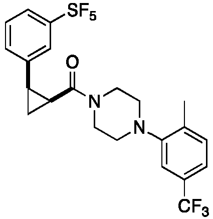

**(4-(2-Methyl-5-(trifluoromethyl)phenyl)piperazine-1-yl)((1*RS*,2*SR*)-2-(3-(pentafluoro-λ6-sulfaneyl)phenyl)cyclopropyl)methanone.** A solution of 1-(4-(2-methyl-5-(trifluoromethyl)phenyl)-piperazine-1-yl)-3-(3-(pentafluoro-λ6-sulfaneyl)phenyl)prop-2-yn-1-one (0.100 g, 0.201 mmol) in EtOAc (2 mL) was treated with Lindlar's catalyst (5% Pd on CaCO₃, lead poisoned, 0.0214 g, equivalent to 5 mol% Pd). The reaction was placed under a balloon of H₂ (3 vacuum/backfill cycles) and stirred at rt for 2 d, filtered through Celite, washed (EtOAc), and the combined filtrates were concentrated under reduced pressure. The crude residue was purified by automated chromatography on SiO₂ (4g column, liquid load CH₂Cl₂, hexanes to 40% EtOAc/hexanes, product eluted at 30% EtOAc/hexanes) to give (Z)-1-(4-(2-methyl-5-(trifluoromethyl)phenyl)piperazine-1-yl)-3-(3-(pentafluoro-λ6-sulfaneyl)phenyl)prop-2-en-1-one (0.0938 g, 0.187 mmol) as a colorless foam.

A solution of CrCl₂ (0.138 g, 1.12 mmol) and (Z)-1-(4-(2-methyl-5-(trifluoromethyl)phenyl)-piperazine-1-yl)-3-(3-(pentafluoro-λ6-sulfaneyl)phenyl)prop-2-en-1-one (0.0938 g, 0.187 mmol) in dry degassed THF (1.9 mL) (degassed by sparging with Ar for 15 min) was treated with CH₂ICl (0.11 mL, 0.937 mmol) at rt, heated for 2 d at 80 °C, cooled to rt, diluted with EtOAc (50 mL), and washed with 1 M aqueous HCl (3 x 20 mL). The organic layer was dried (MgSO₄), filtered and concentrated under reduced pressure. The crude residue was purified by automated chromatography on SiO₂ (4g column, liquid load CH₂Cl₂, 100% hexanes to 40% EtOAc/hexanes, product eluted at 30% EtOAc/hexanes), filtered through basic Al₂O₃ (CH₂Cl₂/EtOAc, 1:1), concentrated under reduced pressure, and dried under high vacuum to give (4-(2-methyl-5-(trifluoromethyl)phenyl)piperazine-1-yl)((1*RS*,2*SR*)-2-(3-(pentafluoro-λ6-sulfaneyl)-phenyl)cyclopropyl)methanone (0.0641 g, 0.125 mmol, 62% (2 steps) (100% purity by ELSD)) as a pale yellow oil: IR (CH₂Cl₂) 2917, 1638, 1438, 1417, 1337, 1307, 1120, 835, 758 cm⁻¹; ¹H NMR (400 MHz, CDCl₃) δ 7.62-7.58 (m, 2 H), 7.38 (t, *J* = 7.8 Hz, 1 H), 7.30 (d, *J* = 7.8 Hz, 1 H), 7.24-7.21 (m, 2 H), 6.96 (s, 1 H), 3.93 (d, *J=* 13.0 Hz, 1 H), 3.79 (dt, *J =* 13.0, 3.8 Hz, 1 H), 3.60 (ddd, *J=* 12.6, 9.2, 3.1 Hz, 1 H), 3.25 (ddd, *J=* 12.6, 9.2, 3.0 Hz, 1 H), 2.79 (ddt, *J=* 20.7, 11.9, 3.8 Hz, 2 H), 2.53 (td, *J=* 8.9, 6.9 Hz, 1 H), 2.31-2.20 (m, 5 H), 2.16-2.10 (m, 1 H), 1.92 (q, *J=* 6.2 Hz, 1 H), 1.43 (td, *J =* 8.4, 5.6 Hz, 1 H); ¹³C NMR (100 MHz, CDCl₃) δ 166.5, 153.9 (quint, *J_{CF} =* 16.9 Hz), 151.0, 139.0, 136.8, 131.3, 130.3, 129.0 (q, *J_{CF} =* 33.1 Hz), 128.5, 125.7 (quint, *J_{CF} =* 4.6 Hz), 124.2 (q, *J_{CF} =* 272.0 Hz), 123.9 (quint, *J_{CF} =* 4.6 Hz), 120.3 (q, *J_{CF}* = 3.9 Hz), 115.9 (q, *J_{CF} =* 3.6 Hz), 51.7, 51.6, 45.5, 42.2, 24.0 (2 C), 24.0, 17.8, 10.9; ¹⁹F NMR (376 MHz, CDCl₃) δ 84.5 (quint, *J=* 150.0 Hz, 1 F), 62.7 (d, *J* = 149.8 Hz, 4 F), -62.4 (s, 3 F); HRMS (ESI) *m*/*z* calcd for C₂₂H₂₃F₈N₂OS ([M+H]⁺) 515.1398, found 515.1380.

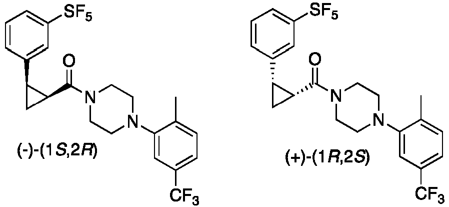

Racemic (4-(2-methyl-5-(trifluoromethyl)phenyl)- 90 -iperazine-1-yl)((1*RS*,2*SR*)-2-(3-(pentafluoro-λ6-sulfaneyl)phenyl)cyclopropyl)methanone was separated on a SFC Chiralpak-IC semiprep (250 x 10 mm) column (30% Methanol: CO₂, 7 mL/min, p = 100 bar, 220 nm) injection volume 90 µL, 20 mg/mL) to give (4-(2-methyl-5-(trifluoromethyl)phenyl)piperazin-1-yl)((1*S*,2*R*)-2-(3-(pentafluoro-λ6-sulfaneyl)phenyl)-cyclopropyl)methanone (retention time 3.25 min) as a colorless viscous oil (100% purity by ELSD): [α]¹⁸_{D} - 104.7 (c 0.84, MeOH); ¹H NMR (300 MHz, CDCl₃) δ 7.62-7.58 (m, 2 H), 7.38 *(t, J=* 7.7 Hz, 1 H), 7.31-7.24 (m, 3 H), 6.96 (s, 1 H), 3.94-3.89 (m, 1 H), 3.81-3.75 (m, 1 H), 3.65-3.56 (m, 1 H), 3.31-3.22 (m, 1 H), 2.84-2.74 (m, 2 H), 2.53 (td, *J* = 9.3, 6.9 Hz, 1 H), 2.32-2.21 (m, 5 H), 2.19-2.11 (m, 1 H), 1.93 (q, *J* = 5.7 Hz, 1 H), 1.43 (td, *J= 8.4,* 5.7 Hz, 1 H). The enantiomeric excess was >99.9% ee (SFC Chiralpak-IC (250 x 10 mm); 30% Methanol:CO₂, 7 mL/min, p = 100 bar, 220 nm; retention time: 3.3 min).

(4-(2-Methyl-5-(trifluoromethyl)phenyl)piperazin-1-yl)((1*R*,2*S*)-2-(3-(pentafluoro-λ6-sulfaneyl)phenyl)-cyclopropyl)methanone (retention time 3.60 min) was obtained as a colorless viscous oil (100% purity by ELSD): [α]¹⁸_{D} +103.4 (c 0.87, MeOH); ¹H NMR (300 MHz, CDCl₃) δ 8.54-7.81 (m, 2 H), 7.62-7.59 (m, 2 H), 7.38 *(t, J=* 7.8 Hz, 1 H), 7.31-7.24 (m, 3 H), 6.96 (s, 1 H), 3.94-3.88 (m, 1 H), 3.81-3.75 (m, 1 H), 3.65-3.56 (m, 1 H), 3.31-3.22 (m, 1 H), 2.84-2.74 (m, 2 H), 2.53 (td, *J* = 9.0*,* 7.2 Hz, 1 H), 2.32-2.22 (m, 5 H), 2.18-2.11 (m, 1 H), 1.93 *(q, J=* 5.7 Hz, 1 H), 1.43 (td, *J =* 8.4, 5.7 Hz, 1 H). The enantiomeric excess was >99.9% ee (SFC Chiralpak-IC (250 x 10 mm); 30% Methanol: CO₂, 7 mL/min, p = 100 bar, 220 nm; retention time: 3.6 min).

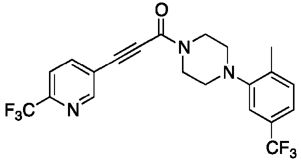

**1-(4-(2-Methyl-5-(trifluoromethyl)phenyl)piperazin-1-yl)-3-(6-(trifluoromethyl)pyridin-3-yl)prop-2-yn-1-one.** A solution of methyl 3-(5-chloropyridin-2-yl)propionate (0.500 g, 2.18 mmol) in THF (4 mL) was treated with 2 M NaOH (1.2 mL, 2.40 mmol) and the mixture was stirred at rt for 3 h, acidified with 1 M aqueous HCl, and extracted with EtOAc (3 x 20 mL). The combined organic layers were dried (MgSO₄), filtered and concentrated under reduced pressure to give the product (0.422 g, 1.96 mmol) as a colorless solid. This solid was taken on to the coupling reaction with no further purification.

A solution of 3-(6-(trifluoromethyl)pyridin-3-vl)propionic acid (0.422 g, 1.96 mmol) and 1-(2-methyl-5-(trifluoromethyl)phenyl)piperazine hydrochloride (0.661 g, 2.35 mmol) in CH₂Cl₂ (19 mL) cooled to 0 °C was treated Et₃N (0.83 mL, 5.88 mmol). The cooled solution was treated with T3P (50% solution in EtOAc) (2.1 mL, 2.94 mmol) dropwise and the reaction was stirred at 0 °C for 30 min, warmed to rt overnight, diluted with EtOAc (50 mL), washed with H₂O (20 mL), satd. aqueous NaHCO₃ (20 mL), dried (MgSO₄), filtered, and concentrated under reduced pressure. The crude material was purified by chromatography on SiO₂ (4g column, 1:1 hexanes:EtOAc), to give the product (0.781 g, 1.77 mmol, 81% (2 steps)) as a pale yellow solid: Mp 110.7-113.5 °C; IR (CH₂Cl₂) 3652, 2981, 2890, 1635, 1382, 1337, 1240, 1150, 1087, 956 cm⁻¹; ¹H NMR (400 MHz, CDCl₃) δ 8.85 (dd, *J* = 1.6, 0.6 Hz, 1 H), 8.04 (ddd, *J* = 8.2,1.6, 0.6 Hz, 1 H), 7.72 (dd, *J=* 8.2, 0.6 Hz, 1 H), 7.32-7.26 (m, 2 H), 7.21 (s, 1 H), 3.99 (t, *J=* 5.0 Hz, 2 H), 3.87 (t, *J=* 5.0 Hz, 2 H), 3.04 *(t, J=* 5.0 Hz, 2 H), 2.95 (t, *J =* 5.0 Hz, 2 H), 2.39 (s, 3 H); ¹³C NMR (100 MHz, CDCl₃) δ 152.5, 152.0, 150.8, 148.2 (q, *J_{CF} =* 35.3 Hz), 140.7, 136.8, 131.6, 129.1 (q, *J_{CF} =* 32.3 Hz), 124.1 *(q, J_{CF} =* 272.1 Hz), 121.0 (q, *J_{CF}* = 274.4 Hz), 120.7, 120.6 (q, *J_{CF} =* 3.9 Hz), 120.0 (q, *J_{CF}* = 2.8 Hz), 116.1 *(q, J_{CF}* = 3.6 Hz), 85.6, 51.8, 51.3, 47.4, 42.0, 17.9; ¹⁹F NMR (376 MHz, CDCl₃) δ -62.3 (s, 3 F). -68.2 (s, 3 F); HRMS (ESI) *m*/*z* calcd for C₂₁H₁₈F₆N₃O ([M+H]⁺) 442.1349, found 442.1345.

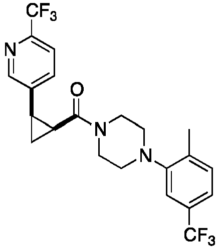

**(4-(2-Methyl-5-(trifluoromethyl)phenyl)piperazin-1-yl)((1RS,2SR)-2-(6-(trifluoromethyl)pyridin-3-yl)cyclopropyl)methanone.** A solution of 1-(4-(2-methyl-5-(trifluoromethyl)phenyl)piperazin-1-yl)-3-(6-(trifluoromethyl)pyridin-3-yl)prop-2-yn-1-one (0.720 g, 1.63 mmol) in EtOAc (16 mL) at rt was treated with quinoline (1.0 mL, 8.16 mmol) and 5% Pd/BaSO₄ (0.0347 g, 0.0163 mmol, equivalent to 1 mol% Pd) and the reaction was stirred under an atmosphere of H₂ (3 x backfill cycles) for 4 h. Analysis by TLC (hexanes/EtOAc, 1:1) indicated that the starting material had been mostly consumed. The reaction was filtered (eluting with EtOAc), and the filtrate was washed with 1 M aqueous HCl (3 x 30 mL), dried (Na₂SO₄), filtered and concentrated under reduced pressure. The crude residue was purified by chromatography on SiO₂ (hexanes/EtOAc, 1:1 to EtOAc) to give (Z)-1-(4-(2-methyl-5-(trifluoromethyl)-phenyl)piperazin-1-yl)-3-(6-(trifluoromethyl)pyridin-3-yl)prop-2-en-1-one (0.640 g, 1.44 mmol) as a yellow/orange oil.

A solution of CrCl₂ (1.06 g, 8.66 mmol) and (Z)-1-(4-(2-methyl-5-(trifluoromethyl)phenyl)-piperazin-1-yl)-3-(6-(trifluoromethyl)pyridin-3-yl)prop-2-en-1-one (0.640 g, 1.44 mmol) in dry degassed THF (14 mL) was sparged with Ar for 5 min and treated with CH₂ICl (0.83 mL, 7.22 mmol) at rt and under Ar atmosphere. The reaction mixture was stirred for 2 d at 80 °C, cooled to rt, diluted with EtOAc (50 mL), and washed with 1 M aqueous HCl (3 x 20 mL). The organic layer was dried (MgSO₄), filtered and concentrated under reduced pressure. The crude material was purified by chromatography on SiO₂ (4: 1, EtOAc:hexanes). filtered through basic Al₂O₃ (eluting with EtOAc) concentrated under reduced pressure, and dried under high vacuum to give (4-(2-methyl-5-(trifluoromethyl)phenyl)piperazin-1-yl)((1RS,2SR)-2-(6-(trifluoromethyl)pyridin-3-yl)cyclopropyl)methanone (0.280 g, 0.612 mmol, 37% (2 steps) (100% purity by ELSD)) as an orange oil: IR (CH₂Cl₂) 2981, 1640, 1418, 1339, 1309, 1166, 1126, 1035, 828 cm⁻¹; ¹H NMR (400 MHz, CDCl₃) δ 8.64 (s, 1 H), 7.60 (t, *J=* 2.0 Hz, 2 H), 7.24 (t, *J=* 2.0 Hz, 2 H), 7.02 (s, 1 H), 3.78-3.65 (m, 3 H), 3.53-3.46 (m, 1 H), 2.92-2.87 (m, 1 H), 2.80-2.75 (m, 1 H), 2.59-2.53 (m, 1 H), 2.47-2.35 (m, 4 H), 2.30 (s, 3 H), 1.95 (q, *J=* 5.6 Hz, 1 H), 1.49 (td, *J=* 8.4, 5.6 Hz, 1 H).; ¹³C NMR (100 MHz, CDCl₃) δ 166.4, 150.8, 150.5, 146.3 (q, *J_{CF} =* 34.8 Hz), 136.9, 136.7, 135.5, 131.4, 129.1 (q, *J_{CF} =* 32.2 Hz), 124.1 (q, *J_{CF} =* 272.1 Hz), 121.6 (q, *J_{CF} =* 273.7 Hz), 120.4 (q, *J_{CF} =* 3.9 Hz), 119.8 (q, *J_{CF} =* 2.6 Hz), 115.9 *(q, J_{CF}* = 3.7 Hz), 52.0, 51.5, 45.6, 42.3, 23.7, 21.5, 17.9, 11.0; ¹⁹F NMR (376 MHz, CDCl₃) δ -62.4 (s, 3 F), -67.8 (s, 3 F); HRMS (ESI) *m*/*z* calcd for C₂₂H₂₂F₆N₃O ([M+H]⁺) 458.1662, found 458.1660.

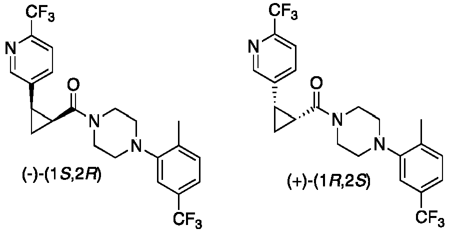

Racemic (4-(2-methyl-5-(trifluoromethyl)phenyl)piperazin-1-yl)((1RS,2SR)-2-(6-(trifluoromethyl)pyridin-3-yl)cyclopropyl)methanone was separated on a SFC Chiralpak-IC semiprep (250 x 10 mm) column (15% Methanol/CO₂, 7 mL/min, 220nm, p =100 bar, 20 mg/mL in MeOH) to give (4-(2-methyl-5-(trifluoromethyl)phenyl)piperazin-1-yl)((1S,2R)-2-(6-(trifluoromethyl)pyridin-3-yl)cyclopropyl)methanone (retention time 7.65 min) as a colorless solid (100% purity by ELSD): [α]¹⁹_{D} -134.8 (c 0.90, MeOH); ¹H NMR (300 MHz, CDCl₃) δ 8.64 (s, 1 H), 7.60 (s, 2 H), 7.26 (s, 4 H), 7.04 (s, 1 H), 3.75-3.64 (m, 3 H), 3.55-3.47 (m, 1 H), 2.94-2.87 (m, 1 H), 2.82-2.74 (m, 1 H), 2.56 *(q, J=* 8.4 Hz, 1 H), 2.49-2.34 (m, 3 H), 2.31 (s, 3 H), 1.96 (q, *J=* 5.8 Hz, 1 H), 1.50 (td, *J =* 8.4, 5.8 Hz, 1 H). The enantiomeric excess was >99.9% ee (SFC Chiralpak-IC (250 x 10 mm); 15% Methanol:CO₂, 7 mL/min, p = 100 bar, 220 nm; retention time: 7.8 min). (4-(2-Methyl-5-(trifluoromethyl)phenyl)piperazin-1-yl)((1*R*,2*S*)-2-(6-(trifluoromethyl)pyridin-3-yl)-cyclopropyl)methanone (retention time 8.38 min) was obtained as a colorless solid (100% purity by ELSD): [α]¹⁹_{D} +128.2 (c 1.08, MeOH); ¹H NMR (300 MHz, CDCl₃) δ 8.64 (s, 1 H), 7.60 (s, 2 H), 7.26 (s, 2 H), 7.04 (s, 1 H), 3.77-3.63 (m, 3 H), 3.55-3.47 (m, 1 H), 2.93-2.87 (m, 1 H), 2.81-2.75 (m, 1 H), 2.61-2.52 (m, 1 H), 2.50-2.35 (m, 2 H), 2.31 (s, 3 H), 1.96 (q, *J=* 5.8 Hz, 1 H), 1.50 (td, *J =* 8.4, 5.8 Hz, 1 H). The enantiomeric excess was >99.9% ee (SFC Chiralpak-IC (250 x 10 mm); 15% Methanol:CO₂, 7 mL/min, p = 100 bar, 220 nm; retention time: 8.4 min).

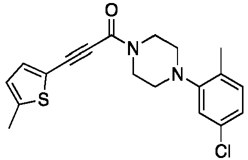

**1-(4-(5-Chloro-2-methylphenyl)piperazin-1-yl)-3-(5-methylthiophen-2-yl)prop-2-yn-1-one.** A solution of 3-(5-methylthiophen-2-yl)propiolic acid (0.0750 g, 0.451 mmol) and 1-(5-chloro-2-methylphenyl)-piperazine hydrochloride (0.134 g, 0.542 mmol) in CH₂Cl₂ (4.5 mL) cooled to 0 °C was treated with Et₃N (0.19 mL, 1.35 mmol). The cooled solution was treated with T3P (50 wt. % solution in EtOAc, 0.48 mL, 0.677 mmol) dropwise and the reaction was stirred at 0 °C for 30 min, warmed to rt overnight, diluted with CH₂Cl₂ (30 mL), washed with 1 M aqueous HCl (20 mL), dried (MgSO₄), filtered, and concentrated under reduced pressure. The crude residue was purified by automated chromatography on SiO₂ (4g column, liquid load CH₂Cl₂, hexanes to 40% EtOAc/hexanes), to give 1-(4-(5-chloro-2-methylphenyl)piperazin-1-yl)-3-(5-methylthiophen-2-yl)prop-2-yn-1-one (0.0620 g, 0.173 mmol, 38%) as a colorless solid: Mp 114.4-116.0 °C; IR (CH₂Cl₂) 2919, 2197, 1643, 1593, 1489, 1426, 1224, 1025, 806 cm⁻¹; ¹H NMR (400 MHz, CDCl₃) δ 7.24 (d, *J =* 3.6 Hz, 1 H), 7.10 (d, *J =* 8.1 Hz, 1 H), 6.98 (dd, *J =* 8.1, 1.9 Hz, 1 H), 6.94 (d, *J =* 1.9 Hz, 1 H), 6.69 (dd, *J=* 3.6, 0.8 Hz, 1 H), 3.91 (app t, *J=* 5.0 Hz, 2 H), 3.81 (app t, *J=* 5.0 Hz, 2 H), 2.94 (app t, *J* = 5.0 Hz, 2 H), 2.86 (app t, *J=* 5.0 Hz, 2 H), 2.49 (s, 3 H), 2.28 (s, 3 H); ¹³C NMR (100 MHz, CDCl₃) δ 153.1, 151.7, 145.5, 135.7, 132.0, 131.8, 130.9, 125.8, 123.6, 119.7, 117.4, 85.4, 84.5, 51.8, 51.3, 47.2, 41.8, 17.4, 15.5; HRMS (ESI) *m*/*z* calcd for C₁₉H₂₀ClN₂OS ([M+H]⁺) 359.0979, found 359.0978.

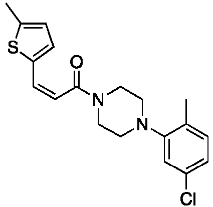

**(Z)-1-(4-(5-Chloro-2-methylphenyl)piperazin-1-yl)-3-(5-methylthiophen-2-yl)prop-2-en-1-one** . A solution of 1-(4-(5-chloro-2-methylphenyl)piperazin-1-yl)-3-(5-methylthiophen-2-yl)prop-2-yn-1-one (0.0620 g, 0.173 mmol) in EtOAc (1.7 mL) was treated with Lindlar's catalyst (5% Pd on CaCO₃, lead poisoned, 0.00735 g, equivalent to 2 mol% Pd) and quinoline (10 µL, 0.172 mmol). The reaction was placed under a balloon of H₂ (4 vacuum/backfill cycles) and stirred at rt for 6 h, filtered through Celite, washed (EtOAc), and the combined filtrates were washed with 1 M aqueous HCl, dried (Na₂SO₄), filtered, and concentrated under reduced pressure. The crude residue was purified by automated chromatography on SiO₂ (4g column, liquid load CH₂Cl₂, 10% EtOAc/hexanes to 40% EtOAc/hexanes) to give (Z)-1-(4-(5-chloro-2-methylphenyl)piperazin-1-yl)-3-(5-methylthiophen-2-yl)prop-2-en-1-one (0.0263 g, 0.0729 mmol, 42% (100% purity by ELSD)) as a colorless solid: Mp 113.2-116.4 °C; IR (CH₂Cl₂) 2917, 2818, 1643, 1593, 1489, 1435, 1226, 1039, 805 cm⁻¹; ¹H NMR (400 MHz, CDCl₃) δ 7.09 (dd, *J* = 8.1, 0.8 Hz, 1 H), 6.98-6.95 (m, 2 H), 6.90 (d, *J=* 2.1 Hz, 1 H), 6.75 (d, *J=* 12.4 Hz, 1 H), 6.67 (dq, *J=* 3.5, 1.1 Hz, 1 H), 5.88 (d*, J =* 12.4 Hz, 1 H), 3.87 (app t, *J=* 5.0 Hz, 2 H), 3.67 (app t, *J=* 5.0 Hz, 2 H), 2.90 (app t, *J=* 5.0 Hz, 2 H), 2.78 (app t, *J=* 5.0 Hz, 2 H), 2.48 (d, *J=* 0.8 Hz, 3 H), 2.26 (s, 3 H); ¹³C NMR (100 MHz, CDCl₃) δ 166.9, 151.9, 143.7, 135.9, 132.0, 131.7, 131.2, 130.9, 128.6, 125.2, 123.6, 119.7, 117.2, 51.7, 51.3, 46.6, 41.7, 17.4, 15.5; HRMS (ESI) *m*/*z* calcd for C₁₉H₂₂ClN₂OS ([M+H]⁺) 361.1136, found 361.1135.

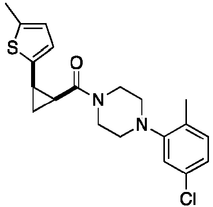

**(4-(5-Chloro-2-methylphenyl)piperazin-1-yl)((1*RS*,2*SR*)-2-(5-methylthiophen-2-yl)cyclopropyl)-methanone.** A solution of CrCl₂ (0.0511 g, 0.416 mmol) and (Z)-1-(4-(5-chloro-2-methylphenyl)piperazin-1-yl)-3-(5-methylthiophen-2-yl)prop-2-en-1-one (0.0250 g, 0.0693 mmol) in dry degassed THF (0.7 mL) was sparged with Ar for 5 min and treated with CH₂ICl (40 uL, 0.346 mmol) at rt, heated for 20 h at 80 °C, cooled to rt, combined, diluted with Et₂O (50 mL), and washed with 1 M aqueous HCl (3 x 20 mL). The organic layer was dried (MgSO₄), filtered and concentrated under reduced pressure. The crude residue was purified by automated chromatography on SiO₂ (4g column, liquid load CH₂Cl₂, hexanes to 40% EtOAc/hexanes) to give the product as a yellow oil. The product was filtered through basic Al₂O₃ (eluting with 1:1 CH₂Cl₂/EtOAc) concentrated under reduced pressure and dried under high vacuum to give (4-(5-chloro-2-methylphenyl)piperazin-1-yl)((1*RS*,2*SR*)-2-(5-methylthiophen-2-yl)cyclopropyl)methanone (0.0154 g, 0.0411 mmol, 59% (100% purity by ELSD)) as a pale yellow oil: IR (CH₂Cl₂) 2918, 1643, 1593, 1490, 1462, 1436, 1226, 1029, 802 cm⁻¹; ¹H NMR (400 MHz, CDCl₃) δ 7.08 (dd, *J* = 8.1, 0.4 Hz, 1 H), 6.97 (dd, *J=* 8.1, 2.1 Hz, 1 H), 6.79 (d, *J=* 2.1 Hz, 1 H), 6.57-6.55 (m, 2 H), 3.96 (d, *J =* 13.0 Hz, 1 H), 3.85 (dt, *J=* 13.0, 3.5 Hz, 1 H), 3.63 (ddd, *J =* 13.0, 8.9, 3.5 Hz, 1 H), 3.34 (ddd, *J* = 13.0, 9.0, 3.2 Hz, 1 H), 2.79 (ddt, *J=* 19.3, 11.2, 4.1 Hz, 2H), 2.52 (td, *J* = 8.9, 6.8 Hz, 1 H), 2.45-2.36 (m, 5 H), 2.24 (s, 3 H), 2.16 (ddd, *J* = 8.9, 8.2, 6.3 Hz, 1 H), 1.77 (td, *J* = 6.3, 5.6 Hz, 1 H), 1.38 (ddd, *J* = 8.9, 8.2, 5.6 Hz, 1 H). ¹³C NMR (100 MHz, CDCl₃) δ 167.2, 152.1, 138.2, 137.5, 131.9, 131.7, 131.1, 125.0, 124.1, 123.5, 119.7, 51.8, 51.7, 45.7, 42.4, 24.1, 19.3, 17.3, 15.3, 12.2; HRMS (ESI) *m*/*z* calcd for C₁₉H₂₄ClN₂OS ([M+H]⁺) 375.1292, found 375.1292.

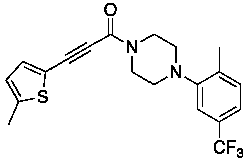

**1-(4-(2-Methyl-5-(trifluoromethyl)phenyl)piperazin-1-yl)-3-(5-methylthiophen-2-yl)prop-2-yn-1-one** . A solution of 3-(5-methylthiophen-2-yl)propiolic acid (0.0592 g, 0.356 mmol) and 1-(2-methyl-5-(trifluoromethyl)phenyl)piperazine hydrochloride (0.100 g, 0.356 mmol) in CH₂Cl₂ (3.6 mL) cooled to 0 °C was treated with Et₃N (0.20 mL, 1.42 mmol). The cooled solution was treated with T3P (50 wt. % solution in EtOAc, 0.38 mL, 0.534 mmol) dropwise and the reaction was stirred at 0 °C for 30 min, warmed to rt overnight, diluted with CH₂Cl₂ (30 mL), washed with 1 M aqueous HCl (20 mL), dried (MgSO₄), filtered, and concentrated under reduced pressure. The crude material was purified by automated chromatography on SiO₂ (4g column, liquid load CH₂Cl₂, hexanes to 40% EtOAc/hexanes), to give 1-(4-(2-methyl-5-(trifluoromethyl)phenyl)piperazin-1-yl)-3-(5-methylthiophen-2-yl)prop-2-yn-1-one (0.0685, 0.175 mmol, 49% (100% purity by ELSD)) as a yellow solid. Mp 145.7-149.3 °C; IR (CH₂Cl₂) 2919, 2199, 1635, 1420, 1340, 1309, 1120, 1029, 955, 732 cm⁻¹; ¹H NMR (400 MHz, CDCl₃) δ 7.31-7.24 (m, 3 H), 7.21 (s, 1 H), 6.69 (dd, *J =* 3.6, 0.8 Hz, 1 H), 3.94 (app t, *J =* 5.0 Hz, 2 H), 3.83 (app t, *J =* 5.0 Hz, 2 H), 3.01 (app t, *J =* 5.0 Hz, 2 H), 2.91 (app t, *J* = 5.0 Hz, 2 H), 2.49 (d, *J =* 0.8 Hz, 3 H), 2.38 (s, 3 H); ¹³C NMR (100 MHz, CDCl₃) δ 153.2, 151.0, 145.5, 136.8, 135.7, 131.5, 129.0 (q, *J_{CF} =* 32.2 Hz), 125.9, 124.1 (q, *J_{CF} =* 272.1 Hz), 120.4 (q, *J_{CF}* = 3.8 Hz), 117.4, 116.0 (q, *J_{CF} =* 3.6 Hz), 85.4, 84.5, 51.8, 51.3, 47.3, 41.8, 17.9, 15.5; ¹⁹F NMR (376 MHz, CDCl₃) δ -62.2 (s, 3 F); HRMS (ESI) *m*/*z* calcd for C₂₀H₂₀F₃N₂OS ([M+H]⁺) 393.1243, found 393.1241.

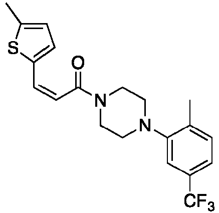

**(Z)-1-(4-(5-Chloro-2-methylphenyl)piperazin-1-yl)-3-(3-(trifluoromethyl)phenyl)prop-2-en-1-one.** A solution of 1-(4-(2-methyl-5-(trifluoromethyl)phenyl)piperazin-1-yl)-3-(5-methylthiophen-2-yl)prop-2-yn-1-one (0.0650 g, 0.166 mmol) in EtOAc (1.7 mL) was treated with Lindlar's catalyst (5% Pd on CaCO₃, lead poisoned, 0.0176 g, equivalent to 5 mol% Pd). The reaction was placed under a balloon of H₂ (3 vacuum/backfill cycles) and stirred at rt for 3 d, filtered through Celite, washed (EtOAc), and the combined filtrates were concentrated under reduced pressure. The crude residue was purified by automated chromatography on SiO₂ (4g column, liquid load CH₂Cl₂, hexanes to 40% EtOAc/hexanes, product eluted at 20% EtOAc/hexanes) to give (Z)-1-(4-(5-chloro-2-methylphenyl)piperazin-1-yl)-3-(3-(trifluoromethyl)-phenyl)prop-2-en-1-one (0.0443 g, 0.112 mmol, 68%) as a colorless solid: Mp 125.8-128.1 °C; IR (CH₂Cl₂) 2919, 2857, 1635, 1435, 1339, 1308, 1118, 1040, 806 cm⁻¹; ¹H NMR (400 MHz, CDCl₃) δ 7.28 (d, *J* = 8.0 Hz, 1 H), 7.25 (dd, *J =* 8.0, 1.3 Hz, 1 H), 7.15 (s, 1 H), 6.96 (d, *J =* 3.5 Hz, 1 H), 6.76 (d, *J =* 12.4 Hz, 1 H), 6.67 (dq, *J =* 3.5, 1.0 Hz, 1 H), 5.89 (d, *J =* 12.4 Hz, 1 H), 3.89 (app t, *J =* 4.8 Hz, 2 H), 3.69 (app t, *J =* 4.8 Hz, 2 H), 2.94 (app t, *J =* 4.8 Hz, 2 H), 2.82 (app t, *J =* 4.8 Hz, 2 H), 2.47 (d, *J =* 1.0 Hz, 3 H), 2.36 (s, 3 H); ¹³C NMR (100 MHz, CDCl₃) δ 166.9, 151.2, 143.7, 136.8, 135.9, 131.5, 131.2, 128.9 (q, *J_{CF}* = 32.2 Hz), 128.7, 125.2, 124.1 (q, *J_{CF} =* 272.1 Hz), 120.3 (q, *J_{CF} =* 3.7 Hz), 117.2, 115.9 (q, *J_{CF} =* 3.7 Hz), 51.6, 51.3, 46.6, 41.7, 17.9, 15.4; ¹⁹F NMR (376 MHz, CDCl₃) δ -62.2 (s, 3 F); HRMS (ESI) *m*/*z* calcd for C₂₀H₂₂F₃N₂OS ([M+H]⁺) 395.1399, found 393.1399.

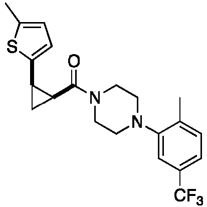

**(4-(2-Methyl-5-(trifluoromethyl)phenyl)piperazin-1-yl)((1RS,2SR)-2-(5-methylthiophen-2-yl)cyclopropyl)methanone.** A solution of CrCl₂ (0.105 g, 0.851 mmol) and (Z)-1-(4-(5-chloro-2-methylphenyl)piperazin-1-yl)-3-(3-(trifluoromethyl)phenyl)prop-2-en-1-one (0.0420 g, 0.142 mmol) in dry degassed THF (1.1 mL) was sparged with Ar for 5 min and treated with CH₂ICl (62uL, 0.532 mmol) at rt, heated for 22 h at 80 °C, cooled to rt, diluted with Et₂O (50 mL), and washed with 1 M aqueous HCl (3 x 20 mL). The organic layer was dried (MgSO₄), filtered and concentrated under reduced pressure. The crude residue was purified by automated chromatography on SiO₂ (4g column, liquid load CH₂Cl₂, 100% hexanes to 40% EtOAc/hexanes) to give a clear oil that was filtered through basic Al₂O₃ (eluting with 1:1 CH₂Cl₂/EtOAc), concentrated under reduced pressure, and dried under high vacuum to give (4-(2-methyl-5-(trifluoromethyl)phenyl)piperazin-1-yl)((1RS,2SR)-2-(5-methylthiophen-2-yl)cyclopropyl)methanone (0.0187 g, 0.0458 mmol, 43% (100% purity by ELSD)) as pale yellow viscous oil: IR (CH₂Cl₂) 2921, 2822, 1639, 1464, 1434, 1417, 1337, 1306, 1116, 1079, 1031, 801 cm⁻¹; ¹H NMR (400 MHz, CDCl₃) δ 7.26 (d, *J =* 8.4 Hz, 1 H), 7.24 (dd, *J =* 8.4, 1.4 Hz, 1 H), 7.04 (s, 1 H), 6.58 (t, *J =* 4.7 Hz, 1 H), 6.55 (dt, *J =* 3.5, 1.0 Hz, 1 H), 4.00 (bd, *J =* 13.0 Hz, 1 H), 3.88 (dt, *J =* 13.0, 3.5 Hz, 1 H), 3.64 (ddd, *J =* 12.4, 9.0, 3.2 Hz, 1 H), 3.34 (ddd, *J =* 13.0, 9.2, 2.9 Hz, 1 H), 2.82 (ddt, *J =* 19.4, 11.6, 3.7 Hz, 2 H), 2.53 (td, *J* = 8.6, 6.6 Hz, 1 H), 2.48-2.40 (m, 1 H), 2.38 (d, *J =* 1.0 Hz, 3 H), 2.34 (s, 3 H), 2.17 (td, *J =* 8.6, 6.6 Hz, 1 H), 1.77 (q, *J =* 6.0 Hz, 1 H), 1.38 (td, *J =* 8.6, 5.3 Hz, 1 H); ¹³C NMR (100 MHz, CDCl₃) δ 167.2, 151.4, 138.3, 137.6, 136.9, 131.4, 128.9 (q, *J_{CF} =* 32.0 Hz), 124.9, 124.3 (q, *J_{CF} =* 272.1 Hz), 124.2, 120.3 (q, *J_{CF} =* 4.0 Hz), 116.0 (q, *J_{CF} =* 3.7 Hz), 51.8, 51.7, 45.7, 42.3, 24.1, 19.4 , 17.9, 15.1, 12.3; ¹⁹F NMR (376 MHz, CDCl₃) δ -62.2 (s, 3 F); HRMS (ESI) *m*/*z* calcd for C₂₁H₂₄F₃N₂OS ([M+H]⁺) 409.1556, found 409.1555.

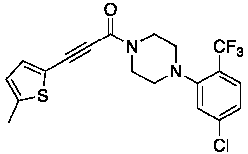

**1-(4-(5-Chloro-2-(trifluoromethyl)phenyl)piperazin-1-yl)-3-(5-methylthiophen-2-yl)prop-2-yn-1-one.** A solution of 3-(5-methylthiophen-2-yl)propiolic acid (0.0770 g, 0.463 mmol) and 1-(5-chloro-2-(trifluoromethyl)phenyl)piperazine hydrochloride (0.153 g, 0.510 mmol) in CH₂Cl₂ (4.6 mL) cooled to 0 °C was treated with Et₃N (0.26 mL, 1.85 mmol). The cooled solution was treated with T3P (50 wt. % solution in EtOAc, 0.49 mL, 0.695 mmol) dropwise and the reaction was stirred at 0 °C for 30 min, warmed to rt overnight, diluted with CH₂Cl₂ (30 mL), washed with H₂O (20 mL), satd. aqueous NaHCO₃ (20 mL), dried (MgSO₄), filtered, and concentrated under reduced pressure. The crude material was purified by automated chromatography on SiO₂ (4g column, liquid load CH₂Cl₂, hexanes to 40% EtOAc/hexanes), to give 1-(4-(5-chloro-2-(trifluoromethyl)phenyl)piperazin-1-yl)-3-(5-methylthiophen-2-yl)prop-2-yn-1-one (0.135 g, 0.326 mmol, 70%) as a light orange waxy solid: IR (CH₂Cl₂) 2918, 2200, 1629, 1596, 1425, 1309, 1224, 1144, 1120, 1027, 809 cm⁻¹; ¹H NMR (400 MHz, CDCl₃) δ 7.58 (d, *J =* 8.4 Hz, 1 H), 7.27-7.23 (m, 3 H), 6.69 (dd, *J =* 3.6, 0.8 Hz, 1 H), 3.91 (app t, *J =* 4.9 Hz, 2 H), 3.81 (app t, *J =* 5.0 Hz, 2 H), 2.98 (app t, *J =* 5.0 Hz, 2 H), 2.91 (app t, *J =* 5.0 Hz, 2 H), 2.49 (d, *J =* 0.8 Hz, 3 H); ¹³C NMR (100 MHz, CDCl₃) δ 153.2, 152.7, 152.6, 145.5, 138.8, 135.7, 128.5 (q, *J_{CF} =* 5.4 Hz), 125.9, 125.8 (q, *J_{CF} =* 29.3 Hz), 125.6, 124.7, 123.6 (q, *J_{CF} =* 273.0 Hz), 117.4, 85.4, 84.5, 53.6, 52.8, 47.3, 41.7, 15.5; ¹⁹F NMR (376 MHz, CDCl₃) δ -60.3 (s, 3 F); HRMS (ESI) *m*/*z* calcd for C₁₉H₁₇ClF₃N₂OS ([M+H]⁺) 413.0697, found 413.0693.

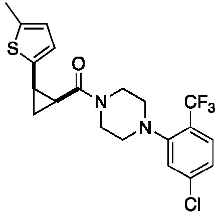

**(4-(5-Chloro-2-(trifluoromethyl)phenyl)piperazin-1-yl)((1*RS*,2*SR*)-2-(5-methylthiophen-2-yl)cyclopropyl)methanone.** A solution of 1-(4-(5-chloro-2-(trifluoromethyl)phenyl)piperazin-1-yl)-3-(5-methylthiophen-2-yl)prop-2-yn-1-one (0.112 g, 0.271 mmol) in EtOAc (2.7 mL) was treated with Lindlar's catalyst (5% Pd on CaCO₃, lead poisoned, 0.0289 g, equivalent to 5 mol% Pd). The reaction was placed under a balloon of H₂ (3 vacuum/backfill cycles) and stirred at rt for 3 d, filtered through Celite, washed (EtOAc), and the combined filtrates were concentrated under reduced pressure. The crude residue was purified by automated chromatography on SiO₂ (4g column, liquid load CH₂Cl₂, hexanes to 40% EtOAc/hexanes, product eluted at 20% EtOAc/hexanes) to give (Z)-1-(4-(5-chloro-2-(trifluoromethyl)-phenyl)piperazin-1-yl)-3-(5-methylthiophen-2-yl)prop-2-en-1-one (0.0655 g, 0.158 mmol) as a colorless foam.

A solution of CrCl₂ (0.116 g, 0.947 mmol) and (Z)-1-(4-(5-chloro-2-(trifluoromethyl)phenyl)-piperazin-1-yl)-3-(5-methylthiophen-2-yl)prop-2-en-1-one (0.0655 g, 0.158 mmol) in dry degassed THF (1.6 mL) was sparged with Ar for 5 min and treated with CH₂ICl (0.091 mL, 0.789 mmol) at rt, heated for 2 days at 80 °C, cooled to rt, diluted with EtOAc (50 mL), and washed with 1 M aqueous HCl (3 x 20 mL). The organic layer was dried (MgSO₄), filtered and concentrated under reduced pressure. The crude residue was purified by automated chromatography on SiO₂ (4g column, liquid load CH₂Cl₂, 100% hexanes to 30% EtOAc/hexanes, product eluted at 20% EtOAc/hexanes), filtered through basic Al₂O₃ (CH₂Cl₂/EtOAc, 1:1), and concentrated under reduced pressure. The resulting oil was crystalized from cyclohexane (~0.5 mL at rt), the crystals were washed with hexanes and dried under high vacuum to give (4-(5-chloro-2-(trifluoromethyl)phenyl)piperazin-1-yl)((1*RS*,2*SR*)-2-(5-methylthiophen-2-yl)cyclopropyl)methanone (0.0313 g, 0.0730 mmol, 27% (2 steps) (100% purity by ELSD)) as a colorless solid: Mp 84.5-86.8 °C; IR (CH₂Cl₂) 3007, 2920, 1642, 1595, 1464, 1435, 1405, 1308, 1144, 1120, 1031, 825, 803 cm⁻¹; ¹H NMR (400 MHz, CDCl₃) δ 7.54 (d, *J =* 8.5 Hz, 1 H), 7.21 (dd, *J* = 8.5, 1.5 Hz, 1 H), 7.03 (d, *J =* 1.5 Hz, 1 H), 6.61 (dd, *J =* 3.4, 1.0 Hz, 1 H), 6.59 (d, *J =* 3.4 Hz, 1 H), 4.08 (d, *J =* 13.0 Hz, 1 H), 3.87 (d, *J=* 13.0 Hz, 1 H), 3.57 (ddd, *J =* 13.0, 9.4, 3.0 Hz, 1 H), 3.23 (ddd, *J =* 13.0, 9.6, 3.0 Hz, 1 H), 2.78 (tt, *J =* 9.6, 4.7 Hz, 2 H), 2.52 (td, *J* = 8.8, 6.8 Hz, 1 H), 2.46-2.40 (m, 4 H), 2.31-2.25 (m, 1 H), 2.16 (ddd, *J =* 8.8, 8.2, 6.0 Hz, 1 H), 1.77 (q, *J =* 6.0 Hz, 1 H), 1.39 (td, *J =* 8.6, 6.0 Hz, 1 H); ¹³C NMR (100 MHz, CDCl₃) δ 167.1, 153.2, 138.6, 138.5, 137.4, 128.4 (q, *J_{CF} =* 5.4 Hz), 126.0 (q, *J_{CF} =* 28.2 Hz), 125.5, 125.1, 124.8, 124.0, 123.6 (q, *J_{CF} =* 273.0 Hz), 53.6, 53.2, 45.7, 42.4, 24.4, 19.4, 15.3, 12.4; ¹⁹F NMR (376 MHz, CDCl₃) δ -60.4 (s, 3 F); HRMS (ESI) *m*/*z* calcd for C₂₀H₂₁ClF₃N₂OS ([M+H]⁺) 429.1010, found 429.1006.

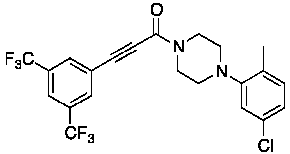

**3-(3,5-Bis(trifluoromethyl)phenyl)-1-(4-(5-chloro-2-methylphenyl)piperazin-1-yl)prop-2-yn-1-one.** A solution of 3-(3,5-bis(trifluoromethyl)phenyl)propiolic acid (0.250 g, 0.886 mmol) and 1-(5-chloro-2-methylphenyl)piperazine hydrochloride (0.263 g, 1.06 mmol) in CH₂Cl₂ (9.0 mL) cooled to 0 °C was treated Et₃N (0.5 mL, 3.54 mmol). The cooled solution was treated with T3P (50% solution in EtOAc) (1.0 mL, 1.33 mmol) dropwise and the reaction was stirred at 0 °C for 30 min and allowed to warm to rt overnight. The reaction was diluted with EtOAc (40 mL) and washed with H₂O (20 mL), satd. aqueous NaHCO₃ (20 mL), dried (MgSO₄), filtered, and concentrated under reduced pressure. The crude material was purified by automated chromatography on SiO₂ (4g column, gradient hexanes to EtOAc), to give the product (0.280 g, 0.590 mmol, 67%) as a pale yellow solid: Mp 129.7-132.4 °C; IR (CH₂Cl₂) 2927, 2221, 1638, 1432, 1381, 1279, 1180, 1137, 1044, 900, 684 cm⁻¹; ¹H NMR (400 MHz, CDCl₃) δ 8.00 (s, 2 H), 7.92 (s, 1 H), 7.13 (dd, *J =* 8.4, 0.4 Hz, 1 H), 7.01 (dd, *J =* 8.4, 2.0 Hz, 1 H), 6.96 (d, *J =* 2.0 Hz, 1 H), 3.97 (t, *J =* 5.0 Hz, 2 H), 3.85 (t, *J =* 5.0 Hz, 2 H), 3.00 (t, *J =* 5.0 Hz, 2 H), 2.91 (t, *J =* 5.0 Hz, 2 H), 2.30 (s, 3 H); ¹³C NMR (100 MHz, CDCl₃) δ 152.0, 151.6, 132.3 (q, *J* = 34.1 Hz), 132.2, 131.9, 131.0, 123.9, 123.5 (q, *J =* 3.6 Hz), 122.8, 122.6 (q, *J =* 273.1 Hz), 119.8, 87.0, 83.5, 51.9, 51.3, 47.5, 42.1, 17.4; ¹⁹F NMR (376 MHz, CDCl₃) δ -63.2 (s, 6 F); HRMS (ESI) *m*/*z* calcd for C₂₂H₁₈ClF₆N₂O ([M+H]⁺) 475.1006, found 475.1004.

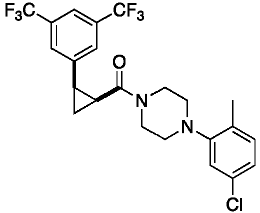

**((1*RS*,2*SR*)-2-(3,5-Bis(trifluoromethyl)phenyl)cyclopropyl)(4-(5-chloro-2-methylphenyl)piperazin-1-**yl)methanone. A solution of 3-(3,5-bis(trifluoromethyl)phenyl)-1-(4-(5-chloro-2-methylphenyl)piperazin-1-yl)prop-2-yn-1-one (0.280 g, 0.590 mmol) in EtOAc (6 mL) was treated with Lindlar's catalyst (5% Pd on CaCO₃, lead poisoned, 0.0628 g, equivalent to 5 mol% Pd). The reaction was placed under a balloon of H₂ (3 vacuum/backfill cycles) and stirred at rt for 3 d, filtered through Celite, washed (EtOAc), and the combined filtrates were concentrated under reduced pressure. The crude residue was purified by automated chromatography on SiO₂ (4g column, liquid load CH₂Cl₂, hexanes to 40% EtOAc/hexanes, pdt eluted at 30% EtOAc/hexanes) to give (*Z*)-3-(3,5-bis(trifluoromethyl)phenyl)-1-(4-(5-chloro-2-methylphenyl)piperazin-1-yl)prop-2-en-1-one (0.0710 g, 0.149 mmol) as a pale yellow oil.

A solution of CrCl₂ (0.110 g, 0.893 mmol) and (Z)-3-(3,5-bis(trifluoromethyl)phenyl)-1-(4-(5-chloro-2-methylphenyl)piperazin-1-yl)prop-2-en-1-one (0.0710 g, 0.149 mmol) in dry degassed THF (1.5 mL) was sparged with Ar for 5 min and treated with CH₂ICl (0.086 mL, 0.744 mmol) at rt, further sparged for 2 min, heated for 20 h at 80 °C, cooled to rt, diluted with EtOAc (50 mL), and washed with 1 M aqueous HCl (3 x 20 mL). The organic layer was dried (MgSO₄), filtered and concentrated under reduced pressure. The crude residue was purified by automated chromatography on SiO₂ (4g column, liquid load CH₂Cl₂, 100% hexanes to 40% EtOAc:hexanes), filtered through basic Al₂O₃ (CH₂Cl₂ /EtOAc, 1:1), recrystallized from cyclohexane, and dried under high vacuum to give ((1*RS*,2S*R*)-2-(3,5-bis(trifluoromethyl)phenyl)cyclopropyl)(4-(5-chloro-2-methylphenyl)piperazin-1-yl)methanone (0.0560 g, 0.114 mmol, 19% (2 steps) (100% purity by ELSD)) as a pale yellow oil: IR (CH₂Cl₂) 2820, 1641, 1466, 1277, 1167, 1132, 1036, 899, 682 cm⁻¹; ¹H NMR (400 MHz, CDCl₃) δ 7.73 (s, 1 H), 7.65 (s, 2 H), 7.07 (dd, *J =* 8.4, 0.6 Hz, 1 H), 6.97 (dd, *J =* 8.4, 2.0 Hz, 1 H), 6.73 (d, *J =* 2.0 Hz, 1 H), 3.81-3.64 (m, 3 H), 3.35 (ddd, *J =* 12.5, 8.8, 3.1 Hz, 1 H), 2.86 (dt, *J =* 11.4, 4.1 Hz, 1 H), 2.76 (dt, *J* = 11.9, 4.0 Hz, 1 H), 2.59 (td, *J* = 8.4, 6.8 Hz, 1 H), 2.38-2.27 (m, 3 H), 2.21 (s, 3 H), 1.96 (dt, *J =* 6.8, 5.6 Hz, 1 H), 1.47 (td, *J =* 8.4, 5.6 Hz, 1 H); ¹³C NMR (100 MHz, CDCl₃) δ 166.4, 151.6, 140.4, 132.0, 131.8, 131.4 (q, *J_{CF} =* 33.1 Hz), 130.9, 128.3 (q, *J_{CF}* = 1.1 Hz), 123.7, 123.3 (q, *J_{CF}* = 272.7 Hz), 120.4 (q, *J_{CF} =* 3.8 Hz), 119.6, 51.8, 51.6, 45.6, 42.3, 23.8, 23.4, 17.3, 11.3; ¹⁹F NMR (376 MHz, CDCl₃) δ -62.7 (s, 6 F); HRMS (ESI) *m*/*z* calcd for C₂₃H₂₂ClF₆N₂O ([M+H]⁺) 491.1319, found 491.1312.

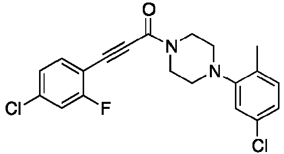

**3-(4-Chloro-2-fluorophenyl)-1-(4-(5-chloro-2-methylphenyl)piperazin-1-yl)prop-2-yn-1-one.** A solution of 3-(4-chloro-2-fluorophenyl)propiolic acid (0.190 g, 0.957 mmol) and 1-(5-chloro-2-methylphenyl)-piperazine hydrochloride (0.284 g, 1.15 mmol) in CH₂Cl₂ (9.6 mL) cooled to 0 °C was treated with Et₃N (0.53 mL, 3.83 mmol). The cooled solution was treated with T3P (50% solution in EtOAc) (1.0 mL, 1.44 mmol) dropwise and the reaction was stirred at 0 °C for 30 min, warmed to rt overnight, diluted with EtOAc (40 mL) and washed with H₂O (20 mL), satd. aqueous NaHCO₃ (20 mL), dried (MgSO₄), filtered, and concentrated under reduced pressure. The crude residue was purified by automated chromatography on SiO₂ (4g column, liquid load CH₂Cl₂, gradient 100% hexanes to 100% EtOAc) to give 3-(4-chloro-2-fluorophenyl)-1-(4-(5-chloro-2-methylphenyl)piperazin-1-yl)prop-2-yn-1-one (0.302 g, 0.772 mmol, 81%) as a pale yellow solid: Mp 135.1-137.3 °C; IR (CH₂Cl₂) 2917, 2820, 2219, 1632, 1489, 1431, 1291, 1224, 1039, 898, 819 cm⁻¹; ¹H NMR (400 MHz, CDCl₃) δ 7.50 (dd, *J =* 8.8, 8.0 Hz, 1 H), 7.17 (t, *J =* 2.0 Hz, 1 H), 7.15 (q, *J =* 2.0 Hz, 1 H), 7.11 (d, *J =* 8.4 Hz, 1 H), 6.98 (dd, *J =* 8.0, 2.0 Hz, 1 H), 6.94 (d, *J =* 2.4 Hz, 1 H), 3.97 (t, *J =* 5.0 Hz, 2 H), 3.83 (t, *J =* 5.0 Hz, 2 H), 2.96 (t, *J =* 5.0 Hz, 2 H), 2.88 (t, *J =* 5.0 Hz, 2 H), 2.28 (s, 3 H); ¹³C NMR (100 MHz, CDCl₃) δ 163.0 (d, *J_{CF} =* 257.4 Hz), 152.5, 151.6, 137.4 (d, *J_{CF} =* 10.1 Hz), 134.7 (d, *J_{CF} =* 1.4 Hz), 131.9 (d, *J_{CF} =* 25.0 Hz), 130.9, 125.0 (d, *J_{CF}* = 3.7 Hz), 123.7, 119.8, 116.8, 116.6, 107.9 (d, *J_{CF} =* 15.9 Hz), 86.6 (d, *J_{CF} =* 3.6 Hz), 83.0, 51.8, 51.2, 47.3, 41.9, 17.4; ¹⁹F NMR (376 MHz, CDCl₃) δ - 105.8 (s, 1 F); HRMS (ESI) *m*/*z* calcd for C₂₀H₁₈Cl₂FN₂O ([M+H]⁺) 391.0775, found 391.0781.

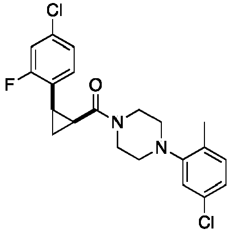

**((1*RS*,2*SR*)-2-(4-Chloro-2-fluorophenyl)cyclopropyl)(4-(5-chloro-2-methylphenyl)piperazin-1-**yl)methanone A solution of 3-(4-chloro-2-fluorophenyl)-1-(4-(5-chloro-2-methylphenyl)piperazin-1-yl)prop-2-yn-1-one (0.300 g, 0.767 mmol) in EtOAc (7.8 mL) was treated with Lindlar's catalyst (5% Pd on CaCO₃, lead poisoned, 0.0816 g, equivalent to 5 mol% Pd). The reaction was placed under a balloon of H₂ (3 vacuum/backfill cycles) and stirred at rt for 3 d, filtered through Celite, washed (EtOAc), and the combined filtrates were concentrated under reduced pressure. The crude residue was purified by automated chromatography on SiO₂ (4g column, liquid load CH₂Cl₂, 100% hexanes to 40% EtOAc/hexanes, pdt eluted at 30% EtOAc/hexanes) to give (Z)-3-(4-chloro-2-fluorophenyl)-1-(4-(5-chloro-2-methylphenyl)piperazin-1-yl)prop-2-en-1-one (0.138 g, 0.351 mmol, 46%) as a pale yellow oil.

A solution of CrCl₂ (0.259 g, 2.11 mmol) and (Z)-3-(4-chloro-2-fluorophenyl)-1-(4-(5-chloro-2-methylphenyl)piperazin-1-yl)prop-2-en-1-one (0.138 g, 0.351 mmol) in dry degassed THF (3.5 mL) was sparged with Ar for 5 min and treated with CH₂ICl (0.20 mL, 1.75 mmol) at rt, further sparged for 5 min, heated for 20 h at 80 °C, cooled to rt, diluted with EtOAc (50 mL) and washed with 1 M aqueous HCl (3 x 20 mL). The organic layer was dried (MgSO₄), filtered, and concentrated under reduced pressure. The crude residue was purified by automated chromatography on SiO₂ (4g column, liquid load CH₂Cl₂, 100% hexanes to 40% EtOAc/hexanes), filtered through basic Al₂O₃ (1:1 CH₂Cl₂/EtOAc) and dried under high vacuum to give ((1RS,2SR)-2-(4-chloro-2-fluorophenyl)cyclopropyl)(4-(5-chloro-2-methylphenyl)piperazin-1-yl)methanone (0.0930 g, 0.228 mmol, 65% (100% purity by ELSD)) as a colorless solid: Mp 100.2-103.0 °C; IR (CH₂Cl₂) 2950, 2819, 1638, 1490, 1435, 1225, 1034, 899, 818, 731 cm⁻¹; ¹H NMR (400 MHz, CDCl₃) δ 7.09-7.03 (m, 4 H), 6.97 (dd, *J =* 8.0, 2.0 Hz, 1 H), 6.81 (d, *J* = 2.0 Hz, 1 H), 3.81-3.66 (m, 3 H), 3.38 (ddd, *J* = 12.4, 8.0, 3.4 Hz, 1 H), 2.91-2.86 (m, 1 H), 2.77-2.72 (m, 1 H), 2.58 (qd, *J* = 8.4, 0.8 Hz, 1 H), 2.50 (ddd, *J =* 11.2, 8.0, 2.8 Hz, 1 H), 2.40 (ddd, *J =* 11.2, 8.0, 2.8 Hz, 1 H), 2.29 (ddd, *J =* 9.2, 8.0, 5.6 Hz, 1 H), 2.23 (s, 3 H), 1.90 (dt, *J =* 6.8, 5.6 Hz, 1 H), 1.34 (td, *J =* 8.4, 5.6 Hz, 1 H); ¹³C NMR (100 MHz, CDCl₃) δ 167.0, 161.9 (d, *J_{CF}* = 247.9 Hz), 151.9, 132.8 (d, *J_{CF} =* 10.5 Hz), 132.0, 131.8, 130.9, 129.8 (d, *J_{CF} =* 4.5 Hz), 124.2 (d, *J_{CF} =* 3.4 Hz), 123.5, 123.1 (d, *J_{CF} =* 14.3 Hz), 119.6, 115.4 (d, *J_{CF}* = 25.3 Hz), 51.8, 51.6, 45.6, 42.2, 22.2, 17.4, 17.4, 9.3; ¹⁹F NMR (376 MHz, CDCl₃) δ -116.4 (s, 1 F); HRMS (ESI) *m*/*z* calcd for C₂₁H₂₂Cl₂FN₂O ([M+H]⁺) 407.1088, found 407.1089.

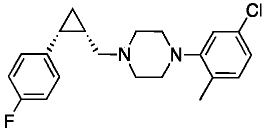

**1-(5-Chloro-2-methylphenyl)-4-(((1*S*,2*R*)-2-(4-fluorophenyl)cyclopropyl)methyl)piperazine (JKJ741.047)** A solution of (4-(5-chloro-2-methylphenyl)piperazin-1-yl)((1S,2R)-2-(4-fluorophenyl)-cyclopropyl)methanone (0.0372 g, 0.0998 mmol) in THF (0.4 mL) was treated with BH₃•DMS (1M solution in THF, 0.4 mL, 0.400 mmol) under Ar and the reaction was heated at reflux for overnight. The reaction was cooled to 0 °C and quenched by the addition of 1M NaOH and stirred for 20 min at 0°C until all of the bubbling subsided. The reaction was extracted with EtOAc (3 x 20 mL), and the combined organic layers were dried (Na₂SO₄), filtered and concentrated in vacuo. The crude material was stirred in 1M HCl overnight at rt. The resulting solution was made basic with 1M NaOH and extracted with EtOAc (3 x 10 mL). The combined organic layers were dried (Na₂SO₄), filtered and concentrated in vacuo. The crude residue was purified by automated chromatography on SiO₂ (4g column, hexanes to EtOAc; pdt eluted at 30% EtOAc/hexanes) to afford the product (0.0157 g, 0.0437 mmol, 44%) as a pale yellow oil: IR (CH₂Cl₂) 2927, 2819, 1591, 1510, 1489, 1224, 1095, 835 cm⁻¹; ¹H NMR (600 MHz, acetone-d6) δ 7.32-7.29 (m, 2 H), 7.14 (d, *J =* 8.0 Hz, 1 H), 7.06-7.02 (m, 2 H), 6.98 (d, *J* = 2.1 Hz, 1 H), 6.95 (dd, *J=* 8.0, 2.2 Hz, 1 H), 2.85 (bs, 4 H), 2.46 (bs, 4 H), 2.26 (dd, *J =* 12.7, 6.2 Hz, 1 H), 2.20 (q, *J =* 8.0 Hz, 4 H), 1.93 (dd, *J =* 12.7, 7.1 Hz, 1 H), 1.35-1.29 (m, 2 H), 1.09 (td, *J* = 8.4, 5.4 Hz, 1 H), 0.84 (q, *J =* 5.4 Hz, 1 H); ¹³C NMR (150 MHz, CDCl₃) δ 162.1 (d, *J =* 242.0 Hz), 154.0, 135.9 (d, *J =* 3.1 Hz), 132.9, 132.1, 131.8, 131.6 (d, *J =* 8.0 Hz), 123.4, 119.9, 115.3 (d, *J* = 21.1 Hz), 58.3, 54.2, 52.4, 20.4, 17.6, 17.1 10.1; ¹⁹F NMR (376 MHz, CDCl₃) δ - 119.1 (s, 1 F); HRMS (ESI) *m*/*z* calcd for C₂₁H₂₅ClFN₂ ([M+H]⁺) 359.1685, found 359.1682. [α]¹⁹_{D} +30.7 (c 0.48, MeOH).

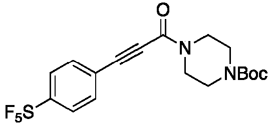

***tert*-Butyl 4-(3-(4-(pentafluoro-16-sulfaneyl)phenyl)propioloyl)piperazine-1-carboxylate (JKJ759.024).** A solution of 3-(4-(pentafluoro-└⁶-sulfanyl)phenyl)propiolic acid (0.400 g, 1.47 mmol) and N-boc-piperazine (0.328 g, 1.76 mmol) in CH₂Cl₂ (15 mL) at 0 °C was treated Et₃N (0.62 mL, 4.41 mmol). The cooled solution was treated with T3P (50% solution in EtOAc) (1.6 mL, 2.20 mmol) dropwise and the reaction was stirred at 0 °C for 30 min and allowed to warm to rt overnight. The reaction was diluted with EtOAc (50 mL) and washed with water (20 mL), satd. aqueous NaHCO₃ (20 mL), dried (MgSO₄), filtered, and concentrated in vacuo. The crude material was purified by chromatography on SiO₂ (1:1 hexanes/ EtOAc), to give *tert*-butyl 4-(3-(4-(pentafluoro-16-sulfaneyl)phenyl)propioloyl)piperazine-1-carboxylate (0.457 g, 1.04 mmol, 71%) as a tan solid: Mp 192.4-195.0 °C; IR (CH₂Cl₂) 2981, 1697, 1635, 1419, 1255, 1239, 1167, 841 cm⁻¹; ¹H NMR (400 MHz, CDCl₃) δ 7 7.76 (dt, *J* = 9.0, 2.0 Hz, 2 H), 7.63 (d, *J =* 9.0 Hz, 2 H), 3.79-3.76 (m, 2 H), 3.67 (t, *J =* 4.8 Hz, 2 H), 3.54-3.51 (m, 2 H), 3.46 (t, *J =* 4.8 Hz, 2 H); ¹³C NMR (100 MHz, CDCl₃) δ 154.4 (quint, *J =* 40.0 Hz), 152.4, 132.5, 126.3 (t, *J* = 4.7 Hz), 123.9, 99.9, 88.4, 83.0, 80.6, 46.9, 43.3, 41.5, 28.3; ¹⁹F NMR (376 MHz, CDCl₃) δ 83.0 (t, *J =* 150.5 Hz, 1 F), 62.4 (d, *J =* 150.2 Hz, 4 F).; HRMS (ESI) *m*/*z* calcd for C₁₈H₂₂F₅N₂O₂S ([M+H]⁺) 441.1266, found 441.1264.

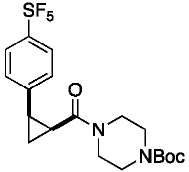

***tert-*Butyl 4-((1*RS*,2*SR*)-2-(4-(pentafluoro-λ6-sulfaneyl)phenyl)cyclopropane-1-carbonyl)piperazine-1-carboxylate (JKJ759.031/035).** A solution of tert-butyl 4-(3-(4-(pentafluorosulfanyl)phenyl)propioloyl)-piperazine-1-carboxylate (0.420 g, 0.954 mmol) in EtOAc (10 mL) at rt was treated with quinoline (0.56 mL, 4.77 mmol) and 5% Pd/BaSO₄ (0.0203 g, 0.0096 mmol, 1 mol% based on Pd) and the reaction was stirred under an atmosphere of H₂ (3 x backfill cycles) for 4 h. Analysis by TLC (1:1 H:EA) indicated that the starting material had been mostly consumed. The reaction was filtered (eluting with EtOAc), and the filtrate was washed with 1M HCl (3 x 25 mL), dried (Na₂SO₄), filtered and concentrated in vacuo. The crude residue was purified by chromatography on SiO₂ (1:1 hexanes/EtOAc to EtOAc) to afford the product (0.408 g, 0.922 mmol) as a colorless solid. This solid was carried on to the cyclopropanation 759.035.

A solution of CrCl₂ (0.667 g, 5.43 mmol) and (Z)-tert-butyl 4-(3-(4-(pentafluorosulfanyl)phenyl)-acryloyl)piperazine-1-carboxylate (0.400 g, 0.904 mmol) in anhydrous THF (9 mL) and the mixture was sparged with Ar for 15 min and added CH₂ICl (0.52 mL, 4.52 mmol) at rt and under Ar atmosphere. The reaction mixture was stirred for 24 h at 80 °C, cooled to rt, diluted with EtOAc (80 mL) and washed with 1M aqueous HCl (3 x 20 mL). The organic layer was dried (MgSO₄), filtered and concentrated in vacuo. The crude material was purified by chromatography on SiO₂ (EtOAc) to afford a the product as a clear oil. The product was filtered through basic Al₂O₃ (eluting with EtOAc) concentrated to afford the product (0.0920 g, 0.202 mmol, 21% (2 steps)) as a colorless solid: Mp 125.3-128.5 °C; IR (neat) 2980, 1679, 1635, 1429, 1364, 1239, 1171, 1030, 829 cm⁻¹; ¹H NMR (400 MHz, CDCl₃) δ 7.62 (d, *J* = 8.6 Hz, 2 H), 7.22 (d, *J =* 8.6 Hz, 2 H), 3.55-3.48 (m, 4 H), 3.40-3.35 (m, 1 H), 3.26-3.20 (m, 1 H), 2.85-2.75 (m, 2 H), 2.48 (q, *J* = 8.8 Hz, 1 H), 2.24 (td, *J =* 8.8, 6.0 Hz, 1 H), 1.89 (q, *J =* 6.0 Hz, 1 H), 1.46-1.38 (m, 10 H); ¹³C NMR (100 MHz, CDCl₃) δ 166.7, 154.4, 152.1 (quint, *J*_{CF} = 17.3 Hz), 141.6, 127.8, 125.6 (quint, *J*_{CF} = 4.6 Hz), 80.3, 45.0, 43.2, 41.6, 28.3, 24.3, 23.7 11.2; ¹⁹F NMR (376 MHz, CDCl₃) δ 84.9 (quint, *J* = 150.2 Hz, 1 F), 63.1 (d, *J* = 150.0 Hz, 4 F).; HRMS (ESI) *m*/*z* calcd for C₁₉H₂₅F₅N₂O₃SNa ([M+Na]⁺) 479.1398, found 479.1395.

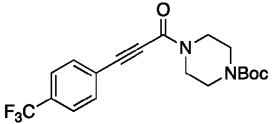

***tert*-Butyl 4-(3-(4-(trifluoromethyl)phenyl)propioloyl)piperazine-1-carboxylate (JKJ759.025).** A solution of 3-(4-trifluoromethylphenyl)propiolic acid (0.500 g, 2.33 mmol) and N-Boc-pipreazine (0.522 g, 2.80 mmol) in CH₂Cl₂ (23 mL) at 0 °C was treated Et₃N (1.0 mL, 7.00 mmol). The cooled solution was treated with T3P (50% solution in EtOAc) (2.5 mL, 3.50 mmol) dropwise and the reaction was stirred at 0 °C for 30 min and allowed to warm to rt overnight. The reaction was diluted with EtOAc (100 mL) and washed with water (20 mL), satd. aqueous NaHCO₃ (20 mL), dried (MgSO₄), filtered, and concentrated in vacuo. The crude material was purified by chromatography on SiO₂ (1:1 hexanes/EtOAc), to give the product (0.841 g, 2.20 mmol, 94%) as a colorless solid: Mp 174.2-175.9 °C; IR (CH₂Cl₂) 2981, 2230, 1678, 1622, 1425, 1321, 1162, 1123, 1064, 837 cm⁻¹; ¹H NMR (400 MHz, CDCl₃) δ 7.65 (d, *J =* 8.8 Hz, 2 H), 7.63 (d, *J* = 8.8 Hz, 2 H), 3.80-3.77 (m, 2 H), 3.66 (t, *J =* 5.0 Hz, 2 H), 3.54-3.51 (m, 2 H), 3.46 (t, *J =* 5.0 Hz, 2 H), 1.47 (s, 9 H); ¹³C NMR (100 MHz, CDCl₃) δ 154.4, 152.6, 132.6, 131.8 *(q, J_{CF} =* 32.8 Hz), 125.5 (q, *J_{CF}* = 3.9 Hz), 124.0, 123.6 (q, *J_{CF}* = 272.7 Hz), 89.2, 82.5, 80.5, 46.8, 41.5, 28.3; ¹⁹F NMR (376 MHz, CDCl₃) δ -63.1 (s, 3 F); HRMS (ESI) *m*/*z* calcd for C₁₉H₂₁F₃N₂O₃ ([M+H]⁺) 383.1577, found 383.1576.

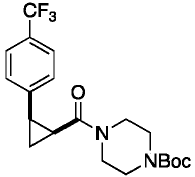

***tert*-Butyl 4-((1R*S*,2S*R*)-2-(4-(trifluoromethyl)phenyl)cyclopropane-1-carbonyl)piperazine-1-carboxylate (JKJ759.043).** A solution of tert-butyl 4-(3-(4-(trifluoromethyl)phenyl)propioloyl)piperazine-1-carboxylate (0.840 g, 2.20 mmol) in EtOAc (22 mL) at rt was treated with quinoline (1.3 mL, 11.0 mmol) and 5% Pd/BaSO₄ (0.0468 g, 0.0220 mmol, 1 mol% based on Pd) and the reaction was stirred under an atmosphere of H₂ (3 x backfill cycles) for 2 h. Analysis by TLC ( 1:1 H:EA) indicated that the starting material had been mostly consumed. The reaction was filtered (eluting with EtOAc), and the filtrate was washed with 1M HCl (3 x 25 mL), dried (Na₂SO₄), filtered and concentrated in vacuo. The crude residue was purified by chromatography on SiO2 (1:1 hexanes/EtOAc to EtOAc) to afford the product (0.718 g, 1.87 mmol, 85%) as a colorless solid.

A solution of CrCl₂ (1.38 g, 11.2 mmol) and tert-butyl (Z)-4-(3-(4-(trifluoromethyl)phenyl)-acryloyl)piperazine-1-carboxylate (0.718 g, 1.87 mmol) in dry degassed THF (19 mL) (previously sparged with Ar for 15 min) and treated with CH₂ICl (1.08 mL, 9.34 mmol) and sparged with Ar for 2 min. The reaction mixture was stirred for 24 h at 80 °C under an atmosphere of Ar, cooled to rt, diluted with EtOAc (80 mL), and washed with 1 M aqueous HCl (3 x 20 mL). The organic layer was dried (MgSO₄), filtered and concentrated in vacuo. The crude material was purified by chromatography on SiO₂ (EtOAc) to afford the product as a colorless oil. The product was filtered through basic Al₂O₃ (eluting with EtOAc) concentrated to give *tert*-butyl 4-((1*RS*,2*SR*)-2-(4-(trifluoromethyl)phenyl)cyclopropane-1-carbonyl)piperazine-1-carboxylate (0.194 g, 0.487 mmol, 26%) as a colorless solid: Mp 108.6-111.9 °C; IR (CH₂Cl₂) 2978, 1693, 1641, 1417, 1324, 1236, 1162, 1116, 1069, 1017, 997, 844 cm⁻¹; ¹H NMR (400 MHz, CDCl₃) δ 7.50 (d, *J =* 8.4 Hz, 2 H), 7.24 (d, *J =* 8.4 Hz, 2 H), 3.59-3.54 (m, 1 H), 3.51-3.36 (m, 4 H), 3.23-3.16 (m, 1 H), 2.82-2.76 (m, 1 H), 2.71-2.66 (m, 1 H), 2.50 (q, *J =* 8.8 Hz, 1 H), 2.23 (td, *J =* 8.8, 6.0 Hz, 1 H), 1.90 (q, *J =* 6.0 Hz, 1 H), 1.46-1.37 (m, 10 H); ¹³C NMR (100 MHz, CDCl₃) δ 166.9, 154.4, 141.6, 128.7 (q, *J =* 32.4 Hz), 127.9, 125.0 (q, *J =* 3.9 Hz), 124.1 (q, *J =* 272.0 Hz), 80.3, 45.0, 41.6, 28.3, 24.3, 24.0, 11.0; ¹⁹F NMR (376 MHz, CDCl₃) δ -62.4 (s, 3 F); HRMS (ESI) *m*/*z* calcd for C₂₀H₂₆F₃N₂O₃ ([M+H]⁺) 399.1890, found 399.1888.

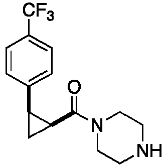

**Piperazin-1-yl((1*S*,2*R*)-2-(4-(trifluoromethyl)phenyl)cyclopropyl)methanone (JKJ759.045).** A solution of tert-butyl 4-(2-(4-(trifluoromethyl)phenyl)cyclopropanecarbonyl)piperazine-1-carboxylate 759.043 (0.169 g, 0.424 mmol) in THF (0.4 mL) was treated with 4M HCl in dioxane (0.5 mL, 2.12 mmol) was added and the reaction was stirred at rt for overnight. The soln was diluted with hexanes (20 mL) and the resulting precipitate was filtered and washed with additional hexanes and Et2O. The product was dried under high vacuum to afford the product (0.141 g, 0.421 mmol, 99%) as a colorless solid.

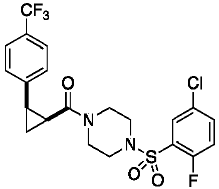

**(4-((5-Chloro-2-fluorophenyl)sulfonyl)piperazin-1-yl)((1*S*,2*R*)-2-(4-(trifluoromethyl)phenyl)-cyclopropyl)methanone (JKJ759.047).** A solution of piperazin-1-yl((1RS,2SR)-2-(4-(trifluoromethyl)-phenyl)cyclopropyl)methanone hydrochloride (0.0610 g, 0.182 mmol) and 5-chloro-2-fluorobenzene-1-sulfonyl chloride (0.0500 g, 0.219 mmol) in CH₂Cl₂ (2.0 mL) at 0 °C was treated with Et₃N (1.0 mL, 7.00 mmol), stirred at 0 °C for 30 min, warmed to rt overnight, and concentrated under reduced pressure. The crude material was purified by chromatography on SiO₂ (EtOAc), to give the product (0.0834 g, 0.170 mmol, 93% (100% purity by ELSD)) as a colorless solid: Mp 139.7-142.3 °C; IR (CH₂Cl₂) 3095, 2859, 1642, 1469 1324, 1163, 1113, 1069, 939, 844, 736 cm⁻¹; ¹H NMR (400 MHz, CDCl₃) δ 7.73 (dd,*J* = 5.6, 2.6 Hz, 1 H), 7.53 (ddd, *J =* 8.8, 4.0, 2.6 Hz, 1 H), 7.39 (d, *J =* 8.4 Hz, 2 H), 7.18 (d, *J* = 8.4 Hz, 2 H), 7.13 (t, *J =* 8.8 Hz, 1 H), 3.86 (bd, *J =* 13.2 Hz, 1 H), 3.72 (bd, *J =* 13.2 Hz, 1 H), 3.51 (ddd, *J* = 13.2, 9.2, 2.8 Hz, 1 H), 3.38 (bd, *J* = 13.2 Hz, 1 H), 3.26-3.14 (m, 2 H), 2.49 (q, *J =* 8.8 Hz, 1 H), 2.35-2.27 (bq, *J =* 11.6 Hz, 2 H), 2.19 (ddd, *J* = 9.2, 8.8, 6.0 Hz, 1 H), 1.86 (q, *J =* 6.0 Hz, 1 H), 1.39 (td, *J* = 8.8, 5.6 Hz, 1 H); ¹³C NMR (100 MHz, CDCl₃) δ 166.6, 157.1 (d, *J =* 255.5 Hz), 141.4, 135.1 (d, *J =* 8.6 Hz), 130.5, 130.0 (d, *J =* 3.6 Hz), 128.5 (q, *J =* 32.5 Hz), 127.7, 126.3 (d, *J =* 16.4 Hz), 124.8 (q, *J =* 3.8 Hz), 124.1 (q, *J* = 271.6 Hz), 118.7 (d, *J =* 23.8 Hz), 46.0, 45.4, 44.9, 41.3, 24.3, 23.9, 10.9;¹⁹F NMR (376 MHz, CDCl₃) δ -62.2 (s, 3 F), -111.2 (s, 1 F); HRMS (ESI) *m*/*z* calcd for C₂₁H₂₀F₄N₂O₃SCl ([M+H]⁺) 491.0814, found 491.0812.

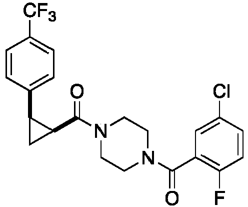

**(4-(5-Chloro-2-fluorobenzoyl)piperazin-1-yl)((1*S*,2*R*)-2-(4-(trifluoromethyl)phenyl)cyclopropyl)-methanone (JKJ759.046).** A solution of piperazin-1-vl((1RS,2SR)-2-(4-(trifluoromethyl)phenyl)-cyclopropyl)methanone hydrochloride (0.0800 g, 0.239 mmol) and 5-chloro-2-fluorobenzoic acid (0.0501 g, 0.287 mmol) in CH₂Cl₂ (2.5 mL) cooled to 0 °C was treated with Et₃N (0.13 mL, 0.956 mmol). The cooled solution was treated with T3P (50% solution in EtOAc) (0.25 mL, 0.358 mmol) dropwise and the reaction was stirred at 0 °C for 30 min, warmed to rt overnight, diluted with EtOAc (50 mL), and washed with water (20 mL), satd. Aqueous NaHCO₃ (20 mL), dried (MgSO₄), filtered, and concentrated under reduced pressure. The crude residue was purified by chromatography on SiO₂ (EtOAc), to give (4-(5-chloro-2-fluorobenzoyl)piperazin-1-yl)((1S,2R)-2-(4-(trifluoromethyl)phenyl)cyclopropyl)methanone (0.0982 g, 0.216 mmol, 90% (100% purity by ELSD) as a colorless solid: Mp 143.2-145.9 °C; IR (CH₂Cl₂) 3010, 2865, 1635, 1432, 1324, 1113, 1068, 1009, 844, 734 cm⁻¹; ¹H NMR (400 MHz, CDCl₃, 50 °C rotomers) δ 7.50 (d, *J =* 8.0 Hz, 2 H), 7.36-7.24 (m, 4 H), 7.01 (t, *J =* 8.8 Hz, 1 H), 3.90 (bs, 1 H), 3.60 (bs, 3 H), 3.21 (bs, 2 H), 2.98 (bs, 1 H), 2.79 (bs, 1 H), 2.51 (bs, 1 H), 2.23 (bs, 1 H), 1.91 (q, *J =* 6.0 Hz, 1 H), 1.40 (td, *J =* 8.4, 5.6 Hz, 1 H); ¹³C NMR (100 MHz, CDCl₃, 20 °C) δ 167.0, 163.8, 156.4 (d, *J =* 248.1 Hz), 141.5, 131.6 (d, *J =* 8.1 Hz), 130.2, 129.2, 128.8 (q, *J =* 32.7 Hz), 127.9, 125.1 (q, *J =* 3.7 Hz), 124.2 (q, *J* = 271.8 Hz), 117.3 (d, *J =* 23.6 Hz), 46.7, 44.8, 42.3, 41.9, 24.2, 24.1, 11.0; ¹⁹F NMR (376 MHz, CDCl₃) δ -62.4 (s, 3 F), -117.7 (s, 1 F); HRMS (ESI) *m*/*z* calcd for C₂₂H₂₀F₄N₂O₂Cl ([M+H]⁺) 455.1144, found 455.1143.

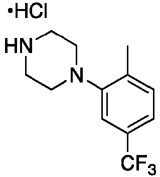

**1-(2-Methyl-5-(trifluoromethyl)phenyl)piperazine hydrochloride.** A solution of Boc-piperazine (2.14 g, 11.5 mmol), KOtBu (2.35 g, 20.9 mmol), (racemic)-BINAP (0.671 g, 1.05 mmol), Pd₂(dba)₃ (0.194 g, 0.209 mmol) in dry toluene (105 mL) was sparged with Ar for 20 min, treated with 2-bromo-1-methyl-4-(trifluoromethyl)benzene (2.50 g, 10.5 mmol), and the mixture was heated under Ar at 100 °C overnight, cooled to rt, diluted with Et₂O (125 mL), filtered through Celite, washed (Et₂O), and the combined filtrates were concentrated under reduced pressure. The crude residue was purified by chromatography on SiO₂ (hexanes/EtOAc, 9:1) to give tert-butyl 4-(2-methyl-5-(trifluoromethyl)phenyl)piperazine-1-carboxylate (2.49 g, 7.23 mmol) as an orange oil.

A solution of tert-butyl 4-(2-methyl-5-(trifluoromethyl)phenyl)piperazine-1-carboxylate (2.49 g, 7.23 mmol) in THF (3.5 mL) was cooled to 0 °C and treated with 4M HCl in dioxane (9.0 mL, 36.2 mmol) was added and the reaction was stirred at 0 °C for 30 min and then rt for 4 h. The solution was concentrated under reduced pressure and the tan solid was precipitated in ether, filtered off from the solution, washed with Et₂O, dried under vacuum to give the crude product. The crude material was recrystallized from EtOH/hexanes (1:1) to give the product (1.71 g, 6.10 mmol, 58% (2 steps)) as colorless needles. Mp 296 °C (decomp); IR (CH₂Cl₂) 2939, 2799, 2498, 1610, 1418, 1338, 1307, 1153, 1076, 950, 827 cm⁻¹; ¹H NMR (400 MHz, DMSO-d₆) δ 9.44 (bs, 2 H), 7.42 (d, *J =* 7.9 Hz, 1 H), 7.35 (dd, *J =* 7.9, 1.0 Hz, 1 H), 7.25 (d, *J =* 1.0 Hz, 1 H), 3.22 (dd, *J =* 6.2, 3.6 Hz, 4 H), 3.12 (dd, *J* = 6.2, 3.6 Hz, 4 H), 2.33 (s, 3 H); ¹³C NMR (100 MHz, DMSO-d₆) δ 150.8, 137.2, 131.9, 127.5 (q, *J_{CF} =* 31.6 Hz), 124.2 (q, *J_{CF} =* 272.1 Hz), 120.1 (q, *J_{CF} =* 3.9 Hz), 115.3 (q, *J_{CF} =* 3.8 Hz), 47.9, 43.1, 17.7; ¹⁹F NMR (376 MHz, CDCl₃) δ -60.7 (s, 3 F); HRMS (ESI) *m*/*z* calcd for C₁₂H₁₆F₃N₂ ([M+H]⁺) 245.1260, found 245.1261.

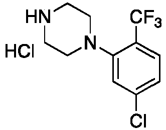

**1-(5-Chloro-2-(trifluoromethyl)phenyl)piperazine hydrochloride.** Two flasks each containing a solution Boc-piperazine (1.58 g, 8.48 mmol), KOtBu (1.73 g, 15.4 mmol), (racemic)-BINAP (0.480 g, 0.771 mmol), Pd₂dba₃ (0.142 g, 0.154 mmol) in toluene (8 mL) was sparged with argon for 15 min and treated with 2-bromo-4-chloro-1-(trifluoromethyl)benzene (2.00 g, 7.71 mmol), heated under N₂ at 80 °C for 24 h, cooled to rt, combined, diluted with Et₂O (100 mL) and added Celite and filtered through Celite, washed (Et₂O), and the combined organic layers were concentrated in vacuo. The crude residue was purified by chromatography on SiO₂ (hexanes/EtOAc, 9:1) to give 4-(5-chloro-2-(trifluoromethyl)phenyl)piperazine-1-carboxylate (3.12 g, 8.55 mmol) as an orange oil.

A solution of tert-butyl 4-(5-chloro-2-(trifluoromethyl)phenyl)piperazine-1-carboxylate (3.12 g, 8.55 mmol) in THF (8 mL) was treated with 4M HCl in dioxane (10.7 mL, 42.8 mmol), stirred at rt overnight, diluted with hexanes (100 mL), and the resulting precipitate was filtered and washed with additional hexanes and Et₂O. The crude material was recrystallized at rt (EtOH/hexanes), crystals were collected by vacuum filtration, washed (hexanes) and dried under high vacuum to give 1-(5-chloro-2-(trifluoromethyl)phenyl)-piperazine hydrochloride (1.88 g, 6.24 mmol, 41% (2 steps)) as tan solid: Mp 272 °C (decomp); IR (neat) 2947, 2726, 2480, 1599, 1576, 1307, 1118, 1037, 946, 819 cm⁻¹; ¹H NMR (400 MHz, DMSO-d₆) δ 9.58 (s, 2 H), 7.72 (d, *J =* 8.8 Hz, 1 H), 7.57 (d, *J =* 1.6 Hz, 1 H), 7.46 (dd, *J* = 8.8, 1.6 Hz, 1 H), 3.13 (bs, 8 H); ¹³C NMR (100 MHz, DMSO-d₆) δ 152.2, 138.3, 129.0 (q, *J =* 5.4 Hz), 125.9, 124.7, 124.2 (q, *J =* 29.7 Hz), 123.7 (q, *J* = 273.0 Hz), 49.6, 43.1; ¹⁹F NMR (376 MHz, CDCl₃) δ -59.0 (s, 3 F); HRMS (ESI) *m*/*z* calcd for C₁₁H₁₃N₂ClF₃ ([M+H]⁺) 265.0714, found 265.0712.

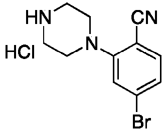

**4-Bromo-2-(piperazin-1-yl)benzonitrile hydrochloride.** A suspension of 4-bromo-2-fluorobenzonitrile (2.00 g, 10.0 mmol), 1-boc-piperazine (1.86 g, 10.0 mmol), and Et₃N (1.4 mL, 10.0 mmol) in anhydrous MeCN (5.0 mL) was heated to 110 °C for 21 h, cooled to rt, and concentrated under reduced pressure. The crude residue was purified by chromatography on SiO₂ (hexanes/EtOAc, 9:1) to give tert-butyl 4-(5-bromo-2-cyanophenyl)piperazine-1-carboxylate (3.03 g, 8.27 mmol) as a yellow oil.

A solution of tert-butyl 4-(5-bromo-2-cyanophenyl)piperazine-1-carboxylate (3.03 g, 8.27 mmol) in THF (4 mL) was cooled to 0 °C and treated with 4M HCl in dioxane (10.3 mL, 41.4 mmol), stirred at 0 °C for 30 min, rt for 16 h, diluted with hexanes (200 mL), and the resulting precipitate was collected by vacuum filtration, washed with hexanes, ether, and dried under high vacuum to give 4-bromo-2-(piperazin-1-yl)benzonitrile hydrochloride (3.82 g, 13.6 mmol, 79% (2 steps)) as a colorless solid: Mp 288 °C (decomp); IR (neat) 2901, 2748, 1459, 1129, 1581, 1411, 1241, 1118, 949, 874 cm⁻¹; ¹H NMR (400 MHz, MeOD-d4) δ 7.59 (d, *J =* 8.0 Hz, 1 H), 7.44 (d, *J =* 1.6 Hz, 1 H), 7.37 (dd, *J =* 8.0, 1.6 Hz, 1 H), 3.49 (td, *J* = 4.0, 1.2 Hz, 4 H), 3.44 (td, *J* = 4.0, 1.2 Hz, 4 H); ¹³C NMR (100 MHz, MeOD-d4) δ 156.5, 136.4, 129.9, 127.8, 124.2, 118.2, 106.7, 49.6, 44.9; HRMS (ESI) *m*/*z* calcd for C₁₁H₁₃N₃Br ([M+H]⁺) 266.0287, found 266.0286.

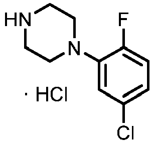

**1-(5-Chloro-2-fluorophenyl)piperazine hydrochloride.** Under N₂ atmosphere, CuBr (0.471 g, 3.22 mmol), 1,1'-bi-2-naphthol (0.692 g, 2.41 mmol) and DMF (8.04 mL) was added to the flame-dried flask. The mixture was stirred for 10 minutes before the addition of 1-Boc-piperazine (4.49 g, 24.1 mmol), K₃PO₄ (7.04 g, 32.2 mmol) and 2-bromo-4-chloro-1-fluorobenzene (2.00 mL, 16.1 mmol). The reaction mixture was stirred at 120 °C for 22.0 h. After cooling to room temperature, the mixture was diluted with EtOAc (50 mL) and filtered through Celite pad. The filtrate was sequentially washed with saturated aqueous NH₄Cl (50 mL) and brine (50 mL×3). The resulting organic phase was dried (MgSO₄), filtered and concentrated in vacuo. The crude product was filtered through a short column of SiO₂ (EtOAc/hexanes, 1:15) to yield a mixture of *tert*-butyl 4-(5-chloro-2-fluorophenyl)piperazine-1-carboxylate (1.50 g) as a light yellow oil. This mixture was used without further purification.

To a solution of the above product (1.50 g) in 1,4-dioxane (11.7 mL), HCl (4 M in 1,4-dioxane, 4.76 mL) was added at 0 °C. The mixture was stirred at RT for 14 h. The thick suspension was diluted with hexanes (50 mL) and the resulting solid was collected by filtration, washed with hexanes and Et₂O, and dried to give the desired compound as a light yellow solid (0.408 g, 10% over 2 steps). Mp: 173-174 °C; IR (neat): 3013, 2956, 2838, 2528, 2484, 2391, 1516, 1478, 1456, 1393, 1269, 1123, 1042, 1019 cm⁻¹; ¹H-NMR (500 MHz; DMSO-d6): δ 9.29 (s, 2 H), 7.23 (ddd, *J =* 12.5, 8.7, 0.8 Hz, 1 H), 7.13 (dd, *J* = 7.7, 2.5 Hz, 1 H), 7.08-7.05 (m, 1 H), 3.30-3.25 (brd, 4 H), 3.25-3.18 (brd, 4 H); ¹³C-NMR (125 MHz; DMSO-d6): δ 153.5 (d, *J=* 244.6 Hz), 139.8 (d, *J=* 9.9 Hz), 128.6 (d, *J=* 2.9 Hz), 122.4 (d, *J=* 8.3 Hz), 119.4 (d, *J=* 3.1 Hz), 117.6 (d, *J* = 22.6 Hz), 46.6 (d, *J* = 3.7 Hz), 42.6; ¹⁹F-NMR (471 MHz; DMSO-d6): δ -124.65 (ddd, *J =* 11.4, 7.0, 3.8 Hz, 1 F); HRMS (ESI): m/z calculated for C₁₀H₁₃ClF ([M+H]⁺) 215.0746, found 215.0747

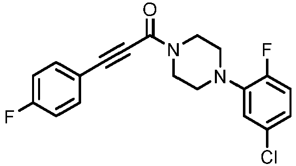

**1-(4-(5-Chloro-2-fluorophenyl)piperazin-1-yl)-3-(4-fluorophenyl)prop-2-yn-1-one.** To a solution of 3-(4-fluorophenyl)propiolic acid (0.200 g, 1.22 mmol) in CH₂Cl₂ (6.09 mL) at 0°C was added 1-(5-chloro-2-fluorophenyl)piperazine hydrochloride (0.367 g, 1.46 mmol), and Et₃N (0.432 mL, 3.05 mmol). T₃P (1.29 mL, 1.83 mmol) was added dropwise and the reaction was stirred at 0 °C for 30 min and allowed to warm to room temperature for 33 h. The reaction was diluted with CH₂Cl₂ (30 mL) and sequentially washed with 1 M HCl (30 mL×2) and saturated aqueous NaHCO₃ (30 mL×2). The resulting organic phase was dried (MgSO₄), filtered and concentrated in vacuo. The crude material was purified by chromatography on SiO₂ (hexanes/EtOAc, 1:1) to give 1-(4-(5-chloro-2-fluorophenyl)piperazin-1-yl)-3-(4-fluorophenyl)prop-2-yn-1-one (0.296 g, 0.821 mmol, 67%) as a light yellow crystal. Mp: 139-140 °C; IR (neat): 2217, 1629, 1601, 1506, 1498, 1432, 1286, 1244, 1227, 1205, 1156, 1039 cm⁻¹; ¹H-NMR (400 MHz; CDCl₃): δ 7.58-7.54 (m, 2 H), 7.11-7.05 (m, 2 H), 7.01-6.89 (m, 3 H), 3.99 (t, *J =* 5.1 Hz, 2 H), 3.86 (t, *J =* 5.1 Hz, 2 H), 3.15 (t, *J =* 5.1 Hz, 2 H), 3.09 (t, *J =* 5.1 Hz, 2 H); ¹³C-NMR (100 MHz; CDCl₃): δ 163.5 (d, *J =* 252.8 Hz), 154.0 (d, *J =* 245.8 Hz), 140.3 (d, *J* = 9.9 Hz), 134.6 (d, *J =* 8.8 Hz), 129.5 (d, *J =* 3.3 Hz), 122.8 (d, *J =* 8.1 Hz), 119.6 (d, *J =* 2.9 Hz), 117.2 (d, *J* = 22.4 Hz), 116.4 (d, *J =* 3.7 Hz), 116.1 (d, *J* = 22.4 Hz), 90.1, 80.7, 50.7 (d, *J =* 3.2 Hz), 50.0 (d, *J* = 3.4 Hz), 46.9, 41.4; ¹⁹F-NMR (376 MHz; CDCl₃): δ -107.24 (tt, *J =* 8.1, 5.5 Hz, 1 F), - 125.19 (ddd, *J =* 11.4, 7.3, 4.6 Hz, 1 F); HRMS (ESI): m/z calculated for C₁₉H₁₆ClON₂F₂ ([M+H]⁺) 361.0914, found 361.0912.

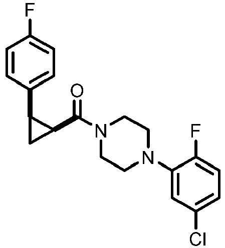

**(4-(5-chloro-2-fluorophenyl)piperazin-1-yl)(2-(4-fluorophenyl)cyclopropyl)methanone.** To a solution of 1-(4-(5-chloro-2-fluorophenyl)piperazin-1-yl)-3-(4-fluorophenyl)prop-2-yn-1-one (0.275 g, 0.763 mmol) in EtOAc (7.63 mL, 0.1 M) was added Lindlar's catalyst (5% Pd on CaCO₃, lead poisoned, 0.0812 g, 5.0 mol%) . The reaction vessel was placed under vacuum and backfilled with H₂ (balloon, 2×) and allowed to stir for 14 h. The reaction mixture was then filtered through celite, washed with EtOAc and concentrated in vacuo. The crude residue was purified by chromatography on SiO₂ (hexanes/EtOAc, 1:1) to afford (Z)-1-(4-(5-chloro-2-fluorophenyl)piperazin-1-yl)-3-(4-fluorophenyl)prop-2-en-1-one (0.107 g, 39%) as a light yellow oil. ¹H-NMR (300 MHz; CDCl₃): δ 7.38 (dd, *J* = 8.6, 5.4 Hz, 2 H), 7.03 (t, *J =* 8.6 Hz, 2 H), 6.95-6.91 (m, 2 H), 6.78 (dd, *J =* 7.7, 1.9 Hz, 1 H), 6.68 (d, *J =* 12.5 Hz, 1 H), 6.05 (d, *J =* 12.5 Hz, 1 H), 3.83 (t, *J =* 5.1 Hz, 2 H), 3.51 (t, *J =* 5.1 Hz, 2 H), 3.02 (t, *J =* 5.1 Hz, 2H), 2.71 (t, *J* = 5.1 Hz, 2 H).

To a solution of anhydrous CrCl₂ (0.169 g, 1.37 mmol) in THF (2.29 mL) that was degassed by sparging with Ar for 30 min followed by the addition of (Z)-1-(4-(5-chloro-2-fluorophenyl)piperazin-1-yl)-3-(4-fluorophenyl)prop-2-en-1-one (83.0 mg, 0.229 mmol) and CH₂ICl (0.132 mL, 1.14 mmol) at RT and under Ar atmosphere. The reaction mixture was stirred at 80 °C. After stirring for 14 h, the mixture was cooled to RT and quenched by the addition of 1.0 M aqueous HCl (10 mL) and extracted with EtOAc (3×20 mL). The combined organic layer was concentrated, then the residue was diluted in EtOAc (3 mL) and the solution was filtered through a plug of basic alumina, and concentrated. The crude material was purified by chromatography on SiO₂ (EtOAc/hexanes, 1:1) to afford (4-(5-chloro-2-fluorophenyl)piperazin-1-yl)(2-(4-fluorophenyl)cyclopropyl)methanone as a white crystal (67 mg, 78 %): Mp: 108-109 °C; IR (neat): 1638, 1606, 1512, 1498, 1436, 1230, 1209, 1032 cm⁻¹; ¹H-NMR (400 MHz; CDCl₃): δ 7.12 (dd, *J =* 8.5, 5.5 Hz, 2 H), 6.97-6.88 (m, 4 H), 6.72 (dd, *J =* 7.3, 1.9 Hz, 1 H), 3.79 (d, *J =* 12.8 Hz, 1 H), 3.69 (t, *J =* 17.1 Hz, 2 H), 3.39 (t, *J =* 9.5 Hz, 1 H), 2.99 (t, *J =* 13.9 Hz, 2 H), 2.52-2.42 (m, 2 H), 2.40-2.34 (m, 1 H), 2.20-2.15 (m, 1 H), 1.84-1.80 (m, 1 H), 1.37-1.32 (m, 1 H). ¹³C-NMR (125 MHz; CDCl₃): δ 167.2, 161.6 (d, *J =* 245.2 Hz), 154.1 (d, *J =* 246.1 Hz), 140.4 (d, *J =* 9.9 Hz), 133.0 (d, *J =* 2.8 Hz), 129.4 (d, *J =* 3.3 Hz), 129.0 (d, *J =* 7.5 Hz), 122.4 (d, *J =* 8.1 Hz), 119.3 (d, *J* = 2.8 Hz), 117.1 (d, *J =* 22.7 Hz), 115.0 *(d, J=* 21.2 Hz), 50.6, 50.1, 45.1, 41.7, 23.7, 23.5, 10.6. HRMS (ESI): m/z calculated for C₂₀H₂₀N₂OClF₂ ([M+H]⁺) 377.1227, found 377.1221.

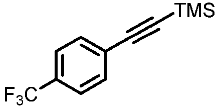

**Trimethyl((4-(trifluoromethyl)phenyl)ethynyl)silane.** A flame-dried flask under Ar was added with Pd(PPh)₂Cl₂ (0.496 g, 0.707 mmol), CuI (0.137 g, 0.707 mmol), and 4-bromobenzotrifluoride (10.0 mL, 70.7 mmol). The flask was purged with Ar before triethylamine (141 mL) and (trimethylsilyl)acetylene (15.0 mL, 106 mmol) were added via syringe and the solution was sparged with Ar for 10 min. The resulting mixture was heated to 80 °C for 22.5 h. After cooling the reaction to RT, the solution was filtered through celite, which was washed with EtOAc until the washes appeared colorless. The filtrate was concentrated in vacuo. The crude product was purified by chromatography on SiO₂ (hexanes) to afford trimethyl((4-(trifluoromethyl)phenyl)ethynyl)silane (17.1 g, >99%) as a light yellow oil: ¹H-NMR (400 MHz; CDCl₃): δ 7.55 (s, 4 H), 0.26 (s, 9 H). The spectra obtained are in agreement with previously reported data (Rahaim et al., J. Org. Chem. 2008, 73:2912-2915).

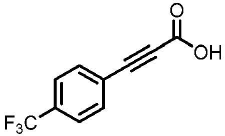

**3-(4-(Trifluoromethyl)phenyl)propionic acid.** A dried and CO₂ (balloon) infused flask, equipped with a magnetic stirrer and a septum, was charged with CsF (13.0 g, 84.7 mmol). A solution of ((4-trifluorophenyl)ethynyl)trimethylsilane (17.1 g, 70.6 mmol) in dry DMSO (141 mL) was added dropwise to a reaction mixture by using equal-dropping funnel and the reaction was stirred under CO₂ at room temperature for 21 h. After that, the reaction mixture was quenched with water (300 mL) at 0 °C. Then the mixture was washed with CH₂Cl₂ (300 mL×2) and the resulting water phase was acidified with 1 M aqueous HCl (300 mL). Then the solution was extracted with Et₂O (500 mL×3), dried (Na₂SO₄), filtered and concentrated in vacuo. The resulting solid was collected during washing it with hexanes. The collecting solid was dried under reduced pressure to yield 3-(4-(trifluoromethyl)phenyl)propionic acid (14.8 g, 98 %) as a light brown solid: ¹H-NMR (400 MHz; DMSO-d₆): δ 14.27-13.92 (brs, 1 H), 7.88-7.83 (m, 4 H). The spectra obtained are in agreement with previously reported data (Cheng et al., Green Chem. 2015, 17:1675-1682).

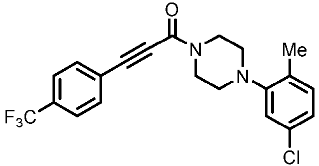

**1-(4-(5-Chloro-2-methylphenyl)piperazin-1-yl)-3-(4-(trifluoromethyl)phenyl)prop-2-yn-1-one.** To a solution of 3-(4-trifluorophenyl)propionic acid(0.20 g, 0.934 mmol) in CH₂Cl₂ (9.34 mL) at 0°C was added 1-(5-chloro-2-methylphenyl)piperazine (0.276 g, 1.12 mmol), and Et₃N (0.530 mL, 3.74 mmol). T₃P (0.991 mL, 1.40 mmol) was added dropwise and the reaction was stirred at 0°C for 30 min and allowed to warm to room temperature for 18.5 h. The reaction was quenched with saturated aqueous NH₄Cl solution (20 mL) and extracted with CH₂Cl₂ (40 mL×3). The combined organic phase was dried (MgSO₄), filtered and concentrated in vacuo. The crude material was purified by chromatography on SiO₂ (EtOAc/hexanes, 1:4) to afford 1-(4-(5-chloro-2-methylphenyl)piperazin-1-yl)-3-(4-(trifluoromethyl)phenyl)prop-2-yn-1-one (0.222 g, 58 %) as a light yellow crystal. Mp: 104-106 °C; IR (neat): 1633, 1593, 1490, 1431, 1322, 1126 cm⁻¹; ¹H-NMR (400 MHz; CDCl₃): δ 7.67 (d, *J =* 8.5 Hz, 2 H), 7.64 (d, *J=* 8.5 Hz, 2 H), 7.12 (dd, *J =* 8.1, 0.4 Hz, 1 H), 7.00 (dd, *J =* 8.1, 2.1 Hz, 1 H), 6.96 (d, *J* = 2.1 Hz, 1 H), 3.97 (t, *J =* 5.0 Hz, 2 H), 3.85 (t, *J =* 5.0 Hz, 2 H), 2.98 (t, *J =* 5.0 Hz, 2 H), 2.90 (t, *J =* 5.0 Hz, 2 H), 2.29 (s, 3 H). ¹³C-NMR (100 MHz; CDCl₃): δ 152.6, 151.6, 132.6, 132.1, 131.7 (q, *J* = 32.9 Hz), 131.0, 125.5 (q, *J =* 3.7 Hz), 124.2, 123.9, 123.6 (q, *J =* 273.7 Hz), 119.8, 89.0, 82.73, 51.9, 51.3, 47.4, 42.0, 17.4. ¹⁹F-NMR (376 MHz; CDCl₃): δ -63.07 (s, 3 F). HRMS (ESI): m/z calculated for C₂₁H₁₉N₂OClF₃ ([M+H]⁺) 407.1133, found 407.1132.

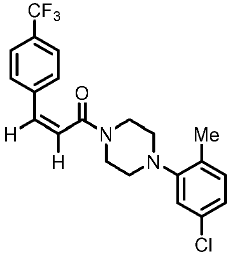

**(*Z*)-1-(4-(5-Chloro-2-methylphenyl)piperazin-1-yl)-3-(4-(trifluoromethyl)phenyl)prop-2-en-1-one.** To a solution of 1-(4-(5-chloro-2-methylphenyl)piperazin-1-yl)-3-(4-(trifluoromethyl)phenyl)prop-2-yn-1-one (0.186 g, 0.457 mmol) in EtOAc (4.60 mL, 0.1 M) was added Lindlar's catalyst (5% Pd on CaCO₃, lead poisoned, 0.0487 g, 5.0 mol%) . The reaction vessel was placed under vacuum and backfilled with H₂ (balloon, 2×) and allowed to stir for 18 h. The reaction mixture was then filtered through celite, washed with EtOAc and concentrated in vacuo. The crude product was purified by chromatography on SiO₂ (hexanes/ EtOAc, 1:1) to afford (Z)-1-(4-(5-chloro-2-methylphenyl)piperazin-1-yl)-3-(4-(trifluoromethyl)phenyl)prop-2-en-1-one (0.184 g, 98%) as a white crystal. Mp: 109-111 °C; IR (neat): 1621, 1594, 1490, 1440, 1325, 1124 cm⁻¹; ¹H-NMR (400 MHz; CDCl₃): δ 7.62 (d, *J =* 8.2 Hz, 2 H), 7.52 (d, *J =* 8.2 Hz, 2 H), 7.07 (d, *J =* 8.1 Hz, 1 H), 6.96 (dd, *J =* 8.1, 2.1 Hz, 1 H), 6.78 (d, *J* = 2.1 Hz, 1 H), 6.74 (d, *J =* 12.6 Hz, 1 H), 6.21 (d, *J* = 12.6 Hz, 1 H), 3.81 (t, *J =* 5.0 Hz, 2 H), 3.49 (t, *J =* 5.0 Hz, 2 H), 2.80 (t, *J =* 5.0 Hz, 2 H), 2.51 (t, *J* = 5.0 Hz, 2 H), 2.21 (s, 3 H). ¹³C-NMR (100 MHz; CDCl₃): δ 166.8, 151.6, 138.9, 132.3, 132.1, 131.9, 131.0, 130.5 (q, *J* = 32.2 Hz), 128.7, 125.6 (q, *J =* 3.9 Hz), 125.3, 123.9 (q, *J =* 270.5 Hz), 123.8, 119.7, 51.5, 51.3, 46.6, 41.6, 17.3. ¹⁹F-NMR (376 MHz; CDCl₃): δ -62.66 (s, 3 F). HRMS (ESI): m/z calculated for C₂₁H₁₉N₂OClF₃ ([M+H]⁺) 407.1133, found 407.1132.

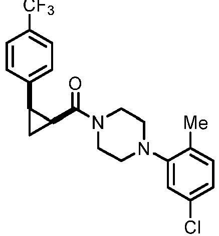

**(4-(5-Chloro-2-methylphenyl)piperazin-1-yl)(2-(4-(trifluoromethyl)phenyl)cyclopropyl)methanone.** To a solution of anhydrous CrCl₂ (0.273 g, 2.16 mmol) in THF (162 mL) that was degassed by sparging with Ar for 30 min followed by the addition of (*Z*)-1-(4-(5-chloro-2-methylphenyl)piperazin-1-yl)-3-(4-trifluorophenyl)prop-2-en-1-one (0.147 g, 0.360 mmol) and CH₂ICl (0.134 mL, 1.80 mmol) at room temperature and under Ar atmosphere. The reaction mixture was stirred at 80 °C. After stirring for 17.5 h, the reactions were was cooled to room temperature, combined, quenched by the addition of 1.0 M aqueous HCl (10 mL) and extracted with EtOAc (3×20 mL). The organic layers were combined, dried (Na₂SO₄), filtered through a plug of basic alumina and concentrated in vacuo. The crude material was purified by chromatography on SiO₂ (EtOAc/hexanes, 1:1) to afford (4-(5-chloro-2-methylphenyl)piperazin-1-yl)(2-(4-(trifluoromethyl)phenyl)cyclopropyl)methanone (127 mg) as a white solid. Then the product was recrystallized with cyclohexane to afford the target product as a white crystal (89 mg, 59 %): Mp: 109-110 °C; IR (neat): 1638, 1619, 1593, 1437, 1324, 1116, 1069 cm⁻¹; ¹H-NMR (400 MHz; CDCl₃): δ 7.54 (d, *J* = 8.1 Hz, 2 H), 7.28 (d, *J =* 8.1 Hz, 2 H), 7.06 (d, *J =* 8.1 Hz, 1 H), 6.95 (dd, *J =* 8.1, 2.1 Hz, 1 H), 6.67 (d, *J* = 2.1 Hz, 1 H), 3.82-3.78 (m, 1 H), 3.70-3.58 (m, 2 H), 3.37-3.31 (m, 1 H), 2.79-2.69 (m, 2 H), 2.51 (q, *J =* 8.0 Hz, 1 H), 2.30-2.23 (m, 2 H), 2.19 (s, 3 H), 2.13-2.08 (m, 1 H), 1.91 (q, *J =* 6.2 Hz, 1 H), 1.45-1.40 (m, 1 H). ¹³C-NMR (100 MHz; CDCl₃): δ 166.7, 151.7, 141.9, 141.9, 131.9, 131.8, 130.9, 128.7 (q, *J* = 32.5 Hz), 127.9, 125.0 (q, *J* = 3.8 Hz), 124.2 (q, *J =* 270.0 Hz), 123.7, 119.6, 51.8, 51.5, 45.5, 42.2, 24.5, 24.0, 17.3, 11.1. ¹⁹F-NMR (376 MHz; CDCl₃): δ -62.31 (s, 1 F). HRMS (ESI): m/z calculated for C₂₂H₂₃N₂OClF₃ ([M+H]⁺) 423.1446, found 423.1443.

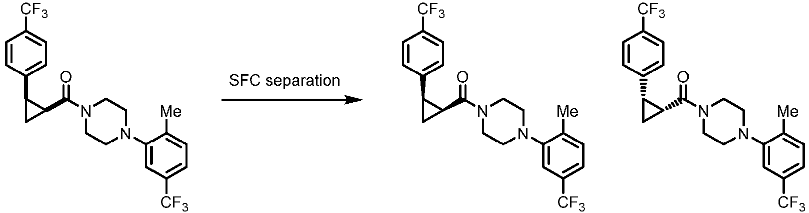

Racemic (2-(4-fluorophenyl)cyclopropyl)(4-(2-methyl-5-(trifluoromethyl)phenyl)piperazin-1-yl)methanone was separated on a SFC Chiralpak-IC semiprep (250× 10 mm) column (30% MeOH, 7 mL/min, 220 nM, P=100) to afford (4-(2-methyl-5-(trifluoromethyl)phenyl)piperazin-1-yl)((*1S*,*2R*)-2-(4-(trifluoromethyl)-phenyl)cyclopropyl)methanone (retention time; 5.12 min) as a colorless oil (>99.9% purity by ELSD): [α]¹⁸_{D} -150.5 (*c* 0.51, MeOH); HRMS (ESI): m/z calculated for C₂₂H₂₃N₂OClF₃ ([M+H]⁺) 423.1446, found 423.1449. The enantiomeric excess was >99.9% ee (SFC Chiralpak-IC semiprep; 30% MeOH, 220 nM, 7 mL/min; retention time: 5.12 min). ¹H-NMR (400 MHz; CDCl₃): δ 7.54 (d, *J =* 8.1 Hz, 2 H), 7.28 (d, *J* = 8.1 Hz, 2 H), 7.06 (d, *J =* 8.1 Hz, 1 H), 6.95 (dd, *J =* 8.1, 2.1 Hz, 1 H), 6.67 (d, *J =* 2.1 Hz, 1 H), 3.82-3.78 (m, 1 H), 3.70-3.58 (m, 2 H), 3.37-3.31 (m, 1 H), 2.79-2.69 (m, 2 H), 2.51 (q, *J =* 8.0 Hz, 1 H), 2.30-2.23 (m, 2 H), 2.19 (s, 3 H), 2.13-2.08 (m, 1 H), 1.91 (q, *J* = 6.2 Hz, 1 H), 1.45-1.40 (m, 1 H).

(4-(2-Methyl-5-(trifluoromethyl)phenyl)piperazin-1-yl)((*1R*,*2S*)-2-(4-(trifluoromethyl)phenyl)-cyclopropyl)methanone (retention time; 6.43 min) as a colorless oil (>99.9% purity by ELSD): [α]¹⁸_{D} +148.4 (c 0.51, MeOH); HRMS (ESI): m/z calculated for C₂₂H₂₃N₂OClF₃ ([M+H]⁺) 423.1446, found 423.1444. The enantiomeric excess was >99.9% ee (SFC Chiralpak-IC semiprep; 30% MeOH, 220 nM, 7 mL/min; retention time: 6.43 min). ¹H-NMR (400 MHz; CDCl₃): δ 7.54 (d, *J =* 8.1 Hz, 2 H), 7.28 (d, *J =* 8.1 Hz, 2 H), 7.06 (d, *J =* 8.1 Hz, 1 H), 6.95 (dd, *J =* 8.1, 2.1 Hz, 1 H), 6.67 (d, *J =* 2.1 Hz, 1 H), 3.82-3.78 (m, 1 H), 3.70-3.58 (m, 2 H), 3.37-3.31 (m, 1 H), 2.79-2.69 (m, 2 H), 2.51 (q, *J* = 8.0 Hz, 1 H), 2.30-2.23 (m, 2 H), 2.19 (s, 3 H), 2.13-2.08 (m, 1 H), 1.91 (q, *J* = 6.2 Hz, 1 H), 1.45-1.40 (m, 1 H).

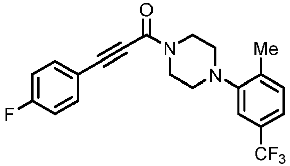

**3-(4-Fluorophenyl)-1-(4-(2-methyl-5-(trifluoromethyl)phenyl)piperazin-1-yl)prop-2-yn-1-one.** To a solution of 3-(4-fluorophenyl)propionic acid(0.100 g, 0.609 mmol) in CH₂Cl₂ (6.09 mL) at 0°C was added 1-(5-trifluoromethyl-2-methylphenyl)piperazine (0.205 g, 0.731 mmol), and Et₃N (0.259 mL, 1.83 mmol). T₃P (0.646 mL, 0.914 mmol) was added dropwise and the reaction was stirred at 0°C for 30 min and allowed to warm to room temperature for 20.5 h. The reaction was diluted with CH₂Cl₂ (40 mL) and washed with 1 M HCl (50 mL). The aqueous phase was extracted with CH₂Cl₂ (30 ×2) and combined organic phase was dried (MgSO₄), filtered and concentrated in vacuo. The crude material was purified by chromatography on SiO₂ (EtOAc/hexanes, 1:4) to afford 3-(4-fluorophenyl)-1-(4-(2-methyl-5-(trifluoromethyl)phenyl)piperazin-1-yl)prop-2-yn-1-one (219 mg, 92%) as a white crystal: Mp: 176-177 °C; IR (neat): 2220, 1630, 1600, 1507, 1419, 1310, 1226, 1120 cm⁻¹; ¹H-NMR (400 MHz; CDCl₃): δ 7.56 (dd, *J* = 8.8, 5.3 Hz, 2 H), 7.31 (d, *J =* 8.1 Hz, 1 H), 7.28 (d, *J =* 8.1 Hz, 1 H), 7.22 (s, 1 H), 7.08 (t, *J =* 8.8 Hz, 2 H), 4.00 (t, *J =* 5.0 Hz, 2 H), 3.87 (t, *J* = 5.0 Hz, 2 H), 3.03 (t, *J =* 4.9 Hz, 2 H), 2.94 (t, *J* = 5.0 Hz, 2 H), 2.40 (s, 3 H). ¹³C-NMR (100 MHz; CDCl₃): δ 163.6 (d, *J =* 252.4 Hz), 153.1, 151.0, 136.8, 134.6, 134.5 , 131.6, 129.1 (q, *J* = 32.2 Hz), 124.1 (q, *J =* 270.4 Hz), 120.5 (q, *J =* 3.8 Hz, ), 116.4 (d, *J =* 3.7 Hz), 116.1 (q, *J* = 3.7 Hz), 116.0 (d, *J* = 22.0 Hz), 90.0, 80.8, 80.8, 77.3, 77.0, 76.7, 51.9, 51.4, 47.4, 41.9, 18.0. ¹⁹F-NMR (376 MHz; CDCl₃): δ -62.29 (s, 3 F), -107.28 - -107.35 (m, 1 F). HRMS (ESI): m/z calculated for C₂₁H₁₉N₂OF₄ ([M+H]⁺) 391.1428, found 391.1402.

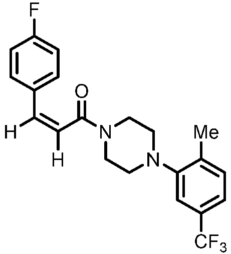

**(*Z*)-3-(4-Fluorophenyl)-1-(4-(2-methyl-5-(trifluoromethyl)phenyl)piperazin-1-yl)prop-2-en-1-one.** To a solution of 3-(4-fluorophenyl)-1-(4-(2-methyl-5-(trifluoromethyl)phenyl)piperazin-1-yl)prop-2-yn-1-one (0.193 g, 0.494 mmol) in EtOAc (4.90 mL, 0.1 M) was added Lindlar's catalyst (5% Pd on CaCO₃, lead poisoned, 0.0526 g, 5.0 mol%) . The reaction vessel was placed under vacuum and backfilled with H₂ (balloon, 2×) and allowed to stir for 18 h. The reaction mixture was then filtered through celite, washed with EtOAc and concentrated in vacuo. The crude residue was purified by chromatography on SiO₂ (hexanes/ EtOAc, 1:1) to afford (*Z*)-3-(4-fluorophenyl)-1-(4-(2-methyl-5-(trifluoromethyl)phenyl)piperazin-1-yl)prop-2-en-1-one (0.107 g, 55%) as a light yellow oil: Mp: 176-177 °C; IR (neat): 2917, 1640, 1617, 1602, 1508, 1439, 1417, 1338, 1309, 1225, 1162, 1119 cm⁻¹; ¹H-NMR (400 MHz; CDCl₃): δ 7.42 (dd, *J =* 8.7, 5.3 Hz, 2 H), 7.30-7.25 (m, 2 H), 7.10-7.06 (m, 3 H), 6.71 (d, *J =* 12.5 Hz, 1 H), 6.09 (d, *J =* 12.5 Hz, 1 H), 3.85 (t, *J =* 5.0 Hz, 2 H), 3.54 (t, *J =* 5.0 Hz, 2 H), 2.87 (t, *J =* 5.0 Hz, 2 H), 2.58 (t, *J =* 5.0 Hz, 2 H), 2.34 (s, 3 H); ¹³C-NMR (100 MHz; CDCl₃): δ 167.3, 162.7 (d, *J* = 249.4 Hz), 151.0, 136.8, 132.7, 131.6 (d, *J =* 3.6 Hz), 131.5, 130.3 (d, *J* = 8.1 Hz), 129.0 (q, *J* = 32.2 Hz), 124.1 (q, *J* = 270.4 Hz), 122.7, 122.7, 120.4 (q, *J* = 3.9 Hz), 115.9 (q, *J =* 3.7 Hz), 115.7, 115.6, 51.4, 51.3, 46.6, 41.5, 17.9: ¹⁹F-NMR (376 MHz; CDCl₃): δ -62.36 (s, 3 F), -112.02 - -112.09 (m, 1 F): HRMS (ESI): m/z calculated for C₂₁H₂₁N₂OF₄ ([M+H]⁺) 393.1585, found 393.1585.

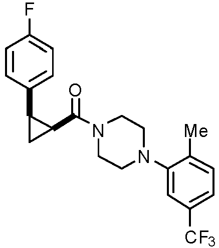

**(2-(4-Fluorophenyl)cyclopropyl)(4-(2-methyl-5-(trifluoromethyl)phenyl)piperazin-1-yl)methanone.** To a solution of anhydrous CrCl₂ (0.172 g, 1.36 mmol) in THF (2.27 mL) that was degassed by sparging with Ar for 10 min followed by the addition of (*Z*)-3-(4-fluorophenyl)-1-(4-(2-methyl-5-(trifluoromethyl)phenyl)-piperazin-1-yl)prop-2-en-1-one (0.0889 g, 0.227 mmol) and CH₂ICl (0.0842 mL, 1.13 mmol) at room temperature and under Ar atmosphere. The reaction mixture was stirred at 80 °C. After stirring for 13 h, the reactions were was cooled to room temperature, combined, quenched by the addition of 1.0 M aqueous HCl (10 mL) and extracted with EtOAc (30 mL). The organic layer was sequentially washed with 1 M aqueous HCl (30 mL) and saturated aqueous sodium thiosulfate (30 mL). Then the organic phase was dried (Na₂SO₄), filtered and concentrated in vacuo. The residue was diluted in minimum amount of 3:2 mixed solvent of EtOAc and hexanes, and the solution was filtered through a plug of basic alumina, and concentrated in vacuo. The crude material was purified by chromatography on SiO₂ (EtOAc/hexanes, 3:2) to afford (2-(4-fluorophenyl)cyclopropyl)(4-(2-methyl-5-(trifluoromethyl)phenyl)piperazin-1-yl)methanone (0.0878 g, 95%) as a brown oil: IR (neat): 2918, 1637, 1513, 1417, 1337, 1307, 1223, 1161, 1118 cm⁻¹; ¹H-NMR (500 MHz; CDCl₃): δ 7.27 (t, *J =* 1.6 Hz, 2 H), 7.17 (dd, *J =* 8.7, 5.3 Hz, 2 H), 7.02-6.98 (m, 3 H), 3.90-3.85 (m, 1 H), 3.78-3.74 (m, 1 H), 3.70-3.65 (m, 1 H), 3.40-3.35 (m, 1 H), 2.86-2.77 (m, 2 H), 2.51-2.46 (m, 1 H), 2.33-2.20 (m, 6 H), 1.86 (q, *J =* 6.2 Hz, 1 H), 1.38 (td, *J =* 8.5, 5.5 Hz, 1 H): ¹³C-NMR (125 MHz; CDCl₃): δ 167.2, 161.6 (d, *J =* 245.2 Hz), 151.1, 136.8, 133.0 (d, *J =* 2.7 Hz), 131.4, 129.0 (d, *J* = 7.9 Hz), 129.0 (q, *J =* 25.5 Hz), 124.1 (q, *J =* 272.0 Hz), 120.3 (q, *J =* 3.7 Hz), 115.9 (q, *J =* 3.7 Hz), 115.0, 114.8, 51.7, 51.6, 45.5, 42.2, 23.9, 23.4, 17.8, 10.6. ¹⁹F-NMR (471 MHz; CDCl₃): δ -62.36 (s, 1 F), -116.30--116.41 (m, 1 F); HRMS (ESI): m/z calculated for C₂₂H₂₃N₂OF₄ ([M+H]⁺) 407.1741, found 407.1732.

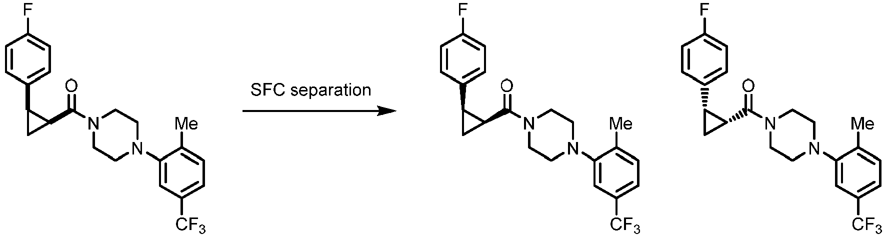

Racemic (2-(4-fluorophenyl)cyclopropyl)(4-(2-methyl-5-(trifluoromethyl)phenyl)piperazin-1-yl)methanone was separated on a SFC Chiralpak-IC semiprep (250× 10 mm) column (30% MeOH, 7 mL/min, 220 nM, P=100) to afford ((*1S*,*2R*)-2-(4-fluorophenyl)cyclopropyl)(4-(2-methyl-5-(trifluoromethyl)phenyl)piperazin-1-yl)methanone (retention time; 4.40 min) as a colorless oil (>99.9% purity by ELSD): [α]¹⁸_{D} -145.6 (c 0.86, MeOH); HRMS (ESI): m/z calculated for C₂₂H₂₃N₂OF₄ ([M+H]⁺) 407.1741, found 407.1741. The enantiomeric excess was >99.9% ee (SFC Chiralpak-IC semiprep; 30% MeOH, 220 nM, 7 mL/min; retention time: 4.40 min). ¹H-NMR (500 MHz; CDCl₃): δ 7.27 (t, *J =* 1.6 Hz, 2 H), 7.17 (dd, *J* = 8.7, 5.3 Hz, 2 H), 7.02-6.98 (m, 3 H), 3.90-3.85 (m, 1 H), 3.78-3.74 (m, 1 H), 3.70-3.65 (m, 1 H), 3.40-3.35 (m, 1 H), 2.86-2.77 (m, 2 H), 2.51-2.46 (m, 1 H), 2.33-2.20 (m, 6 H), 1.86 (q, *J =* 6.2 Hz, 1 H), 1.38 (td, *J =* 8.5, 5.5 Hz, 1 H).

((*1R*,*2S*)-2-(4-Fluorophenyl)cyclopropyl)(4-(2-methyl-5-(trifluoromethyl)phenyl)piperazin-1-yl)methanone (retention time; 4.74 min) as a colorless oil (>99.9% purity by ELSD): [α]¹⁸_{D} +139.8 (c 1.14, MeOH); HRMS (ESI): m/z calculated for C₂₂H₂₃N₂OF₄ ([M+H]⁺) 407.1741, found 407.1741. The enantiomeric excess was >99.9% ee (SFC Chiralpak-IC semiprep; 30% MeOH, 220 nM, 7 mL/min; retention time: 4.74 min). ¹H-NMR (500 MHz; CDCl₃): δ 7.27 (t, *J =* 1.6 Hz, 2 H), 7.17 (dd, *J =* 8.7, 5.3 Hz, 2 H), 7.02-6.98 (m, 3 H), 3.90-3.85 (m, 1 H), 3.78-3.74 (m, 1 H), 3.70-3.65 (m, 1 H), 3.40-3.35 (m, 1 H), 2.86-2.77 (m, 2 H), 2.51-2.46 (m, 1 H), 2.33-2.20 (m, 6 H), 1.86 (q, *J =* 6.2 Hz, 1 H), 1.38 (td, *J =* 8.5, 5.5 Hz, 1 H).

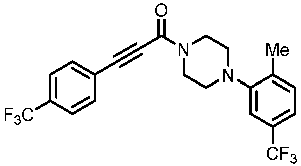

**1-(4-(2-Methyl-5-(trifluoromethyl)phenyl)piperazin-1-yl)-3-(4-(trifluoromethyl)phenyl)prop-2-yn-1-one.** To a solution of 3-(4-trifluorophenyl)propionic acid(0.100 g, 0.467 mmol) in CH₂Cl₂ (6.09 mL) at 0°C was added 1-(5-trifluoromethyl-2-methylphenyl)piperazine (0.157 g, 0.560 mmol), and Et₃N (0.199 mL, 1.40 mmol). T₃P (0.495 mL, 0.700 mmol) was added dropwise and the reaction was stirred at 0°C for 30 min and allowed to warm to room temperature for 20.5 h. The reaction was diluted with CH₂Cl₂ (40 mL) and washed with 1 M HCl (50 mL). The aqueous phase was extracted with CH₂Cl₂ (30 mL×2) and combined organic phase was dried (MgSO₄), filtered and concentrated in vacuo. The crude material was purified by chromatography on SiO₂ (EtOAc/hexanes, 1:4) to afford 1-(4-(2-methyl-5-(trifluoromethyl)-phenyl)piperazin-1-yl)-3-(4-(trifluoromethyl)phenyl)prop-2-yn-1-one (188 mg, 91%) as a white crystal: Mp: 159-160 °C; IR (neat): 2224, 1631, 1458, 1431, 1418, 1339, 1321, 1310, 1279, 1165, 1119 cm⁻¹; ¹H-NMR (400 MHz; CDCl₃): δ 7.68 (d, *J =* 8.5 Hz, 2 H), 7.65 (d, *J =* 8.5 Hz, 2 H), 7.32 (d, *J =* 8.3 Hz, 1 H), 7.28 (dd, *J =* 8.3, 1.2 Hz, 1H), 7.22 (s, 1 H), 4.00 (t, *J =* 5.0 Hz, 2 H), 3.88 (t, *J =* 5.0 Hz, 2 H), 3.04 (t, *J =* 5.0 Hz, 2 H), 2.95 (t, *J =* 5.0 Hz, 2 H), 2.40 (s, 3 H); ¹³C-NMR (100 MHz; CDCl₃): δ 152.6, 150.9, 136.8, 132.6, 131.8 (q, *J =* 32.4 Hz), 131.6, 129.2 (q, *J* = 32.2 Hz), 125.5 (q, *J* = 3.7 Hz), 124.1 (q, *J* = 270.5 Hz), 123.6 *(q, J =* 271.1 Hz), 120.6 (q, *J* = 3.8 Hz), 116.1(q, *J =* 3.7 Hz), 89.1, 82.7, 77.4 77.0, 76.7, 51.9, 51.4, 47.5, 42.0, 18.0; ¹⁹F-NMR (376 MHz; CDCl₃): δ -62.29 (s, 3 F), -63.09 (s, 3 F); HRMS (ESI): m/z calculated for C₂₂H₁₉N₂OF₆ ([M+H]⁺) 441.1396, found 441.1385.

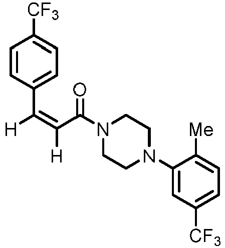

**(*Z*)-1-(4-(2-Methyl-5-(trifluoromethyl)phenyl)piperazin-1-yl)-3-(4-(trifluoromethyl)phenyl)prop-2-en-1-one.** To a solution of 1-(4-(2-methyl-5-(trifluoromethyl)phenyl)piperazin-1-yl)-3-(4-(trifluoromethyl)-phenyl)prop-2-yn-1-one (0.164 g, 0.372 mmol) in EtOAc (3.72 mL, 0.1 M) was added Lindlar's catalyst (5% Pd on CaCO₃, lead poisoned, 0.0396 g, 5.0 mol%) . The reaction vessel was placed under vacuum and backfilled with H₂ (balloon, 2×) and allowed to stir for 18 h. The reaction mixture was then filtered through celite, washed with EtOAc and concentrated in vacuo. The crude residue was purified by chromatography on SiO₂ (hexanes/EtOAc, 1:1) to afford (*Z*)-1-(4-(2-methyl-5-(trifluoromethyl)phenyl)piperazin-1-yl)-3-(4-(trifluoromethyl)phenyl)prop-2-en-1-one (0.0998 g, 61%) as a light yellow oil: IR (neat): 2978, 1622, 1442, 1417, 1327, 1118 cm⁻¹; ¹H-NMR (400 MHz; CDCl₃): δ 7.40 (dd, *J =* 8.7, 5.4 Hz, 2 H), 7.28-7.23 (m, 2 H), 7.05 (t, *J =* 8.7 Hz, 3 H), 6.68 (d, *J =* 12.5 Hz, 1 H), 6.07 (d, *J =* 12.5 Hz, 1 H), 3.83 (t, *J =* 4.9 Hz, 2 H), 3.52 (t, *J =* 4.9 Hz, 2 H), 2.85 (d, *J =* 4.9 Hz, 2 H), 2.57 (d, *J* = 4.9 Hz, 2 H), 2.32 (s, 3 H); ¹³C-NMR (125 MHz; CDCl₃): δ 166.8, 150.9, 138.8, 136.8, 132.4, 131.5, 130.5 (q, *J =* 32.7 Hz), 129.1 (q, *J =* 29.3 Hz), 128.7, 125.6 (q, *J =* 3.7 Hz), 125.3, 124.1 (q, *J =* 216.1 Hz), 123.9 (q, *J =* 216.4 Hz), 120.5 (q, *J =* 3.9 Hz), 115.9 (q, *J =* 3.6 Hz), 77.3, 77.0, 76.8, 51.4, 51.3, 46.6, 41.6, 17.9; ¹⁹F-NMR (376 MHz; CDCl₃): δ -62.41 (s, 3 F), -62.79 (s, 3 F); HRMS (ESI): m/z calculated for C₂₂H₂₁N₂OF₆ ([M+H]⁺) 443.1553, found 443.1551.

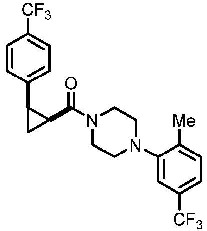

**(4-(2-Methyl-5-(trifluoromethyl)phenyl)piperazin-1-yl)(2-(4-(trifluoromethyl)phenyl)cyclopropyl)-methanone.** To a solution of anhydrous CrCl₂ (0.134 g, 1.06 mmol) in THF (1.80 mL) that was degassed by sparging with Ar for 10 min followed by the addition of (*Z*)-1-(4-(2-methyl-5-(trifluoromethyl)phenyl)-piperazin-1-yl)-3-(4-(trifluoromethyl)phenyl)prop-2-en-1-one (0.0782 g, 0.177 mmol) and CH₂ICl (0.0657 mL, 0.884 mmol) at room temperature and under Ar atmosphere. The reaction mixture was stirred at 80 °C. After stirring for 16.5 h, the reactions were was cooled to room temperature, combined, quenched by the addition of 1.0 M aqueous HCl (10 mL) and extracted with EtOAc (30 mL). The organic layer was sequentially washed with 1 M aqueous HCl (30 mL×2) and saturated aqueous sodium thiosulfate (30 mL). Then the organic phase was dried (Na₂SO₄), filtered and concentrated in vacuo. The residue was diluted in EtOAc, and the solution was filtered through a plug of basic alumina, and concentrated in vacuo. The crude material was purified by chromatography on SiO₂ (EtOAc/hexanes, 3:2) to afford (4-(2-methyl-5-(trifluoromethyl)phenyl)piperazin-1-yl)(2-(4-(trifluoromethyl)phenyl)cyclopropyl)methanone (0.0878 g, 61 %) as a white solid: Mp: 99-101 °C; IR (neat): 1639, 1619, 1438, 1418, 1325, 1308, 1162, 1114 cm⁻¹; ¹H-NMR (400 MHz; CDCl₃): δ 7.53 (d, *J =* 8.1 Hz, 2 H), 7.29 (d, *J =* 8.1 Hz, 2 H), 7.24 (d, *J =* 1.6 Hz, 2 H), 6.94 (s, 1 H), 3.88-3.82 (m, 1 H), 3.73-3.60 (m, 2 H), 3.38-3.31 (m, 1 H), 2.84-2.73 (m, 2 H), 2.52 (q, *J =* 7.9 Hz, 1 H), 2.31-2.25 (m, 5 H), 2.16-2.10 (m, 1 H), 1.92 (q, *J =* 6.3 Hz, 1 H), 1.43 (td, *J* = 8.4, 5.6 Hz, 1 H); ¹³C-NMR (100 MHz; CDCl₃): δ 166.7, 151.0, 141.9, 141.9, 136.8, 131.4, 129.1 (q, *J =* 31.9 Hz), 128.8 (q, *J* = 32.1 Hz), 127.9, 125.0 (q, *J =* 3.7 Hz), 124.2 (q, *J =* 270.3 Hz), 124.1 (q, *J =* 270.4 Hz), 120.4 (q, *J =* 3.9 Hz), 115.8 (q, *J =* 3.7 Hz), 77.4, 77.1, 76.7, 51.8, 51.6, 45.5, 42.2, 24.6, 24.0, 17.8, 11.1; ¹⁹F-NMR (376 MHz; CDCl₃): δ -62.46 (s, 3 F), -62.52 (s, 3 F); HRMS (ESI): m/z calculated for C₂₃H₂₃N₂OF₆ ([M+H]⁺) 457.1709, found 457.1709.

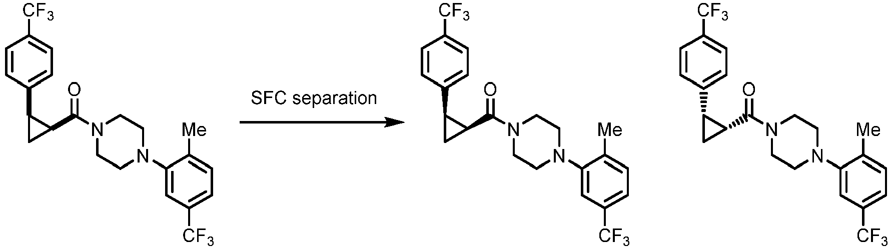

Racemic (4-(2-methyl-5-(trifluoromethyl)phenyl)piperazin-1-yl)(2-(4-(trifluoromethyl)phenyl)cyclopropyl)-methanone was separated on a SFC Chiralpak-IC semiprep (250×10 mm) column (30% MeOH, 7 mL/min, 220 nM, P=100) to afford (4-(2-methyl-5-(trifluoromethyl)phenyl)piperazin-1-yl)((*1S*,*2R*)-2-(4-(trifluoromethyl)phenyl)cyclopropyl)methanone (retention time; 3.36 min) as a colorless oil (>99.9% purity by ELSD): [α]¹⁸_{D} -139.3 (*c* 0.77, MeOH); HRMS (ESI): m/z calculated for C₂₃H₂₃N₂OF₆ ([M+H]⁺) 457.1709, found 457.1709. The enantiomeric excess was >99.9% ee (SFC Chiralpak-IC semiprep; 30% MeOH, 220 nM, 7 mL/min; retention time: 3.36 min). ¹H-NMR (400 MHz; CDCl₃): δ 7.53 (d, *J =* 8.1 Hz, 2 H), 7.29 (d, *J =* 8.1 Hz, 2 H), 7.24 (d, *J* = 1.6 Hz, 2 H), 6.94 (s, 1 H), 3.88-3.82 (m, 1 H), 3.73-3.60 (m, 2 H), 3.38-3.31 (m, 1 H), 2.84-2.73 (m, 2 H), 2.52 (q, *J =* 7.9 Hz, 1 H), 2.31-2.25 (m, 5 H), 2.16-2.10 (m, 1 H), 1.92 (q, *J =* 6.3 Hz, 1 H), 1.43 (td, *J =* 8.4, 5.6 Hz, 1H).

(4-(2-Methyl-5-(trifluoromethyl)phenyl)piperazin-1-yl)((*1R*,*2S*)-2-(4-(trifluoromethyl)phenyl)cyclopropyl)-methanone (retention time; 3.74 min) as a colorless oil (>99.9% purity by ELSD): [α]¹⁸_{D} +135.7 (c 0.91, MeOH); HRMS (ESI): m/z calculated for C₂₃H₂₃N₂OF₆ ([M+H]⁺) 457.1709, found 457.1704. The enantiomeric excess was >99.9% ee (SFC Chiralpak-IC semiprep; 30% MeOH, 220 nM, 7 mL/min; retention time: 3.74 min). ¹H-NMR (400 MHz; CDCl₃): δ 7.53 (d, *J =* 8.1 Hz, 2 H), 7.29 (d, *J =* 8.1 Hz, 2 H), 7.24 (d, *J =* 1.6 Hz, 2 H), 6.94 (s, 1 H), 3.88-3.82 (m, 1 H), 3.73-3.60 (m, 2 H), 3.38-3.31 (m, 1 H), 2.84-2.73 (m, 2 H), 2.52 (q, *J =* 7.9 Hz, 1 H), 2.31-2.25 (m, 5 H), 2.16-2.10 (m, 1 H), 1.92 (q, *J =* 6.3 Hz, 1 H), 1.43 (td, *J =* 8.4, 5.6 Hz, 1 H).

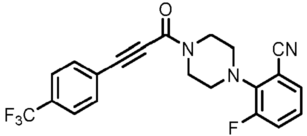

**3-Fluoro-2-(4-(3-(4-(trifluoromethyl)phenyl)propioloyl)piperazin-1-yl)benzonitrile.** Under N₂ atmosphere, CuBr (0.717 g, 4.90 mmol), 1,1'-bi-2-naphthol (1.05 g, 3.67 mmol) and DMF (12.3 mL) was added to the flame-dried flask. The mixture was stirred for 10 minutes before the addition of 1-Boc-piperazine (6.84 g, 36.7 mmol), K₃PO₄ (10.7 g, 49.0 mmol) and 2-bromo-3-fluorobenzonitrile (5.00 g, 24.5 mmol). The reaction mixture was stirred at 120°C for 22.5 h. After cooling to room temperature, the mixture was diluted with dichloromethane and filtered through Celite pad. The filtrate was concentrated under reduced pressure. The residue was diluted with Et₂O (100 mL) and the solution was sequentially washed with saturated aqueous NH₄Cl (100 mL×2) and brine (100 mL×2), dried (Na₂SO₄), filtered and concentrated in vacuo. The crude product was filtered through a short column of SiO₂ (EtOAc/hexanes, 1:9) to remove the remained piperazine. And the obtained mixture (1.10 g) was used without further purification. To a solution of the above mixture (1.10 g) in 1,4-dioxane (8.40 mL), HCl (4 M in 1,4-dioxane, 3.60 mL) was added at 0 °C. The mixture was stirred at RT for 12 h. The thick suspension was diluted with hexanes (50 mL) and the resulting solid was collected by filtration, washed with hexanes and Et₂O and dried to give a yellow solid (0.3827 g). This product was used without further purification.

To a solution of 3-(4-(trifluoromethyl)phenyl)propiolic acid (0.200 g, 0.934 mmol) in dry CH₂Cl₂ (12.2 mL) at 0°C was added the above solid (0.271 g), and Et₃N (0.398 mL, 2.80 mmol). T₃P (0.991 mL, 1.40 mmol) was added dropwise and the reaction was stirred at 0°C for 30 min and allowed to warm to RT for 6.5 h. The reaction was diluted with CH₂Cl₂ (40 mL) and washed with 1 M HCl (50 mL). The aqueous phase was extracted with CH₂Cl₂ (30 mL×2) and combined organic phase was dried (MgSO₄), filtered, and concentrated in vacuo. The crude material was purified by chromatography on SiO₂ (EtOAc/hexanes, 1:2) to afford 3-fluoro-2-(4-(3-(4-(trifluoromethyl)phenyl)propioloyl)piperazin-1-yl)benzonitrile (260 mg, 3% over 3 steps) as a light yellow solid. Mp: 127-129 °C; IR (neat): 2224, 1629, 1464, 1432, 1321, 1281, 1127, 1067, 1032 cm⁻¹; ¹H-NMR (500 MHz; CDCl₃): δ 7.68 (d, J = 8.4 Hz, 2 H), 7.65 (d, J = 8.5 Hz, 2 H), 7.41 (d, *J =* 7.7 Hz, 1 H), 7.29 (dd, *J =* 8.4, 1.4 Hz, 1 H), 7.28-7.26 (dd, *J =* 8.5, 1.4 Hz, 1 H), 4.01 (t, *J =* 4.9 Hz, 2 H), 3.89 (t, *J =* 5.0 Hz, 2 H), 3.39 (t, *J =* 4.0 Hz, 2 H), 3.32 (t, *J =* 4.0 Hz, 2 H). ¹³C-NMR (125 MHz; CDCl₃): δ 158.4 (d, *J =* 250.3 Hz), 152.7, 141.7 (d, *J =* 12.7 Hz), 132.6, 131.8 (d, *J=* 32.8 Hz), 129.6 (d, *J* = 3.4 Hz), 125.5 (q, *J =* 3.8 Hz), 125.4 (d, *J =* 8.4 Hz), 124.2 (d, *J =* 1.4 Hz), 123.6 (q, *J =* 108.4 Hz), 121.8, 121.6, 117.0 (d, *J =* 4.5 Hz), 111.7 (d, *J =* 6.3 Hz), 51.9 (d, *J =* 4.4 Hz), 51.2 (d, *J=* 4.6 Hz), 47.9, 42.4; ¹⁹F-NMR (470 MHz; CDCl₃): δ -63.08 (s, 3 F), -119.85 (d, *J =* 11.3 Hz, 1 F); HRMS (ESI): m/z calculated for C₂₁H₁₆F₄N₃O ([M+H]⁺) 402.1224, found 402.1223.

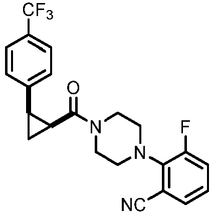

**3-Fluoro-2-(4-(2-(4-(trifluoromethyl)phenyl)cyclopropane-1-carbonyl)piperazin-1-yl)benzonitrile.** To a solution of 3-fluoro-2-(4-(3-(4-(trifluoromethyl)phenyl)propioloyl)piperazin-1-yl)benzonitrile (0.240 g, 0.598 mmol) in EtOAc (5.98 mL, 0.1 M) was added Pd/BaSO₄ (5% Pd on CaCO₃, lead poisoned, 0.0636 g, 5.0 mol%) . The reaction vessel was placed under vacuum and backfilled with H2 (balloon, 2x) and allowed to stir for 21 h. Then the reaction mixture was then filtered through celite, washed with EtOAc and concentrated in vacuo. The crude residue was purified by chromatography on SiO₂ (hexanes/EtOAc, 1:1) to afford 3-fluoro-2-(4-(2-(4-(trifluoromethyl)phenyl)cyclopropane-1-carbonyl)piperazin-1-yl)benzonitrile (0.213 g) as a light yellow oil. This product was used without further purification.

To a solution of anhydrous CrCl₂ (0.364 g, 2.87 mmol) in THF (4.78 mL) that was degassed by sparging with Ar for 30 min followed by the addition of the above product (0.193 g, 0.478 mmol) and CH₂ICl (0.178 mL, 2.39 mmol) at RT and under Ar atmosphere. The reaction mixture was stirred at 80 °C. After stirring for 14.5 h, the reactions were was cooled to RT and added to EtOAc (30 mL). Then the mixture was sequentially washed with 1.0 M aqueous HCl (30 mL×3) and saturated aqueous sodium thiosulfate (30 mL×2). The resulting organic phase was dried (Na₂SO₄), filtered and concentrated in vacuo. The crude material was diluted with EtOAc (5 mL) and filtered through basic aluminum. This process was repeated two times to remove residual chromium. Then the mixture was purified by chromatography on SiO₂ (EtOAc/hexanes, 1:1) to afford 3-fluoro-2-(4-(2-(4-(trifluoromethyl)phenyl)cyclopropane-1-carbonyl)-piperazin-1-yl)benzonitrile as a brown viscous oil (21.4 mg, 10 % over 2 steps): IR (neat): 2233, 1639, 1465, 1436, 1325, 1163, 1116, 1069, 1028 cm⁻¹; ¹H-NMR (400 MHz; CDCl₃): δ 7.53 (d, *J =* 8.1 Hz, 2 H), 7.34 (ddd, *J =* 7.7, 1.4, 0.8 Hz, 1 H), 7.28 (d, *J =* 8.3 Hz, 2 H), 7.20 (ddd, *J* = 12.0, 8.3, 1.4 Hz, 1 H), 7.07 (td, *J =* 8.1, 4.7 Hz, 1 H), 3.97-3.92 (m, 1 H), 3.80-3.75 (m, 1 H), 3.66-3.60 (m, 1 H), 3.33-3.27 (m, 1 H), 3.11-3.04 (m, 2 H), 2.72-2.67 (m, 1 H), 2.54-2.45 (m, 2 H), 2.27 (ddd, *J =* 9.2, 8.4, 6.2 Hz, 1 H), 1.92 (q, J = 6.2 Hz, 1 H), 1.43 (td, J = 8.4, 5.6 Hz, 1 H); ¹³C-NMR (125 MHz; CDCl₃): δ 166.7, 158.5 (d, *J =* 251.5 Hz), 141.8 (d, *J =* 1.8 Hz), 141.7 (d, *J =* 9.4 Hz), 129.4 (d, *J =* 3.2 Hz), 128.7 (q, *J =* 32.3 Hz), 127.8, 125.2 (q, *J =* 7.1 Hz), 125.1 (q, *J =* 3.7 Hz), 124.2 (q, *J =* 270.1 Hz), 121.7, 121.5, 116.9 (d, *J =* 4.5 Hz), 111.8 (d, *J =* 6.4 Hz), 51.9 (d, *J =* 4.2 Hz), 51.3 (d, *J =* 5.0 Hz), 46.0, 42.6, 24.5, 24.0, 11.1; ¹⁹F-NMR (376 MHz; CDCl₃): δ -62.47 (s, 3 F), -119.74 (d, *J =* 11.3 Hz, 1 F). HRMS (ESI): m/z calculated for C₂₂H₂₀O N₃F₄ ([M+H]⁺) 418.1537, found 418.1537.

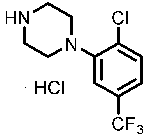

**1-(2-Chloro-5-(trifluoromethyl)phenyl)piperazine hydrochloride.** Under N₂ atmosphere, CuBr (0.565 g, 3.86 mmol), 1,1'-bi-2-naphthol (0.830 g, 2.89 mmol) and DMF (9.65 mL) was added to the flame-dried flask. The mixture was stirred for 10 minutes before the addition of 1-Boc-piperazine (5.39 g, 28.9 mmol), K₃PO₄ (8.44 g, 38.6 mmol) and 2-bromo-1-chloro-4-(trifluoromethyl)benzene (3.00 mL, 19.3 mmol). The reaction mixture was stirred at RT for 24 h. Then the mixture was stirred at 100 °C for 22 h. After cooling to room temperature, the mixture was diluted with EtOAc (20 mL) and filtered through Celite pad. The filtrate was concentrated under reduced pressure. Then the residue was sequentially washed with sat. NH₄Cl aq. (150 mL×2) and brine (150 mL x 2). The resulting organic layer was dried (Na₂SO₄), filtered and concentrated under reduced pressure. The crude product was filtered through a short column of SiO₂ (EtOAc/hexanes, 1:9) to remove unreacted piperazines. And the obtained mixture (2.96 g) was used without further purification.

To a solution of the above product (2.96 g) in 1,4-dioxane (19.9 mL), HCl (4 M in 1,4-dioxane, 8.11 mL) was added at 0 °C. The mixture was stirred at RT for 14 h. The thick suspension was diluted with hexanes (50 mL) and the resulting solid was collected by filtration, washed with hexanes and Et₂O, and dried to give 1-(2-chloro-5-(trifluoromethyl)phenyl)piperazine hydrochloride as a light yellow solid (0.408 g, 11% yield over 2 steps). Mp: 270 °C (decomposition); IR (neat): 2937, 2816, 2725, 2495, 1423, 1305, 1178, 1156, 1114, 1084, 1044 cm⁻¹; ¹H-NMR (400 MHz; DMSO-d6): δ 9.49 (s, 1 H), 9.41 (s, 1 H), 7.70 (d, *J =* 8.0 Hz, 1 H), 7.46 (d, *J =* 8.8 Hz, 1 H), 7.45 (s, 1 H), 3.24 (brs, 4 H); ¹³C-NMR (125 MHz; DMSO-d6): δ 148.6, 131.8, 131.5, 128.8 (q, *J =* 32.2 Hz), 123.7 (q, *J* = 272.5 Hz), 121.1 (q, *J =* 3.9 Hz), 117.6 (q, *J =* 3.6 Hz), 47.4, 42.9; ¹⁹F-NMR (376 MHz; DMSO-d6): δ -60.94 (d, *J* = 2.8 Hz, 1 F); HRMS (ESI): m/z calculated for C₁₁H₁₃F₃N₂Cl ([M+H]⁺) 265.0714, found 265.0710.

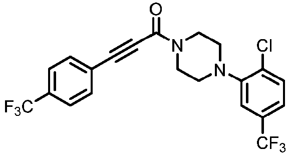

**1-(4-(2-Chloro-5-(trifluoromethyl)phenyl)piperazin-1-yl)-3-(4-(trifluoromethyl)phenyl)prop-2-yn-1-one.** To a solution of 3-(4-(trifluoromethyl)phenyl)propiolic acid (0.300 g, 1.40 mmol) in CH₂Cl₂ (14.0 mL) at 0°C was added 1-(2-chloro-5-(trifluoromethyl)phenyl)piperazine hydrochloride (0.506 g, 1.68 mmol), and Et₃N (0.597 mL, 4.20 mmol). T₃P (1.49 mL, 2.10 mmol) was added dropwise and the reaction was stirred at 0 °C for 30 min and allowed to warm to RT for 23.5 h. Then the reaction was diluted with CH₂Cl₂ (30 mL) and washed with 1 M HCl (30 mL). The aqueous phase was extracted with CH₂Cl₂ (30 mL×3) and combined organic phase was dried (Na₂SO₄), filtered and concentrated in vacuo. The crude material was purified by chromatography on SiO₂ (EtOAc/hexanes, 1:3) to afford1-(4-(2-chloro-5-(trifluoromethyl)-phenyl)piperazin-1-yl)-3-(4-(trifluoromethyl)phenyl)prop-2-yn-1-one (641 mg, 99%) as a yellow solid: Mp: 124-125 °C; IR (neat): 2225, 1631, 1419, 1321, 1310, 1276, 1167, 1122, 1107, 1086, 1068, 1035, 1017 cm⁻¹; ¹H-NMR (500 MHz; CDCl₃): δ 7.68 (d, *J* = 8.4 Hz, 2 H), 7.65 (d, *J =* 8.4 Hz, 2 H), 7.51 (d, *J* = 8.3 Hz, 1 H), 7.28 (d, *J =* 8.3 Hz, 1 H), 7.24 (d, *J =* 1.5 Hz, 1 H), 4.03 (t, *J =* 4.9 Hz, 2 H), 3.91 (t, *J =* 4.9 Hz, 2 H), 3.18 (t, *J* = 4.9 Hz, 2 H), 3.11 (t, *J* = 4.9 Hz, 2 H); ¹³C-NMR (125 MHz; CDCl₃): δ 152.6, 149.0, 132.7, 132.6, 131.8 (q, *J* = 32.9 Hz), 131.3, 130.2 (q, *J =* 32.9 Hz), 125.5 (q, *J* = 3.7 Hz), 124.1, 123.6 (q, *J =* 271.0 Hz), 121.0 (q, *J* = 3.8 Hz), 117.5 (q, *J* = 3.6 Hz), 89.1, 82. 7, 51.4, 50.7, 47.2, 41.7; ¹⁹F-NMR (471 MHz; CDCl₃): δ-62.58 (s, 1 F), -63.10 (s, 1 F); HRMS (ESI): m/z calculated for C₂₀H₁₄ON₃ClF₆ ([M+H]⁺) 461.0724, found 461.0639.

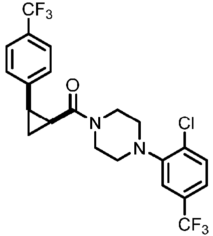

**(4-(2-Chloro-5-(trifluoromethyl)phenyl)piperazin-1-yl)(2-(4-(trifluoromethyl)phenyl)cyclopropyl)-methanone.** Under Ar atmosphere, a solution of 1-(4-(2-chloro-5-(trifluoromethyl)phenyl)piperazin-1-yl)-3-(4-(trifluoromethyl)phenyl)prop-2-yn-1-one (0.610 g, 1.32 mmol) in EtOAc (13.2 mL) was treated with quinoline (0.806 mL, 6.62 mmol) and 5% Pd/BaSO₄ (0.0282 g, 5 mol% based on Pd). The reaction was placed under a balloon of H₂ (3 vacuum/backfill cycles) and stirred at RT for 4 h. The reaction was filtered through Celite (eluting with EtOAc (100 mL) and the filtrates were washed with 1M aqueous HCl (100 mL). The organic phase was concentrated in vacuo to afford a crude product as a brown solid. The crude product was purified by chromatography on SiO₂ (EtOAc/hexanes, 1:2) to give the corresponding alkene (0.530 g, 86%) as a white crystal. ¹H-NMR (400 MHz; CDCl₃): δ 7.61 (d, *J =* 8.2 Hz, 2 H), 7.52 (d, *J =* 8.2 Hz, 2 H), 7.46 (dd, J = 8.4, 0.7 Hz, 1 H), 7.24 (dd, J = 8.4, 0.7 Hz, 1 H), 7.06 (d, *J =* 1.8 Hz, 1 H), 6.76 (d, *J =* 12.6 Hz, 1 H), 6.21 (d, *J =* 12.6 Hz, 1 H), 3.86 (t, *J =* 4.8 Hz, 2 H), 3.54 (t, *J =* 5.0 Hz, 2 H), 3.00 (t, *J* = 5.0 Hz, 2 H), 2.66 (t, *J =* 4.9 Hz, 2 H). ¹⁹F-NMR (376 MHz; CDCl₃): δ -62.69 (s, 1 F), -62.79 (s, 1 F).

To a solution of anhydrous CrCl₂ (0.134 g, 1.06 mmol) in THF (1.80 mL) that was degassed by sparging with Ar for 10 min followed by the addition of (*Z*)-1-(4-(2-chloro-5-(trifluoromethyl)phenyl)-piperazin-1-yl)-3-(4-(trifluoromethyl)phenyl)prop-2-en-1-one (0.469 g, 1.01 mmol) and CH₂ICl (0.376 mL, 5.06 mmol) at RT and under Ar atmosphere. The reaction mixture was stirred at 80 °C. After stirring for 21 h, the reaction was cooled to RT and then diluted with EtOAc (50 mL). The organic layer was sequentially washed with 1 M aqueous HCl (50 mL×3) and sat. aqueous sodium thiosulfate (50 mL×2). Then the organic phase was dried (Na₂SO₄), filtered and concentrated in vacuo. The residue was diluted in minimum amount of EtOAc and the solution was filtered through a plug of basic alumina two times. The resulting crude material was purified by chromatography on SiO₂ (EtOAc/hexanes, 1:2) to afford (4-(2-chloro-5-(trifluoromethyl)phenyl)piperazin-1-yl)(2-(4-(trifluoromethyl)phenyl)cyclopropyl)methanone (0.332 g, 69%) as a white solid: Mp: 122-123 °C; IR (neat): 1641, 1619, 1437, 1418, 1326, 1308, 1166, 1117, 1085, 1070, 1031 cm⁻¹; ¹H-NMR (500 MHz; CDCl₃): δ 7.55 (d, *J* = 8.2 Hz, 2 H), 7.46 (dd, *J =* 8.3, 0.6 Hz, 1 H), 7.31 (d, *J =* 8.2 Hz, 2 H), 7.25 (dd, *J =* 8.3, 1.7 Hz, 1 H), 6.98 (d, *J =* 1.7 Hz, 1 H), 3.94 (d, *J =* 13.0 Hz, 1 H), 3.77 (d, *J =* 13.0 Hz, 1 H), 3.67 (td, *J =* 10.7, 2.8 Hz, 1 H), 3.37 (ddd, *J* = 12.5, 9.2, 2.8 Hz, 1H), 3.00 (t, *J =* 12.5 Hz, 2 H), 2.54 (q, *J* = 8.0 Hz, 1 H), 2.38 (t, *J* = 8.8 Hz, 1 H), 2.30 (ddd, *J* = 9.3, 8.4, 6.2 Hz, 1 H), 2.16 (t, *J* = 8.8 Hz, 1 H), 1.94 (q, *J* = 6.2 Hz, 1 H), 1.46 (td, *J =* 8.4, 5.6 Hz, 1 H); ¹³C-NMR (125 MHz; CDCl₃): δ 166.7, 149.0, 141.9, 132.5 (d, *J =* 1.2 Hz), 131.1, 130.2 (q, *J =* 32.7 Hz), 128.8 (q, *J =* 32.5 Hz), 127.8, 125.0 (q, *J =* 3.7 Hz), 124.1 (q, *J =* 270.5 Hz), 123.5 (q, *J =* 270.8 Hz), 120.8 (q, *J =* 3.8 Hz), 117.2 (q, *J* = 3.7 Hz), 51.4, 50.8, 45.2, 41.9, 24.6, 24.0, 11.2; ¹⁹F-NMR (471 MHz; CDCl₃): δ -62.53 (s, 1 F), -62.76 (s, 1 F); HRMS (ESI): m/z calculated for C₂₂H₂₀F₆N₂ClO ([M+H]⁺) 477.1163, found 477.1152.

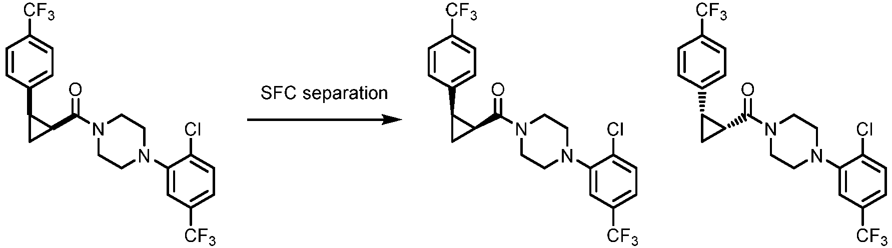

Racemic (4-(2-chloro-5-(trifluoromethyl)phenyl)piperazin-1-yl)(2-(4-(trifluoromethyl)phenyl)cyclopropyl)-methanone was separated on a SFC Chiralpak-IC semiprep (250×10 mm) column (15% MeOH, 7 mL/min, 220 nM, P=100) to afford (4-(2-chloro-5-(trifluoromethyl)phenyl)piperazin-1-yl)((*1S*,*2R*)-2-(4-(trifluoromethyl)phenyl)cyclopropyl)methanone (retention time; 5.70 min) as a white solid (>99.9% purity by ELSD): [α]¹⁸_{D} -127.0 (*c* = 0.29, MeOH); HRMS (ESI): m/z calculated for C₂₂H₂₀F₆ON₂Cl ([M+H]⁺) 477.1163, found 477.1164. The enantiomeric excess was >99.9% ee (SFC Chiralpak-IC semiprep; 15% MeOH, 220 nM, 7 mL/min; retention time: 5.70 min). ¹H-NMR (500 MHz; CDCl₃): δ 7.55 (d, *J =* 8.2 Hz, 2 H), 7.46 (dd, *J =* 8.3, 0.6 Hz, 1 H), 7.31 (d, *J =* 8.2 Hz, 2 H), 7.25 (dd, *J =* 8.3, 1.7 Hz, 1 H), 6.98 (d, *J =* 1.7 Hz, 1 H), 3.94 (d, *J =* 13.0 Hz, 1 H), 3.77 (d, *J =* 13.0 Hz, 1 H), 3.67 (td, *J =* 10.7, 2.8 Hz, 1 H), 3.37 (ddd, *J* = 12.5, 9.2, 2.8 Hz, 1H), 3.00 (t, *J =* 12.5 Hz, 2 H), 2.54 (q, *J =* 8.0 Hz, 1 H), 2.38 (t, *J =* 8.8 Hz, 1 H), 2.30 (ddd, *J =* 9.3, 8.4, 6.2 Hz, 1 H), 2.16 (t, *J =* 8.8 Hz, 1 H), 1.94 (q, *J =* 6.2 Hz, 1 H), 1.46 (td, *J =* 8.4, 5.6 Hz, 1 H).

(4-(2,5-bis(trifluoromethyl)phenyl)piperazin-1-yl)((*1R*,*2S*)-2-(4-(trifluoromethyl)phenyl)-cyclopropyl)methanone; (retention time; 6.40 min) as a white solid (>99.9% purity by ELSD): [α]¹⁸_{D} +130.0 (*c* = 0.23, MeOH); HRMS (ESI): m/z calculated for C₂₂H₂₀F₆ON₂Cl ([M+H]⁺) 477.1163, found 477.1160. The enantiomeric excess was >99.9% ee (SFC Chiralpak-IC semiprep; 15% MeOH, 220 nM, 7 mL/min; retention time: 6.40 min). ¹H-NMR (500 MHz; CDCl₃): δ 7.55 (d, *J =* 8.2 Hz, 2 H), 7.46 (dd, *J =* 8.3, 0.6 Hz, 1 H), 7.31 (d, *J =* 8.2 Hz, 2 H), 7.25 (dd, *J =* 8.3, 1.7 Hz, 1 H), 6.98 (d, *J =* 1.7 Hz, 1 H), 3.94 (d, *J =* 13.0 Hz, 1 H), 3.77 (d, *J =* 13.0 Hz, 1 H), 3.67 (td, *J* = 10.7, 2.8 Hz, 1 H), 3.37 (ddd, *J =* 12.5, 9.2, 2.8 Hz, 1H), 3.00 (t, *J =* 12.5 Hz, 2 H), 2.54 (q, *J =* 8.0 Hz, 1 H), 2.38 (t, *J =* 8.8 Hz, 1 H), 2.30 (ddd, *J =* 9.3, 8.4, 6.2 Hz, 1 H), 2.16 (t, *J =* 8.8 Hz, 1 H), 1.94 (q, *J =* 6.2 Hz, 1 H), 1.46 (td, *J =* 8.4, 5.6 Hz, 1 H).

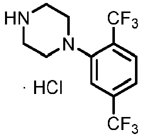

**1-(2,5-Bis(trifluoromethyl)phenyl)piperazine hydrochloride.** Under N₂ atmosphere, CuBr (0.511 g, 3.49 mmol), 1,1'-bi-2-naphthol (0.751 g, 2.62 mmol) and DMF (8.73 mL) was added to the flame-dried flask. The mixture was stirred for 10 minutes before the addition of 1-Boc-piperazine (4.88 g, 26.2 mol), K₃PO₄ (7.64 g, 34.9 mmol) and the 2,5-bis(trifluoromethyl)bromobenzene (3.00 mL, 17.5 mmol). The mixture was stirred at 100 °C for 26.5 h. After cooling to room temperature, the mixture was diluted with EtOAc (20 mL) and filtered through celite pad. The filtrate was concentrated under reduced pressure. Then the residue was sequentially washed with sat. NH₄Cl aq. (150 mL×2) and brine (150 mL×2). The resulting organic layer was dried (Na₂SO₄), filtered and concentrated under reduced pressure. The crude product was filtered through a short column of SiO₂ (EtOAc/hexanes 1:9) to remove unreacted piperazines. And the obtained mixture (2.76 g) was used without further purification.

To a solution of the above product (2.76 g) in 1,4-dioxane (17.0 mL), HCl (4 M in 1,4-dioxane, 6.93 mL) was added at 0 °C. The mixture was stirred at RT for 16 h. The thick suspension was diluted with hexanes (50 mL) and the resulting solid was collected by filtration, washed with hexanes and dried to give 1-(2,5-bis(trifluoromethyl)phenyl)piperazine hydrochloride as a white fluffy solid (0.811 g, 16 % over 2 steps): Mp: 268 °C; IR (neat): 2722, 2478, 1569, 1424, 1311, 1180, 1117, 1081, 1038 cm⁻¹; ¹H-NMR (500 MHz; DMSO-d6): δ 9.47-9.29 (brs, 2 H), 7.82 (s, 1 H), 7.78 (d, *J =* 8.2 Hz, 1 H), 3.18 (s, 4 H); ¹³C-NMR (125 MHz; DMSO-d6): δ 152.1, 134.3 (q, *J* = 32.5 Hz), 129.6 (q, *J* = 28.7 Hz), 129.3 (q, *J* = 5.1 Hz), 123.6 (q, *J =* 274.0 Hz), 123.1 (d, *J=* 3.6 Hz), 121.8 (d, *J* = 3.4 Hz), 50.0, 43.6; ¹⁹F-NMR (471 MHz; DMSO-d6): δ -59.66 (s, 3 F), -61.67 (s, 3 F); HRMS (ESI): m/z calculated for C₁₂H₁₃F₆N₂ ([M+H]⁺) 299.0977, found 299.0971.

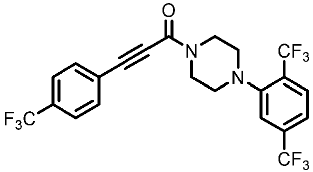

**1-(4-(2,5-Bis(trifluoromethyl)phenyl)piperazin-1-yl)-3-(4-(trifluoromethyl)phenyl)prop-2-yn-1-one.** To a solution of 3-(4-(trifluoromethyl)phenyl)propiolic acid (0.300 g, 1.40 mmol) in CH₂Cl₂ (14.0 mL) at 0°C was added 1-(2,5-bis(trifluoromethyl)phenyl)piperazine hydrochloride (0.563 g, 1.68 mmol), and Et₃N (0.597 mL, 4.20 mmol). T₃P (1.49 mL, 2.10 mmol) was added dropwise and the reaction was stirred at 0°C for 30 min and allowed to warm to RT for 23.5 h. The reaction was diluted with CH₂Cl₂ (30 mL) and washed with 1 M HCl (30 mL). The aqueous phase was extracted with CH₂Cl₂ (30 mL×3) and combined organic phase was dried (Na₂SO₄), filtered and concentrated in vacuo. The crude material was purified by chromatography on SiO₂ (EtOAc/hexanes, 1:3) to afford 1-(4-(2,5-bis(trifluoromethyl)phenyl)piperazin-1-yl)-3-(4-(trifluoromethyl)phenyl)prop-2-yn-1-one (155 mg, 50%) as a light yellow oil: IR (neat): 1632, 1424, 1311, 1285, 1274, 1172, 1121, 1107, 1034 cm⁻¹; ¹H-NMR (500 MHz; CDCl₃): δ 7.80 (d, *J =* 8.1 Hz, 1 H), 7.67 (d, *J =* 8.4 Hz, 2 H), 7.64 (d, *J =* 8.4 Hz, 2 H), 7.56 (s, 1 H), 7.55 (d, *J =* 8.1 Hz, 1 H), 3.99 (t, *J*= 4.9 Hz, 2 H), 3.87 (t, *J =* 4.9 Hz, 2 H), 3.06 (t, *J =* 4.9 Hz, 2 H), 2.99 (t, *J* = 4.9 Hz, 2 H); ¹³C-NMR (125 MHz; CDCl₃): δ 152.6, 152.1, 135.2 (q, *J* = 33.3 Hz), 132.6, 131.8 (q, *J* = 32.9 Hz), 130.7 (q, *J* = 29.6 Hz), 128.4 (q, *J =* 5.4 Hz), 125.5 (q, *J =* 3.7 Hz), 124.1 (d, *J* = 1.8 Hz), 123.6 (q, *J* = 270.8 Hz), 123.2 (q, *J* = 272.0 Hz), 123.1 (q, *J* = 271.0 Hz), 122.4 (q, *J* = 3.6 Hz), 121.1 (q, *J* = 3.5 Hz), 89.1, 82.7, 53.6, 52.8, 47.4, 41.9. ¹⁹F-NMR (471 MHz; CDCl₃): δ -60.91 (s, 3 F), -63.12 (s, 3 F), -63.23 (s, 3 F); HRMS (ESI): m/z calculated for C₂₂H₁₆F₉ON₂ ([M+H]⁺) 495.1113, found 495.0934

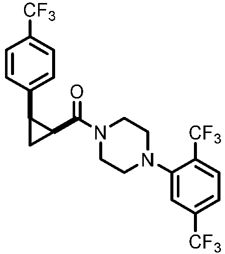

**(4-(2,5-bis(trifluoromethyl)phenyl)piperazin-1-yl)(2-(4-(trifluoromethyl)phenyl)cyclopropyl)-**methanone. Under Ar atmosphere, a solution of 1-(4-(2,5-bis(trifluoromethyl)phenyl)piperazin-1-yl)-3-(4-(trifluoromethyl)phenyl)prop-2-yn-1-one (0.657 g, 1.33 mmol) in EtOAc (13.3 mL) was treated with quinoline (0.810 mL, 6.65 mmol) and 5% Pd/BaSO₄ (0.0283 g, 10 mol% based on Pd). The reaction was placed under a balloon of H₂ (3 vacuum/backfill cycles) and stirred at RT for 6 h. The reaction was filtered through Celite (eluting with EtOAc (100 mL) and the filtrates were washed with 1M aqueous HCl (100 mL×2). The organic phase was dried (Na₂SO₄), filtered and concentrated in vacuo. The crude product was purified by chromatography on SiO₂ (EtOAc/hexanes, 2:3) to give (Z)-1-(4-(2,5-bis(trifluoromethyl)phenyl)-piperazin-1-yl)-3-(4-(trifluoromethyl)phenyl)prop-2-en-1-one (0.655 g, 99%) as a light yellow oil. ¹H-NMR (400 MHz; CDCl₃): δ 7.75 (d, *J =* 8.2 Hz, 1 H), 7.64 (d, *J =* 8.4 Hz, 2 H), 7.53 (d, *J =* 8.4 Hz, 2 H), 7.50 (d, *J* = 8.2 Hz, 1 H), 7.33 (s, 1 H), 6.75 (d, *J =* 12.5 Hz, 1 H), 6.21 (d, *J =* 12.5 Hz, 1 H), 3.82 (t, *J* = 4.9 Hz, 2 H), 3.51 (t, *J =* 4.9 Hz, 2 H), 2.87 (t, *J =* 4.9 Hz, 2 H), 2.51 (t, *J =* 4.9 Hz, 2 H). ¹⁹F-NMR (376 MHz; CDCl₃): δ - 60.96 (s, 3 F), -62.82 (s, 3 F), -63.35 (s, 3 F).

To a solution of anhydrous CrCl₂ (0.850 g, 6.71 mmol) in THF (11.2 mL) that was degassed by sparging with Ar for 10 min followed by the addition of (Z)-1-(4-(2,5-bis(trifluoromethyl)phenyl)piperazin-1-yl)-3-(4-(trifluoromethyl)phenyl)prop-2-en-1-one (0.555 g, 1.12 mmol) and CH₂ICl (0.416 mL, 5.59 mmol) at RT and under Ar atmosphere. The reaction mixture was stirred at 80 °C. After stirring for 22.5 h, the reaction was cooled to RT and then diluted with EtOAc (80 mL). The solution was sequentially washed with 1 M aqueous HCl (80 mL×3) and sat. aqueous sodium thiosulfate (100 mL×2). Then the organic phase was dried (Na₂SO₄), filtered and concentrated in vacuo. The residue was diluted with minimum amount of EtOAc, and the solution was filtered through a plug of basic alumina. This process was repeated two times. Then the resulting crude material was purified by chromatography on SiO₂ (EtOAc/hexanes, 2:1) to afford (4-(2,5-bis(trifluoromethyl)phenyl)piperazin-1-yl)(2-(4-(trifluoromethyl)-phenyl)cyclopropyl)methanone (0.472 g, 83%) as a white solid: Mp: 111-112 °C; IR (neat): 1644, 1425, 1327, 1314, 1123, 1032 cm⁻¹; ¹H-NMR (500 MHz; CDCl₃): δ 7.72 (d, *J* = 8.2 Hz, 1 H), 7.54 (d, *J = 8.2* Hz, 2 H), 7.47 (d, *J =* 8.2 Hz, 1 H), 7.29 (d, *J =* 8.2 Hz, 2 H), 7.18 (s, 1 H), 3.96 (d, *J =* 12.0 Hz, 1 H), 3.75 (d, *J* = 12.6 Hz, 1 H), 3.56 (t, *J =* 9.9 Hz, 1 H), 3.23 (t, *J =* 9.9 Hz, 1 H), 2.78 (t, *J =* 15.3 Hz, 2 H), 2.51 (q, *J =* 7.9 Hz, 1 H), 2.29-2.23 (m, 2 H), 2.05 (t, *J* = 9.0 Hz, 1 H), 1.92 (q, *J* = 6.2 Hz, 1 H), 1.43 (td, *J* = 8.4, 5.6 Hz, 1 H); ¹³C-NMR (125 MHz; CDCl₃): δ 166.6, 152.2, 142.0, 135.1 (q, *J* = 33.0 Hz), 128.8 (q, *J* = 32.6 Hz), 128.1 (q, *J* = 5.4 Hz), 127.9, 124.9 (q, *J* = 3.7 Hz), 124.1 (q, *J* = 269.9 Hz), 123.1 (q, *J* = 270.9 Hz), 123.0 (q, *J =* 271.1 Hz), 122.0 (q, *J* = 3.6 Hz), 120.7 (q, *J* = 3.4 Hz), 53.7, 52.9, 45.4, 42.1, 24.6, 23.9, 11.1. ¹⁹F-NMR (471 MHz; CDCl₃): δ -60.98 (s, 3 F), -62.58 (s, 3 F), -63.48 (s, 3 F); HRMS (ESI): m/z calculated for C₂₃H₂₀F₉ON₂ ([M+H]⁺) 511.1426, found 511.1426. 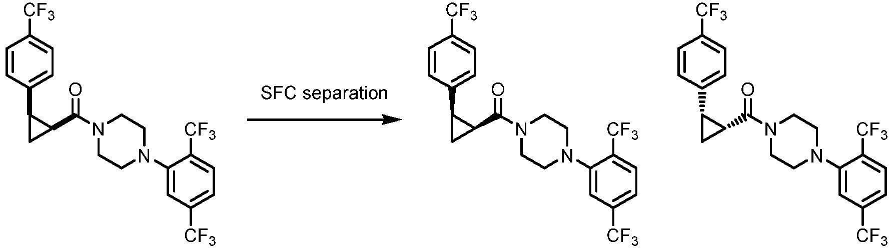 Racemic (4-(2,5-bis(trifluoromethyl)phenyl)piperazin-1-yl)(2-(4-(trifluoromethyl)phenyl)cyclopropyl)-methanone was separated on a SFC Chiralpak-IC semiprep (250 x 10 mm) column (5% MeOH, 7 mL/min, 220 nM, P=100) to afford (4-(2,5-bis(trifluoromethyl)phenyl)piperazin-1-yl)((*1S,2R*)-2-(4-(trifluoromethyl)-phenyl)cyclopropyl)methanone (retention time; 7.60 min) as a colorless oil (>99.9% purity by ELSD): [α]¹⁸_{D} -109.7 (c = 0.25, MeOH); HRMS (ESI): m/z calculated for C₂₃H₂₀F₉ON₂ ([M+H]⁺) 511.1426, found 511.1423. The enantiomeric excess was >99.9% ee (SFC Chiralpak-IC semiprep; 5% MeOH, 220 nM, 7 mL/min; retention time: 7.60 min). ¹H-NMR (500 MHz; CDCl₃): δ 7.72 (d, *J =* 8.2 Hz, 1 H), 7.54 (d, *J =* 8.2 Hz, 2 H), 7.47 (d, *J =* 8.2 Hz, 1 H), 7.29 (d, *J =* 8.2 Hz, 2 H), 7.18 (s, 1 H), 3.96 (d, *J =* 12.0 Hz, 1 H), 3.75 (d, *J* = 12.6 Hz, 1 H), 3.56 (t, *J =* 9.9 Hz, 1 H), 3.23 (t, *J =* 9.9 Hz, 1 H), 2.78 (t, *J =* 15.3 Hz, 2 H), 2.51 (q, *J* = 7.9 Hz, 1 H), 2.29-2.23 (m, 2 H), 2.05 (t, *J =* 9.0 Hz, 1 H), 1.92 (q, *J =* 6.2 Hz, 1 H), 1.43 (td, *J =* 8.4, 5.6 Hz, 1 H).

(4-(2,5-bis(trifluoromethyl)phenyl)piperazin-1-yl)((*1R,2S*)-2-(4-(trifluoromethyl)phenyl)-cyclopropyl)methanone; (retention time; 9.20 min) as a colorless oil (>99.9% purity by ELSD): [α]¹⁸_{D} +108.8 (*c =* 0.22, MeOH); HRMS (ESI): m/z calculated for C₂₃H₂₃F₆O₂N₂ ([M+H]⁺) 511.1426, found 511.1425. The enantiomeric excess was >99.9% ee (SFC Chiralpak-IC semiprep; 5% MeOH, 220 nM, 7 mL/min; retention time: 9.20 min). ¹H-NMR (500 MHz; CDCl₃): δ 7.72 (d, *J =* 8.2 Hz, 1 H), 7.54 (d, *J* = 8.2 Hz, 2 H), 7.47 (d, *J =* 8.2 Hz, 1 H), 7.29 (d, *J =* 8.2 Hz, 2 H), 7.18 (s, 1 H), 3.96 (d, *J =* 12.0 Hz, 1 H), 3.75 (d, J = 12.6 Hz, 1 H), 3.56 (t, *J =* 9.9 Hz, 1 H), 3.23 (t, *J =* 9.9 Hz, 1 H), 2.78 (t, *J =* 15.3 Hz, 2 H), 2.51 (q, *J* = 7.9 Hz, 1 H), 2.29-2.23 (m, 2 H), 2.05 (t, *J =* 9.0 Hz, 1 H), 1.92 (q, *J =* 6.2 Hz, 1 H), 1.43 (td, *J =* 8.4, 5.6 Hz, 1 H).

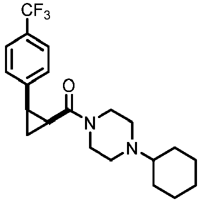

**(4-Cyclohexylpiperazin-1-yl)(2-(4-(trifluoromethyl)phenyl)cyclopropyl)methanone.** To a solution of piperazin-1-yl(2-(4-(trifluoromethyl)phenyl)cyclopropyl)methanone hydrochloride (0.09 g, 0.269 mmol) and cyclohexanone (0.0279 mL, 0.269 mmol) in dichloromethane (1.00 mL) was added NaBH(OAc)₃ (0.256 g, 1.21 mmol) and acetic acid (0.0154 mL, 0.269 mmol). The mixture was stirred for 45 h at room temperature. Then the mixture was quenched with 1N aqueous NaOH (40 mL) and extracted with EtOAc (40 mL×3). The combined organic phase was dried (Na₂SO₄), filtered and concentrated in vacuo. The crude product was purified by chromatography on SiO₂ (MeOH/CH₂Cl₂, 1:9) to yield (4-cyclohexylpiperazin-1-yl)(2-(4-(trifluoromethyl)phenyl)cyclopropyl)methanone (0.0883 g, 86 %) as a light yellow solid: Mp: 99-101 °C; IR (neat): 2933, 1634, 1618, 1468, 1439, 1325, 1161, 1116, 1070, 1017 cm⁻¹;^{.1}H-NMR (500 MHz; CDCl₃): δ 7.49 (d, *J =* 8.2 Hz, 2 H), 7.24 (d, *J =* 8.2 Hz, 2 H), 3.78 (d, *J =* 12.8 Hz, 1 H), 3.58 (d, *J =* 12.8 Hz, 1 H), 3.38 (ddd, *J =* 12.5, 9.4, 3.0 Hz, 1 H), 3.06 (ddd, *J =* 12.5, 9.4, 3.0 Hz, 1 H), 2.47-2.41 (m, 3 H), 2.22 (td, *J* = 8.8, 6.3 Hz, 1 H), 2.09 (tt, *J =* 11.4, 3.1 Hz, 1 H), 1.87-1.82 (m, 2 H), 1.73 (d, *J* = 12.8 Hz, 2 H), 1.61-1.57 (m, 4 H), 1.39 (td, *J =* 8.4, 5.6 Hz, 1 H), 1.19-1.10 (m, 2 H), 1.06-0.94 (m, 3 H); ¹³C-NMR (125 MHz; CDCl₃): δ 166.3, 142.2, 128.5 (q, *J =* 32.4 Hz), 127.8, 124.9 (q, *J* = 3.9 Hz), 124.2 (q, *J* = 270.0 Hz), 63.5, 49.2, 48.4, 45.6, 42.3, 28.6, 28.5, 26.2, 25.8, 24.8, 23.8, 11.2; ¹⁹F-NMR (471 MHz; CDCl₃): δ -62.43 (s, 3 F); HRMS (ESI): m/z calculated for C₂₃H₂₀F₉ON₂ ([M+H]⁺) 381.2154, found 381.2147.

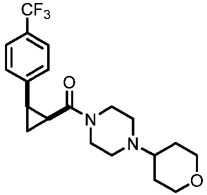

**(4-(Tetrahydro-2H-pyran-4-yl)piperazin-1-yl)(2-(4-(trifluoromethyl)phenyl)cyclopropyl)methanone.** To a solution of piperazin-1-yl(2-(4-(trifluoromethyl)phenyl)cyclopropyl)methanone hydrochloride (0.09 g, 0.269 mmol) and tetrahydro-4H-pyran-4-one (0.0269 mL, 0.269 mmol) in dichloromethane (1.00 mL) was added NaBH(OAc)₃ (0.205 g, 0.269 mmol) and acetic acid (0.0154 mL, 0.269 mmol). The mixture was stirred for 13 h at room temperature. Then the mixture was quenched with 1N aqueous NaOH (30 mL) and extracted with CH₂Cl₂ (30 mL×3). The combined organic phase was dried (Na₂SO₄), filtered and concentrated in vacuo. The crude product was purified by chromatography on SiO₂ (MeOH:CH₂Cl₂ = 1:9) to give (4-(tetrahydro-2H-pyran-4-yl)piperazin-1-yl)(2-(4-(trifluoromethyl)phenyl)cyclopropyl)methanone (0.0845 g, 82 %) as a light yellow solid: Mp: 84-86 °C; IR (neat): 2949, 2845, 1637, 1619, 1468, 1439, 1325, 1161, 1115, 1069 cm⁻¹; ¹H-NMR (500 MHz; CDCl₃): δ 7.49 (d, *J* = 8.2 Hz, 2 H), 7.24 (d, *J* = 8.2 Hz, 2 H), 3.95 (dd, *J =* 11.5, 3.4 Hz, 2 H), 3.83 (d, *J =* 13.1 Hz, 1 H), 3.61 (d, *J =* 12.9 Hz, 1H), 3.37 (ddd, *J* = 12.7, 9.5, 3.1 Hz, 1 H), 3.28 (tt, *J =* 11.8, 2.8 Hz, 2 H), 3.04 (ddd, *J* = 12.7, 9.5, 3.1 Hz, 1 H), 2.47-2.43 (m, 3 H), 2.28-2.19 (m, 2 H), 1.87-1.78 (m, 2 H), 1.51-1.30 (m, 6 H). ¹³C-NMR (125 MHz; CDCl₃): δ 166.3, 142.2, 128.5 (q, *J* = 32.4 Hz), 127.7, 125.0 (q, *J* = 3.9 Hz), 124.2 (q, *J* = 270.4 Hz), 67.4, 67.3, 60.8, 49.0, 48.6, 45.4, 42.1, 29.1, 28.9, 24.9, 23.8, 11.3; ¹⁹F-NMR (471 MHz; CDCl₃): δ -62.37 (s, 3 F); HRMS (ESI): m/z calculated for C₂₀H₂₆F₃O₂N₂ ([M+H]⁺) 383.1946, found 383.1938.

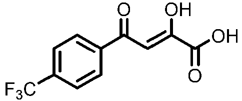

**2-Hydroxy-4-oxo-4-(4-(trifluoromethyl)phenyl)but-2-enoic acid.** To a heterogeneous solution of Na (0.121 g, 5.26 mmol) in Et₂O (2.56 mL) was added dropwise dry EtOH (0.307 mL) at 0 °C. The mixture was stirred for 20 min at that temperature. Then a mixture of dimethyl oxalate (0.583 mL, 5.26 mmol) and 4'-(trifluoromethyl)acetophenone (1.00 g, 5.26 mmol) in dry Et₂O (0.850 mL) was added carefully in small portions at -20 °C and stirred for 19.5 h at that temperature. The mixture was then poured into 1N HCl and extracted with ethyl acetate (50 mL×3). The combined organic layers were dried (Na₂SO₄), filtered and concentrated under vacuum. The resulting crude product was used without further purification.

To a 250-mL round-bottom flask was added the above product (1.51 g) in THF (6.55 mL), followed by LiOH monohydrate (1.10 g, 26.2 mmol) in water (6.55 mL) in one portion. Saponification was terminated when the starting material was consumed completely by TLC analysis (petroleum ether/EtOAc, 1:1). The mixture was concentrated under vacuum to remove the THF and then was extracted with CH₂Cl₂ (100 mL×3). The aqueous layer was acidified with 1N HCl (100 mL) and extracted with EtOAc (100 mL×3). The combined organic layer was dried (Na₂SO₄), filtered and concentrated in vacuo to yield 2-hydroxy-4-oxo-4-(4-(trifluoromethyl)phenyl)but-2-enoic acid (0.311 g, 23% over 2 steps) as a white solid. ¹H-NMR (300 MHz; CDCl₃): δ 8.12 (d, *J=* 8.2 Hz, 2 H), 7.79 (d, *J* = 8.2 Hz, 2 H), 7.20 (s, 1 H). The spectra obtained are in agreement with previously reported data (Zeng et al., Bioorg. Med. Chem. 2008, 16:7777-7787).

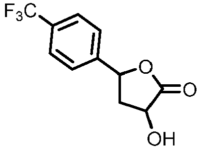

**3-Hydroxy-5-(4-(trifluoromethyl)phenyl)dihydrofuran-2(3H)-one.** To a 100-mL round-bottom flask containing a stirrer, 2-hydroxy-4-oxo-4-(4-(trifluoromethyl)phenyl)but-2-enoic acid (1.03 g, 3.96 mmol) in 13.2 mL MeOH was added, followed by NaBH₄ (0.611 g, 15.8 mmol) in small portions at room temperature. The mixture was stirred for 3 h. 1 mL water was added to decompose residual NaBH₄ and the mixture was concentrated under vacuum to remove the solvent. Then the mixture was acidified to pH 1.0-2.0 by adding 1N HCl (10 mL) and refluxed for 17.5 h. Then the mixture was extracted with EtOAc (50 mL×3) and the combined organic layer was dried (Na₂SO₄), filtered and concentrated in vacuo. The crude mixture was purified by chromatography on SiO₂ (CH₂Cl₂/MeOH, 20:1) to afford 3-Hydroxy-5-(4-(trifluoromethyl)-phenyl)dihydrofuran-2(3H)-one (0.456 g, 47%, dr = 1 : 2) as a white solid: IR (neat): 3421, 1772, 1322, 1166, 1110, 1067, 1016 cm⁻¹; (Major diastereomer) ¹H-NMR (500 MHz; CDCl₃): δ 7.68 (d, *J =* 8.1 Hz, 2 H), 7.50 (d, *J =* 8.1 Hz, 2 H), 5.43 (dd, *J =* 10.9, 5.4 Hz, 1 H), 4.73 (t, *J =* 9.6 Hz, 1 H), 3.06 (ddd, *J=* 12.9, 7.9, 5.2 Hz, 1 H), 3.01 (d, *J =* 0.4 Hz, 1 H), 2.21 (q, *J =* 11.8 Hz, 1 H); ¹³C-NMR (125 MHz; CDCl₃): δ 176.9, 141.8, 131.1 (q, *J =* 32.7 Hz), 126.0, 125.9 (q, *J* = 4.0 Hz), 123.8 (q, *J* = 272.4 Hz), 77.9, 68.8, 39.5; ¹⁹F-NMR (471 MHz; CDCl₃): δ -62.74 (s, 3 F).

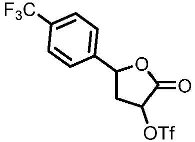

**2-Oxo-5-(4-(trifluoromethyl)phenyl)tetrahydrofuran-3-yl trifluoromethanesulfonate.** To a solution of 3-hydroxy-5-(4-(trifluoromethyl)phenyl)dihydrofuran-2(3H)-one (0.140 g, 0.569 mmol) and pyridine (0.0696 mL, 0.853 mmol) in dry CH₂Cl₂ (2.84 mL) was added trifluoromethanesulfonic anhydride (0.116 mL) at 0 °C. The mixture was stirred for 1.5 h at that temperature. Then the mixture was diluted with CH₂Cl₂ (15 mL) and quenched with 1N aqueous HCl (15 mL). The aqueous layer was extracted with CH₂Cl₂ (15 mL×3) and a combined organic layer was dried (Na₂SO₄), filtered and concentrated in vacuo. The crude mixture was purified by chromatography on SiO₂ (hexanes/EtOAc, 3: 1) to yield 2-oxo-5-(4-(trifluoromethyl)phenyl)tetrahydrofuran-3-yl trifluoromethanesulfonate (0.586 g, 89%, dr = 2 : 1) as a light yellow solid. (Major-diastereomer) ¹H-NMR (400 MHz; CDCl₃): δ 7.72 (d, *J =* 8.3 Hz, 3 H), 7.50 (d, *J = 8.2* Hz, 2 H), 5.61 (dd, *J =* 10.6, 8.4 Hz, 1 H), 5.52 (dd, *J =* 10.6, 5.5 Hz, 1 H), 3.29 (ddd, *J =* 13.0, 8.4, 5.5 Hz, 1 H), 2.52 (dt, *J* = 13.0, 10.7 Hz, 1 H).

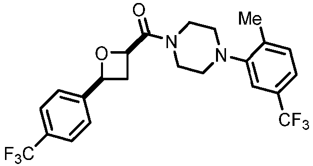

**(4-(2-Methyl-5-(trifluoromethyl)phenyl)piperazin-1-yl)(4-(4-(trifluoromethyl)phenyl)oxetan-2-**yl)methanone. To a solution of 2-oxo-5-(4-(trifluoromethyl)phenyl)tetrahydrofuran-3-yl trifluoromethanesulfonate (0.0680 g, 0.180 mmol) in dry MeOH (0.899 mL) was added potassium carbonate (0.0301 g, 0.216 mmol). The mixture was stirred for 1 h at 0 °C. Then the mixture was quenched with water, extracted with EtOAc, dried (Na₂SO₄), filtered and concentrated in vacuo. The crude product was filtered through a short column of SiO₂ (hexanes/EtOAc, 3: 1) to yield a mixture of methyl 4-(4-(trifluoromethyl)-phenyl)-oxetane-2-carboxylate as a light yellow oil. This product was used without further purification.

To a solution of the above product (97.0 mg) in THF/H₂O (1.85mL / 1.85 mL) was added LiOH (62.6 mg, 1.49 mmol) at room temperature. After stirring for 16 h, the mixture was diluted with H₂O (10 mL) and extracted with CH₂Cl₂ (15 mL×2). Then the aqueous layer was acidified with 1N HCl (20 mL) and extracted with EtOAc (30 mL×3). The combined organic phase was dried (Na₂SO₄), filtered and concentrated in vacuo. The crude product was used without further purification.

To a solution of the above crude product (0.095 g) in CH₂Cl₂ (3.86 mL) at 0 °C was added 1-(2-methyl-5-(trifluoromethyl)phenyl)piperazine hydrochloride (0.130 g, 0.463 mmol) and Et₃N (0.164 mL, 1.16 mmol). T₃P (0.409 mL, 0.579 mmol) was added dropwise and the reaction was stirred at 0 °C for 30 min and allowed to warm to RT for 12.5 h. The reaction was diluted with CH₂Cl₂ (20 mL) and washed with 1 M HCl (20 mL). The aqueous phase was extracted with CH₂Cl₂ (30 mL×3) and combined organic phase was dried (Na₂SO₄), filtered and concentrated in vacuo. The crude material was purified by chromatography on SiO₂ (EtOAc/hexanes, 2:3) to afford (4-(2-methyl-5-(trifluoromethyl)phenyl)piperazin-1-yl)(4-(4-(trifluoromethyl)phenyl)oxetan-2-yl)methanone (cis-diastereomer (light yellow oil): 96.2 mg , *trans*diastereomer (white solid): 52.55 mg). (cis-isomer) IR (neat): 2913, 1652, 1445, 1419, 1325, 1310, 1163, 1120, 1067 cm⁻¹; ¹H-NMR (500 MHz; CDCl₃): δ 7.64 (d, *J =* 8.2 Hz, 2 H), 7.55 (d, *J* = 8.2 Hz, 2 H), 7.30 (d, *J =* 8.0 Hz, 1 H), 7.26 (d, *J =* 8.0 Hz, 1 H), 7.19 (s, 1 H), 5.73 (t, *J =* 7.7 Hz, 2 H), 5.43 (t, *J =* 7.7 Hz, 2 H), 3.91-3.87 (m, 1 H), 3.79-3.72 (m, 2 H), 3.61-3.57 (m, 1 H), 3.21-3.09 (m, 2 H), 2.99-2.91 (m, 4 H), 2.37 (s, 3 H); ¹³C-NMR (125 MHz; CDCl₃): δ 168.87, 151.09, 145.93, 136.79, 131.53, 130.35 (q, *J* = 32.4 Hz), 129.05 (q, *J* = 32.1 Hz), 126.08, 125.52 (q, *J* = 3.8 Hz), 124.22 (d, *J =* 272.3 Hz), 124.0 (d, *J* = 270.5 Hz), 120.37 (q, *J* = 3.8 Hz), 115.99 (q, *J* = 3.6 Hz), 78.66, 74.22, 51.89, 51.63, 45.40, 42.39, 32.58, 17.92; ¹⁹F-NMR (471 MHz; CDCl₃): δ -62.28 (s, 3F), -62.59 (s, 3F); HRMS (ESI): m/z calculated for C₂₃H₂₃F₆O₂N₂ ([M+H]⁺) 473.1658, found 473.1649; (*trans-isomer*) IR (neat): 2918, 1651, 1444, 1418, 1324, 1309, 1163, 1119, 1067 cm⁻¹;¹H-NMR (500 MHz; CDCl₃): δ 7.67 (d, *J =* 8.2 Hz, 2 H), 7.56 (d, *J = 8.2* Hz, 2 H), 7.30 (d, *J =* 8.2 Hz, 1 H), 7.26 (d, *J =* 8.2 Hz, 1 H), 7.21 (s, 1 H), 5.80 (t, *J =* 7.4 Hz, 1 H), 5.39 (dd, *J =* 8.8, 5.9 Hz, 1 H), 3.95-3.92 (m, 1 H), 3.79-3.76 (m, 1 H), 3.72 (ddd, *J =* 13.0, 6.4, 3.0 Hz, 1 H), 3.59-3.53 (m, 2 H), 3.01-2.91 (m, 4 H), 2.77 (ddd, *J =* 11.5, 8.8, 6.5 Hz, 1 H), 2.38 (s, 3 H); ¹³C-NMR (125 MHz; CDCl₃): δ 169.2, 151.1, 146.6, 136.8 (d, *J* = 1.3 Hz), 131.5, 130.2 (q, *J* = 32.4 Hz), 129.1 (q, *J* = 32.2 Hz), 125.6 (q, *J*= 3.8 Hz), 125.4, 124.1 (q, *J* = 272.3 Hz), 124.0 (q, *J* = 270.5 Hz), 120.4 (q, *J* = 3.8 Hz), 116.1 (q, *J* = 3.7 Hz), 79.6, 75.4, 51.8, 51.6, 45.5, 42.4, 32.3, 18.0; ¹⁹F-NMR (471 MHz; CDCl₃): δ -62.27 (s, 3 F), -62.53 (s, 3 F); HRMS (ESI): m/z calculated for C₂₃H₂₃F₆O₂N₂ ([M+H]⁺) 473.1658, found 473.1656.

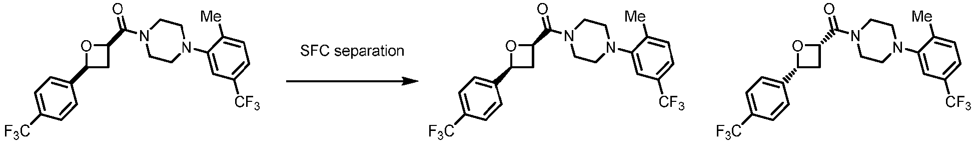

Racemic (4-(2-methyl-5-(trifluoromethyl)phenyl)piperazin-1-yl)(4-(4-(trifluoromethyl)phenyl)-oxetan-2-yl)methanone was separated on a SFC Chiralpak-IC semiprep (250×10 mm) column (9% MeOH, 5 mL/min, 220 nM, P=100) to afford (+)-enantiomer (retention time; 12.60 min) as a colorless oil (>99.9% purity by ELSD): [α]²⁰_{D} +55.5 (MeOH); HRMS (ESI): m/z calculated for C₂₃H₂₃F₆O₂N₂ ([M+H]⁺) 473.1658, found 473.1656. The enantiomeric excess was >99.9% ee (SFC Chiralpak-IC semiprep; 9% MeOH, 220 nM, 5 mL/min; retention time: 12.60 min). ¹H-NMR (500 MHz; CDCl₃): δ 7.64 (d, *J =* 8.2 Hz, 2 H), 7.55 (d, *J* = 8.2 Hz, 2 H), 7.30 (d, *J =* 8.0 Hz, 1 H), 7.26 (d, *J =* 8.0 Hz, 1 H), 7.19 (s, 1 H), 5.73 (t, *J* = 7.7 Hz, 2 H), 5.43 (t, *J =* 7.7 Hz, 2 H), 3.91-3.87 (m, 1 H), 3.79-3.72 (m, 2 H), 3.61-3.57 (m, 1 H), 3.21-3.09 (m, 2 H), 2.99-2.91 (m, 4 H), 2.37 (s, 3 H).

(-)-enantiomer; (retention time; 13.80 min) as a colorless oil (>99.9% purity by ELSD): [α]²⁰_{D} -70.8 (MeOH); HRMS (ESI): m/z calculated for C₂₃H₂₃F₆O₂N₂ ([M+H]⁺) 473.1658, found 473.1657. The enantiomeric excess was >99.9% ee (SFC Chiralpak-IC semiprep; 9% MeOH, 220 nM, 5 mL/min; retention time: 13.80 min). ¹H-NMR (500 MHz; CDCl₃): δ 7.64 (d, *J*= 8.2 Hz, 2 H), 7.55 (d, *J* = 8.2 Hz, 2 H), 7.30 (d, *J* = 8.0 Hz, 1 H), 7.26 (d, *J* = 8.0 Hz, 1 H), 7.19 (s, 1 H), 5.73 (t, *J* = 7.7 Hz, 2 H), 5.43 (t, *J* = 7.7 Hz, 2 H), 3.91-3.87 (m, 1 H), 3.79-3.72 (m, 2 H), 3.61-3.57 (m, 1 H), 3.21-3.09 (m, 2 H), 2.99-2.91 (m, 4 H), 2.37 (s, 3 H).

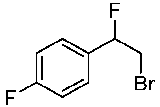

**1-(2-Bromo-1-fluoroethyl)-4-fluorobenzene** (Schlossera et al., Tetrahedron 2004, 7731-7742; Rosen et al., J. Med. Chem. 2004, 47:5860-5871). To a stirred solution of 4-fluorostyrene (2.00 g, 1.95 mL, 15.9 mmol) in distilled CH₂Cl₂ (16 mL) was added triethylamine trihydrofluoride (3.96 mL, 23.8 mmol) and NBS (3.43 g, 19.1 mmol) at 0 °C. After 15 min, the ice bath was removed and the mixture was stirred at room temperature for 18 h. The reaction mixture was poured into ice water (200 mL) and made slightly basic with NH₄OH. The mixture was extracted with CH₂Cl₂ (4 x 40 mL). The combined organic layers were washed with 0.1 M HCl (2 x 40 mL), 5% NaHCO₃ (2 x 40 mL), dried over anhydrous MgSO₄ and concentrated under reduced pressure. The crude product was purified via flash column chromatography on SiO₂ (1:20 EtOAc/hexanes) to afford **1-(2-bromo-1-fluoroethyl)-4-fluorobenzene** (2.67 g, 12.1 mmol, 76%) as a colorless oil: ¹H-NMR (500 MHz, CDCl₃) δ 7.35 (dd, *J =* 8.3, 5.3 Hz, 2 H), 7.10 (t, *J =* 8.6 Hz, 2 H), 5.61 (ddd, *J* = 46.4, 7.5, 4.5 Hz, 1 H), 3.68 (ddd, *J =* 15.0, 11.3, 7.6 Hz, 1 H), 3.59 (ddd, *J =* 24.2, 11.3, 4.4 Hz, 1 H).

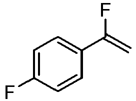

**1-Fluoro-4-(1-fluorovinyl)benzene** (Schlossera et al., Tetrahedron 2004, 7731-7742). To a flame-dried 25-mL round-bottom flask containing **1-(2-Bromo-1-fluoroethyl)-4-fluorobenzene** (2.66 g, 12.0 mmol) was added potassium tert-butoxide (1.49 g, 13.2 mmol) dissolved in distilled THF (6 mL). The mixture was stirred at room temperature for 2 h and filtered. The red-color filtrate was purified via distillation to give **1-fluoro-4-(1-fluorovinyl)benzene** (1.16 g, 8.27 mmol, 69%) as a colorless oil (bp 144-146 °C): ¹H NMR (500 MHz, CDCl₃) *δ* 7.54 (dd, *J* = 8.7, 5.3 Hz, 2 H), 7.07 (t, *J* = 8.4 Hz, 2 H), 4.96 (dd, *J* = 49.6, 3.6 Hz, 2 H), 4.83 (dd, *J =* 17.9, 3.6 Hz, 2 H).

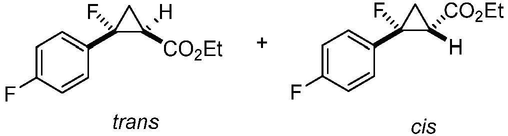

***cis-* and *trans*-Ethyl 2-fluoro-2-(4-fluorophenyl)cyclopropane-1-carboxylate** (Rosen et al., J. Med. Chem. 2004, 47:5860-5871). In a flame-dried 3-neck round-bottom flask equipped with a stir bar, reflux condenser, septum, and stopper, Cu(acac)₂ (64.9 mg, 0.248 mmol, 3 mol%) was dissolved in distilled CH₂Cl₂ (11 mL). The solution was stirred for several minutes. A few drops of phenylhydrazine were added and the stirring continued. **1-fluoro-4-(1-fluorovinyl)benzene** (1.16 g, 8.27 mmol) was added and the mixture was heated to reflux. A solution of ethyl diazoacetate (1.48 mL, 12.4 mmol) dissolved in distilled CH₂Cl₂ (22 mL) was added via syringe pump over 8 h. The solution was then refluxed for an additional 4 hr, after which it was cooled and diluted with CH₂Cl₂ (150 mL). The solution was washed with saturated NaHCO₃ solution and distilled water (300 mL). The organic layer was dried over anhydrous MgSO₄, filtered, and concentrated under reduced pressure. NMR analysis of the crude product revealed a conversion of 65%. Purification of the crude product with flash column chromatography on SiO₂ (1:1 CH₂Cl₂/hexanes) afforded a mixture of 1:1 cis/trans diastereomers as a yellow oil. The diastereomers were then separated by flash column chromatography on SiO₂ (1:2 CH₂Cl₂/hexanes) to give ***cis-* and *trans*-ethyl 2-fluoro-2-(4-fluorophenyl)cyclopropane-1-carboxylate** respectively as colorless oil: *cis*-(474 mg, 2.10 mmol, 25%); ¹H NMR (500 MHz, CDCl₃) *δ* 7.32 (dd, *J =* 8.1, 5.2 Hz, 2 H), 7.08 (t, *J =* 8.4 Hz, 2 H), 4.29-4.19 (m, 2 H), 2.28 (dt, *J =* 20.1, 7.4 Hz, 1 H), 2.15 (ddd, *J =* 9.5, 7.7, 2.7 Hz, 1 H), 1.59 (ddd, *J =* 10.5, 9.5, 7.1 Hz, 1 H), 1.30 (t, *J =* 7.1 Hz, 3 H); ¹³C NMR (75 MHz; CDCl₃) *δ* 167.9 (d, *J*_{C-F} = 2 Hz), 162.9 (dd, *J*_{C-F} = 248, 2 Hz), 133.4 (dd, *J*_{C-F} = 22, 3 Hz), 127.3 (dd, *J*_{C-F} = 8, 6 Hz), 115.8 (d, *J*_{C-F} = 22 Hz), 80.7 (d, *J*_{C-F} = 228 Hz), 61.4, 28.8 (d, *J*_{C-F} = 12 Hz), 18.8 (d, *J*_{C-F} = 13 Hz), 14.4; ¹⁹F NMR (CDCl₃, 470 MHz) δ -113.2 (s, 1 F), -184.9 (s, 1 F).

*trans*-(481 mg, 2.13 mmol, 26%); ¹H NMR (CDCl₃, 500 MHz) δ 7.48-7.44 (m, 2 H), 7.06 (t, *J =* 8.4 Hz, 2 H), 3.98-3.90 (m, 2 H), 2.56 (ddd, *J* = 17.8, 10.3, 7.6 Hz, 1 H), 1.95 (dt, *J =* 12.2, 7.3 Hz, 1 H), 1.82 (ddd, J= 19.2, 10.3, 7.1 Hz, 1 H), 1.04 (t, *J =* 7.1 Hz, 3 H); ¹³C NMR (CDCl₃, 125 MHz) δ 169.0 (d, *J*_{C-F} = 2 Hz), 163.3 (dd, *J*_{C-F} = 249, 3 Hz), 130.6 (dd, *J*_{C-F} = 8, 4 Hz), 129.3 (dd, *J*_{C-F} = 21, 3 Hz), 115.5 (dd, *J*_{C-F} = 22, 2 Hz), 82.6 (d, *J* _{C-F} = 221 Hz), 60.98, 27.9 (d, *J* _{C-F} = 17 Hz), 16.8 (d, *J* _{C-F} = 11 Hz), 14.14; ¹⁹F NMR (CDCl₃, 470 MHz) δ -111.8 (s, 1 F), -152.6 (s, 1 F).

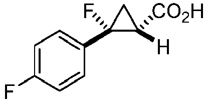

***cis*-2-Fluoro-2-(4-fluorophenyl)cyclopropane-1-carboxylic acid.²** To a solution of ***cis*-ethyl 2-fluoro-2-(4-fluorophenyl)cyclopropane-1-carboxylate** (0.200 g, 0.884 mmol) in methanol (1.8 mL) was added KOH (0.500 g, 8.84 mmol) dissolved in methanol (4.4 ml) at 0 °C. The reaction mixture was warm to room temperature and stirred for 16 h. The mixture was poured into water and extracted with CH₂Cl₂ (25 mL).

The organic layer was discarded and the aqueous layer was acidified with 6 M HCl and extracted with CH₂Cl₂ (2 x 25 mL). The combined organic layers were dried over anhydrous Na₂SO₄ and concentrated under reduced pressure to afford ***cis-*2-fluoro-2-(4-fluorophenyl)cyclopropane-1-carboxylic** acid (0.171 g, 0.864 mmol, 98%) as a white solid: ¹H NMR (CDCl₃, 500 MHz) δ 7.36-7.32 (m, 2 H), 7.12-7.07 (m, 2 H), 2.32 (dt, *J =* 20.1, 7.5 Hz, 1 H), 2.18 (ddd, *J =* 9.5, 7.5, 2.2 Hz, 1 H), 1.70 (ddd, *J =* 10.7, 9.4, 7.2 Hz, 1 H); ¹³C NMR (CDCl₃, 125 MHz) δ 173.0, 163.0 (dd, *J* _{C-F} = 248, 2 Hz), 132.8 (dd, *J*_{C-F} = 22, 3 Hz), 127.5 (dd, *J* _{C-F} = 9, 6 Hz), 115.9 (d, *J*_{C-F} = 22 Hz), 81.3 (d, *J* _{C-F} = 229 Hz), 28.3 (d, *J* _{C-F} = 12 Hz), 19.3 (d, *J* _{C-F} = 12 Hz).

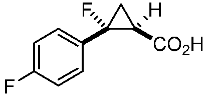

***trans*-2-Fluoro-2-(4-fluorophenyl)cyclopropane-1-carboxylic acid** (Rosen et al., J. Med. Chem. 2004, 47:5860-5871). To a solution of ***trans*-ethyl 2-fluoro-2-(4-fluorophenyl)cyclopropane-1-carboxylate** (0.200 g, 0.884 mmol) in methanol (1.8 mL) was added KOH (0.500 g, 8.84 mmol) dissolved in methanol (4.4 ml) at 0 °C. The reaction mixture was warm to room temperature and stirred for 16 h. The mixture was poured into water and extracted with CH₂Cl₂ (25 mL). The organic layer was discarded and the aqueous layer was acidified with 6 M HCl and extracted with CH₂Cl₂ (2 x 25 mL). The combined organic layers were dried over anhydrous Na₂SO₄ and concentrated under reduced pressure to afford ***trans*-2-fluoro-2-(4-fluorophenyl)cyclopropane-1-carboxylic acid** (0.175 g, 0.884 mmol, quant.) as a white solid: ¹H NMR (CDCl₃, 500 MHz) δ 7.45-7.41 (m, 2 H), 7.05 (t, *J =* 8.6 Hz, 2 H), 2.52 (ddd, *J =* 17.4, 10.0, 7.5 Hz, 1 H), 1.94-1.83 (m, 2 H); ¹³C NMR (CDCl₃, 100 MHz) δ 174.3, 163.4 (dd, *J*_{C-F} = 249, 3 Hz), 130.6 (dd, *J* _{C-F} = 9, 4 Hz), 128.6 (dd, *J* _{C-F} = 21, 3 Hz), 115.5 (d, *J* _{C-F} = 22 Hz), 83.1 (d, *J* _{C-F} = 222 Hz), 27.5 (d, *J* _{C-F} = 17 Hz), 17.5 (d, *J* _{C-F} = 10 Hz).

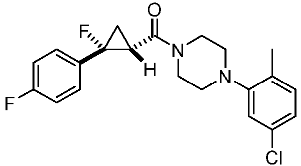

***cis*-(4-(5-Chloro-2-methylphenyl)piperazin-1-yl)((1SR,2RS)-2-fluoro-2-(4-fluorophenyl)cyclopropyl)-methanone.** To a solution of ***cis*-2-fluoro-2-(4-fluorophenyl)cyclopropane-1-carboxylic acid** (0.0500 g, 0.252 mmol) in distilled CH₂Cl₂ (2.5 mL) at 0 °C was added 1-(5-chloro-2-methylphenyl)piperazine hydrochloride (0.0750 g, 0.303 mmol) and triethylamine (0.11 mL, 0.757 mmol). The cooled solution was then treated with T3P (50 wt.% solution in EtOAc, 0.27 mL, 0.378 mmol) dropwise. The reaction mixture was stirred at 0 °C for 30 min and allowed to warm to room temperature and stirred for 16 h. The reaction was diluted with CH₂Cl₂ (30 mL) and washed with 1 M HCl (20 mL), saturated NaHCO₃ (20 mL), dried over anhydrous MgSO₄, filtered and concentrated in vacuo. The crude brown oil residue was purified via flash column chromatography on SiO₂ (2:3 EtOAc/hexanes) to afford ***cis*-(4-(5-chloro-2-methylphenyl)-piperazin-1-yl)((1SR,2RS)-2-fluoro-2-(4-fluorophenyl)cyclopropyl)methanone** (79.1 mg, 0.200 mmol, 79 %) as a white solid: Mp 110.2 - 110.8 °C (hexanes); IR (CDCl₃) 2918, 2819, 1646, 1593, 1516, 1430, 1223, 831, 809, 730 cm⁻¹; ¹H NMR (CDCl₃, 500 MHz) δ 7.26 (m, 2 H), 7.10 (t, *J* = 7.8 Hz, 3 H), 6.98 (dd, *J* = 8.1, 1.9 Hz, 1 H), 6.93 (d, *J =* 1.8 Hz, 1 H), 4.04-4.01 (m, 1 H), 3.67-3.57 (m, 3 H), 2.92-2.79 (m, 4 H), 2.39 (dt, *J* = 20.7, 7.6 Hz, 1 H), 2.25 (s, 3 H), 2.19 (ddd, *J* = 9.8, 7.9, 4.6 Hz, 1 H), 1.63-1.58 (m, 2 H); ¹³C NMR (CDCl₃, 150 MHz) δ 164.7, 162.6 (d, *J*_{C-F} = 248 Hz), 152.0, 133.9 (d, *J*_{C-F} = 22 Hz), 132.2 (d, *J* _{C-F} = 24 Hz), 132.0, 131.1, 125.6-125.5 (m), 123.8 (d, *J*_{C-F} = 25 Hz), 119.9 (d, *J*_{C-F} = 22 Hz), 116.2-115.9 (m), 79.9 (d, *J* _{C-F} = 222 Hz), 52.1, 51.7, 46.2, 43.0, 30.5 (d, *J*_{C-F} = 14 Hz), 17.7 (d, *J*_{C-F} = 12 Hz), 17.6; ¹⁹F NMR (CDCl₃, 470 MHz) δ -113.88 (s, 1 F), -188.55 (s, 1 F); HRMS (ESI) *m*/*z* calcd for C₂₁H₂₂ClF₂N₂O ([M+H]⁺) 391.1383, found 391.1386.

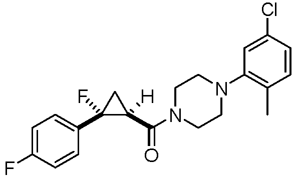

***trans-(4-(5-Chloro-2-methylphenyl)piperazin-1-yl)((1SR,2RS)-2-fluoro-2-(4-fluorophenyl)-***cyclopropyl)methanone. To a solution of ***trans*-2-fluoro-2-(4-fluorophenyl)cyclopropane-1-carboxylic** acid (0.0500 g, 0.252 mmol) in distilled CH₂Cl₂ (2.5 mL) at 0 °C was added 1-(5-chloro-2-methylphenyl)-piperazine hydrochloride (0.0750 g, 0.303 mmol) and triethylamine (0.11 mL, 0.757 mmol). The cooled solution was then treated with T3P (50 wt.% solution in EtOAc, 0.27 mL, 0.378 mmol) dropwise. The reaction mixture was stirred at 0 °C for 30 min and allowed to warm to room temperature and stirred for 16 h. The reaction was diluted with CH₂Cl₂ (30 mL) and washed with 1 M HCl (20 mL), saturated NaHCO₃ (20 mL), dried over anhydrous MgSO4, filtered and concentrated in vacuo. The crude brown oil residue was purified via flash column chromatography on SiO2 (2:3 EtOAc/hexanes) to afford *trans*-(4-(5-Chloro-2-methylphenyl)piperazin-1-yl)((1SR,2RS)-2-fluoro-2-(4-fluorophenyl)cyclopropyl)methanone (76.0 mg, 0.195 mmol, 77 %) as a colorless oil: IR (CDCl₃) 2922, 2855, 1641, 1593, 1517, 1432, 1225, 1193, 812, 735. ¹H NMR (CDCl₃, 500 MHz) δ 7.34-7.30 (m, 2 H), 7.10-7.06 (m, 3 H), 6.97 (dd, *J* = 8.1, 2.1 Hz, 1 H), 6.74 (d, *J =* 2.1 Hz, 1 H), 3.78-3.75 (m, 1 H), 3.66 (t, *J* = 5.0 Hz, 2 H), 3.40 (ddd, *J =* 12.4, 8.6, 3.3 Hz, 1 H), 2.85-2.81 (m, 1 H), 2.77-2.73 (m, 1 H), 2.68 (ddd, *J* = 18.8, 10.9, 7.9 Hz, 1 H), 2.38 (ddd, *J =* 11.5, 8.3, 3.0 Hz, 1 H), 2.30 (dt, 1 H, *J =* 11.4, 5.7 Hz), 2.22 (s, 3 H), 2.11 (dt, *J =* 12.3, 7.6 Hz, 1 H), 1.84 (ddd, *J* = 20.6, 10.8, 7.4 Hz, 1 h); ¹³C NMR (CDCl₃, 100 MHz) δ 165.3, 162.8 (d, *J*_{C-F} = 248 Hz,), 151.8, 132.1 (d, *J* _{C-F} = 21 Hz), 131.1, 130.6 (dd, *J* _{C-F} = 21, 3 Hz), 127.6 (d, *J*_{C-F} = 7 Hz), 127.5, 123.9, 119.8, 115.5 (d, *J* _{C-F} = 22 Hz), 81.5 (d, *J* _{C-F} = 222 Hz), 51.9, 51.6, 46.1, 42.5, 29.6 (d, *J* _{C-F} = 13 Hz), 17.5, 16.5 (d, *J* _{C-F} = 10 Hz); ¹⁹F NMR (CDCl₃, 470 MHz) δ -113.19 (s, 1 F), -164.01 (s, 1 F); HRMS (ESI) *m*/*z* calcd for C₂₁H₂₂ClF₂N₂O ([M+H]⁺) 391.1383, found 391.1384.

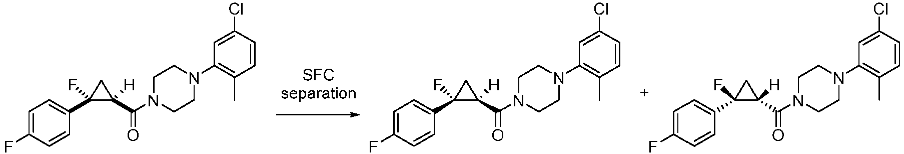

Racemic ***trans*-(4-(5-Chloro-2-methylphenyl)piperazin-1-yl)((1SR,2RS)-2-fluoro-2-(4-fluorophenyl)-cyclopropyl)methanone** was separated on a SFC Chiralpak-IC semiprep (250 x 10 mm) column (30% MeOH, 7 mL/min, 220 nM, P=100) to afford **(-)-(4-(5-Chloro-2-methylphenyl)piperazin-1-yl)(-2-fluoro-2-(4-fluorophenyl)cyclopropyl)methanone** (retention time 4.76 min) as a colorless oil (100% purity by ESLD): [a]²⁰_{D} -141.9 (c 0.84, MeOH); ¹H NMR (CDCl₃, 500 MHz) δ 7.35-7.31 (m, 2 H), 7.10-7.06 (m, 3 H), 6.97 (dd, *J =* 8.1, 2.1 Hz, 1 H), 6.75 (d, *J* = 2.1 Hz, 1 H), 3.78-3.74 (m, 1 H), 3.69-3.64 (m, 2 H), 3.43-3.38 (m, 1 H), 2.85-2.81 (m, 1 H), 2.77-2.73 (m, 1 H), 2.68 (ddd, *J* = 18.8, 10.9, 7.9 Hz, 1 H), 2.41-2.37 (m, 1 H), 2.31 (td, *J =* 11.0, 5.4 Hz, 1 H), 2.22 (s, 3 H), 2.11 (dt, *J =* 12.3, 7.6 Hz, 1 H), 1.84 (ddd, *J =* 20.5, 10.8, 7.4 Hz,1 H); HRMS (ESI) *m*/*z* calcd for C₂₁H₂₂ClF₂N₂O ([M+H]⁺) 391.1383, found 391.1379. The enantiomeric excess was >99% ee (SFC Chiralpak-IC (250 x 10 mm); 30% MeOH, 220 nm, 7 mL/min; retention time: 4.77 min).

**(+)-(4-(5-Chloro-2-methylphenyl)piperazin-1-yl)(-2-fluoro-2-(4-fluorophenyl)cyclopropyl)-methanone** (retention time 5.60 min) was obtained as a colorless oil (100% purity by ESLD): [a]²⁰_{D} +145.1 (c 0.86, MeOH); ¹H NMR (CDCl₃, 500 MHz) δ 7.34-7.31 (m, 2 H), 7.10-7.06 (m, 3 H), 6.97 (dd, *J* = 8.1, 2.1 Hz, 1 H), 6.75 (d, *J =* 2.0 Hz, 1 H), 3.79-3.74 (m, 1 H), 3.69-3.65 (m, 2 H), 3.43-3.38 (m, 1 H), 2.85-2.81 (m, 1 H), 2.77-2.73 (m, 1 H), 2.68 (ddd, *J* = 18.9, 10.9, 8.0 Hz, 1 H), 2.39 (ddd, *J =* 11.2, 8.8, 2.6 Hz, 1 H), 2.31 (dt, *J =* 11.3, 5.8 Hz, 1 H), 2.22 (s, 3 H), 2.11 (dt, *J =* 12.3, 7.6 Hz, 1 H), 1.84 (ddd, *J =* 20.5, 10.8, 7.4 Hz, 1 H); HRMS (ESI) *m*/*z* calcd for C₂₁H₂₂ClF₂N₂O ([M+H]⁺) 391.1383, found 391.1374. The enantiomeric excess was >99% ee (SFC Chiralpak-IC (250 x 10 mm); 30% MeOH, 220 nm, 7 mL/min; retention time: 5.60 min).

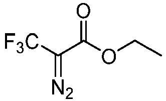

**Ethyl 2-diazo-3,3,3-trifluoropropanoate** (Bartrum et al., Chem. Eur. J. 2011, 17:9586-9589; Shi et al., J. Org. Chem. 1990, 55:3383-3386). To a stirred solution of p-toluenesulfonyl hydrazide (11.5 g, 60.5 mmol) in distilled CH₂Cl₂ (65 mL) was added ethyl trifluoropyruvate (7.79 mL, 57.6 mmol). The reaction was stirred at room temperature for 18 h. Phosphorus oxychloride (7.05 mL, 74.9 mmol) was added dropwise followed by pyridine (6.11 mL, 74.9 mmol) (Note: when pyridine was added dropwise the reaction mixture warmed up to a self-sustaining gentle reflux). The reaction mixture was stirred at room temperature for another 18 h. The mixture was washed with water and the organic layer was separated. The aqueous layer was extracted with CH₂Cl₂ (3 x 100 mL). The combined organic layers were washed with brine, dried over anhydrous MgSO₄, filtered and concentrated in vacuo to give **ethyl 3,3,3-trifluoro-2-(2-tosylhydrazineylidene)propanoate** as a yellow liquid which solidified to a white solid upon standing. The crude product was used in the next step without further purification.

To a solution of **ethyl 3,3,3-trifluoro-2-(2-tosylhydrazineylidene)propanoate** (18.3 g, 54.1 mmol) in anhydrous CH₂Cl₂ (433 mL) was added triethylamine (15.2 mL, 0.108 mol) dropwise. The reaction mixture was stirred at room temperature for 2 days. The solution was carefully concentrated in vacuo and the crude product was purified via distillation. The distillate was washed with 1 M HCl (20 mL x 2) to remove the excess triethylamine. The organic layers collected were dried over anhydrous MgSO₄, filtered and carefully concentrated in vacuo to afford **ethyl 2-diazo-3,3,3-trifluoropropanoate** (4.89 g, 26.9 mmol, 50 % over 2 steps) as a yellow oil: Bp lit: 60 - 62 °C (100 mmHg)⁴; ¹H NMR (CDCl₃, 500 MHz) δ 4.32 (q, *J* = 7.1 Hz, 2 H), 1.32 (t, *J =* 7.1 Hz, 3 H); ¹³C NMR (CDCl₃, 125 MHz) δ 161.0, 122.8 (q, *J*_{C-F} = 269 Hz), 62.3, 14.4 (C=N signal not observed). 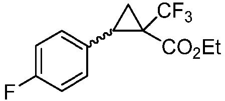 ***cis-*** and ***trans*-Ethyl 2-(4-fluorophenyl)-1-(trifluoromethyl)cyclopropane-1-carboxylate.** To a flame-dried flask containing 4-fluorostyrene (3.3 mL, 27.5 mmol) and Rh₂(OAc)₄ (0.121 g, 0.275 mmol, 5 mol%) in distilled CH₂Cl₂ (25 mL) was added ethyl **2-diazo-3,3,3-trifluoropropanoate** (1.00 g, 5.49 mmol) in distilled CH₂Cl₂ (20 mL) via a syringe pump over 16 h at room temperature. After consumption of the diazo compound by TLC, the mixture was passed through a pad of silica gel to remove the rhodium catalyst and washed with CH₂Cl₂. The solvent was removed in vacuo. The crude product was purified by column chromatography (1:2 CH₂Cl₂/hexanes) to give an inseparable mixture of ***cis-* and *trans*-ethyl 2-(4-fluorophenyl)-1-(trifluoromethyl)cyclopropane-1-carboxylate** (cis/trans ratio 1: 1.6) as an yellow oil *(Note: The mixture also contained side product from the dimerization of the diazo compound): **cis-*** (597 mg, 2.16 mmol, 34 % calcd yield); ¹H NMR (CDCl₃, 500 MHz) δ 7.26 (m, 2 H), 7.00 (t*, J =* 8.7 Hz, 2 H), 4.33-4.25 (m, 2 H), 3.04 (t, *J* = 8.9 Hz, 1 H), 1.95 (ddq, *J =* 9.5, 5.4, 1.8 Hz, 1 H), 1.88 (dd, *J =* 8.2, 5.3 Hz, 1 H), 1.34 (t, *J* = 7.1 Hz, 3 H); ¹⁹F NMR (CDCl₃, 470 MHz) δ -61.1 (s, 3 F), -114.5 (s, 1 F).

*trans-* (955 mg, 3.46 mmol, 54 % calcd yield); ¹H NMR (CDCl₃, 500 MHz) δ 7.22 (dd, *J =* 8.2, 5.4 Hz, 2 H), 6.98 (t, *J =* 8.7 Hz, 2 H), 3.96-3.87 (m, 2 H), 2.90 (t, *J =* 9.0 Hz, 1 H), 2.14-2.11 (m, 1 H), 1.78 (dd, *J =* 9.7, 5.7 Hz, 1 H), 0.94 (t, *J* = 7.1 Hz, 3 H); ¹⁹F NMR (CDCl₃, 470 MHz) δ -66.8 (s, 3 F), -114.5 (s, 1 F). 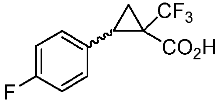 ***cis-* and *trans*-2-(4-Fluorophenyl)-1-(trifluoromethyl)cyclopropane-1-carboxylic acid.** To a solution of to KOH (1.02 g, 18.1 mmol) in methanol (9 ml) was added ***cis-* and *trans*-ethyl 2-(4-fluorophenyl)-1-(trifluoromethyl)cyclopropane-1-carboxylate** (0.500 g, 1.81 mmol) dissolved in methanol (3.8 mL). The reaction mixture was heated to 55 °C and stirred at this temperature for 24 h. The mixture was cooled to room temperature and poured into water and extracted with CH₂Cl₂ (25 mL). The organic layer was discarded and the aqueous layer acidified with 6 M HCl and extracted with CH₂Cl₂ (2 x 25 mL). The combined organic layers were dried over anhydrous Na₂SO₄ and concentrated under reduced pressure to give a mixture of ***cis-* and trans-2-(4-Fluorophenyl)-1-(trifluoromethyl)cyclopropane-1-carboxylic acid** as a pale yellow solid (cis/trans ratio 1:1.16) which was used in the next step without purification: ***cis-*** (168 mg, 0.677 mmol, 37 % calcd yield); ¹H NMR (CDCl₃, 500 MHz) δ 7.29-7.26 (m, 2 H), 7.02 (t, *J =* 8.6 Hz, 2 H), 3.16 (t, *J =* 9.0 Hz, 1 H), 2.04-1.97 (m, 2 H); ¹⁹F NMR (CDCl₃, 471 MHz) δ -61.5 (s, 3 F), -114.0 (s, 1 F).

***trans-*** (262 mg, 1.06 mmol, 58 % calcd yield); ¹H NMR (CDCl₃, 500 MHz) δ 7.18 (dd, *J =* 8.4, 5.4 Hz, 2 H), 6.97 (t, *J =* 8.6 Hz, 2 H), 2.99 (t, *J =* 9.0 Hz, 1 H), 2.09 (t, *J =* 6.5 Hz, 1 H), 1.84 (dd, *J =* 9.7, 5.8 Hz, 1 H); ¹⁹F NMR (CDCl₃, 470 MHz) δ -66.9 (s, 3 F), -114.1 (s, 1 F). 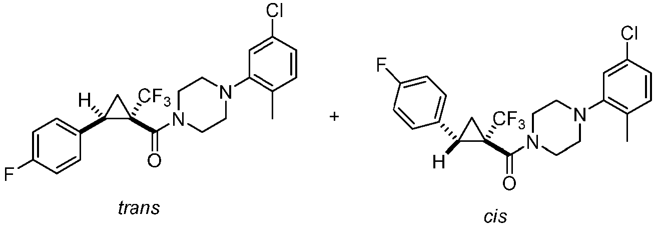 ***cis-* and *trans*-(4-(5-Chloro-2-methylphenyl)piperazin-1-yl)(2-(4-fluorophenyl)-1-(trifluoromethyl)-cyclopropyl)methanone.** To a suspension of ***cis-* and *trans*-2-(4-Fluorophenyl)-1-(trifluoromethyl)-cyclopropane-1-carboxylic acid** (0.250 g, 1.01 mmol) in distilled CH₂Cl₂ (10 mL) cooled to 0 °C was added oxalyl chloride (0.18 mL, 2.01 mmol) followed by a catalytic amount of DMF and the reaction was gradually warmed to room temperature and stirred for another 2 h. The reaction was concentrated in vacuo (water bath 25°C) to afford the yellow crude acid chloride. The crude residue formed above was dissolved in distilled CH₂Cl₂ (10 mL) cooled to 0°C and added with 1-(5-chloro-2-methylphenyl)piperazine hydrochloride (0.349 g, 1.41 mmol) and Et₃N (0.50 mL, 3.60 mmol) and the reaction was allowed to warm to room temperature and stirred for 20 h. LCMS and NMR analysis showed presence of starting material carboxylic acid (possibly due to hydrolysis of the acyl chloride). The reaction mixture was cooled to 0 °C and T3P (50wt.% in EtOAc, 0.85 mL, 1.21 mmol) was added to couple the remaining of the carboxylic acid to the piperazine. The mixture was stirred at room temperature for 3 days until no starting material was seen in LCMS. The reaction was diluted with CH₂Cl₂ (30 mL) and washed with saturated NaHCO₃ (20 mL), dried over anhydrous MgSO₄, filtered and concentrated in vacuo. The crude oil residue was dissolved purified via flash column chromatography on SiO₂ (1:2 Et₂O/hexanes) to afford the ***cis-* and *trans*-(4-(5-Chloro-2-methylphenyl)piperazin-1-yl)(2-(4-fluorophenyl)-1-(trifluoromethyl)cyclopropyl)methanone** respectively as yellow oil: *cis-* (107 mg, 0.244 mmol, 24%); IR (CDCl₃) 2926, 1647, 1515, 1438, 1226, 1153, 1125, 842, 738 cm⁻¹; ¹H NMR (CDCl₃, 500 MHz) δ 7.32 (dd, *J =* 8.5, 5.4 Hz, 2 H), 7.12 (d, *J =* 8.1 Hz, 1 H), 7.05-7.00 (m, 3 H), 6.96 (d, *J =* 2.1 Hz, 1 H), 3.88 (br s, 4 H), 2.94 (br s, 4 H), 2.79 (t, *J* = 8.7 Hz, 1 H), 2.30 (s, 3 H), 1.98 (dd, *J =* 7.9, 6.2 Hz, 1 H), 1.73-1.69 (m, 1 H); ¹³C NMR (CDCl₃, 151 MHz) δ **164.6,** 162.3 (d, *J* = 246 Hz), 151.8, 132.3, 132.1, 131.1, 130.7 (d, *J*_{C-F} = 8 Hz), 129.3 (d, *J* _{C-F} = 3 Hz), 124.7 (q, *J* _{C-F} = 276 Hz), 124.0, 120.0, 115.5 (d, *J*_{C-F} = 21 Hz), 51.8, 47.1, 43.4, 34.8 (q, *J* _{C-F} = 32 Hz), 27.6, 17.6, 14.8 ; ¹⁹F NMR (CDCl₃, 470 MHz) δ -60.7 (s, 3 F), -114.8 (s, 1 F); HRMS (ESI) *m*/*z* calcd for C₂₂H₂₂ClF₄N₂O ([M+H]⁺) 441.1351, found 441.1352.

***trans-*** (151 mg, 0.342 mmol, 34 %); IR (CDCl₃) 2925, 2860, 1643, 1516, 1437, 1226, 1149, 1132, 845, 812, 733 cm⁻¹; ¹H NMR (CDCl₃, 500 MHz) δ 7.12-7.04 (m, 5 H), 6.95 (dd, *J =* 8.1, 2.1 Hz, 1 H), 6.65 (d, *J =* 1.7 Hz, 1 H), 3.77 (s, 1 H), 3.49-3.47 (m, 2 H), 3.03 (s, 1 H), 2.80 (dd, *J* = 9.8*,* 7.5 Hz, 1 H), 2.74-2.71 (m, 1 H), 2.53-2.42 (m, 2 H), 2.16 (s, 3 H), 1.87 (t, *J=* 7.0 Hz, 1 H), 1.79 (dd, *J* = 9.8, 6.6 Hz, 1 H), 1.51 (s, 1 H); ¹³C NMR (CDCl₃, 101 MHz) δ 162.6 (d, *J*_{C-F} = 248 Hz), 161.7, 151.7, 132.1, 132.0, 131.1, 130.9 (d, *J*_{C-F} = 3 Hz), 128.8 (d, *J* _{C-F} = 8 Hz), 124.6 (q, *J* _{C-F} = 272 Hz), 123.9, 119.8, 116.0 (d, *J*_{C-F} = 22 Hz), 51.3, 50.9, 46.8, 43.2, 36.8 (q, *J*_{C-F} = 33 Hz), 27.3, 17.4, 15.5; ¹⁹F NMR (CDCl₃, 470 MHz) δ -66.3 (s, 3 F), -114.0 (s, 1 F); HRMS (ESI) *m*/*z* calcd for C₂₂H₂₂ClF₄N₂O ([M+H]⁺) 441.1351, found 441.1349.

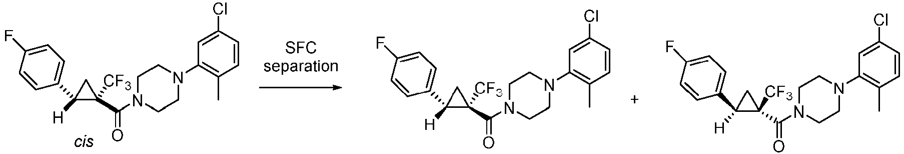

Racemic ***cis*-(4-(5-chloro-2-methylphenyl)piperazin-1-yl)((1SR,2SR)-2-(4-fluorophenyl)-1-(trifluoromethyl)cyclopropyl)methanone** was separated on a SFC Chiralpak-IC semiprep (250 x 10 mm) column (10% MeOH, 6 mL/min, 220 nM, P=100) to afford **(-)-(4-(5-chloro-2-methylphenyl)piperazin-1-yl)(-2-(4-fluorophenyl)-1-(trifluoromethyl)cyclopropyl)methanone** (retention time 14.46 min) as a colorless oil which foamed up upon drying (100% purity by ESLD): [a]²⁰_{D} -57.3 (c 0.44, MeOH); ¹H NMR (CDCl₃, 500 MHz) δ 7.32 (dd, *J* = 8.5, 5.4 Hz, 2 H), 7.13 (d, *J =* 8.1 Hz, 1 H), 7.05-7.00 (m, 3 H), 6.96 (d, *J* = 2.1 Hz, 1 H), 3.89-3.88 (m, 4 H), 2.94 (s, 4 H), 2.79 (t, *J =* 8.7 Hz, 1 H), 2.30 (s, 3 H), 1.98 (dd, *J* = 7.9, 6.2 Hz, 1 H), 1.71 (ddq, *J* = 9.5, 6.0, 1.8 Hz, 1 H). HRMS (ESI) *m*/*z* calcd for C₂₂H₂₂ClF₄N₂O ([M+H]⁺) 441.1348, found 441.1351. The enantiomeric excess was >99% ee (SFC Chiralpak-IC (250 x 10 mm); 10% MeOH, 220 nm, 6 mL/min; retention time: 14.55 min).

**(+)-(4-(5-chloro-2-methylphenyl)piperazin-1-yl)(-2-(4-fluorophenyl)-1-(trifluoromethyl)-cyclopropyl)methanone** (retention time 16.17 min) was obtained as a colorless oil which foamed up upon drying (100% purity by ESLD): [a]²⁰_{D} +58.4 (c 0.42, MeOH); ¹H NMR (CDCl₃, 500 MHz) δ 7.32 (dd, *J* = 8.5, 5.3 Hz, 2 H), 7.13 (d, *J* = 8.1 Hz, 1 H), 7.05-7.00 (m, 3 H), 6.96 (d, *J* = 2.1 Hz, 1 H), 3.89-3.88 (m, 4 H), 2.94 (s, 4 H), 2.79 (t, *J =* 8.7 Hz, 1 H), 2.30 (s, 3 H), 1.98 (dd, *J =* 7.9, 6.2 Hz, 1 H), 1.71 (ddq, *J* = 9.6, 6.0, 1.9 Hz, 1 H); HRMS (ESI) *m*/*z* calcd for C₂₂H₂₂ClF₄N₂O ([M+H]⁺) 441.1348, found 441.1351. The enantiomeric excess was >99% ee (SFC Chiralpak-IC (250 x 10 mm); 10% MeOH, 220 nm, 6 mL/min; retention time: 16.31 min).

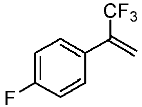

**1-fluoro-4-(3,3,3-trifluoroprop-1-en-2-yl)benzene** (Miura et al., Chem. Lett. 2008, 37:1006-1007; Kobayashi et al., J. Fluorine Chem. 2009, 130:591-594). To a solution of 1,2-dibromo-3,3,3-trifluoropropane (6.35 mL, 52.0 mmol) in THF/DME/H2O (1:1:1, 105 mL), 4-fluorophenylboronic acid (5.00 g, 34.7 mmol), PdCl₂(PPh₃)₂ (0.973 g, 1.39 mmol), AsPh₃ (1.64 g, 5.20 mmol), and KOH (11.7 g, 0.208 mol) were added under an inert atmosphere. The reaction mixture was stirred in an ice bath for 10 min, and gradually warmed up to room temperature and then heated at 75 °C for 13 h. After completion of reaction, water (200 mL) was added, then the mixture was extracted with diethyl ether (2 x 200 mL). The combined organic layers were washed with brine (300 mL) and dried over anhydrous MgSO₄. The solvent was carefully removed in vacuo. The crude material was purified via vacuum distillation to give **1-fluoro-4-(3,3,3-trifluoroprop-1-en-2-yl)benzene** (5.25 g, 82 %) as a colorless oil: Bp 53 °C @ 20 mmHg; ¹H NMR (CDCl₃, 500 MHz) δ 7.43 (dd, *J =* 8.6, 5.5 Hz, 2 H), 7.10-7.05 (m, 2 H), 5.95 (d, *J =* 1.2 Hz, 1 H), 5.73 (q, *J* = 1.6 Hz, 1 H); ¹³C NMR (CDCl₃, 125 MHz) δ 163.4 (d, *J*_{C-F} = 249 Hz), 138.3 (q, *J* _{C-F} = 30 Hz), 129.9 (d, *J* _{C-F} = 4 Hz), 129.5 (d, *J* _{C-F} = 8 Hz), 123.4 (q, *J* _{C-F} = 274 Hz), 120.6 (q, *J*_{C-F} = 6 Hz), 115.7 (d, *J* _{C-F} = 22 Hz); ¹⁹F NMR (CDCl₃, 471 MHz) δ -65.1 (s, 3 F), -112.5 (s, 1 F).

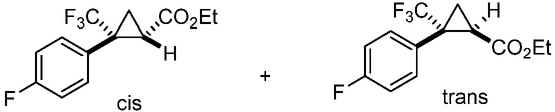

***cis-* and *trans*-Ethyl-2-(4-fluorophenyl)-2-(trifluoromethyl)cyclopropane-1-carboxylate.** To a flame-dried round-bottom flask was added Rh₂(OAc)₄ (128 mg, 0.290 mmol, 5 mol%) and **1-fluoro-4-(3,3,3-trifluoroprop-1-en-2-yl)benzene** (1.20 g, 5.81 mmol) in distilled CH₂Cl₂ (5 mL). A solution of ethyl diazoacetate (1.04 mL, 8.71 mmol) in CH₂Cl₂ (18 mL) was added via syringe pump over 16 h at room temperature. After addition of the diazo compound, TLC analysis showed that there was still presence of the alkene. Another 2 mol% of the Rh₂(OAc)₄ catalyst was added and 0.5 equiv of diazo compound dissolved in CH₂Cl₂ (10 mL) was added dropwise over 9 h. The mixture was filtered through a silica gel plug and washed with CH₂Cl₂. The solution was concentrated under reduced pressure and the crude mixture was purified via flash column chromatography on SiO₂ (1:2 CH₂Cl₂/hexanes) to afford ***cis-* and *trans*-ethyl-2-(4-fluorophenyl)-2-(trifluoromethyl)cyclopropane-1-carboxylate** respectively as yellow oils: *cis-* (554 mg, 2.01 mmol, 35%); ¹H NMR (CDCl₃, 500 MHz) δ 7.47 (dd, *J* = 8.6, 5.3 Hz, 2 H), 7.08-7.02 (m, 2 H), 4.32-4.18 (m, 2 H), 2.23 (t, *J =* 8.1 Hz, 1 H), 2.04 (dd, *J =* 7.2, 5.9 Hz, 1 H), 1.46 (ddq, *J* = 8.9, 5.7, 1.7 Hz, 1 H), 1.31 (t, *J =* 7.1 Hz, 3 H); ¹³C NMR (CDCl₃, 125 MHz) δ 168.0, 163.0 (d, *J* _{C-F} = 248 Hz), 132.8 (d, *J* _{C-F} = 9 Hz), 131.8 (d, *J* _{C-F} = 3 Hz), 125.2 (q, *J* _{C-F} = 276 Hz), 115.7 (d, *J* _{C-F} = 22 Hz), 61.7, 35.0 (q, *J* _{C-F} = 34 Hz), 27.5, 14.5 (q, *J* _{C-F} = 2 Hz), 14.2; ¹⁹F NMR (CDCl₃, 471 MHz) δ -65.6 (s, 3 F), -112.4 (s, 1 F).

***trans-*** (662 mg, 2.40 mmol, 41%); ¹H NMR (CDCl₃, 500 MHz) δ 7.34 (dd, *J* = 8.6, 5.3 Hz, 2 H), 7.05-7.00 (m, 2 H), 4.01-3.91 (m, 2 H), 2.48 (dd, *J* = 8.8, 6.3 Hz, 1 H), 1.83 (tq, *J* = 5.8, 1.8 Hz, 1 H), 1.72 (dd, *J =* 8.8, 5.5 Hz, 1 H), 1.07 (t, *J =* 7.1 Hz, 3 H); ¹³C NMR (CDCl₃, 100 MHz) δ 168.7, 163.0 (d, *J* _{C-F} = 248 Hz), 133.0 (d, *J* _{C-F} = 9 Hz), 127.4 (d, *J* _{C-F} = 3 Hz), 125.0 (q, *J* _{C-F} = 274 Hz), 115.6 (d, *J* _{C-F} = 22 Hz), 61.3, 35.2 (q, *J* _{C-F} = 34 Hz), 23.8 (q, *J* _{C-F} = 2 Hz), 14.7 (q, *J* _{C-F} = 2 Hz), 14.1; ¹⁹F NMR (CDCl₃, 471 MHz) δ -70.7 (s, 3 F), -112.4 (s, 1 F). A fraction containing a mixture of both diastereomers was also obtained (192 mg, 0.694 mmol, 12%).

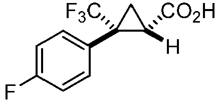

***cis*-2-(4-fluorophenyl)-2-(trifluoromethyl)cyclopropane-1-carboxylic acid.** To a solution of KOH (0.463 g, 8.25 mmol) in methanol (3.6 ml) was added ***cis*-ethyl-2-(4-fluorophenyl)-2-(trifluoromethyl)-cyclopropane-1-carboxylate** (0.228 g, 0.825 mmol) dissolved in methanol (1.5 mL) at 0 °C. The reaction mixture was gradually warm to room temperature and stirred for 12 h. The mixture was poured into water and extracted with CH₂Cl₂ (25 mL). The organic layer was discarded and the aqueous layer acidified with 6 M HCl and extracted with CH₂Cl₂ (2 x 25 mL). The combined organic layers were dried over anhydrous Na₂SO₄ and concentrated under reduced pressure to give the cis-2-(4-fluorophenyl)-2-(trifluoromethyl)-cyclopropane-1-carboxylic acid (0.202 g, 0.812 mmol, 98 %) as a white solid: ¹H NMR (CDCl₃, 500 MHz) δ 7.47 (dd, *J =* 8.6, 5.2 Hz, 2 H), 7.09-7.02 (m, 2 H), 2.28 (t, *J =* 8.0 Hz, 1 H), 2.09 (dd, *J =* 7.3, 5.9 Hz, 1 H), 1.57-1.53 (m, 1 H); ¹⁹F NMR (CDCl₃, 471 MHz) δ -65.3 (s, 3 F), -112.1 (s, 1 F).

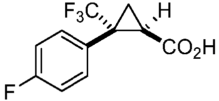

***trans*-2-(4-fluorophenyl)-2-(trifluoromethyl)cyclopropane-1-carboxylic acid.** To a solution of KOH (0.406 g, 7.24 mmol) in methanol (3.6 ml) was added ***trans*-ethyl-2-(4-fluorophenyl)-2-(trifluoromethyl)-**cyclopropane-1-carboxylate (0.200 g, 0.724 mmol) dissolved in methanol (1.5 mL) at 0 °C. The reaction mixture was gradually warm to room temperature and stirred for 12 h. The mixture was poured into water and extracted with CH₂Cl₂ (25 mL). The organic layer was discarded and the aqueous layer acidified with 6 M HCl and extracted with CH₂Cl₂ (2 x 25 mL). The combined organic layers were dried over anhydrous Na₂SO₄ and concentrated under reduced pressure to give the ***trans*-2-(4-fluorophenyl)-2-(trifluoromethyl)-cyclopropane-1-carboxylic acid** (0.181 g, 0.731 mmol, 98 %) as a white solid: ¹H NMR (CDCl₃, 500 MHz) δ 7.32 (dd, *J =* 8.3, 5.4 Hz, 2 H), 7.02 (t, *J =* 8.6 Hz, 2 H), 2.49-2.45 (m, 1 H), 1.77 (dq, *J =* 8.3, 2.9 Hz, 2 H); ¹⁹F NMR (CDCl₃, 471 MHz) δ -70.9 (s, 3 F), -112.1 (s, 1 F).

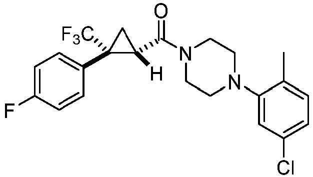

***cis*-(4-(5-Chloro-2-methylphenyl)piperazin-1-yl)((1SR,2RS)-2-(4-fluorophenyl)-2-(trifluoromethyl)-cyclopropyl)methanone.** A solution of ***cis*-2-(4-fluorophenyl)-2-(trifluoromethyl)cyclopropane-1-**carboxylic acid (0.100 g, 0.403 mmol) and 1-(5-chloro-2-methylphenyl)piperazine hydrochloride (0.120 g, 0.484 mmol) in distilled CH₂Cl₂ (4 mL) was cooled to 0 °C and then added with triethylamine (0.168 mL, 1.21 mmol). The cooled solution was then treated with T3P (50wt.% solution in EtOAc), 0.43 mL, 0.604 mmol) dropwise. The reaction mixture was stirred at 0 °C for 30 min and allowed to warm to room temperature and stirred for 18 h. After completion of the reaction by TLC analysis, the reaction was diluted with CH₂Cl₂ (30 mL) and washed with 1 M HCl (30 mL), saturated NaHCO₃ solution (20 mL), dried over anhydrous MgSO₄, filtered and concentrated in vacuo. The brown crude oil residue was purified via automated flash column chromatography on SiO₂ (100 hexanes - 40% EtOAc/hexanes gradient) to afford

***cis*-(4-(5-chloro-2-methylphenyl)piperazin-1-yl)((1SR,2RS)-2-(4-fluorophenyl)-2-(trifluoromethyl)-cyclopropyl)methanone** (108 mg, 0.245 mmol, 61%) as a colorless oil which foamed up upon drying: IR (CDCl₃) 2917, 2825, 1651, 1593, 1513, 1435, 1227, 1159, 1137, 835, 727 cm⁻¹; ¹H NMR (CDCl₃, 500 MHz) δ 7.47 (dd, *J =* 8.7, 5.2 Hz, 2 H), 7.14-7.06 (m, 3 H), 7.00 (dd, *J =* 8.1, 2.1 Hz, 1 H), 6.97 (d, *J =* 2.1 Hz, 1 H), 3.99-3.87 (m, 3 H), 3.81-3.76 (m, 1 H), 3.01 (t, *J =* 5.0 Hz, 2 H), 2.94-2.86 (m, 2 H), 2.31 (s, 3 H), 2.22-2.17 (m, 2 H), 1.60-1.56 (m, 1 H); ¹³C NMR (CDCl₃, 125 MHz) δ 164.9, 162.9 (d, *J* _{C-F} = 249 Hz), 152.0, 132.3, 132.0, 131.8 (d, *J* _{C-F} = 8 Hz), 131.1, 125.6 (q, *J*_{C-F} = 276 Hz), 123.9, 119.9, 116.0 (d, *J* _{C-F} = 22 Hz), 51.7, 51.6, 46.3, 42.9, 35.1 (q, *J* _{C-F} = 34 Hz), 28.1, 17.60, 14.55; ¹⁹F NMR (CDCl₃, 471 MHz) δ -64.7 (s, 3 F), -112.2 (s, 1 F); HRMS (ESI) *m*/*z* calcd for C₂₂H₂₂ClF₄N₂O ([M+H]⁺) 441.1351, found 441.1350.

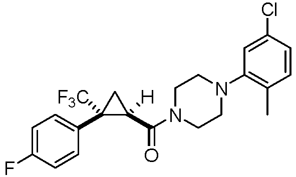

***trans*-(4-(5-chloro-2-methylphenyl)piperazin-1-yl)((1RS,2RS)-2-(4-fluorophenyl)-2-(trifluoromethyl)-cyclopropyl)methanone.** A solution of ***trans*-2-(4-fluorophenyl)-2-(trifluoromethyl)cyclopropane-1-carboxylic acid** (0.100 g, 0.403 mmol) and 1-(5-chloro-2-methylphenyl)piperazine hydrochloride (0.120 g, 0.484 mmol) in distilled CH₂Cl₂ (4 mL) was cooled to 0 °C and then added with triethylamine (0.168 mL, 1.21 mmol). The cooled solution was then treated with T3P (50wt.% solution in EtOAc), 0.43 mL, 0.604 mmol) dropwise. The reaction mixture was stirred at 0 °C for 30 min and allowed to warm to room temperature and stirred for 18 h. After completion of the reaction by TLC analysis, the reaction was diluted with CH₂Cl₂ (30 mL) and washed with 1 M HCl (30 mL), saturated NaHCO₃ solution (20 mL), dried over anhydrous MgSO₄, filtered and concentrated in vacuo. The brown crude oil residue was purified via automated flash column chromatography on SiO₂ (100 hexanes - 40% EtOAc/hexanes gradient) to afford trans-(4-(5-chloro-2-methylphenyl)piperazin-1-yl)((1SR,2RS)-2-(4-fluorophenyl)-2-(trifluoromethyl)-cyclopropyl)methanone (162 mg, 0.367 mmol, 91%) as a colorless oil which foamed up upon drying: IR (CDCl₃) 2894, 2820, 1652, 1593, 1514, 1436, 1296, 1226, 1160, 1143, 830, 735; ¹H NMR (CDCl₃, 500 MHz) δ 7.31 (dd, *J =* 8.6, 5.3 Hz, 2 H), 7.13 (d, *J =* 8.1 Hz, 1 H), 7.06-7.00 (m, 3 H), 6.96 (d, *J =* 2.0 Hz, 1 H), 3.99-3.86 (m, 2 H), 3.70-3.56 (m, 2 H), 3.07-2.99 (m, 2 H), 2.86-2.75 (m, 2 H), 2.59 (dd, *J =* 8.8, 6.2 Hz, 1 H), 2.30 (s, 3 H), 2.12-2.09 (m, 1 H), 1.70 (dd, *J* = 8.8, 5.3 Hz, 1 H); ¹³C NMR (CDCl₃, 101 MHz) δ 165.3, 162.9 (d, *J*_{C-F} = 248 Hz), 151.8, 132.8 (d, *J* _{C-F} = 8 Hz), 132.3, 132.1, 131.1, 127.1 (d, *J*_{C-F} = 3 Hz), 125.4 (q, *J*_{C-F} = 274 Hz), 124.0, 119.8, 115.8 (d, *J*_{C-F} = 22 Hz), 52.1, 51.7, 46.2, 42.8, 34.9 (q, *J*_{C-F} = 33 Hz), 22.8, 17.6, 13.9. ¹⁹F NMR (CDCl₃, 471 MHz) δ -69.6 (s, 3 F), -112.4 (s, 1 F); HRMS (ESI) *m*/*z* calcd for C₂₂H₂₂ClF₄N₂O ([M+H]⁺) 441.1351, found 441.1351.

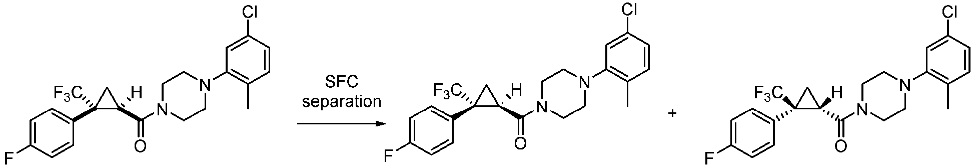

Racemic ***trans*-(4-(5-chloro-2-methylphenyl)piperazin-1-yl)((1SR,2RS)-2-(4-fluorophenyl)-2-(trifluoromethyl)cyclopropyl)methanone** was separated on a SFC Chiralpak-IC semiprep (250 x 10 mm) column (13% MeOH, 7 mL/min, 220 nM, P=100) to afford **(-)-(4-(5-chloro-2-methylphenyl)piperazin-1-yl)(2-(4-fluorophenyl)-2-(trifluoromethyl)cyclopropyl)methanone** (retention time 6.01 min) as a colorless oil which foamed up upon drying (100% purity by ESLD): [a]²⁰_{D} -179.6 (c 0.69, MeOH); ¹H NMR (CDCl₃, 400 MHz) δ 7.31 (dd, *J =* 8.6, 5.3 Hz, 2 H), 7.13 (d, *J =* 7.6 Hz, 1 H), 7.07-7.00 (m, 3 H), 6.96 (d, *J* = 2.1 Hz, 1 H), 3.99-3.85 (m, 2 H), 3.71-3.54 (m, 2 H), 3.08-2.98 (m, 2 H), 2.86-2.74 (m, 2 H), 2.59 (dd, *J* = 8.8, 6.1 Hz, 1 H), 2.30 (s, 3 H), 2.13-2.08 (m, 1 H), 1.70 (dd, *J* = 8.8, 5.3 Hz, 1 H); HRMS (ESI) *m*/*z* calcd for C₂₂H₂₂ClF₄N₂O ([M+H]⁺) 441.1344, found 441.1351. The enantiomeric excess was >99% ee (SFC Chiralpak-IC (250 x 10 mm); 10% MeOH, 220 nm, 7 mL/min; retention time: 6.01 min).

**(+)-(4-(5-chloro-2-methylphenyl)piperazin-1-yl)(2-(4-fluorophenyl)-2-(trifluoromethyl)-cyclopropyl)methanone** (retention time 7.16 min) was obtained as a colorless oil which foamed up upon drying (100% purity by ESLD): [a]²⁰_{D} -177.9 (c 0.67, MeOH); ¹H NMR (CDCl₃, 400 MHz) δ 7.31 (dd, *J* = 8.6, 5.3 Hz, 2 H), 7.13 (d, *J =* 8.1 Hz, 1 H), 7.07-7.00 (m, 3 H), 6.96 (d, *J* = 2.1 Hz, 1 H), 3.99-3.85 (m, 2 H), 3.71-3.54 (m, 2 H), 3.07-3.00 (m, 2 H), 2.87-2.74 (m, 2 H), 2.59 (dd, *J* = 8.9, 6.1 Hz, 1 H), 2.30 (s, 3 H), 2.12-2.08 (m, 1 H), 1.70 (dd, *J* = 8.8, 5.3 Hz, 1 H); HRMS (ESI) *m*/*z* calcd for C₂₂H₂₂ClF₄N₂O ([M+H]⁺) 441.1340, found 441.1351. The enantiomeric excess was >99% ee (SFC Chiralpak-IC (250 x 10 mm); 13% MeOH, 220 nm, 7 mL/min; retention time: 7.12 min).

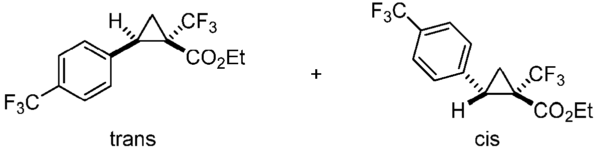

***cis-* and *trans*-Ethyl-1-(trifluoromethyl)-2-(4-(trifluoromethyl)phenyl)cyclopropane-1-carboxylate.** To a flame-dried round-bottom flask was added rhodium acetate (64.2 mg, 0.145 mmol) and 4-(trifluoromethyl)styrene in distilled CH₂Cl₂ (3 mL). A solution of **ethyl 2-diazo-3,3,3-trifluoropropanoate** (0.793 g, 4.36 mmol) in distilled CH₂Cl₂ (10 mL) was added via a syringe pump over 18 h. The mixture was stirred for another 2 hour and then filtered through a silica gel plug and washed with CH₂Cl₂. The solution was concentrated under reduced pressure and the crude residue was purified via flash column chromatography on SiO₂ (1:2 CH₂Cl₂/hexanes) to afford ***cis-* and *trans*-ethyl-1-(trifluoromethyl)-2-(4-(trifluoromethyl)phenyl)cyclopropane-1-carboxylate** respectively as pale yellow oil: *cis-* (224 mg, 0.686 mmol, 24%); ¹H NMR (CDCl₃, 500 MHz) δ 7.58 (d, *J =* 8.2 Hz, 2 H), 7.42 (d, *J =* 8.1 Hz, 2 H), 4.35-4.27 (m, 2 H), 3.11 (t, *J =* 8.9 Hz, 1 H), 1.99 (ddq, *J* = 9.5, 5.5, 1.8 Hz, 1 H), 1.94 (dd, *J* = 8.3, 5.4 Hz, 1 H), 1.35 (t, *J =* 7.1 Hz, 3 H); ¹⁹F NMR (CDCl₃, 470 MHz) δ -61.1 (s, 3 F), -62.6 (s, 3 F).

*trans-* (435 mg, 1.33 mmol, 46%); ¹H NMR (CDCl₃, 500 MHz) δ 7.56 (d, *J =* 8.1 Hz, 2 H), 7.37 (d, *J* = 8.6 Hz, 2 H), 3.91 (q, *J* = 7.1 Hz, 2 H), 2.97 (t, *J* = 8.9 Hz, 1 H), 2.18 (ddq, *J* = 8.0, 6.0, 2.0 Hz, 1 H), 1.84 (dd, *J =* 9.7, 5.8 Hz, 1 H), 0.91 (t, *J* = 7.1 Hz, 3 H); ¹⁹F NMR (CDCl₃, 470 MHz) δ -62.6 (s, 3 F), -67.0 (s, 3 F). A fraction containing a mixture of both diastereomers was also obtained (157 mg, 0.481 mmol, 17%).

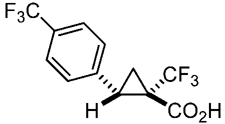

***cis*-1-(trifluoromethyl)-2-(4-(trifluoromethyl)phenyl)cyclopropane-1-carboxylic** acid. A solution of *cis-***ethyl-1-(trifluoromethyl)-2-(4-(trifluoromethyl)phenyl)cyclopropane-1-carboxylate** (0.220 g, 0.674 mmol) in methanol (1.4 mL) was added to KOH (0.378 g, 6.74 mmol) in methanol (3.4 ml). The reaction mixture was heated to 55 °C and stirred for 3 d. The mixture was cooled to room temperature and poured into water and extracted with CH₂Cl₂ (15 mL). The organic layer was discarded and the aqueous layer acidified with 6 M HCl and extracted with CH₂Cl₂ (2 x 15 mL). The combined organic layers were dried over anhydrous Na₂SO₄ and concentrated under reduced pressure to give ***cis*-1-(trifluoromethyl)-2-(4-(trifluoromethyl)phenyl)cyclopropane-1-carboxylic acid** (0.190 g, 0.636 mmol, 94%) as a white solid: ¹H NMR (CDCl₃, 500 MHz) δ 7.60 (d, *J* = 8.2 Hz, 2 H), 7.44 (d, *J* = 8.2 Hz, 2 H), 3.24 (t, *J* = 9.1 Hz, 1 H), 2.10-2.04 (m, 2 H); ¹³C NMR (CDCl₃, 125 MHz) δ 173.7, 137.1, 130.4 (q, *J*_{C-F} = 33 Hz), 130.0, 125.6 (q, *J* _{C-F} = 4 Hz), 124.1 (q, *J*_{C-F} = 272 Hz), 123.6 (q, *J*_{C-F} = 275 Hz), 32.9 (q, *J*_{C-F} = 34 Hz), 32.6, 17.0; ¹⁹F NMR (CDCl₃, 470 MHz) δ -61.6 (s, 3 F), -62.7 (s, 3 F).

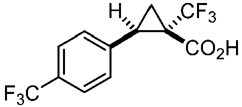

***trans*-1-(trifluoromethyl)-2-(4-(trifluoromethyl)phenyl)cyclopropane-1-carboxylic acid.** A solution of ***trans*-ethyl-1-(trifluoromethyl)-2-(4-(trifluoromethyl)phenyl)cyclopropane-1-carboxylate** (0.200 g, 0.613 mmol) in methanol (1.3 mL) was added to KOH (0.344 g, 6.13 mmol) in methanol (3 mL). The reaction mixture was heated to 55 °C and stirred for 3 d. The mixture was cooled to room temperature and poured into water and extracted with CH₂Cl₂ (15 mL). The organic layer was discarded and the aqueous layer acidified with 6 M HCl and extracted with CH₂Cl₂ (2 x 15 mL). The combined organic layers were dried over anhydrous Na₂SO₄ and concentrated under reduced pressure to give ***trans*-1-(trifluoromethyl)-2-(4-(trifluoromethyl)phenyl)cyclopropane-1-carboxylic acid** (187 mg, 0.627 mmol, quant.) as a white solid: ¹H NMR (CDCl₃, 500 MHz) δ 7.54 (d, *J =* 8.2 Hz, 2 H), 7.33 (d, *J =* 8.2 Hz, 2 H), 3.04 (t, *J* = 9.1 Hz, 1 H), 2.16-2.13 (m, 1 H), 1.89 (dd, *J* = 9.7, 5.9 Hz, 1 H); ¹³C NMR (CDCl₃, 125 MHz) δ 170.7, 137.1 (d, *J*_{C*-* F} = 1 Hz), 130.3 (q, *J*_{C-F} = 33 Hz), 129.6, 125.4 (q, *J*_{C-F} = 4 Hz), 124.1 (q, *J*_{C-F} = 272 Hz), 123.9 (q, *J*_{C-F} = 274 Hz), 34.3 (q, *J*_{C-F} = 34 Hz), 30.1 (d, *J*_{C-F} = 1 Hz), 16.2; ¹⁹F NMR (CDCl₃, 470 MHz) δ -62.7 (s, 3 F), - 67.1 (s, 3 F).

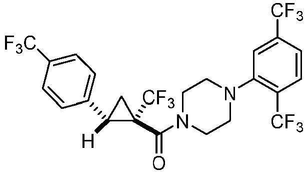

***cis*-(4-(2,5-bis(trifluoromethyl)phenyl)piperazin-1-yl)(1-(trifluoromethyl)-2-(4-(trifluoromethyl)phenyl)cyclopropyl)methanone.** A solution of ***cis*-1-(trifluoromethyl)-2-(4-(trifluoromethyl)phenyl)cyclopropane-1-carboxylic** acid (50.0 mg, 0.168 mmol) and 1-(2,5-trifluoromethylphenyl)piperazine hydrochloride (73 mg, 0.218 mmol) in distilled CH₂Cl₂ (0.85 mL) was cooled to 0 °C and then added with triethylamine (0.12 mL, 0.838 mmol). The cooled solution was then treated with T3P (50wt.% solution in EtOAc, 0.14 mL, 0.201 mmol) dropwise. The reaction mixture was stirred at 0 °C for 30 min and allowed to warm to room temperature and stirred for 3 d. After completion of the reaction by TLC and LCMS analysis, the reaction mixture was diluted with CH₂Cl₂ (10 mL) and washed with 1 M HCl (10 mL), saturated NaHCO₃ (10 mL), dried over anhydrous MgSO₄, filtered and concentrated in vacuo. The crude residue was purified via automated flash column chromatography on SiO₂ (1:3 EtOAc/hexanes) to afford ***cis*-(4-(2,5-bis(trifluoromethyl)phenyl)piperazin-1-yl)(1-(trifluoromethyl)-2-(4-(trifluoromethyl)phenyl)cyclopropyl)methanone** as a white solid (77.6 mg, 0.134 mmol, 80%): Mp 128.4 - 131.7 °C; IR (CDCl₃) 2925, 2833, 1648, 1425, 1326, 1313, 1122, 1085, 846, 736; ¹H NMR (CDCl₃, 500 MHz) δ 7.81 (d, *J =* 8.1 Hz, 1 H), 7.60 (d, *J =* 8.2 Hz, 2 H), 7.56 (d, *J =* 8.3 Hz, 2 H), 7.48 (d, *J* = 8.1 Hz, 2 H), 3.91-3.87 (m, 4 H), 3.02 (t, *J* = 4.6 Hz, 4 H), 2.88 (t, *J* = 8.6 Hz, 1 H), 2.07 (t, *J =* 7.1 Hz, 1 H), 1.75 (t*, J =* 7.8 Hz, 1 H); ¹³C NMR (CDCl₃, 126 MHz) δ 164.2, 152.2, 137.8, 135.3 (q, *J*_{C-F} = 33 Hz), 130.9 (q, *J*_{C-F} = 29 Hz), 130.1 (t, *J*_{C-F} = 33 Hz), 129.5, 128.5 (q, *J*_{C-F} = 5 Hz), 125.5 (q, *J*_{C-F} = 4 Hz), 124.5 (q, *J*_{C-F} = 281 Hz), 124.2 (q, *J*_{C-F} = 272 Hz), 123.4 (q, *J*_{C-F} = 274 Hz), 123.2 (q, *J*_{C-F} = 273 Hz), 122.6 (q, *J*_{C-F} = 4 Hz), 121.2 (q, *J*_{C-F} = 4 Hz), 53.3, 53.2, 46.9, 43.1, 35.1 (q, *J*_{C-F} = 33 Hz), 27.9, 14.8 (d, *J*_{C-F} = 2 Hz); ¹⁹F NMR (CDCl₃, 470 MHz) δ -60.9 (d, 6 F), -62.6 (s, 3 F), -63.2 (s, 3 F); HRMS (ESI) *m*/*z* calcd for C₂₄H₁₉F₁₂N₂O ([M+H]⁺) 579.1300, found 579.1298.

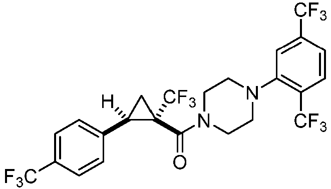

***trans-*(4-(2,5-bis(trifluoromethyl)phenyl)piperazin-1-yl)(1-(trifluoromethyl)-2-(4-(trifluoromethyl)-phenyl)cyclopropyl)methanone.** A solution of ***trans*-1-(trifluoromethyl)-2-(4-(trifluoromethyl)phenyl)-cyclopropane-1-carboxylic acid** (0.100 g, 0.335 mmol) and 1-(2,5-trifluoromethylphenyl)piperazine hydrochloride (0.146 g, 0.436 mmol) in distilled CH₂Cl₂ (1.7 mL) was cooled to 0 °C and then added with triethylamine (0.24 mL, 1.68 mmol). The cooled solution was then treated with T3P (50wt.% solution in EtOAc, 0.28 mL, 0.402 mmol) dropwise. The reaction mixture was stirred at 0 °C for 30 min and allowed to warm to room temperature and stirred for 4 d. After completion of the reaction by TLC and LCMS analysis, the reaction mixture was diluted with CH₂Cl₂ (15 mL) and washed with 1 M HCl (15 mL), saturated NaHCO₃ (15 mL), dried over anhydrous MgSO₄, filtered and concentrated in vacuo. The crude residue was purified via automated flash column chromatography on SiO₂ (1:3 EtOAc/hexanes) to afford ***trans*-(4-(2,5-bis(trifluoromethyl)phenyl)piperazin-1-yl)(1-(trifluoromethyl)-2-(4-(trifluoromethyl)phenyl)-cyclopropyl)methanone** as colorless oil (51.5 mg, 0.0890 mmol, 27%): IR (CDCl₃) 2925, 2833, 1643, 1510, 1424, 1310, 1115, 1070, 908, 851, 732 cm⁻¹; ¹H NMR (CDCl₃, 500 MHz) δ 7.71 (d, *J =* 8.2 Hz, 1 H), 7.63 (d, *J =* 8.2 Hz, 2 H), 7.48 (d, *J =* 8.2 Hz, 1 H), 7.27 (d, *J =* 8.0 Hz, 2 H), 7.14 (s, 1 H), 4.06-4.01 (m, 1 H), 3.44-3.29 (m, 2 H), 3.19-3.15 (m, 1 H), 2.86 (dd, *J* = 9.7*,* 7.5 Hz, 1 H), 2.82-2.80 (m, 1 H), 2.56-2.43 (m, 2 H), 1.98 (t, *J =* 7.0 Hz, 1 H), 1.86 (dd, *J* = 9.7, 6.7 Hz, 1 H), 1.20 (s, 1 H); ¹³C NMR (CDCl₃, 125 MHz) δ 161.4, 152.0, 139.6, 135.3 (q, *J*_{C-F} = 33 Hz), 130.7 (q, *J*_{C-F} = 29 Hz), 130.4 (q, *J*_{C-F} = 33 Hz), 128.4 (q, *J*_{C-F} = 5 Hz), 127.7, 125.8 (q, *J*_{C-F} = 4 Hz), 124.5 (q, *J*_{C-F} = 271 Hz), 123.9 (q, *J*_{C-F} = 272 Hz), 123.2 (q, *J*_{C-F} = 274 Hz), 123.1 (q, *J*_{C-F} = 273 Hz), 122.4 (q, *J*_{C-F} = 4 Hz), 120.6 (q, *J*_{C-F} = 3 Hz), 52.7, 52.4, 46.7, 43.1, 37.4 (q, *J*_{C-F} = 33 Hz), 27.5, 15.7; ¹⁹F NMR (CDCl₃, 470 MHz) δ -61.1 (s, 3 F), -62.8 (s, 3 F), -63.5 (s, 3 F), - 66.2 (s, 3 F); HRMS (ESI) *m*/*z* calcd for C₂₄H₁₉F₁₂N₂O ([M+H]⁺) 579.1300, found 579.1299.

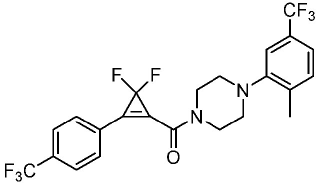

**(3,3-Difluoro-2-(4-(trifluoromethyl)phenyl)cycloprop-1-en-1-yl)(4-(2-methyl-5-(trifluoromethyl)-**phenyl)piperazin-1-yl)methanone. Under an inert atmosphere, 1-(4-(2-methyl-5-(trifluoromethyl)-**phenyl)piperazin-1-yl)-3-(4-(trifluoromethyl)phenyl)prop-2-yn-1-one** (0.400 g, 0.908 mmol), TMSCF₂Br (0.28 mL, 1.82 mmol), *n*Bu₄NBr (14.6 mg, 0.0454 mmol), and toluene (3.6 mL) were added into an oven-dried pressure tube at room temperature. After being heated at 110 °C for 20 h, The reaction mixture was cooled to room temperature and poured into saturated NaHCO₃ solution (15 mL) and extracted with EtOAc (2 x 20 mL). The combined organic layers were dried over anhydrous Na₂SO₄, filtered, and concentrated under reduced pressure. The crude product was subjected to flash column chromatography on SiO₂ (1:9 EtOAc/hexanes). The fractions collected contained ~10% of impurities. The product was resubjected to flash column chromatography on SiO₂ (1:2 CH₂Cl₂/hexanes) to afford **(3,3-difluoro-2-(4-(trifluoromethyl)phenyl)cycloprop-1-en-1-yl)(4-(2-methyl-5-(trifluoromethyl)phenyl)piperazin-1-yl)methanone** (0.325 g, 0.663 mmol, 73%) as a pale yellow oil that foamed up upon drying under vacuum: IR (CDCl₃) 2925, 2825, 1776, 1643, 1442, 1303, 1120, 1061, 1031, 909, 850, 730 cm⁻¹; ¹H NMR (CDCl₃, 500 MHz) δ 8.13 (d, *J =* 8.0 Hz, 2 H), 7.79 (d, *J =* 8.1 Hz, 2 H), 7.31 (q, *J =* 8.7 Hz, 2 H), 7.22 (s, 1 H), 3.94 (t, *J* = 4.7 Hz, 2 H), 3.88 (t, *J* = 5.0 Hz, 2 H), 3.04 (dt, *J* = 25.5, 5.0 Hz, 4 H), 2.41 (s, 3 H); ¹³C NMR (CDCl₃, 125 MHz) δ 155.1, 150.9, 137.0, 135.5 (t, *J =* 10 Hz), 134.4 (q, *J* = 33 Hz), 132.6, 131.8, 129.4 (q, *J* = 32 Hz), 126.3 (q, *J =* 3.6 Hz), 126.0, 124.3 (q, *J* = 272 Hz), 123.6 (q, *J* = 273 Hz), 120.9 (q, *J* = 3.9 Hz), 119.7 (t, *J* = 13 Hz), 116.3 (q, *J* = 3.6 Hz), 98.8 (t, *J* = 278 Hz), 52.2, 51.5, 46.9, 42.6, 18.1; ¹⁹F NMR (CDCl₃, 470 MHz) δ -62.3 (s, 3 F), -63.2 (s, 3 F), -102.3 (s, 2 F) ; HRMS (ESI) *m*/*z* calcd for C₂₃H₁₉ON₂F₈ ([M+H]⁺) 491.1364, found 491.1363.

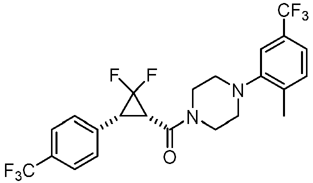

***cis*-((1SR,3RS)-2,2-difluoro-3-(4-(trifluoromethyl)phenyl)cyclopropyl)(4-(2-methyl-5-(trifluoromethyl)phenyl)piperazin-1-yl)methanone.** A solution of **(3,3-difluoro-2-(4-(trifluoromethyl)-phenyl)cycloprop-1-en-1-yl)(4-(2-methyl-5-(trifluoromethyl)phenyl)piperazin-1-yl)methanone** (260 mg, 0.530 mmol) in EtOAc (4.6 mL) was added Pd/C (10% Pd on carbon, 56.4 mg, 10.0 mol%) . The reaction vessel was placed in the parr hydrogenator (7 bar) and stirred for 24 h at room temperature. The mixture was filtered through celite and concentrated in vacuo. The crude oil was then passed through a plug of silica gel to remove baseline impurities. The crude residue (270 mg) contained a mixture of the desired cis-product and ring-opening side products which was inseparable by normal phase column chromatography.

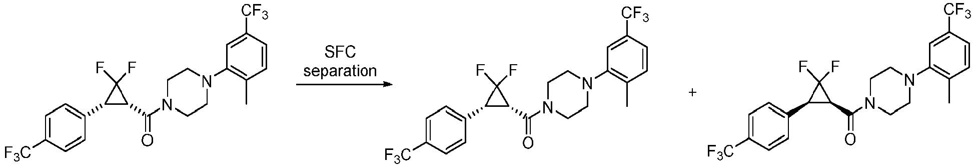

The crude racemic ***cis*-((1SR,3RS)-2,2-difluoro-3-(4-(trifluoromethyl)phenyl)cyclopropyl)(4-(2-methyl-5-(trifluoromethyl)phenyl)piperazin-1-yl)methanone** was purified and separated on a SFC Chiralpak-IC semiprep (250 x 10 mm) column (15% iPrOH, 6 mL/min, 220 nM, P=100) to afford the (-)-enantiomer (45.1 mg, 0.0916 mmol, 17%) and (+)-enantiomer (41.3 mg, 0.0839 mmol, 16%) respectively as a white solid: Mp 123.5 - 127.8 °C; IR (CDCl₃) 2917, 2820, 1648, 1416, 1323, 1308, 1115, 1070, 986, 856, 731 cm⁻¹. **(-)-2,2-difluoro-3-(4-(trifluoromethyl)phenyl)cyclopropyl)(4-(2-methyl-5-(trifluoromethyl)-phenyl)piperazin-1-yl)methanone** (retention time 7.52 min) was obtained as a white solid (99.5% purity by ESLD): [a]²⁰_{D} -32.1 (c 1.03, iPrOH); ¹H NMR (CDCl₃, 500 MHz) δ 7.60 (d, *J =* 8.3 Hz, 2 H), 7.44 (d, *J* = 8.1 Hz, 2 H), 7.28-7.24 (m, 4 H), 7.02 (s, 1 H), 3.80-3.77 (m, 1 H), 3.66-3.53 (m, 3 H), 3.13 (td, *J =* 12.6, 2.0 Hz, 1 H), 2.90 (td, *J =* 12.5, 2.4 Hz, 1 H), 2.83 (dtd, *J =* 11.5, 5.8, 3.4 Hz, 2 H), 2.61 (ddd, *J =* 11.2, 8.0, 3.0 Hz, 1 H), 2.39-2.33 (m, 1 H), 2.31 (s, 3 H); ¹⁹F NMR (CDCl₃, 470 MHz) δ -62.4 (s, 3 F), -62.8 (s, 3 F), - 118.9 (d, *J*_{F-F} = 161 Hz, 1 F), -147.2 (d, *J*_{F-F} = 161 Hz, 1 F); HRMS (ESI) *m*/*z* calcd for C₂₃H₂₁ON₂F₈ ([M+H]⁺) 493.1521, found 493.1522. The enantiomeric excess was >99% ee (SFC Chiralpak-IC (250 x 10 mm); 15% iPrOH, 220 nm, 6 mL/min; retention time: 7.54 min).

**(+)-2,2-difluoro-3-(4-(trifluoromethyl)phenyl)cyclopropyl)(4-(2-methyl-5-(trifluoromethyl)-phenyl)piperazin-1-yl)methanone** (retention time 9.64 min) was obtained as a white solid (99.5% purity by ESLD): [a]²⁰_{D} +33.5 (c 0.60, iPrOH); ¹H NMR (CDCl₃, 500 MHz) δ 7.60 (d, *J =* 8.2 Hz, 2 H), 7.44 (d, *J =* 8.1 Hz, 2 H), 7.28-7.24 (m, 5 H), 7.02 (s, 1 H), 3.81-3.76 (m, 1 H), 3.66-3.52 (m, 3 H), 3.13 (td, *J =* 12.6, 2.2 Hz, 1 H), 2.90 (td, *J =* 12.5, 2.6 Hz, 1 H), 2.83 (dtd, *J* = 11.5, 5.8, 3.3 Hz, 2 H), 2.61 (ddd, *J =* 11.4, 7.9, 3.0 Hz, 1 H), 2.39-2.33 (m, 1 H), 2.31 (s, 3 H); ¹³C NMR (CDCl₃, 126 MHz) δ 160.94 (s, 1 C), 150.94 (s, 1 C), 136.92 (s, 1 C), 134.96 (s, 1 C), 131.67 (s, 1 C), 130.13 (q, *J =* 32.8 Hz, 1 C), 129.53 (d, *J =* 2.7 Hz, 1 C), 129.30 (q, *J =* 34.0 Hz, 1 C), 125.39 (q, *J =* 3.6 Hz, 1 C), 124.22 (q, *J =* 272.0 Hz, 1 C), 124.06 (q, *J =* 272.1 Hz, 1 C), 120.72 (q, *J =* 3.7 Hz, 1 C), 116.01 (q, *J =* 3.6 Hz, 1 C), 110.99 (t, *J* = 289.7 Hz, 1 C), 51.70 (s, 1 C), 51.49 (s, 1 C), 46.17 (s, 1 C), 42.12 (s, 1 C), 31.35 (dd, *J =* 12.9, 9.8 Hz, 1 C), 30.28 (dd, *J =* 10.4, 8.9 Hz, 1 C), 18.00 (s, 1 C). ¹⁹F NMR (CDCl₃, 470 MHz) δ -62.4 (s, 3 F), -62.8 (s, 3 F), -118.9 (d, *J*_{F-F} = 161 Hz, 1 F), -147.2 (d, *J*_{F-F} = 161 Hz, 1 F). HRMS (ESI) *m*/*z* calcd for C₂₃H₂₁ON₂F₈ ([M+H]⁺) 493.1521, found 493.1522. The enantiomeric excess was >99% ee (SFC Chiralpak-IC (250 x 10 mm); 15% iPrOH, 220 nm, 6 mL/min; retention time: 9.66 min).

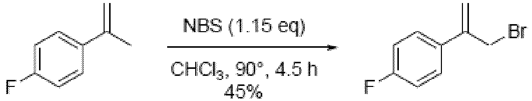

**1-(3-Bromoprop-1-en-2-yl)-4-fluorobenzene.** A solution of 4-fluoro-α-methylstyrene (4.78 g, 35.1 mmol) in CHCl₃ (7 mL) was treated with NBS (8.50 g, 47.7 mmol, 1.36 eq). The reaction was heated to reflux (~90 °C) for 6 h then it was cooled and filtered through Celite. The filter cake was then washed with hexane (50 mL) and the eluent was concentrated. The residue was purified by chromatography on SiO₂ (hexane) to afford the allylic bromide (3.38 g, 15.7 mmol, 45%) as a colorless oil: ¹H NMR (400 MHz, CDCl₃) δ 7.48-7.43 (m, 2 H), 7.13-7.01 (m, 2 H), 5.50 (s, 1 H), 5.48 (s, 1 H), 4.36 (s, 2 H); ¹⁹F NMR (376 MHz, CDCl₃) δ - 113.7.

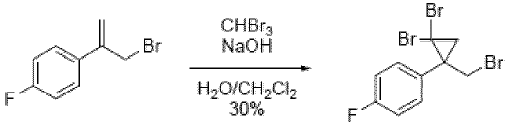

**1-(2,2-Dibromo-1-(bromomethyl)cyclopropyl)-4-fluorobenzene.** A solution of allylic bromide (3.17 g, 14.7 mmol) and cetyltrimethyl ammonium bromide (0.054 g, 0.15 mmol, 1 mol %) in CH₂Cl₂ (4 mL) was charged to a 100 mL 3-neck flask affixed with an N₂ inlet, mechanical stirrer, and septum. Bromoform (3.9 mL, 44 mmol, 3.0 eq) was added by syringe, and the solution was cooled to 0 °C. Sodium hydroxide (7.07 g, 0.177 mol) was added as a solution in water (15 mL, ~50 g/100 mL) via syringe over 5 min. The ice bath was removed and the flask stirred for 26 h at 300 rpm over which period the color changed from clear to a dark, opaque brown. The reaction mixture was then partitioned between water (50 mL) and CH₂Cl₂ (50 mL). The layers were separated, and the aqueous phase was extracted with CH₂Cl₂ (50 mL). The combined extracts were washed with water (25 mL), 1:1 water/brine (25 mL), and then were dried (Na₂SO₄) and concentrated. The residue was purified by column chromatography on SiO₂ (10% EtOAc in hexane). Upon concentration of the product-containing fractions, a solid mass formed. This material was then recrystallized from ~1:1 EtOAc/hexanes (~15 mL), and the crystals were washed with cold hexanes (30 mL). After drying under vacuum, the tribromide (1.68 g, 4.35 mmol, 30%) was obtained as a colorless crystalline solid: Mp 101-103 °C; ¹H NMR (300 MHz, CDCl₃) δ 7.38-7.30 (m, 2 H), 7.08 (app t, 2 H, *J =* 3.3 Hz), 3.91 (dd, 2 H, *J =* 7.8 Hz, 1.2 Hz), 3.82 (d, 1 H, *J =* 7.8 Hz), 2.21 (dd, 1 H, *J =* 6.0 Hz, 0.9 Hz), 2.01 (d, 1 H, *J* = 6.3 Hz); ¹⁹F NMR (376 MHz, CDCl₃) δ -113.2.

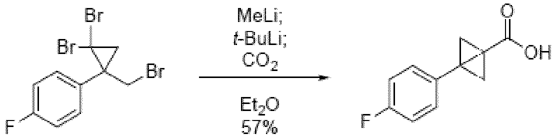

**3-(4-Fluorophenyl)bicyclo[1.1.0]butane-1-carboxylic acid.** A solution of tribromide (0.880 g, 2.57 mmol) in Et₂O (7 mL) and THF (3 mL, necessary for solubility) in a 50 mL round bottom flask was placed under N₂ and cooled to -78 °C. MeLi (1.6 mL, 2.6 mmol, 1.0 eq, 1.6 M in ether) was added via syringe over 10 min. After 1.75 h at -78 °C, t-BuLi (1.5 mL, 2.6 mmol, 1.0 eq, 1.7 M in pentane) was added via syringe over 10 min. After an additional 1 h at -78 °C, a ballon of dry CO₂ was bubbled through the solution via a needle for 10 min (external bubbler was used, and a continuous stream of CO₂ was passed through the flask). The cooling bath was then removed, and the opaque grey reaction mixture was warmed to 0 °C over 20 min. The reaction mixture was then quenched with 1 M NaOH (10 mL), then the material was partitioned between ether and 1 M NaOH (50 mL each). The light brown aqueous layer was acidified with con HCl, forming an opaque solution with a light brown precipitate. This solution was then extracted with ether (2 x 40 mL). The organic layer was dried (NaSO₄) and concentrated to afford the acid (0.228 g, 1.19 mmol, 57%) as a buff solid: Mp 158-160 °C; IR (ATR) 1646, 1527, 1226, 841 cm⁻¹; ¹H NMR (300 MHz, CD₃OD) δ 7.41-7.32 (m, 2 H), 7.05 (app t, 2 H, *J =* 9.0 Hz), 2.88 (t, 2 H, *J =* 1.2 Hz), 1.60 (s, 2 H); ¹³C NMR (100 MHz, CD₃OD) δ 172.2, 127.5 (³*J*_{CF} = 8 Hz), 114.9 (²*J*_{CF} = 21 Hz), 34.9, 31.8, 22.4; ¹⁹F NMR (376 MHz, CD₃OD) δ -117.7; HRMS *m*/*z* calcd for C₁₁H₁₀FO₂ [M+H] 193.0665, found 193.0660.

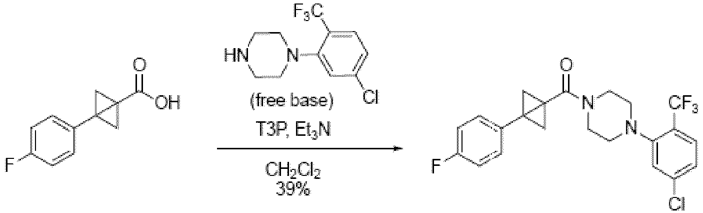

**(4-(5-Chloro-2-(trifluoromethyl)phenyl)piperazin-1-yl)(3-(4-fluorophenyl)bicyclo[1.1.0]butan-1-yl)methanone.** A solution of bicyclobutane acid (0.018 g, 0.096 mmol, 1.1 eq) and piperazine free base (0.023 g, 0.087 mmol) in CH₂Cl₂ (1 mL) was cooled to 0°C and treated with Et₃N (0.02 mL, 0.2 mmol, 2 eq). The transparent beige solution was then treated with T3P (50% solution in EtOAc) (0.09 mL, 0.13 mmol, 1.5 eq) dropwise via syringe over ~1 min. The reaction was stirred at 0°C for 30 min, then the ice bath was removed and the reaction was warmed to rt for 19.5 h. The reaction mixture was then directly purified by chromatography on SiO₂ (15-25% EtOAc/hexanes) without workup to afford the amide (0.015 g, 0.038 mmol, 39%) as a colorless oil: IR (ATR) 2923, 1620, 1596, 1434, 1307, 1221, 1120, 1027, 906, 933, 725 cm⁻¹; ¹H NMR (400 MHz, CDCl₃) δ 7.56 (d, 1 H, *J* = 8.4 Hz), 7.34-7.27 (m, 2 H), 7.25-7.19 (m, 2 H), 7.01 (app t, 2 H, *J =* 8.8), 4.1-3.5 (br m, 4 H), 2.9-2.7 (br m, 4 H), 2.75 (s, 2 H), 1.63 (s, 2 H); ¹³C NMR (100 MHz, CDCl₃) δ 167.7, 161.9 (d, ¹*J*_{CF} = 243 Hz), 152.9, 138.8, 135.1, 129.7 (d, ⁴*J*_{CF} = 3 Hz), 128.6 (q, ³*J*_{CF} = 5 Hz), 128.0 (d, ³*J*_{CF} = 8 Hz), 126.3 (q, ¹*J*_{CF} = 272 Hz), 125.4 (q, ²*J*_{CF} = 30 Hz), 115.4 (d, ²*J*_{CF} = 21 Hz), 36.9, 30.6, 21.0; ¹⁹F NMR (376 MHz, CDCl₃) δ -60.3, -115.9; MS (ESI)⁺ *m*/*z* 439 (100), 265 (3), 175 (5), 147 (45); HRMS (ESI)⁺ *m*/*z* cald for C₂₂H₂₀ClF₄N₂O [M+H] 439.1195, found 439.1193.

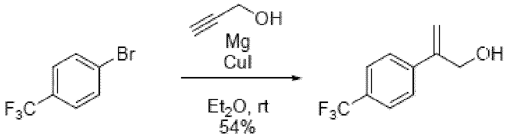

**2-(4-(Trifluoromethyl)phenyl)prop-2-en-1-ol** (Garzan et al., Chem. Eur. J. 2013, 19:9015-9021). Magnesium turnings (2.71 g, 0.111 mol) were charged to a 500 mL 3-neck round bottomed flask fitted with a reflux condenser, septum, and internal thermometer. After flushing with N₂, the turnings were suspended in ether (120 mL). 4-Trifluoromethylbromobenzene (15.8 mL, 0.111 mol) was added by syringe in ~1 mL portions over ~15 min, which caused the formation of a dark brown Gringard solution and spontaneous heating of the reaction to reflux. After ~1 h, the reaction had cooled from reflux back to rt. To the freshly prepared Grignard reagent was added CuI (1.27 g, 6.67 mmol) and the black suspension was stirred for 15 min. A solution of propargyl alcohol (2.60 mL, 44.6 mmol) in ether (50 mL) was added via cannula over 1.75 h, causing an exotherm to 29 °C. The reaction was then stirred at rt for 18 h, at which point a quenched aliquot showed consumption of propargyl alcohol by ¹H NMR. The reaction was then cooled to 0 °C and quenched with sat aq NH₄Cl (100 mL). The layers were separated and the aqueous layer was extracted with ether (2 x 100 mL). The organic extracts were washed with water (100 mL) and brine (100 mL), then they were dried (MgSO₄), filtered, and concentrated. The crude product was purified by chromatography on SiO₂ (25% EtOAc/hexanes) to afford the alcohol (4.86 g, 24.0 mmol, 54%) as a red-tinted oil: ¹H NMR (400 MHz, CDCl₃) δ 7.58 (AB q, *J* = 11.2 Hz, ∂_{AB} = 0.05 ppm), 5.55 (s, 1 H), 5.46 (s, 1 H), 4.56 (s, 2 H).

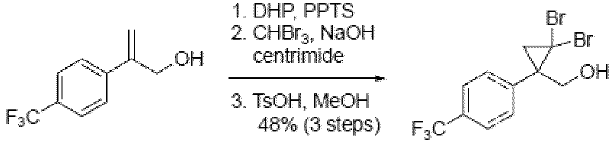

**(2,2-Dibromo-1-(4-(trifluoromethyl)phenyl)cyclopropyl)methanol.** A solution of alcohol (4.86 g, 24.0 mmol) in CH₂Cl₂ (40 mL) was treated with 3,4-dihydro-2H-pyran (3.1 mL, 34 mmol, 1.4 eq) followed by PPTS (0.604 g, 2.40 mmol, 0.10 eq). The resulting solution was stirred at rt for 2.5 h. The reaction mixture was then concentrated on the rotovap. The residue was partitioned between ether and water (75 mL each). The layers were separated, and the organic layer was washed with sat aq NaHCO₃ (75 mL). The organic layer was then dried (Na₂SO₄) and concentrated to afford the tetrahydropyran (7.11 g) as a red-tinted oil. The material contained residual solvents and was carried on without further purification.

A solution of protected alcohol (7.11 g, 24.8 mmol) and cetyltrimethyl ammonium bromide (0.091 g, 0.25 mmol, 1 mol %) in CH₂Cl₂ (12 mL) was placed in a 250 mL 3-neck flask was affixed with an N₂ inlet, mechanical stirrer, and septum. Bromoform (6.5 mL, 75 mmol, 3.0 eq) was added, and the solution was cooled to 0 °C. Sodium hydroxide (11.9 g, 298 mmol, 12.0 eq) was added as a solution in water (24 mL, ~50 g/100 mL) via syringe over 10 min. The ice bath was removed and the flask stirred at rt for 24 h at 300 rpm. ¹H NMR of an aliquot showed consumption of the alkene. The reaction mixture was then partitioned between water (50 mL) and CH₂Cl₂ (50 mL), the layers were separated, and the aqueous phase was extracted with CH₂Cl₂ (50 mL). The combined extracts were washed with water (25 mL), 1:1 water/brine (25 mL), then were dried (Na₂SO₄) and concentrated.

This crude dark red oil was dissolved in MeOH (25 mL) and treated with tosic acid monohydrate (0.472 g, 2.48 mmol, 10 mol %). After stirring at rt for 3 h, the reaction mixture was concentrated. The residue was partitioned between water and ether (75 mL each). The layers were separated and the organic layer was washed with water (75 mL), sat aq NaHCO₃ (75 mL), and brine (75 mL). The organic layer was then dried (Na₂SO₄) and concentrated. Purification by chromatography on SiO₂ afforded the dibromocyclopropane (4.45 g, 11.9 mmol, 48% over 3 steps) as a colorless oil: ¹H NMR (400 MHz, CDCl₃) δ 7.65 (app d, 2 H, *J =* 8.4 Hz), 7.52 (app d, 2 H, *J =* 8.0 Hz), 4.01 (AB q, *J =* 12.0 Hz, ∂_{AB} = 0.04 ppm), 2.11 (AB q, *J =* 7.6 Hz, ∂_{AB} = 0.03 ppm); ¹³C NMR (100 MHz, CDCl₃) δ 142.4, 130.2, 125.4 (³*J*_{CF} = 4 Hz), 70.1, 40.7, 31.9, 31.3, 21.1; ¹⁹F NMR (376 MHz, CDCl₃) δ -62.6.

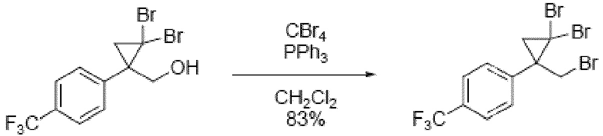

**1-(2,2-Dibromo-1-(bromomethyl)cyclopropyl)-4-(trifluoromethyl)benzene.** A solution of alcohol (4.45 g, 11.9 mmol) in CH₂Cl₂ (35 mL) was cooled to 0 °C under N₂. PPh₃ (3.75 g, 13.1 mmol, 1.20 eq) was then charged, followed by CBr₄ (4.34 g, 13.1 mmol, 1.10 eq). The reaction was warmed to rt and stirred for 20 h, then it was concentrated. Purification by chromatography on SiO₂ (0-15% EtOAc in hexanes) afforded tribromide (4.31 g, 9.85 mmol, 83%) as a colorless oil: ¹H NMR (400 MHz, CDCl₃) δ 7.66 (d, 2 H, *J =* 8.0 Hz), 7.49 (d, 2 H, *J =* 8.0 Hz), 3.93 (dd, 1 H, *J =* 10.8 Hz, 1.2 Hz), 3.83 (d, 1 H, *J =* 10.8 Hz), 2.27 (dd, 1 H, *J =* 8.0 Hz, 1.2 Hz), 2.06 (d, 1 H, *J =* 8.0 Hz); ¹³C NMR (100 MHz, CDCl₃) δ 142.0, 130.6, 130.3, 125.4 (q, ³*J*_{CF} = 4 Hz), 41.7, 39.8, 34.8, 34.7, 33.4, 31.6, 14.1; ¹⁹F NMR (376 MHz, CDCl₃) δ -62.6.

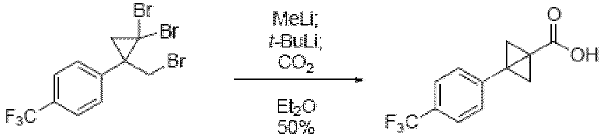

**3-(4-(Trifluoromethyl)phenyl)bicyclo[1.1.0]butane-1-carboxylic acid.** A solution of tribromide (1.50 g, 3.43 mmol) in Et₂O (15 mL) in a 50 mL round bottom flask was placed under N₂ and cooled to -78 °C. MeLi (2.1 mL, 3.4 mmol, 1.6 M in Et₂O) was added via syringe over 5 min. After 1 h at -78 °C, t-BuLi (2.0 mL, 3.4 mmol, 1.7 M in pentane, 1.0 eq) was added via syringe over 5 min. After an additional 1 h at -78 °C, a balloon of dry CO₂ was bubbled through the solution via a needle for 10 min (external bubbler was used and a continuous stream of CO₂ was passed through the flask). The cooling bath was then removed, and the opaque grey reaction mixture was warmed to 0 °C over 20 min while maintaining CO₂ bubbling. The reaction mixture was then quenched with 1 M NaOH (10 mL), then it was partitioned between ether and 1 M NaOH (50 mL each). The light yellow aqueous layer was acidified with concentrated HCl, forming a colorless precipitate. This suspension was then extracted with ether (2 x 40 mL). The organic layers were dried (Na₂SO₄) and concentrated to afford the acid (0.416 g, 1.72 mmol, 50%) as a colorless solid: Mp 146-148 °C; IR (ATR) 2980, 1648, 1616, 1463, 1318, 1167, 1116, 1061, 907, 842, 689 cm⁻¹; ¹H NMR (400 MHz, CD₃OD) δ 7.61 (d, 2 H, *J =* 8.4 Hz), 7.52 (d, 2 H, *J =* 8.4 Hz), 4.88 (s, 1 H), 2.99 (t, 2 H, *J =* 1.2 Hz), 1.68 (s, 2 H); ¹³C NMR (100 MHz, CD₃OD) δ 171.5, 140.0, 128.4 (q, ²*J*_{CF} = 32 Hz), 126.2, 124.9 (q, ³*J*_{CF} = 4 Hz), 124.4 (q, ¹*J*_{CF} = 269 Hz), 35.0, 31.0, 24.2; ¹⁹F NMR (376 MHz, CDCl₃) δ -64.0; MS (ESI)⁺ *m*/*z* 243 (80), 233 (50), 231 (50), 197 (60), 177 (40), 155 (100); HRMS (ESI)⁺ *m*/*z* cald for C₁₂H₁₀F₃O₂ [M+H] 243.0633, found 243.0626.

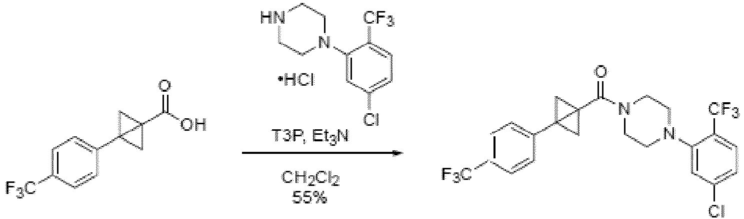

**(4-(5-Chloro-2-(trifluoromethyl)phenyl)piperazin-1-yl)(3-(4-(trifluoromethyl)phenyl)-bicyclo[1.1.0]butan-1-yl)methanone.** A solution ofbicyclobutane acid (0.046 g, 0.19 mmol, 1.0 eq) and piperazine HCl salt (0.057 g, 0.19 mmol) in CH₂Cl₂ (4 mL) cooled to 0 °C was treated Et₃N (0.08 mL, 0.6 mmol, 3 eq). The transparent beige solution was then treated with T3P (50% solution in EtOAc) (0.20 mL, 0.28 mmol, 1.5 eq) dropwise via syringe over ~1 min. The reaction was stirred at 0 °C for 30 min, then the ice bath was removed and the reaction was warmed to rt for 22 h. The reaction mixture was then partitioned between water and CH₂Cl₂ (30 mL each). The layers were separated, and the aqueous layer was extracted with additional CH₂Cl₂ (30 mL). The combined extracts were washed with water (30 mL) and sat aq NaHCO₃ (30 mL), then were dried (Na₂SO₄) and concentrated. Purification by chromatography on SiO₂ (15-25% EtOAc/hexanes) afforded the amide (0.051 g, 0.10 mmol, 55%) as a colorless solid: Mp 137-139 °C; IR (ATR) 2902, 1604, 1466, 1438, 1312, 1100, 1027, 924, 838, 825 cm⁻¹; ¹H NMR (400 MHz, CDCl₃) δ 7.56 (app d, 3 H, *J =* 8.4 Hz), 7.43 (app d, 2 H, *J =* 8.4 Hz), 7.23 (app q, 2 H, *J =* 8.4 Hz), 4.1-3.5 (br, 4 H), 3.0-2.7 (br, 4 H), 2.83 (s, 2 H), 1.69 (s, 2 H); ¹³C NMR (100 MHz, CDCl₃) δ 167.1, 152.8, 138.8, 138.6, 128.5 (q, ²*J*_{CF} = 32), 128.5 (q, ³*J*_{CF} = 4), 126.6, 125.7 (q, ²*J*_{CF} = 29), 125.5, 125.3 (q, ³*J*_{CF} = 3), 124.6, 124.3 (q, ¹*J*_{CF} = 270), 123.6 (q, ¹*J*_{CF} = 272), 37.0, 30.0, 29.7, 22.7; ¹⁹F NMR (376 MHz, CDCl₃) δ -60.3, -62.4; MS (ESI)⁺ *m*/*z* 489, 256 (5); HRMS (ESI)⁺ *m*/*z* calcd for C₂₃H₂₀F₆ClN₂O [M+H] 489.1168, found 489.1158.

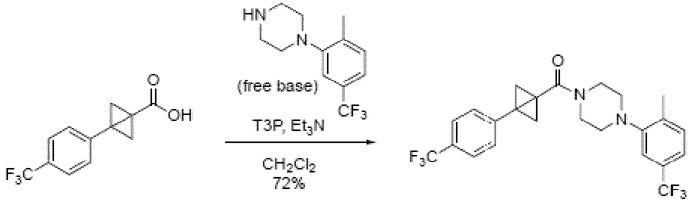

**(4-(2-Methyl-5-(trifluoromethyl)phenyl)piperazin-1-yl)(3-(4-(trifluoromethyl)phenyl)-bicyclo[1.1.0]butan-1-yl)methanone.** Before this reaction was performed, the piperazine was free-based by shaking a solution of 66 mg (0.24 mmol, 1.1 eq) of HCl salt in CH₂Cl₂ (30 mL) with 1 M NaOH (2 x 30 mL). The organic layer was then dried, concentrated, and the free base was used in the following reaction.

A solution of bicyclobutane acid (0.052 g, 0.21 mmol) and piperazine free base in CH₂Cl₂ (2 mL) cooled to 0 °C and treated with Et₃N (0.06 mL, 0.43 mmol, 2.0 eq). The transparent beige solution was treated with T3P (0.23 mL, 0.32 mmol, 1.5 eq, 50% solution in EtOAc) dropwise via syringe. The reaction was stirred at 0 °C for 30 min, then the ice bath was removed and the reaction was warmed to rt for 17 h. The reaction mixture was partitioned between water and CH₂Cl₂ (50 mL each). The layers were separated, and the aqueous layer was extracted with additional CH₂Cl₂ (30 mL). The combined extracts were washed with water (50 mL) and sat aq NaHCO₃ (40 mL), then were dried (Na₂SO₄) and concentrated. Purification by chromatography on SiO₂ (15-25% EtOAc/hexanes) afforded the amide (0.072 g, 0.154 mmol, 72%) as a colorless oil: IR (ATR) 2821, 1618, 1435, 1418, 1323, 1162, 1114, 1030, 840, 732 cm⁻¹; ¹H NMR (400 MHz, CDCl₃) δ 7.56 (d, 2 H, *J =* 8.0 Hz), 7.43 (d, 2 H, *J =* 8.4 Hz), 7.28 (app q, 2 H, *J =* 8.0 Hz), 7.18 (s, 1 H), 4.1-3.5 (br, 4 H), 3.0-2.7 (br, 4 H), 2.84 (s, 2 H), 2.36 (s, 3 H), 1.70 (s, 2 H); ¹³C NMR (100 MHz, CDCl₃) δ 167.1, 151.2, 138.6, 136.8, 131.6, 129.1 (q, ²*J*_{CF} = 32), 128.5 (q, ²*J*_{CF} = 32), 126.6, 125.3 (q, ³*J*_{CF} = 4), 124.3 (q, ¹*J*_{CF} = 270), 124.2 (q, ¹*J*_{CF} = 271), 120.4 (q, ³*J*_{CF} = 3), 116.0 (q, ³*J*_{CF} = 4), 37.0, 30.0, 22.8, 18.0; ¹⁹F NMR (376 MHz, CDCl₃) δ -62.2, -62.4; MS (ESI)⁺ *m*/*z* 469 (100), 197 (5); HRMS (ESI)⁺ *m*/*z* cald for C₂₄H₂₃F₆N₂O [M+H] 469.1709, found 469.1705.

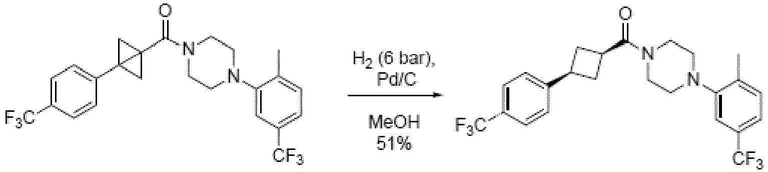

**(4-(2-methyl-5-(trifluoromethyl)phenyl)piperazin-1-yl)(3-(4-(trifluoromethyl)phenyl)cyclobutyl)-methanone.** A solution of amide (0.072 g, 0.153 mmol) in MeOH (3 mL) was placed under N₂ and treated with Pd/C (3 mg, ~20 mol %). A hydrogen atmosphere of 6.43 bar was then established on the Parr hydrogenator. The reaction was stirred at rt for 18 h, at which point the hydrogen was vented and the solution was purified by chromatography on SiO₂ (25-40% EtOAc/hexanes) to afford the syn-cyclobutane (0.037 g, 0.079 mmol, 51%) as a colorless oil: IR (ATR) 2939, 1641, 1618, 1417, 1324, 1308, 1161, 1112, 1067, 834, 732 cm⁻¹; ¹H NMR (400 MHz, CDCl₃) δ 7.55 (d, 2 H, *J* = 8.4 Hz), 7.36 (d, 2 H, *J =* 8.4 Hz), 7.32-7.24 (m, 2 H, *J =* 8.0 Hz), 7.19 (s, 1 H), 3.79 (t, 2 H, *J =* 4.8 Hz), 3.60 (t, 2 H, *J =* 5.2 Hz), 3.57-3.48 (m, 1 H), 3.37-3.26 (m, 1 H), 2.95-2.86 (m, 4 H), 2.68-2.59 (m, 2 H), 2.53 (ddd, 2 H, *J* = 19.2 Hz, 9.6 Hz, 2.4 Hz), 2.84 (s, 2 H), 2.37 (s, 3 H); ¹³C NMR (100 MHz, CDCl₃) δ 172.4, 151.2, 148.6, 136.8, 131.6, 129.1 (q, ²*J*_{CF} = 32), 128.5 (q, ²*J*_{CF} = 32), 127.4, 126.9, 126.7, 125.3 (q, ³*J*_{CF} = 4), 124.3 (q, ¹*J*_{CF} = 270), 124.2 (q, ¹*J*_{CF} = 270), 120.4 (q, ³*J*_{CF} = 4), 116.0 (q, ³*J*_{CF} = 5), 51.9, 51.7, 45.5, 42.2, 35.5, 33.1, 32.6, 17.6; ¹⁹F NMR (376 MHz, CDCl₃) δ -62.3; MS (ESI)⁺ *m*/*z* 471 (100), 355 (1), 246 (3), 179 (3); HRMS (ESI)⁺ *m*/*z* cald for C₂₄H₂₅F₆N₂O [M+H] 471.1871, found 471.1865.

### Example 6

### Androgen Receptor and Estrogen Receptor Alpha Competitor Assays

The disclosed compounds are assayed for androgen receptor activity using the PolarScreen^{™} Androgen Receptor Competitor Assay Kit, Green (ThermoFisher Scientific, catalog #A15880). The kit uses rat AR-ligand binding domain tagged with gluthathione-S-transferase (GST) and histidine [AR-LBD(Hist-GST)] to determine the IC₅₀ of competitive androgen receptor compounds. AR-LBD(His-GST) is added to fluorescently-tagged androgen ligand (FluormoneAL Green) in the presence of competitor test compounds. Effective competitors prevent the formation of a AL Green/AR-LBD(His-GST) complex, resulting in a decrease of polarization due to ligand displacement by the competitor. The shift in polarization values is used to determine the IC₅₀ of test compounds.

The disclosed compounds are assayed for estrogen receptor (ER) alpha activity using the PolarScreen^{™} ER Alpha Compeitor Asssay Kit, Green (ThermoFisher Scientific, catalog #A15883). The kit uses full length, native (untagged), human estrogen receptor alpha to determine the IC₅₀ of competitive estrogen receptor compounds. Full length ER alpha is added to a fluorescent estrogen ligand (Fluormone ES2 Green) to form an ER-Fluormone ES2 complex. An effective ER alpha competitor will displace the Fluormone ES2 ligand from the ER alpha and will result in a decrease in polarization. The shift in fluorescence polarization is used to determine the relative affinity of test compounds for ER alpha.

In view of the many possible embodiments to which the principles of the disclosed compounds, compositions and methods may be applied, it should be recognized that the illustrated embodiments are only preferred examples and should not be taken as limiting the scope of the invention.

## Claims

1. A compound selected from: 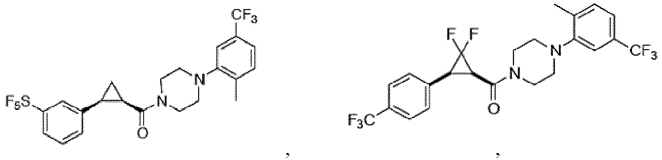 and 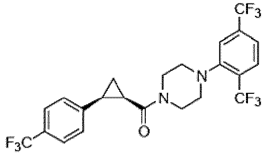 or a pharmaceutically acceptable salt thereof.

2. A compound selected from: 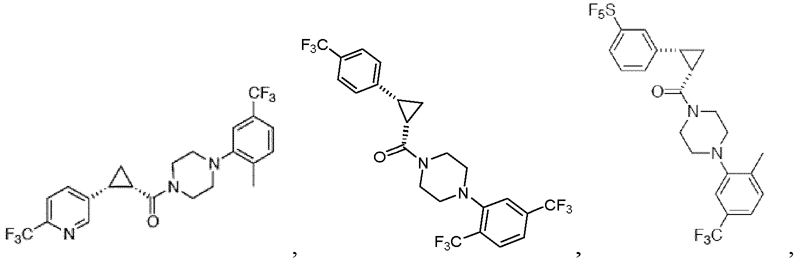 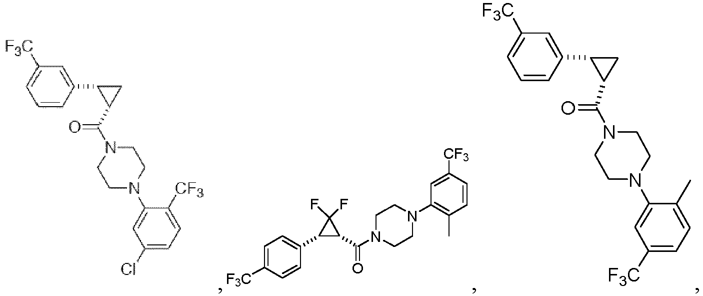 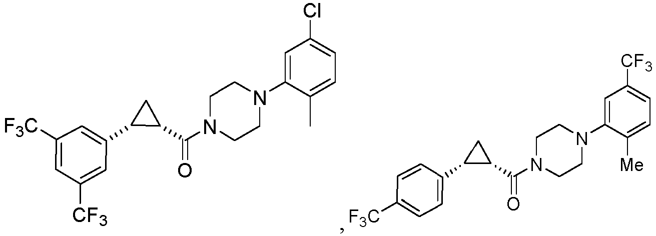 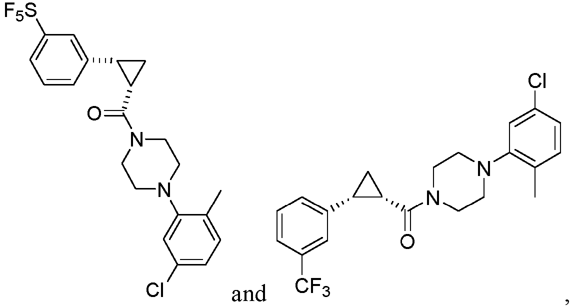 or a pharmaceutically acceptable salt thereof.

3. A compound selected from: 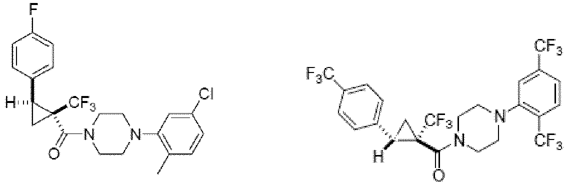 and 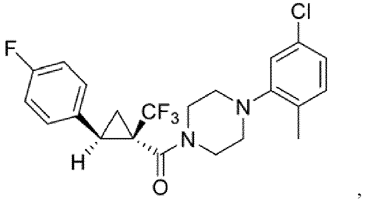 or a pharmaceutically acceptable salt thereof.

4. A pharmaceutical composition comprising at least one pharmaceutically acceptable additive, and 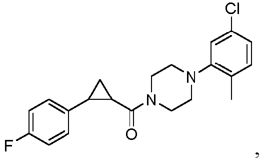 or a pharmaceutically acceptable salt thereof.

5. A pharmaceutical composition comprising at least one pharmaceutically acceptable additive, and a compound of any one of claims 1 to 3, or a pharmaceutically acceptable salt thereof.

6. A compound for use in a method for treating prostate cancer in a subject, wherein the subject is administered a therapeutically effective amount of the compound, wherein the compound is 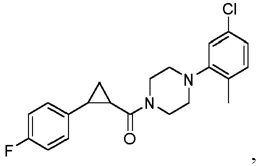 or a pharmaceutically acceptable salt thereof, optionally wherein the prostate cancer is castration-resistant cancer.

7. The compound for the use of claim 6, wherein the compound is orally administered.

8. The compound for the use of claim 6 or claim 7, wherein the compound is used in combination with androgen deprivation therapy.

9. The compound for the use of claim 6 or claim 7, wherein
the compound is co-administered with abiraterone; or
the compound is co-administered with enzalutamide.

10. The compound for the use of any one of claims 6 to 9, wherein the compound is: 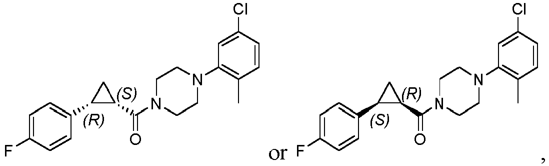 or a pharmaceutically acceptable salt thereof.

11. A compound for use in a method for treating prostate cancer in a subject, wherein the subject is administered a therapeutically effective amount of the compound, wherein the compound is a compound according to any one of claims 1 to 3, or a pharmaceutically acceptable salt thereof;
optionally wherein the prostate cancer is castration-resistant cancer.

12. The compound for the use of claim 11, wherein the compound is orally administered.

13. The compound for the use of claim 11 of claim 12, wherein the compound is used in combination with androgen deprivation therapy.

14. The compound for the use of claim 11 or claim 12, wherein
the compound is co-administered with abiraterone; or
the compound is co-administered with enzalutamide.

## Patentansprüche

1. Verbindung, ausgewählt aus: 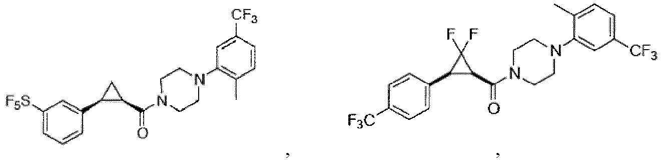 und 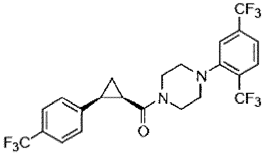 oder ein pharmazeutisch unbedenkliches Salzes davon.

2. Verbindung, ausgewählt aus: 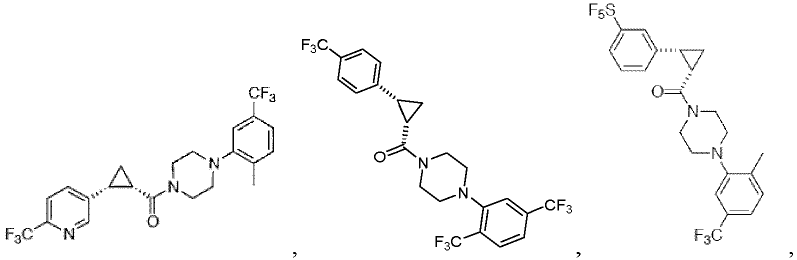 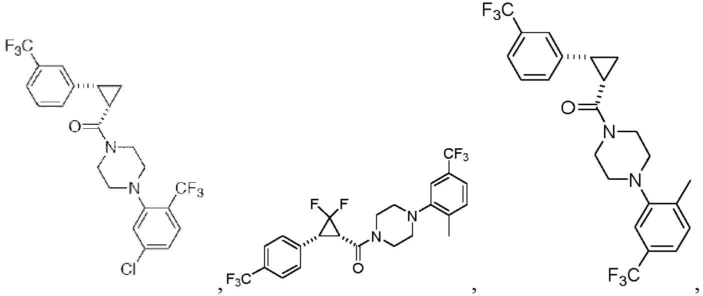 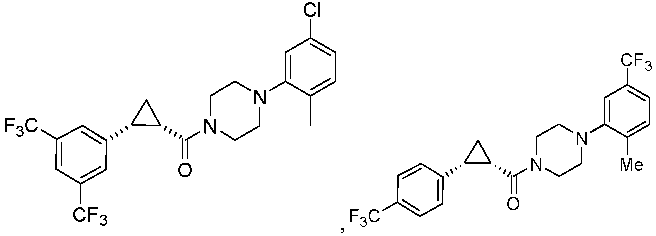 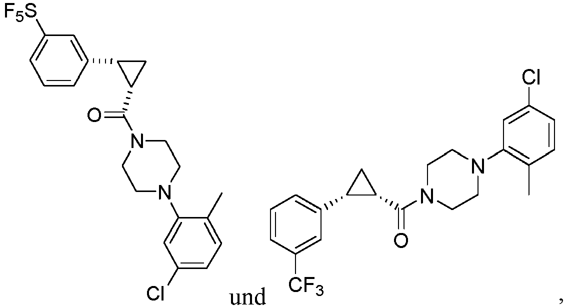 oder ein pharmazeutisch unbedenkliches Salzes davon.

3. Verbindung, ausgewählt aus: 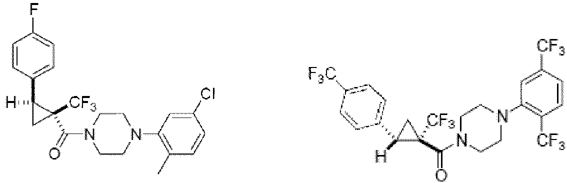 und 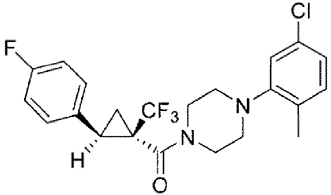 oder ein pharmazeutisch unbedenkliches Salzes davon.

4. Pharmazeutische Zusammensetzung, umfassend mindestens einen pharmazeutisch unbedenklichen Zusatzstoff und 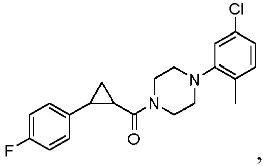 oder ein pharmazeutisch unbedenkliches Salz davon.

5. Pharmazeutische Zusammensetzung, umfassend mindestens einen pharmazeutisch unbedenklichen Zusatzstoff und eine Verbindung nach einem der Ansprüche 1 bis 3 oder ein pharmazeutisch unbedenkliches Salz davon.

6. Verbindung zur Verwendung in einem Verfahren zur Behandlung von Prostatakrebs bei einem Subjekt, wobei dem Subjekt eine therapeutisch wirksame Menge der Verbindung verabreicht wird, wobei die Verbindung 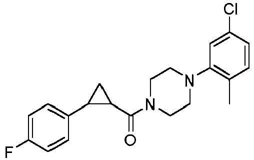 oder ein pharmazeutisch unbedenkliches Salz davon ist, optional wobei der Prostatakrebs kastrationsresistenter Krebs ist.

7. Verbindung zur Verwendung nach Anspruch 6, wobei die Verbindung oral verabreicht wird.

8. Verbindung zur Verwendung nach Anspruch 6 oder Anspruch 7, wobei die Verbindung in Kombination mit einer Androgendeprivationstherapie verwendet wird.

9. Verbindung zur Verwendung nach Anspruch 6 oder Anspruch 7, wobei die Verbindung gleichzeitig mit Abirateron verabreicht wird, oder wobei die Verbindung gleichzeitig mit Enzalutamid verabreicht wird.

10. Verbindung zur Verwendung nach einem der Ansprüche 6 bis 9, wobei die Verbindung: 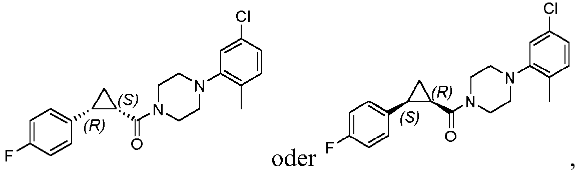 oder ein pharmazeutisch unbedenkliches Salzes davon ist.

11. Verbindung zur Verwendung in einem Verfahren zum Behandeln von Prostatakrebs bei einem Subjekt, wobei dem Subjekt eine therapeutisch wirksame Menge der Verbindung verabreicht wird, wobei die Verbindung eine Verbindung nach einem der Ansprüche 1 bis 3 oder ein pharmazeutisch unbedenkliches Salz davon ist;
optional wobei der Prostatakrebs kastrationsresistenter Krebs ist.

12. Verbindung zur Verwendung nach Anspruch 11, wobei die Verbindung oral verabreicht wird.

13. Verbindung zur Verwendung nach Anspruch 11 oder Anspruch 12, wobei die Verbindung in Kombination mit einer Androgendeprivationstherapie verwendet wird.

14. Verbindung zur Verwendung nach Anspruch 11 oder Anspruch 12, wobei die Verbindung gleichzeitig mit Abirateron verabreicht wird, oder wobei die Verbindung gleichzeitig mit Enzalutamid verabreicht wird.

## Revendications

1. Composé choisi parmi : 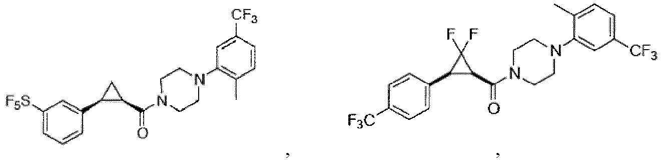 et 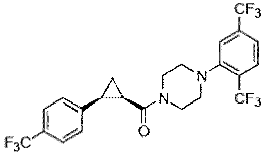 ou sel acceptable pharmaceutiquement de celui-ci.

2. Composé choisi parmi : 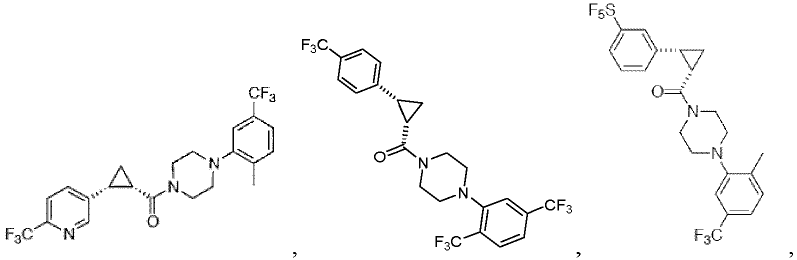 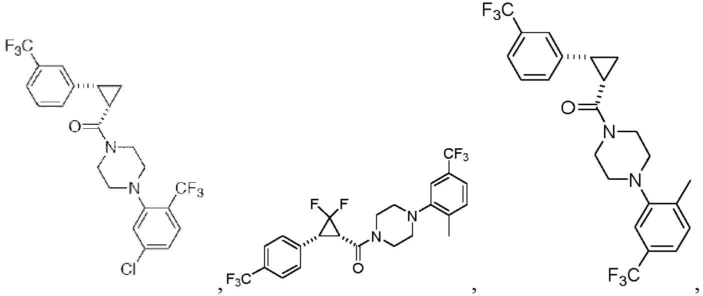 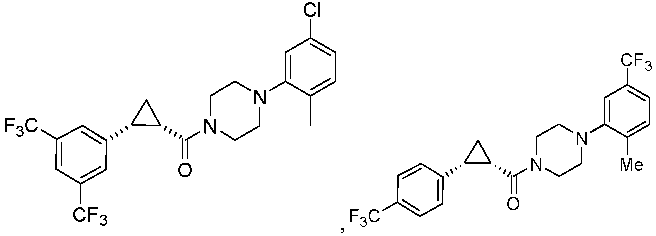 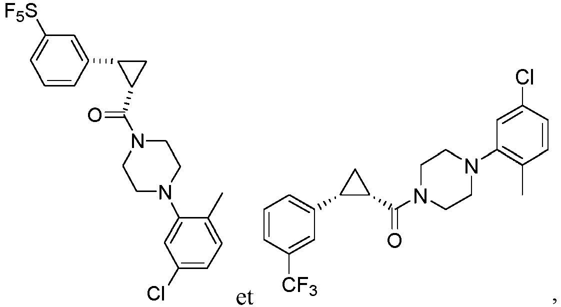 ou sel acceptable pharmaceutiquement de celui-ci.

3. Composé choisi parmi : 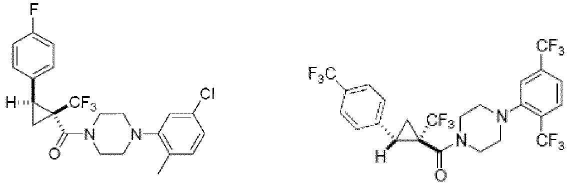 et 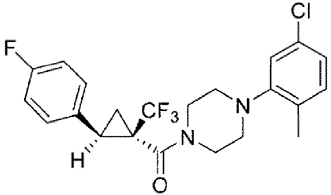 ou sel acceptable pharmaceutiquement de celui-ci.

4. Composition pharmaceutique comprenant au moins un additif acceptable pharmaceutiquement, et 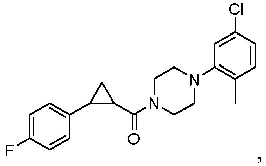 ou un sel acceptable pharmaceutiquement de celui-ci.

5. Composition pharmaceutique comprenant au moins un additif acceptable pharmaceutiquement, et un composé de l'une quelconque des revendications 1 à 3, ou un sel acceptable pharmaceutiquement de celui-ci.

6. Composé destiné à être utilisé dans un procédé permettant le traitement d'un cancer de la prostate chez un sujet, dans lequel une quantité thérapeutiquement efficace du composé est administrée au sujet, dans lequel le composé est 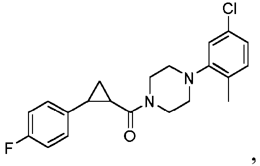 ou sel acceptable pharmaceutiquement de celui-ci, éventuellement dans lequel le cancer de la prostate est un cancer résistant à la castration.

7. Composé destiné à être utilisé selon la revendication 6, dans lequel le composé est administré par voie orale.

8. Composé destiné à être utilisé selon la revendication 6 ou la revendication 7, dans lequel le composé est utilisé en combinaison avec une thérapie par suppression androgénique.

9. Composé destiné à être utilisé selon la revendication 6 ou la revendication 7, dans lequel le composé est co-administré avec l'abiratérone ; ou le composé est co-administré avec l'enzalutamide.

10. Composé destiné à être utilisé selon l'une quelconque des revendications 6 à 9, dans lequel le composé est : 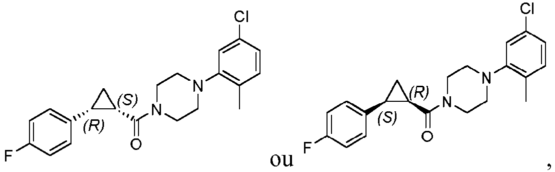 ou sel acceptable pharmaceutiquement de celui-ci.

11. Composé destiné à être utilisé dans un procédé permettant le traitement du cancer de la prostate chez un sujet, dans lequel une quantité thérapeutiquement efficace du composé est administrée au sujet, dans lequel le composé est un composé selon l'une quelconque des revendications 1 à 3, ou un sel acceptable pharmaceutiquement de celui-ci ;
éventuellement dans lequel le cancer de la prostate est un cancer résistant à la castration.

12. Composé destiné à être utilisé selon la revendication 11, dans lequel le composé est administré par voie orale.

13. Composé destiné à être utilisé selon la revendication 11 ou la revendication 12, dans lequel le composé est utilisé en combinaison avec une thérapie par suppression androgénique.

14. Composé destiné à être utilisé selon la revendication 11 ou la revendication 12, dans lequel le composé est co-administré avec l'abiratérone ; ou le composé est co-administré avec l'enzalutamide.
